(19)

Europäisches
Patentamt
European
Patent Office
Office européen
des brevets

(11)  **EP 3 838 260 A1**

(12)  **EUROPEAN PATENT APPLICATION**

(43) Date of publication:
**23.06.2021 Bulletin 2021/25**

(21) Application number: **19219008.0**

(22) Date of filing: **20.12.2019**

(51) Int Cl.:
*A61K 9/00* (2006.01)          *A61K 9/06* (2006.01)
*A61K 47/02* (2006.01)        *A61K 47/14* (2017.01)
*A61K 47/18* (2017.01)        *A61K 47/26* (2006.01)
*A61K 38/00* (2006.01)        *A61P 35/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(71) Applicants:
- **Ares Trading S.A.
  1170 Aubonne (CH)**
- **Glaxosmithkline Intellectual Property (No. 4) Ltd.
  Brentford Middlesex
  TW8 9GS (GB)**

(72) Inventors:
- **IORIO, Chiara
  00012 Guidonia Montecelio RM (IT)**

- **PERGOLA, Carlo
  00012 Guidonia Montecelio RM (IT)**
- **CANAL, Fabiana
  00012 Guidonia Montecelio RM (IT)**
- **RINALDI, Gianluca
  00012 Guidonia Montecelio RM (IT)**
- **GRIESER, Katrin
  64293 Darmstadt (DE)**
- **WEIGANDT, Markus
  64293 Darmstadt (DE)**
- **WINZER, Matthias
  64293 Darmstadt (DE)**

(74) Representative: **Merck Serono S.A.
Intellectual Property
Terre Bonne Business Park
Building Z0
Route de Crassier 1
1262 Eysins (CH)**

(54)  **IGG:TGF RII FUSION PROTEIN COMPOSITION**

(57)    The present invention relates to a pharmaceutical composition, particularly a pharmaceutical composition comprising an IgG:TGFβRII (such as an anti-PD-L1:TGFp-inhibiting) fusion protein. The present invention also relates *inter alia* to a method of manufacturing the composition, to a kit including the composition, to a package including the composition, to a method of manufacturing the package, and to methods of treatment using the composition and/or package, especially cancer treatments.

**EP 3 838 260 A1**

SEQUENCE LISTING

<110> ARES TRADING SA
      Glaxosmithkline Intellectual Property (No. 4) Ltd.

<120> IgG:TGFbRII FUSION PROTEIN COMPOSITION

<130> P19-242 EP-EPA

<160> 35

<170> BiSSAP 1.3.6

<210> 1
<211> 5
<212> PRT
<213> Artificial Sequence


<220>
<223> from human Fab library

<400> 1
Ser Tyr Ile Met Met
1               5

<210> 2
<211> 17
<212> PRT
<213> Artificial Sequence


<220>
<223> from human Fab library

<400> 2
Ser Ile Tyr Pro Ser Gly Gly Ile Thr Phe Tyr Ala Asp Thr Val Lys
1               5                   10                  15
Gly


<210> 3
<211> 11
<212> PRT
<213> Artificial Sequence


<220>
<223> from human Fab library

<400> 3
Ile Lys Leu Gly Thr Val Thr Thr Val Asp Tyr
1               5                   10

<210> 4
<211> 14
<212> PRT
<213> Artificial Sequence


<220>
<223> from human Fab library

<400> 4
Thr Gly Thr Ser Ser Asp Val Gly Gly Tyr Asn Tyr Val Ser
1               5                   10

<210> 5
<211> 7
<212> PRT
<213> Artificial Sequence

<220>
<223> from human Fab library

<400> 5
Asp Val Ser Asn Arg Pro Ser
1               5

<210> 6
<211> 10
<212> PRT
<213> Artificial Sequence

<220>
<223> from human Fab library

<400> 6
Ser Ser Tyr Thr Ser Ser Ser Thr Arg Val
1               5                   10

<210> 7
<211> 216
<212> PRT
<213> Artificial Sequence

<220>
<223> from human Fab library

<400> 7
Gln Ser Ala Leu Thr Gln Pro Ala Ser Val Ser Gly Ser Pro Gly Gln
1               5                   10                  15
Ser Ile Thr Ile Ser Cys Thr Gly Thr Ser Ser Asp Val Gly Gly Tyr
                20              25                  30
Asn Tyr Val Ser Trp Tyr Gln Gln His Pro Gly Lys Ala Pro Lys Leu
            35                  40                  45
Met Ile Tyr Asp Val Ser Asn Arg Pro Ser Gly Val Ser Asn Arg Phe
        50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Tyr Thr Ser Ser
                85                  90                  95
Ser Thr Arg Val Phe Gly Thr Gly Thr Lys Val Thr Val Leu Gly Gln
                100                 105                 110
Pro Lys Ala Asn Pro Thr Val Thr Leu Phe Pro Pro Ser Ser Glu Glu
        115                 120                 125
Leu Gln Ala Asn Lys Ala Thr Leu Val Cys Leu Ile Ser Asp Phe Tyr
        130                 135                 140
Pro Gly Ala Val Thr Val Ala Trp Lys Ala Asp Gly Ser Pro Val Lys
145                 150                 155                 160
Ala Gly Val Glu Thr Thr Lys Pro Ser Lys Gln Ser Asn Asn Lys Tyr
                165                 170                 175
Ala Ala Ser Ser Tyr Leu Ser Leu Thr Pro Glu Gln Trp Lys Ser His

```
                    180                    185                    190
Arg Ser Tyr Ser Cys Gln Val Thr His Glu Gly Ser Thr Val Glu Lys
    195                    200                    205
Thr Val Ala Pro Thr Glu Cys Ser
    210                    215


<210> 8
<211> 607
<212> PRT
<213> Artificial Sequence


<220>
<223> Synthetic peptide


<400> 8
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
        20                  25                  30
Ile Met Met Trp Val Arg Gln Ala Pro Gly Lys Gly Leu Glu Trp Val
        35                  40                  45
Ser Ser Ile Tyr Pro Ser Gly Gly Ile Thr Phe Tyr Ala Asp Thr Val
    50                  55                  60
Lys Gly Arg Phe Thr Ile Ser Arg Asp Asn Ser Lys Asn Thr Leu Tyr
65                  70                  75                      80
Leu Gln Met Asn Ser Leu Arg Ala Glu Asp Thr Ala Val Tyr Tyr Cys
                85                  90                  95
Ala Arg Ile Lys Leu Gly Thr Val Thr Val Asp Tyr Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
        130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Arg Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
    275                 280                 285
Asn Ala Lys Thr Lys Pro Arg Glu Glu Gln Tyr Asn Ser Thr Tyr Arg
        290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
            325                 330                 335
Lys Thr Ile Ser Lys Ala Lys Gly Gln Pro Arg Glu Pro Gln Val Tyr
            340                 345                 350
Thr Leu Pro Pro Ser Arg Glu Glu Met Thr Lys Asn Gln Val Ser Leu
        355                 360                 365
Thr Cys Leu Val Lys Gly Phe Tyr Pro Ser Asp Ile Ala Val Glu Trp
```

```
        370                    375                    380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
            435                 440                 445
Gly Ala Gly Gly Gly Gly Ser Gly Gly Gly Gly Ser Gly Gly Gly Gly
    450                 455                 460
Ser Gly Gly Gly Gly Ser Gly Ile Pro Pro His Val Gln Lys Ser Val
465                 470                 475                 480
Asn Asn Asp Met Ile Val Thr Asp Asn Asn Gly Ala Val Lys Phe Pro
                485                 490                 495
Gln Leu Cys Lys Phe Cys Asp Val Arg Phe Ser Thr Cys Asp Asn Gln
            500                 505                 510
Lys Ser Cys Met Ser Asn Cys Ser Ile Thr Ser Ile Cys Glu Lys Pro
            515                 520                 525
Gln Glu Val Cys Val Ala Val Trp Arg Lys Asn Asp Glu Asn Ile Thr
    530                 535                 540
Leu Glu Thr Val Cys His Asp Pro Lys Leu Pro Tyr His Asp Phe Ile
545                 550                 555                 560
Leu Glu Asp Ala Ala Ser Pro Lys Cys Ile Met Lys Glu Lys Lys Lys
            565                 570                 575
Pro Gly Glu Thr Phe Phe Met Cys Ser Cys Ser Ser Asp Glu Cys Asn
            580                 585                 590
Asp Asn Ile Ile Phe Ser Glu Glu Tyr Asn Thr Ser Asn Pro Asp
    595                 600                 605
```

<210> 9
<211> 592
<212> PRT
<213> Homo sapiens


<400> 9
```
Met Gly Arg Gly Leu Leu Arg Gly Leu Trp Pro Leu His Ile Val Leu
1               5                   10                  15
Trp Thr Arg Ile Ala Ser Thr Ile Pro Pro His Val Gln Lys Ser Asp
            20                  25                  30
Val Glu Met Glu Ala Gln Lys Asp Glu Ile Ile Cys Pro Ser Cys Asn
    35                  40                  45
Arg Thr Ala His Pro Leu Arg His Ile Asn Asn Asp Met Ile Val Thr
    50                  55                  60
Asp Asn Asn Gly Ala Val Lys Phe Pro Gln Leu Cys Lys Phe Cys Asp
65                  70                  75                  80
Val Arg Phe Ser Thr Cys Asp Asn Gln Lys Ser Cys Met Ser Asn Cys
            85                  90                  95
Ser Ile Thr Ser Ile Cys Glu Lys Pro Gln Glu Val Cys Val Ala Val
            100                 105                 110
Trp Arg Lys Asn Asp Glu Asn Ile Thr Leu Glu Thr Val Cys His Asp
            115                 120                 125
Pro Lys Leu Pro Tyr His Asp Phe Ile Leu Glu Asp Ala Ala Ser Pro
            130                 135                 140
Lys Cys Ile Met Lys Glu Lys Lys Lys Pro Gly Glu Thr Phe Phe Met
145                 150                 155                 160
Cys Ser Cys Ser Ser Asp Glu Cys Asn Asp Asn Ile Ile Phe Ser Glu
            165                 170                 175
Glu Tyr Asn Thr Ser Asn Pro Asp Leu Leu Leu Val Ile Phe Gln Val
            180                 185                 190
Thr Gly Ile Ser Leu Leu Pro Pro Leu Gly Val Ala Ile Ser Val Ile
            195                 200                 205
```

```
Ile Ile Phe Tyr Cys Tyr Arg Val Asn Arg Gln Gln Lys Leu Ser Ser
    210                 215                 220
Thr Trp Glu Thr Gly Lys Thr Arg Lys Leu Met Glu Phe Ser Glu His
225                 230                 235                 240
Cys Ala Ile Ile Leu Glu Asp Asp Arg Ser Asp Ile Ser Ser Thr Cys
                245                 250                 255
Ala Asn Asn Ile Asn His Asn Thr Glu Leu Leu Pro Ile Glu Leu Asp
            260                 265                 270
Thr Leu Val Gly Lys Gly Arg Phe Ala Glu Val Tyr Lys Ala Lys Leu
        275                 280                 285
Lys Gln Asn Thr Ser Glu Gln Phe Glu Thr Val Ala Val Lys Ile Phe
    290                 295                 300
Pro Tyr Glu Glu Tyr Ala Ser Trp Lys Thr Glu Lys Asp Ile Phe Ser
305                 310                 315                 320
Asp Ile Asn Leu Lys His Glu Asn Ile Leu Gln Phe Leu Thr Ala Glu
                325                 330                 335
Glu Arg Lys Thr Glu Leu Gly Lys Gln Tyr Trp Leu Ile Thr Ala Phe
                340                 345                 350
His Ala Lys Gly Asn Leu Gln Glu Tyr Leu Thr Arg His Val Ile Ser
        355                 360                 365
Trp Glu Asp Leu Arg Lys Leu Gly Ser Ser Leu Ala Arg Gly Ile Ala
    370                 375                 380
His Leu His Ser Asp His Thr Pro Cys Gly Arg Pro Lys Met Pro Ile
385                 390                 395                 400
Val His Arg Asp Leu Lys Ser Ser Asn Ile Leu Val Lys Asn Asp Leu
                405                 410                 415
Thr Cys Cys Leu Cys Asp Phe Gly Leu Ser Leu Arg Leu Asp Pro Thr
                420                 425                 430
Leu Ser Val Asp Asp Leu Ala Asn Ser Gly Gln Val Gly Thr Ala Arg
        435                 440                 445
Tyr Met Ala Pro Glu Val Leu Glu Ser Arg Met Asn Leu Glu Asn Val
    450                 455                 460
Glu Ser Phe Lys Gln Thr Asp Val Tyr Ser Met Ala Leu Val Leu Trp
465                 470                 475                 480
Glu Met Thr Ser Arg Cys Asn Ala Val Gly Glu Val Lys Asp Tyr Glu
                485                 490                 495
Pro Pro Phe Gly Ser Lys Val Arg Glu His Pro Cys Val Glu Ser Met
            500                 505                 510
Lys Asp Asn Val Leu Arg Asp Arg Gly Arg Pro Glu Ile Pro Ser Phe
        515                 520                 525
Trp Leu Asn His Gln Gly Ile Gln Met Val Cys Glu Thr Leu Thr Glu
    530                 535                 540
Cys Trp Asp His Asp Pro Glu Ala Arg Leu Thr Ala Gln Cys Val Ala
545                 550                 555                 560
Glu Arg Phe Ser Glu Leu Glu His Leu Asp Arg Leu Ser Gly Arg Ser
                565                 570                 575
Cys Ser Glu Glu Lys Ile Pro Glu Asp Gly Ser Leu Asn Thr Thr Lys
            580                 585                 590
```

<210> 10
<211> 567
<212> PRT
<213> Homo sapiens


<400> 10
```
Met Gly Arg Gly Leu Leu Arg Gly Leu Trp Pro Leu His Ile Val Leu
1               5                   10                  15
Trp Thr Arg Ile Ala Ser Thr Ile Pro Pro His Val Gln Lys Ser Val
                20                  25                  30
Asn Asn Asp Met Ile Val Thr Asp Asn Asn Gly Ala Val Lys Phe Pro
```

99

<pre>
              35                    40                    45
Gln Leu Cys Lys Phe Cys Asp Val Arg Phe Ser Thr Cys Asp Asn Gln
    50                    55                    60
Lys Ser Cys Met Ser Asn Cys Ser Ile Thr Ser Ile Cys Glu Lys Pro
65                    70                    75                    80
Gln Glu Val Cys Val Ala Val Trp Arg Lys Asn Asp Glu Asn Ile Thr
                  85                    90                    95
Leu Glu Thr Val Cys His Asp Pro Lys Leu Pro Tyr His Asp Phe Ile
                 100                   105                   110
Leu Glu Asp Ala Ala Ser Pro Lys Cys Ile Met Lys Glu Lys Lys Lys
             115                   120                   125
Pro Gly Glu Thr Phe Phe Met Cys Ser Cys Ser Ser Asp Glu Cys Asn
    130                   135                   140
Asp Asn Ile Ile Phe Ser Glu Glu Tyr Asn Thr Ser Asn Pro Asp Leu
145                   150                   155                   160
Leu Leu Val Ile Phe Gln Val Thr Gly Ile Ser Leu Leu Pro Pro Leu
                 165                   170                   175
Gly Val Ala Ile Ser Val Ile Ile Ile Phe Tyr Cys Tyr Arg Val Asn
                 180                   185                   190
Arg Gln Gln Lys Leu Ser Ser Thr Trp Glu Thr Gly Lys Thr Arg Lys
             195                   200                   205
Leu Met Glu Phe Ser Glu His Cys Ala Ile Ile Leu Glu Asp Asp Arg
    210                   215                   220
Ser Asp Ile Ser Ser Thr Cys Ala Asn Asn Ile Asn His Asn Thr Glu
225                   230                   235                   240
Leu Leu Pro Ile Glu Leu Asp Thr Leu Val Gly Lys Gly Arg Phe Ala
                 245                   250                   255
Glu Val Tyr Lys Ala Lys Leu Lys Gln Asn Thr Ser Glu Gln Phe Glu
                 260                   265                   270
Thr Val Ala Val Lys Ile Phe Pro Tyr Glu Glu Tyr Ala Ser Trp Lys
             275                   280                   285
Thr Glu Lys Asp Ile Phe Ser Asp Ile Asn Leu Lys His Glu Asn Ile
    290                   295                   300
Leu Gln Phe Leu Thr Ala Glu Glu Arg Lys Thr Glu Leu Gly Lys Gln
305                   310                   315                   320
Tyr Trp Leu Ile Thr Ala Phe His Ala Lys Gly Asn Leu Gln Glu Tyr
                 325                   330                   335
Leu Thr Arg His Val Ile Ser Trp Glu Asp Leu Arg Lys Leu Gly Ser
             340                   345                   350
Ser Leu Ala Arg Gly Ile Ala His Leu His Ser Asp His Thr Pro Cys
    355                   360                   365
Gly Arg Pro Lys Met Pro Ile Val His Arg Asp Leu Lys Ser Ser Asn
    370                   375                   380
Ile Leu Val Lys Asn Asp Leu Thr Cys Cys Leu Cys Asp Phe Gly Leu
385                   390                   395                   400
Ser Leu Arg Leu Asp Pro Thr Leu Ser Val Asp Asp Leu Ala Asn Ser
                 405                   410                   415
Gly Gln Val Gly Thr Ala Arg Tyr Met Ala Pro Glu Val Leu Glu Ser
             420                   425                   430
Arg Met Asn Leu Glu Asn Val Glu Ser Phe Lys Gln Thr Asp Val Tyr
    435                   440                   445
Ser Met Ala Leu Val Leu Trp Glu Met Thr Ser Arg Cys Asn Ala Val
    450                   455                   460
Gly Glu Val Lys Asp Tyr Glu Pro Pro Phe Gly Ser Lys Val Arg Glu
465                   470                   475                   480
His Pro Cys Val Glu Ser Met Lys Asp Asn Val Leu Arg Asp Arg Gly
                 485                   490                   495
Arg Pro Glu Ile Pro Ser Phe Trp Leu Asn His Gln Gly Ile Gln Met
                 500                   505                   510
Val Cys Glu Thr Leu Thr Glu Cys Trp Asp His Asp Pro Glu Ala Arg
             515                   520                   525
Leu Thr Ala Gln Cys Val Ala Glu Arg Phe Ser Glu Leu Glu His Leu
    530                   535                   540
</pre>

100

Asp Arg Leu Ser Gly Arg Ser Cys Ser Glu Glu Lys Ile Pro Glu Asp
545                 550                 555                 560
Gly Ser Leu Asn Thr Thr Lys
                565

<210> 11
<211> 136
<212> PRT
<213> Homo sapiens


<400> 11
Ile Pro Pro His Val Gln Lys Ser Val Asn Asn Asp Met Ile Val Thr
1               5                   10                  15
Asp Asn Asn Gly Ala Val Lys Phe Pro Gln Leu Cys Lys Phe Cys Asp
            20                  25                  30
Val Arg Phe Ser Thr Cys Asp Asn Gln Lys Ser Cys Met Ser Asn Cys
        35                  40                  45
Ser Ile Thr Ser Ile Cys Glu Lys Pro Gln Glu Val Cys Val Ala Val
    50                  55                  60
Trp Arg Lys Asn Asp Glu Asn Ile Thr Leu Glu Thr Val Cys His Asp
65                  70                  75                  80
Pro Lys Leu Pro Tyr His Asp Phe Ile Leu Glu Asp Ala Ala Ser Pro
                85                  90                  95
Lys Cys Ile Met Lys Glu Lys Lys Lys Pro Gly Glu Thr Phe Phe Met
            100                 105                 110
Cys Ser Cys Ser Ser Asp Glu Cys Asn Asp Asn Ile Ile Phe Ser Glu
        115                 120                 125
Glu Tyr Asn Thr Ser Asn Pro Asp
    130                 135

<210> 12
<211> 117
<212> PRT
<213> Homo sapiens


<400> 12
Gly Ala Val Lys Phe Pro Gln Leu Cys Lys Phe Cys Asp Val Arg Phe
1               5                   10                  15
Ser Thr Cys Asp Asn Gln Lys Ser Cys Met Ser Asn Cys Ser Ile Thr
            20                  25                  30
Ser Ile Cys Glu Lys Pro Gln Glu Val Cys Val Ala Val Trp Arg Lys
        35                  40                  45
Asn Asp Glu Asn Ile Thr Leu Glu Thr Val Cys His Asp Pro Lys Leu
    50                  55                  60
Pro Tyr His Asp Phe Ile Leu Glu Asp Ala Ala Ser Pro Lys Cys Ile
65                  70                  75                  80
Met Lys Glu Lys Lys Lys Pro Gly Glu Thr Phe Phe Met Cys Ser Cys
                85                  90                  95
Ser Ser Asp Glu Cys Asn Asp Asn Ile Ile Phe Ser Glu Glu Tyr Asn
            100                 105                 110
Thr Ser Asn Pro Asp
            115

<210> 13
<211> 115
<212> PRT
<213> Homo sapiens


<400> 13
Val Lys Phe Pro Gln Leu Cys Lys Phe Cys Asp Val Arg Phe Ser Thr

```
        1                   5                       10                      15
        Cys Asp Asn Gln Lys Ser Cys Met Ser Asn Cys Ser Ile Thr Ser Ile
                        20                      25                      30
        Cys Glu Lys Pro Gln Glu Val Cys Val Ala Val Trp Arg Lys Asn Asp
                        35                      40                      45
        Glu Asn Ile Thr Leu Glu Thr Val Cys His Asp Pro Lys Leu Pro Tyr
                        50                      55                      60
        His Asp Phe Ile Leu Glu Asp Ala Ala Ser Pro Lys Cys Ile Met Lys
        65                      70                      75                      80
        Glu Lys Lys Lys Pro Gly Glu Thr Phe Phe Met Cys Ser Cys Ser Ser
                        85                      90                      95
        Asp Glu Cys Asn Asp Asn Ile Ile Phe Ser Glu Glu Tyr Asn Thr Ser
                        100                     105                     110
        Asn Pro Asp
                        115


        <210> 14
        <211> 446
        <212> PRT
        <213> Artificial Sequence


        <220>
        <223> Description of Artificial Sequence: Synthetic polypeptide

        <400> 14
        Gln Val Gln Leu Val Gln Ser Gly Ala Glu Val Lys Lys Pro Gly Ala
        1                   5                       10                      15
        Ser Val Lys Val Ser Cys Lys Ala Ser Gly Tyr Thr Phe Thr Ser Tyr
                        20                      25                      30
        Trp Met His Trp Val Arg Gln Ala Pro Gly Gln Gly Leu Glu Trp Met
                        35                      40                      45
        Gly Arg Ile Gly Pro Asn Ser Gly Phe Thr Ser Tyr Asn Glu Lys Phe
                        50                      55                      60
        Lys Asn Arg Val Thr Met Thr Arg Asp Thr Ser Thr Ser Thr Val Tyr
        65                      70                      75                      80
        Met Glu Leu Ser Ser Leu Arg Ser Glu Asp Thr Ala Val Tyr Tyr Cys
                        85                      90                      95
        Ala Arg Gly Gly Ser Ser Tyr Asp Tyr Phe Asp Tyr Trp Gly Gln Gly
                        100                     105                     110
        Thr Thr Val Thr Val Ser Ser Ala Ser Thr Lys Gly Pro Ser Val Phe
                        115                     120                     125
        Pro Leu Ala Pro Cys Ser Arg Ser Thr Ser Glu Ser Thr Ala Ala Leu
                        130                     135                     140
        Gly Cys Leu Val Lys Asp Tyr Phe Pro Glu Pro Val Thr Val Ser Trp
        145                     150                     155                     160
        Asn Ser Gly Ala Leu Thr Ser Gly Val His Thr Phe Pro Ala Val Leu
                        165                     170                     175
        Gln Ser Ser Gly Leu Tyr Ser Leu Ser Ser Val Val Thr Val Pro Ser
                        180                     185                     190
        Ser Ser Leu Gly Thr Lys Thr Tyr Thr Cys Asn Val Asp His Lys Pro
                        195                     200                     205
        Ser Asn Thr Lys Val Asp Lys Arg Val Glu Ser Lys Tyr Gly Pro Pro
                        210                     215                     220
        Cys Pro Pro Cys Pro Ala Pro Glu Ala Ala Gly Gly Pro Ser Val Phe
        225                     230                     235                     240
        Leu Phe Pro Pro Lys Pro Lys Asp Thr Leu Met Ile Ser Arg Thr Pro
                        245                     250                     255
        Glu Val Thr Cys Val Val Val Asp Val Ser Gln Glu Asp Pro Glu Val
                        260                     265                     270
        Gln Phe Asn Trp Tyr Val Asp Gly Val Glu Val His Asn Ala Lys Thr
                        275                     280                     285
        Lys Pro Arg Glu Glu Gln Phe Asn Ser Thr Tyr Arg Val Val Ser Val
```

```
        290                    295                    300
Leu Thr Val Leu His Gln Asp Trp Leu Asn Gly Lys Glu Tyr Lys Cys
305                    310                    315                    320
Lys Val Ser Asn Lys Gly Leu Pro Ser Ser Ile Glu Lys Thr Ile Ser
                325                    330                    335
Lys Ala Lys Gly Gln Pro Arg Glu Pro Gln Val Tyr Thr Leu Pro Pro
            340                    345                    350
Ser Gln Glu Glu Met Thr Lys Asn Gln Val Ser Leu Thr Cys Leu Val
        355                    360                    365
Lys Gly Phe Tyr Pro Ser Asp Ile Ala Val Glu Trp Glu Ser Asn Gly
    370                    375                    380
Gln Pro Glu Asn Asn Tyr Lys Thr Thr Pro Pro Val Leu Asp Ser Asp
385                    390                    395                    400
Gly Ser Phe Phe Leu Tyr Ser Arg Leu Thr Val Asp Lys Ser Arg Trp
                405                    410                    415
Gln Glu Gly Asn Val Phe Ser Cys Ser Val Met His Glu Ala Leu His
            420                    425                    430
Asn His Tyr Thr Gln Lys Ser Leu Ser Leu Ser Leu Gly Ala
            435                    440                    445
```

```
<210> 15
<211> 218
<212> PRT
<213> Artificial Sequence


<220>
<223> Description of Artificial Sequence: Synthetic polypeptide

<400> 15
Asp Ile Val Leu Thr Gln Ser Pro Ala Ser Leu Ala Val Ser Pro Gly
1               5                   10                  15
Gln Arg Ala Thr Ile Thr Cys Arg Ala Ser Glu Ser Val Ser Ile His
            20                  25                  30
Gly Thr His Leu Met His Trp Tyr Gln Gln Lys Pro Gly Gln Pro Pro
        35                  40                  45
Lys Leu Leu Ile Tyr Ala Ala Ser Asn Leu Glu Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Glu Asp Thr Ala Asn Tyr Tyr Cys Gln Gln Ser Phe
                85                  90                  95
Glu Asp Pro Leu Thr Phe Gly Gln Gly Thr Lys Leu Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210> 16
<211> 450
<212> PRT
<213> Artificial Sequence
```

<220>
<223> Description of Artificial Sequence: Synthetic polypeptide

<400> 16

```
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ile Met Met Trp Val Arg Gln Ala Pro Gly Lys Gly Leu Glu Trp Val
        35                  40                  45
Ser Ser Ile Tyr Pro Ser Gly Gly Ile Thr Phe Tyr Ala Asp Thr Val
        50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Ile Lys Leu Gly Thr Val Thr Thr Val Asp Tyr Trp Gly Gln
            100                 105                 110
Gly Thr Leu Val Thr Val Ser Ser Ala Ser Thr Lys Gly Pro Ser Val
            115                 120                 125
Phe Pro Leu Ala Pro Ser Ser Lys Ser Thr Ser Gly Gly Thr Ala Ala
        130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
            165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
            195                 200                 205
Pro Ser Asn Thr Lys Val Asp Lys Arg Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
            245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
    275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
            325                 330                 335
Lys Thr Ile Ser Lys Ala Lys Gly Gln Pro Arg Glu Pro Gln Val Tyr
            340                 345                 350
Thr Leu Pro Pro Ser Arg Glu Glu Met Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
            405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
            435                 440                 445
Gly Ala
```

450

<210> 17
<211> 584
<212> PRT
<213> Artificial Sequence


<220>
<223> Description of Artificial Sequence: Synthetic polypeptide


<400> 17
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Gly Pro Asn Ser Gly Phe Thr Ser Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Ala Ala Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
            355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp

```
                     405                        410                        415
      Gln Glu Gly Asn Val Phe Ser Cys Ser Val Met His Glu Ala Leu His
                     420                        425                   430
      Asn His Tyr Thr Gln Lys Ser Leu Ser Leu Ser Leu Gly Ala Gly Gly
                     435                        440                   445
      Gly Gly Ser Gly Gly Gly Gly Ser Gly Gly Gly Gly Ser Gly Gly Gly
                     450                        455                   460
      Gly Ser Gly Gly Ala Val Lys Phe Pro Gln Leu Cys Lys Phe Cys Asp
      465                        470                   475                   480
      Val Arg Phe Ser Thr Cys Asp Asn Gln Lys Ser Cys Met Ser Asn Cys
                     485                        490                   495
      Ser Ile Thr Ser Ile Cys Glu Lys Pro Gln Glu Val Cys Val Ala Val
                     500                        505                   510
      Trp Arg Lys Asn Asp Glu Asn Ile Thr Leu Glu Thr Val Cys His Asp
                     515                        520                   525
      Pro Lys Leu Pro Tyr His Asp Phe Ile Leu Glu Asp Ala Ala Ser Pro
                     530                        535                   540
      Lys Cys Ile Met Lys Glu Lys Lys Lys Pro Gly Glu Thr Phe Phe Met
      545                        550                   555                   560
      Cys Ser Cys Ser Ser Asp Glu Cys Asn Asp Asn Ile Ile Phe Ser Glu
                     565                        570                   575
      Glu Tyr Asn Thr Ser Asn Pro Asp
                     580
```

```
<210> 18
<211> 587
<212> PRT
<213> Artificial Sequence


<220>
<223> Description of Artificial Sequence: Synthetic polypeptide

<400> 18
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
                20                  25                  30
Trp Met His Trp Val Arg Gln Ala Pro Gly Gln Gly Leu Glu Trp Met
            35                  40                  45
Gly Arg Ile Gly Pro Asn Ser Gly Phe Thr Ser Tyr Asn Glu Lys Phe
        50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
        130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
            165                 170                 175
Gln Ser Ser Gly Leu Tyr Ser Leu Ser Ser Val Val Thr Val Pro Ser
        180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
        210                 215                 220
Cys Pro Pro Cys Pro Ala Pro Glu Ala Ala Gly Gly Pro Ser Val Phe
```

```
          225                      230                      235                      240
Leu Phe Pro Pro Lys Pro Lys Asp Thr Leu Met Ile Ser Arg Thr Pro
                   245                      250                      255
Glu Val Thr Cys Val Val Val Asp Val Ser Gln Glu Asp Pro Glu Val
                   260                      265                      270
Gln Phe Asn Trp Tyr Val Asp Gly Val Glu Val His Asn Ala Lys Thr
                   275                      280                      285
Lys Pro Arg Glu Glu Gln Phe Asn Ser Thr Tyr Arg Val Val Ser Val
                   290                      295                      300
Leu Thr Val Leu His Gln Asp Trp Leu Asn Gly Lys Glu Tyr Lys Cys
305                      310                      315                      320
Lys Val Ser Asn Lys Gly Leu Pro Ser Ser Ile Glu Lys Thr Ile Ser
                   325                      330                      335
Lys Ala Lys Gly Gln Pro Arg Glu Pro Gln Val Tyr Thr Leu Pro Pro
                   340                      345                      350
Ser Gln Glu Glu Met Thr Lys Asn Gln Val Ser Leu Thr Cys Leu Val
                   355                      360                      365
Lys Gly Phe Tyr Pro Ser Asp Ile Ala Val Glu Trp Glu Ser Asn Gly
370                      375                      380
Gln Pro Glu Asn Asn Tyr Lys Thr Thr Pro Pro Val Leu Asp Ser Asp
385                      390                      395                      400
Gly Ser Phe Phe Leu Tyr Ser Arg Leu Thr Val Asp Lys Ser Arg Trp
                   405                      410                      415
Gln Glu Gly Asn Val Phe Ser Cys Ser Val Met His Glu Ala Leu His
                   420                      425                      430
Asn His Tyr Thr Gln Lys Ser Leu Ser Leu Ser Leu Gly Ala Gly Gly
                   435                      440                      445
Gly Gly Ser Gly Gly Gly Gly Ser Gly Gly Gly Gly Ser Gly Gly Gly
                   450                      455                      460
Gly Ser Gly Gly Gly Gly Ser Gly Val Lys Phe Pro Gln Leu Cys Lys
465                      470                      475                      480
Phe Cys Asp Val Arg Phe Ser Thr Cys Asp Asn Gln Lys Ser Cys Met
                   485                      490                      495
Ser Asn Cys Ser Ile Thr Ser Ile Cys Glu Lys Pro Gln Glu Val Cys
                   500                      505                      510
Val Ala Val Trp Arg Lys Asn Asp Glu Asn Ile Thr Leu Glu Thr Val
                   515                      520                      525
Cys His Asp Pro Lys Leu Pro Tyr His Asp Phe Ile Leu Glu Asp Ala
                   530                      535                      540
Ala Ser Pro Lys Cys Ile Met Lys Glu Lys Lys Lys Pro Gly Glu Thr
545                      550                      555                      560
Phe Phe Met Cys Ser Cys Ser Ser Asp Glu Cys Asn Asp Asn Ile Ile
                   565                      570                      575
Phe Ser Glu Glu Tyr Asn Thr Ser Asn Pro Asp
                   580                      585
```

```
<210> 19
<211> 5
<212> PRT
<213> Mus musculus


<400> 19
Ser Tyr Trp Met His
1               5

<210> 20
<211> 17
<212> PRT
<213> Artificial sequence


<220>
```

<223> Description of Artificial Sequence: Synthetic polypeptide

<220>
<221> VARIANT
<222> 3
<223> His or Gly

<220>
<221> VARIANT
<222> 8
<223> Gly or Phe

<400> 20
Arg Ile Xaa Pro Asn Ser Gly Xaa Thr Ser Tyr Asn Glu Lys Phe Lys
1               5                   10                  15
Asn


<210> 21
<211> 10
<212> PRT
<213> Mus musculus


<400> 21
Gly Gly Ser Ser Tyr Asp Tyr Phe Asp Tyr
1               5                   10

<210> 22
<211> 15
<212> PRT
<213> Mus musculus


<400> 22
Arg Ala Ser Glu Ser Val Ser Ile His Gly Thr His Leu Met His
1               5                   10                  15

<210> 23
<211> 7
<212> PRT
<213> Mus musculus


<400> 23
Ala Ala Ser Asn Leu Glu Ser
1               5

<210> 24
<211> 9
<212> PRT
<213> Mus musculus


<400> 24
Gln Gln Ser Phe Glu Asp Pro Leu Thr
1               5

<210> 25
<211> 110
<212> PRT
<213> Artificial Sequence

<220>
<223> From human Fab library

<400> 25
Gln Ser Ala Leu Thr Gln Pro Ala Ser Val Ser Gly Ser Pro Gly Gln
1                   5                   10                  15
Ser Ile Thr Ile Ser Cys Thr Gly Thr Ser Ser Asp Val Gly Gly Tyr
            20                  25                  30
Asn Tyr Val Ser Trp Tyr Gln Gln His Pro Gly Lys Ala Pro Lys Leu
        35                  40                  45
Met Ile Tyr Asp Val Ser Asn Arg Pro Ser Gly Val Ser Asn Arg Phe
        50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Tyr Thr Ser Ser
                85                  90                  95
Ser Thr Arg Val Phe Gly Thr Gly Thr Lys Val Thr Val Leu
            100                 105                 110

<210> 26
<211> 120
<212> PRT
<213> Artificial Sequence

<220>
<223> From human Fab library

<400> 26
Glu Val Gln Leu Leu Glu Ser Gly Gly Gly Leu Val Gln Pro Gly Gly
1                   5                   10                  15
Ser Leu Arg Leu Ser Cys Ala Ala Ser Gly Phe Thr Phe Ser Ser Tyr
            20                  25                  30
Ile Met Met Trp Val Arg Gln Ala Pro Gly Lys Gly Leu Glu Trp Val
        35                  40                  45
Ser Ser Ile Tyr Pro Ser Gly Gly Ile Thr Phe Tyr Ala Asp Thr Val
        50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Ile Lys Leu Gly Thr Val Thr Thr Val Asp Tyr Trp Gly Gln
            100                 105                 110
Gly Thr Leu Val Thr Val Ser Ser
            115                 120

<210> 27
<211> 5
<212> PRT
<213> Artificial Sequence

<220>
<223> Sequence is synthesized

<400> 27
Asp Ser Trp Ile His
1                   5

<210> 28
<211> 10
<212> PRT

<213> Artificial Sequence


<220>
<223> Sequence is synthesized

<400> 28
Trp Ile Ser Pro Tyr Gly Gly Ser Thr Tyr
1               5                   10

<210> 29
<211> 7
<212> PRT
<213> Artificial Sequence


<220>
<223> Sequence is synthesized

<400> 29
Arg His Trp Pro Gly Gly Phe
1               5

<210> 30
<211> 7
<212> PRT
<213> Artificial Sequence


<220>
<223> Sequence is synthesized

<400> 30
Asp Val Ser Thr Ala Val Ala
1               5

<210> 31
<211> 6
<212> PRT
<213> Artificial Sequence


<220>
<223> Sequence is synthesized

<400> 31
Ser Ala Ser Phe Leu Tyr
1               5

<210> 32
<211> 9
<212> PRT
<213> Artificial Sequence


<220>
<223> Sequence is synthesized

<400> 32
Gln Gln Tyr Leu Tyr His Pro Ala Thr
1               5

<210> 33

<211> 214
<212> PRT
<213> Artificial Sequence

<220>
<223> Sequence is synthesized

<400> 33
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Val Ser Thr Ala
            20                  25                  30
Val Ala Trp Tyr Gln Gln Lys Pro Gly Lys Ala Pro Lys Leu Leu Ile
        35                  40                  45
Tyr Ser Ala Ser Phe Leu Tyr Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Leu Tyr His Pro Ala
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
            165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
        210

<210> 34
<211> 605
<212> PRT
<213> Artificial Sequence

<220>
<223> Sequence is synthesized

<400> 34
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Ser
            20                  25                  30
Trp Ile His Trp Val Arg Gln Ala Pro Gly Lys Gly Leu Glu Trp Val
        35                  40                  45
Ala Trp Ile Ser Pro Tyr Gly Gly Ser Thr Tyr Tyr Ala Asp Ser Val
        50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ala Arg Arg His Trp Pro Gly Gly Phe Asp Tyr Trp Gly Gln Gly Thr
            100                 105                 110
Leu Val Thr Val Ser Ala Ala Ser Thr Lys Gly Pro Ser Val Phe Pro

```
              115                     120                     125
         Leu Ala Pro Ser Ser Lys Ser Thr Ser Gly Gly Thr Ala Ala Leu Gly
         130                     135                     140
         Cys Leu Val Lys Asp Tyr Phe Pro Glu Pro Val Thr Val Ser Trp Asn
         145                     150                     155                 160
         Ser Gly Ala Leu Thr Ser Gly Val His Thr Phe Pro Ala Val Leu Gln
                         165                     170                     175
         Ser Ser Gly Leu Tyr Ser Leu Ser Ser Val Val Thr Val Pro Ser Ser
                         180                     185                     190
         Ser Leu Gly Thr Gln Thr Tyr Ile Cys Asn Val Asn His Lys Pro Ser
                         195                     200                     205
         Asn Thr Lys Val Asp Lys Arg Val Glu Pro Lys Ser Cys Asp Lys Thr
         210                     215                     220
         His Thr Cys Pro Pro Cys Pro Ala Pro Glu Leu Leu Gly Gly Pro Ser
         225                     230                     235                 240
         Val Phe Leu Phe Pro Pro Lys Pro Lys Asp Thr Leu Met Ile Ser Arg
                         245                     250                     255
         Thr Pro Glu Val Thr Cys Val Val Val Asp Val Ser His Glu Asp Pro
                         260                     265                     270
         Glu Val Lys Phe Asn Trp Tyr Val Asp Gly Val Glu Val His Asn Ala
                         275                     280                     285
         Lys Thr Lys Pro Arg Glu Glu Gln Tyr Asn Ser Thr Tyr Arg Val Val
         290                     295                     300
         Ser Val Leu Thr Val Leu His Gln Asp Trp Leu Asn Gly Lys Glu Tyr
         305                     310                     315                 320
         Lys Cys Lys Val Ser Asn Lys Ala Leu Pro Ala Pro Ile Glu Lys Thr
                         325                     330                     335
         Ile Ser Lys Ala Lys Gly Gln Pro Arg Glu Pro Gln Val Tyr Thr Leu
                         340                     345                     350
         Pro Pro Ser Arg Glu Glu Met Thr Lys Asn Gln Val Ser Leu Thr Cys
                         355                     360                     365
         Leu Val Lys Gly Phe Tyr Pro Ser Asp Ile Ala Val Glu Trp Glu Ser
         370                     375                     380
         Asn Gly Gln Pro Glu Asn Asn Tyr Lys Thr Thr Pro Pro Val Leu Asp
         385                     390                     395                 400
         Ser Asp Gly Ser Phe Phe Leu Tyr Ser Lys Leu Thr Val Asp Lys Ser
                         405                     410                     415
         Arg Trp Gln Gln Gly Asn Val Phe Ser Cys Ser Val Met His Glu Ala
                         420                     425                     430
         Leu His Asn His Tyr Thr Gln Lys Ser Leu Ser Leu Ser Pro Gly Ala
                         435                     440                     445
         Gly Gly Gly Gly Ser Gly Gly Gly Gly Ser Gly Gly Gly Gly Ser Gly
                         450                     455                     460
         Gly Gly Gly Ser Gly Ile Pro Pro His Val Gln Lys Ser Val Asn Asn
         465                     470                     475                 480
         Asp Met Ile Val Thr Asp Asn Asn Gly Ala Val Lys Phe Pro Gln Leu
                         485                     490                     495
         Cys Lys Phe Cys Asp Val Arg Phe Ser Thr Cys Asp Asn Gln Lys Ser
                         500                     505                     510
         Cys Met Ser Asn Cys Ser Ile Thr Ser Ile Cys Glu Lys Pro Gln Glu
                         515                     520                     525
         Val Cys Val Ala Val Trp Arg Lys Asn Asp Glu Asn Ile Thr Leu Glu
                         530                     535                     540
         Thr Val Cys His Asp Pro Lys Leu Pro Tyr His Asp Phe Ile Leu Glu
         545                     550                     555                 560
         Asp Ala Ala Ser Pro Lys Cys Ile Met Lys Glu Lys Lys Lys Pro Gly
                         565                     570                     575
         Glu Thr Phe Phe Met Cys Ser Cys Ser Ser Asp Glu Cys Asn Asp Asn
                         580                     585                     590
         Ile Ile Phe Ser Glu Glu Tyr Asn Thr Ser Asn Pro Asp
                         595                     600                     605

         <210> 35
```

<211> 448
<212> PRT
<213> Artificial Sequence


<220>
<223> Sequence is synthesized

<400> 35
```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Ser
            20                  25                  30
Trp Ile His Trp Val Arg Gln Ala Pro Gly Lys Gly Leu Glu Trp Val
        35                  40                  45
Ala Trp Ile Ser Pro Tyr Gly Gly Ser Thr Tyr Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ala Arg Arg His Trp Pro Gly Gly Phe Asp Tyr Trp Gly Gln Gly Thr
            100                 105                 110
Leu Val Thr Val Ser Ala Ala Ser Thr Lys Gly Pro Ser Val Phe Pro
            115                 120                 125
Leu Ala Pro Ser Ser Lys Ser Thr Ser Gly Gly Thr Ala Ala Leu Gly
            130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
            195                 200                 205
Asn Thr Lys Val Asp Lys Arg Val Glu Pro Lys Ser Cys Asp Lys Thr
    210                 215                 220
His Thr Cys Pro Pro Cys Pro Ala Pro Glu Leu Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser His Glu Asp Pro
            260                 265                 270
Glu Val Lys Phe Asn Trp Tyr Val Asp Gly Val Glu Val His Asn Ala
            275                 280                 285
Lys Thr Lys Pro Arg Glu Glu Gln Tyr Asn Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Glu Lys Thr
                325                 330                 335
Ile Ser Lys Ala Lys Gly Gln Pro Arg Glu Pro Gln Val Tyr Thr Leu
            340                 345                 350
Pro Pro Ser Arg Glu Glu Met Thr Lys Asn Gln Val Ser Leu Thr Cys
            355                 360                 365
Leu Val Lys Gly Phe Tyr Pro Ser Asp Ile Ala Val Glu Trp Glu Ser
            370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
```

```
Leu His Asn His Tyr Thr Gln Lys Ser Leu Ser Leu Ser Pro Gly Ala
        435                 440                 445
```

**Description**

*INTRODUCTION*

The present invention relates to a pharmaceutical composition, particularly a pharmaceutical composition comprising an IgG:TGFβRII fusion protein. The present invention also relates *inter alia* to a method of manufacturing the composition, to a kit including the composition, to a container or drug delivery device including the composition, to a method of manufacturing the container or drug delivery device, and to methods of treatment using the composition and/or container or drug delivery device, especially cancer treatments.

*BACKGROUND*

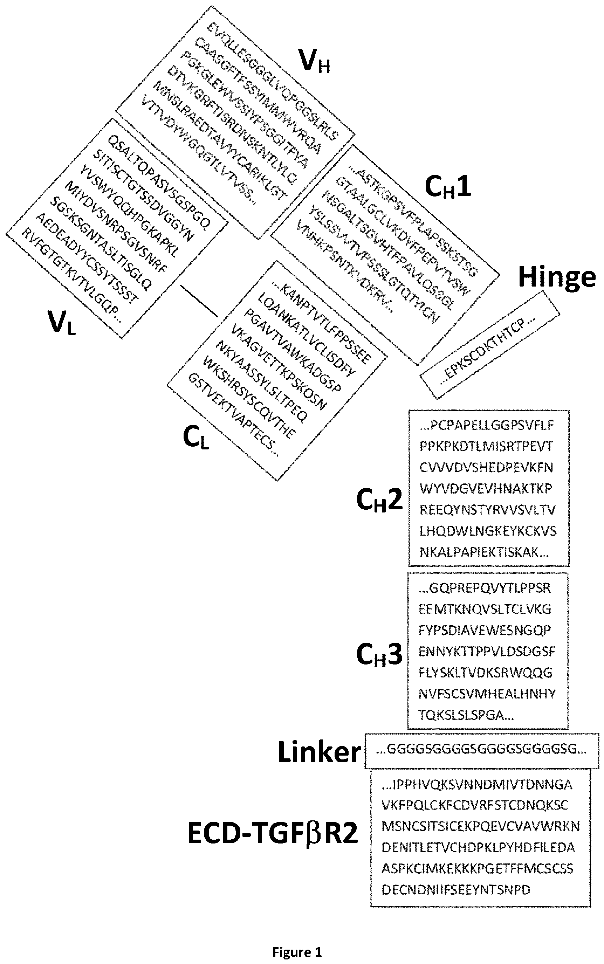

WO2015118175 describes a bi-functional IgG:TGFβRII fusion protein that combines an anti-programmed death ligand 1 (anti-PD-L1) antibody with the soluble extracellular domain of tumor growth factor beta receptor type II (TGFβRII) as a TGFβ neutralizing "Trap," into a single molecule. Specifically, the protein is a heterotetramer, consisting of the two immunoglobulin light chains of the anti-PD-L1 antibody, and two heavy chains comprising the heavy chain of the anti-PD-L1 antibody genetically fused via a flexible glycine-serine linker to the extracellular domain of the human TGFβRII (*see* Fig. 1). This anti-PD-L1/TGFβ Trap molecule is designed to target two major mechanisms of immunosuppression in the tumor microenvironment, and may thus be used in treating cancer or inhibiting tumor growth.

An object of the invention is the provision of viable pharmaceutical compositions of an IgG:TGFβRII fusion protein. Unpredictabilities inherent in the art of formulating biologics, especially antibody- or antibody fragment-containing biologics, cause difficulties in the discovery of such viable pharmaceutical compositions, since most formulations of a given biopharmaceutical (if said formulation is arbitrarily chosen) are unstable over prolonged periods and/or under stressed conditions owing to the variety of degradation pathways open to biologics formulations, especially aqueous formulations. Degradation factors may, for instance, include one or more (typically two or more, and potentially three or more) of the following:

- Physical effects, such as:

  ○ Inadequate inhibition of aggregation of the relevant protein molecules;

  ○ Inadequate inhibition of precipitation;

  ○ Inadequate inhibition of adsorption of the relevant protein molecules at the interface of water and air or at the contact surface of any packaging material;

  ○ Inadequate regulation of osmotic pressure;

- Chemical effects, such as:

  ○ Inadequate regulation of oxidation;

  ○ Inadequate inhibition of photo-oxidation;

  ○ inadequate inhibition of hydrolysis of ester bonds leading to the formation of acid, aldehyde and peroxide products, thus affecting the stability of the fusion protein;

  ○ Inadequate stabilisation and maintenance of pH;

  ○ Inadequate inhibition of protein fragmentation;

  ○ Inadequate inhibition of protein unfolding.

Any, some, or all of the above factors can lead to either an unviable drug product (which may be unsafe for use in medical treatments) or a drug product whose viability is variable and unpredictable, especially in view of the variable stresses (agitation, freeze-thaw, heat, light) different batches of drug product may be exposed to during manufacture, transport, and storage.

The present invention preferably seeks to address one or more of the aforesaid stability issues and, in so doing, furnish viable pharmaceutical formulations.

## SUMMARY OF THE INVENTION

In some embodiments, there is provided a pharmaceutical composition comprising an IgG:TGFβR fusion protein. Pharmaceutical compositions of the invention may preferably comprise, consist of, or exclude any, some, or all of the ingredients described herein (e.g. include any of a buffer, surfactant, sugar component, amino acid component, tonicifier, antioxidant, chelator; or indeed exclude any of the aforesaid), in any relevant amounts described herein, and/or may be preferably characterised by any, some, or all parameters (e.g. pH, pl, osmolality) described herein. The pharmaceutical composition may be a liquid (e.g. an aqueous) pharmaceutical composition. Alternatively, the pharmaceutical composition may be a lyophilised composition.

In some embodiments, there is provided a container or a drug delivery device comprising or containing a pharmaceutical composition as defined herein. Such drug delivery device can be, e.g., a vial, ampoule, syringe, pre-filled syringe, injection pen (e.g. essentially incorporating a syringe), autoinjector, or intravenous bag, or a package/container containing any of the aforementioned.

In some embodiments, there is provided a kit of parts comprising a drug delivery device, a pharmaceutical composition as defined herein, and optionally a set of instructions with directions regarding the administration (e.g. intravenous, subcutaneous) of the pharmaceutical composition.

In some embodiments, there is provided a method of manufacturing a pharmaceutical composition, the method comprising mixing together an IgG:TGFβR fusion protein with one or more pharmaceutically-acceptable excipients and/or carriers.

In some embodiments, there is provided a method of treating a disease or medical disorder in a patient in need of such treatment, said method comprising administering to said patient a therapeutically effective amount of a pharmaceutical composition as defined herein.

In some embodiments, there is provided a pharmaceutical composition as defined herein for use in treating a disease or medical disorder in a patient in need of such treatment.

In some embodiments, there is provided a use of a pharmaceutical composition as defined herein in the manufacture of a medicament for the treatment of a disease or disorder.

In some embodiments, there are provided a method of treating a disease or medical disorder, a pharmaceutical composition for use in treating a disease or medical disorder, and a use of a pharmaceutical composition in the manufacture of a medicament for a treatment of a disease or disorder as defined herein, wherein the disease or medical disorder is a PD-L1-related disease, a TGFβ-related disease and/or a proliferative disease or disorder, preferably cancer.

In some embodiments, said treating or treatment involves combination therapy whereby the pharmaceutical composition is administered in combination with one or more other pharmaceutical or biopharmaceutical active(s); wherein said combination therapy involves simultaneous, sequential or separate dosing of the individual components of the treatment. In some embodiments, the additional pharmaceutical or biopharmaceutical active may be present within any of the pharmaceutical compositions as defined herein.

All of the aforesaid methods of treatment, composition(s) for use, and use of composition(s) in the manufacture of a medicament, are equally applicable to the relevant containers, drug-delivery devices, and kits incorporating said composition(s).

Any features, including optional, suitable, and preferred features, described in relation to any particular aspect of the invention may also be features, including optional, suitable and preferred features, of any other aspect of the present invention unless incompatible therewith.

## BRIEF DESCRIPTION OF THE DRAWINGS

For a better understanding of the invention, and to show how embodiments of the same are put into effect, reference is now made, by way of example, to the following diagrammatic drawings, in which:

FIG. 1 shows the sequence of one half of the bintrafusp alfa fusion protein, containing a light chain (with designated $V_L$ and $C_L$ regions) and a heavy chain (with designated $V_H$, $C_H1$, $C_H2$, and $C_H3$ regions) connected *via* the designated linker to the extracellular domain (ECD) of a TGFβ Receptor II.

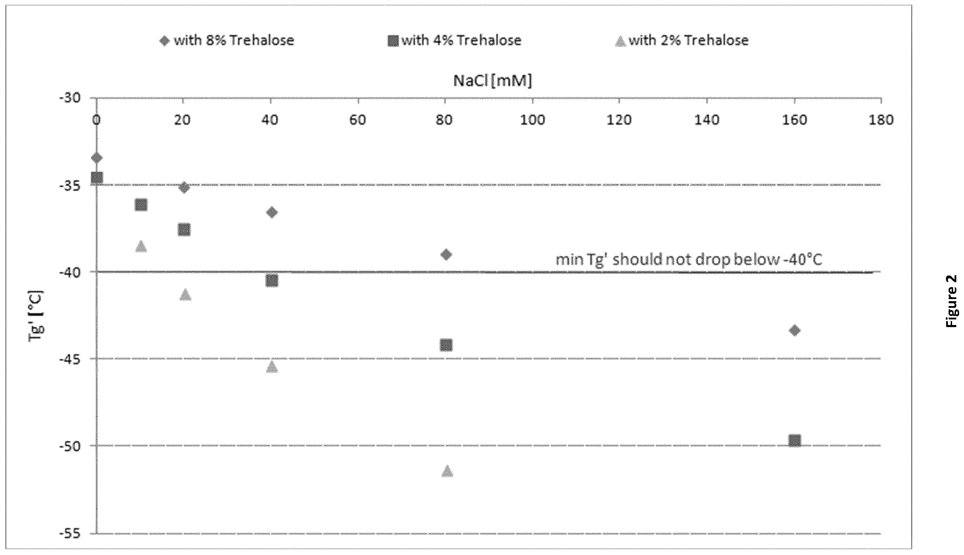

FIG. 2 shows a graphical representation of the relationship between Tg' (in °C) and NaCl concentration for formulations containing 8% trehalose (diamonds), 4% trehalose (squares), and 2% trehalose (triangles). It is undesirable for Tg' values to be below -40°C from the point of view of practicalities when freeze drying.

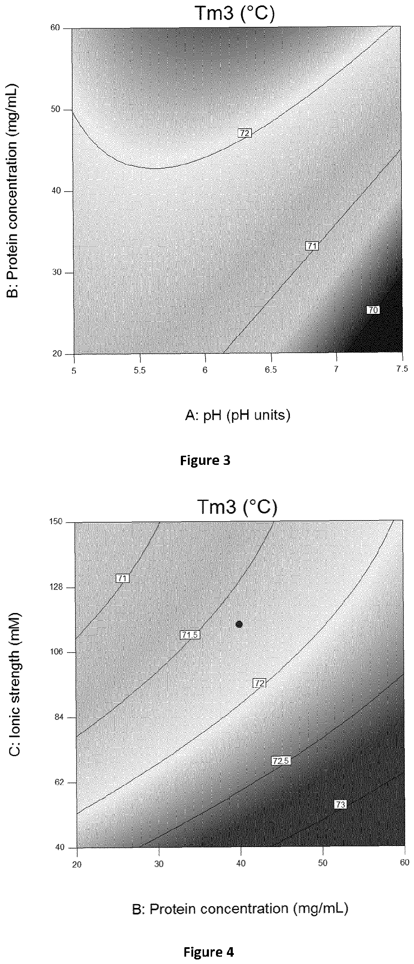

FIG. 3 is a contour plot with $T_m3$ unfolding temperatures represented as contours (ranging from 69.98-72.72) within a formulation space (having fixed ionic strength, 95 mM NaCl) with variable protein concentration (mg/mL of bintrafusp alfa) and pH.

FIG. 4 is a contour plot with $T_m3$ unfolding temperatures represented as contours (ranging from 69.98-72.72) within a formulation space (having fixed pH of pH 6.25) with variable protein concentration (mg/mL of bintrafusp alfa) and ionic strength (mM NaCl).

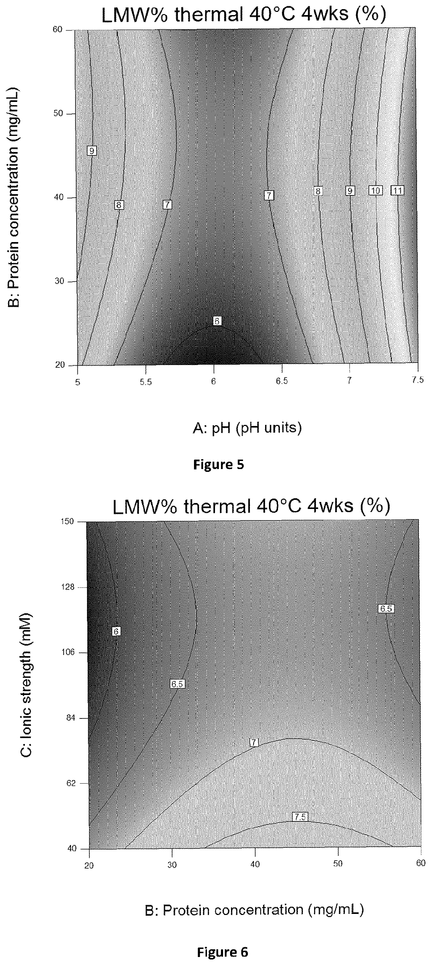

FIG. 5 is a contour plot with LMW% species after 4 weeks at 40°C represented as contours (ranging from 5.71-12.35) within a formulation space (having fixed ionic strength, 95 mM NaCl) with variable protein concentration (mg/mL of bintrafusp alfa) and pH. The data was obtained by CGE NR, i.e. CGE under non-reducing conditions.

FIG. 6 is a contour plot with LMW% species after 4 weeks at 40°C represented as contours (see contour lines at 6, 6.5, 7, and 7.5) within a formulation space (having fixed pH of pH 6.25) with variable protein concentration (mg/mL of bintrafusp alfa) and ionic strength (mM NaCl). The data was obtained by CGE NR.

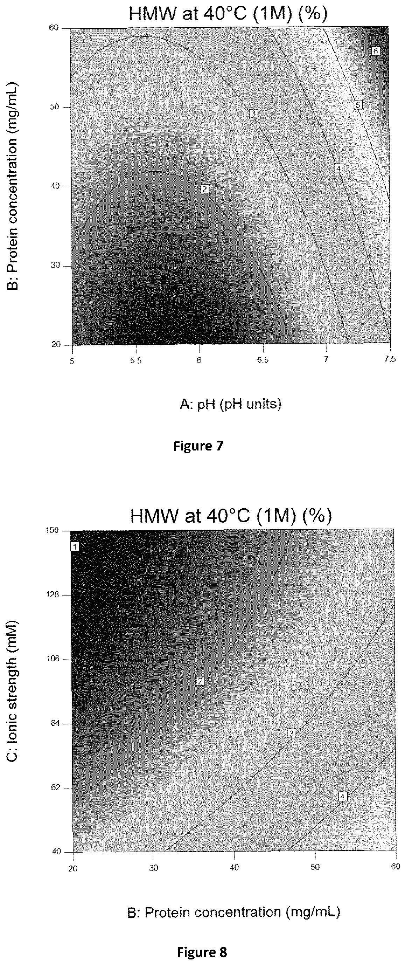

FIG. 7 is a contour plot with HMW% species after 4 weeks at 40°C represented as contours (ranging from 1.2-6.3) within a formulation space (having fixed ionic strength, 95 mM NaCl) with variable protein concentration (mg/mL of bintrafusp alfa) and pH. The data was obtained by SE-UPLC.

FIG. 8 is a contour plot with HMW% species after 4 weeks at 40°C represented as contours (see contour lines at 1, 2, 3, and 4) within a formulation space (having fixed pH of pH 6.25) with variable protein concentration (mg/mL of bintrafusp alfa) and ionic strength (mM NaCl). The data was obtained by SE-UPLC.

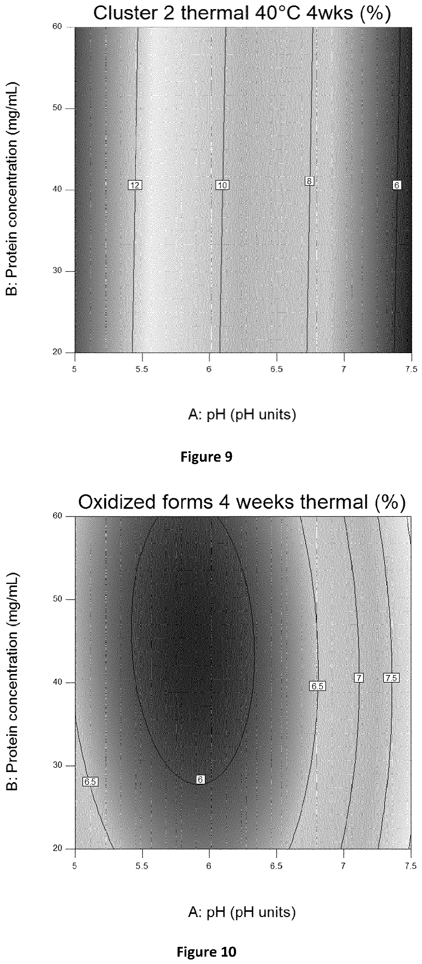

FIG. 9 is a contour plot with Cluster 2 isoforms after 4 weeks at 40°C represented as contours (ranging from 5.5-13.68) within a formulation space (having fixed ionic strength, 95 mM NaCl) with variable protein concentration (mg/mL of bintrafusp alfa) and pH. The data was obtained *via* cIEF identification and isoforms distribution by iCE3.

FIG. 10 is a contour plot with % oxidation levels of Met516 after 4 weeks at 40°C represented as contours (ranging from 5.77-8.76) within a formulation space (having fixed ionic strength, 95 mM NaCl) with variable protein concentration (mg/mL of bintrafusp alfa) and pH. The data was obtained by RP-UPLC.

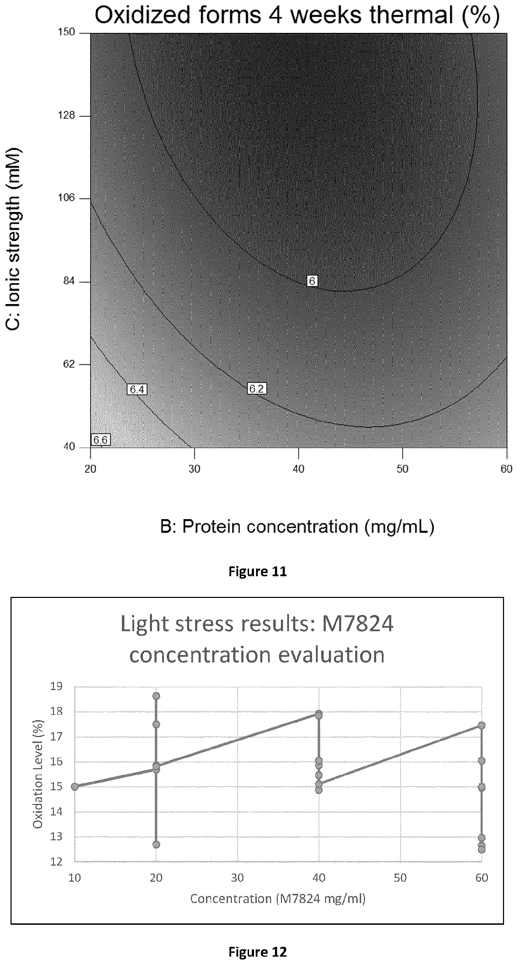

FIG. 11 is a contour plot with % oxidation of Met516 after 4 weeks at 40°C represented as contours (see contour lines at 6, 6.2, 6.4, and 6.6) within a formulation space (having fixed pH of pH 6.25) with variable protein concentration (mg/mL of bintrafusp alfa) and ionic strength (mM NaCl). The data was obtained by RP-UPLC.

FIG. 12 is a scatter plot for all samples showing % oxidation vs bintrafusp alfa concentration after light stress.

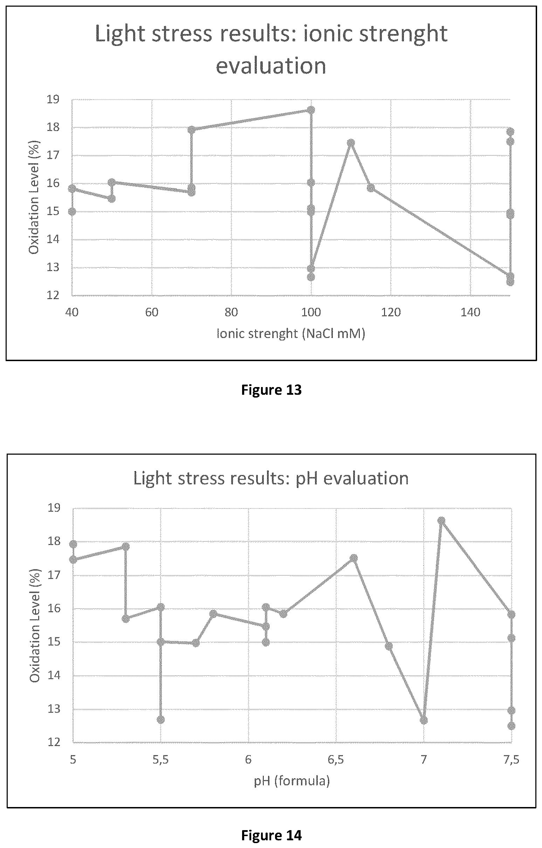

FIG. 13 is a scatter plot for all samples showing % oxidation vs ionic strength after light stress.

FIG. 14 is a scatter plot for all samples showing % oxidation vs pH after light stress.

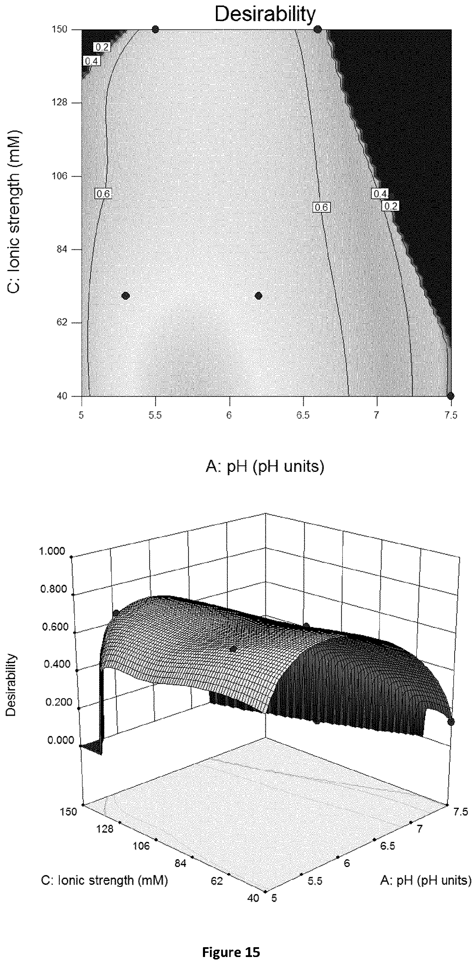

FIG. 15 is a 2D and a 3D contour plot with a desirability parameter (reflecting a balance of factors in the overall response evaluation) represented as contours (in the 2D plot) and curved surface (in the 3D plot) within a formulation space (having a fixed protein concentration of 20 mg/mL) with variable pH and ionic strength (given as mM NaCl). This suggests optimal conditions at this concentration of IgG:TGFβR2 are pH 5.7 and an ionic strength of 40 mM NaCl.

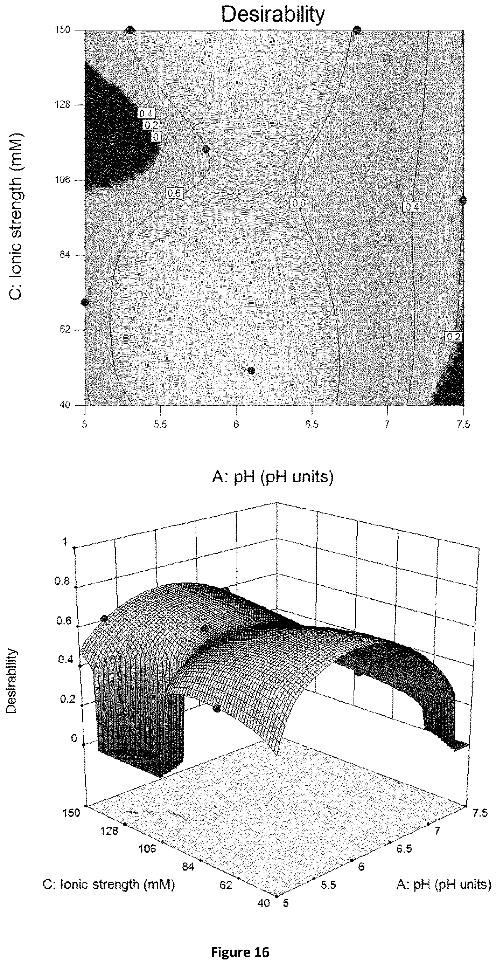

FIG. 16 is a 2D and a 3D contour plot with a desirability parameter (reflecting a balance of factors in the overall response evaluation) represented as contours (in the 2D plot) and curved surface (in the 3D plot) within a formulation space (having a fixed protein concentration of 40 mg/mL) with variable pH and ionic strength (given as mM NaCl). This suggests optimal conditions at this concentration of IgG:TGFβR2 are pH 5.9 and an ionic strength of 60 mM NaCl.

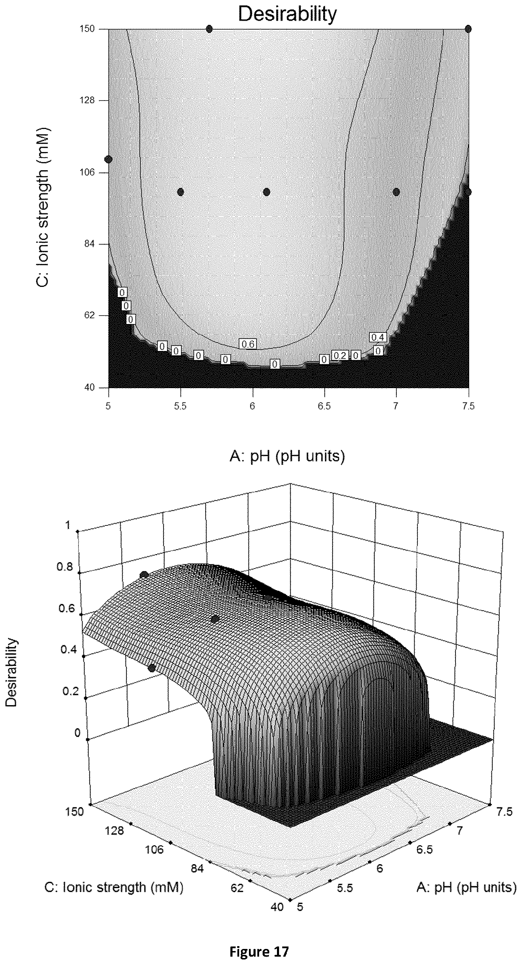

FIG. 17 is a 2D and a 3D contour plot with a desirability parameter (reflecting a balance of factors in the overall response evaluation) represented as contours (in the 2D plot) and curved surface (in the 3D plot) within a formulation space (having a fixed protein concentration of 60 mg/mL) with variable pH and ionic strength (given as mM NaCl). This suggests optimal conditions at this concentration of IgG:TGFβR2 are pH 5.9 and an ionic strength of 150 mM NaCl.

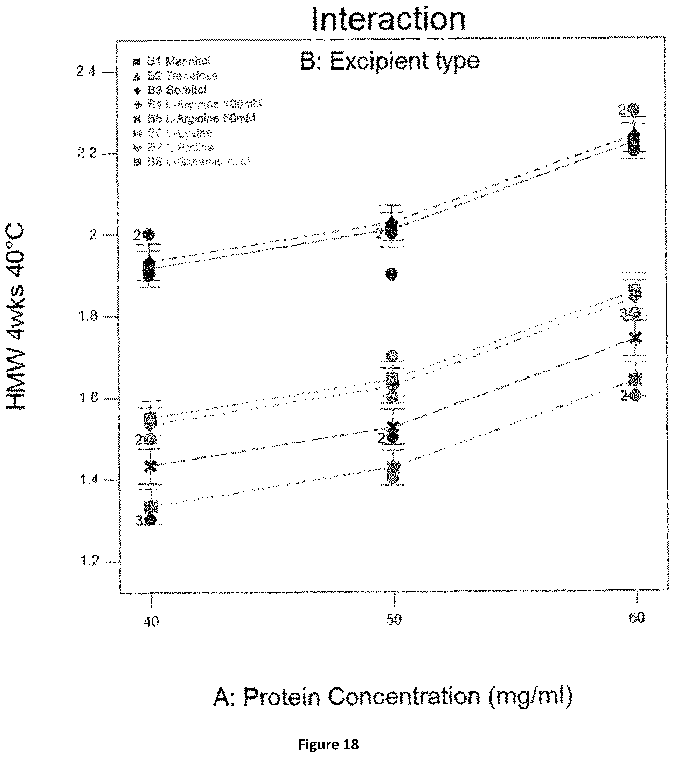

FIG. 18 is a graph showing how HMW%, after 4 weeks thermal stress at 40°C, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

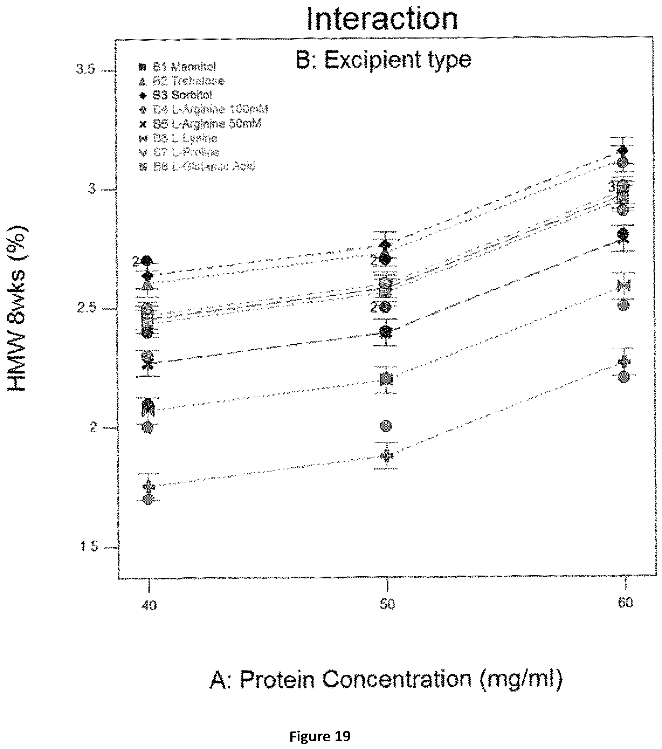

FIG. 19 is a graph showing how HMW%, after 8 weeks thermal stress at 40°C, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

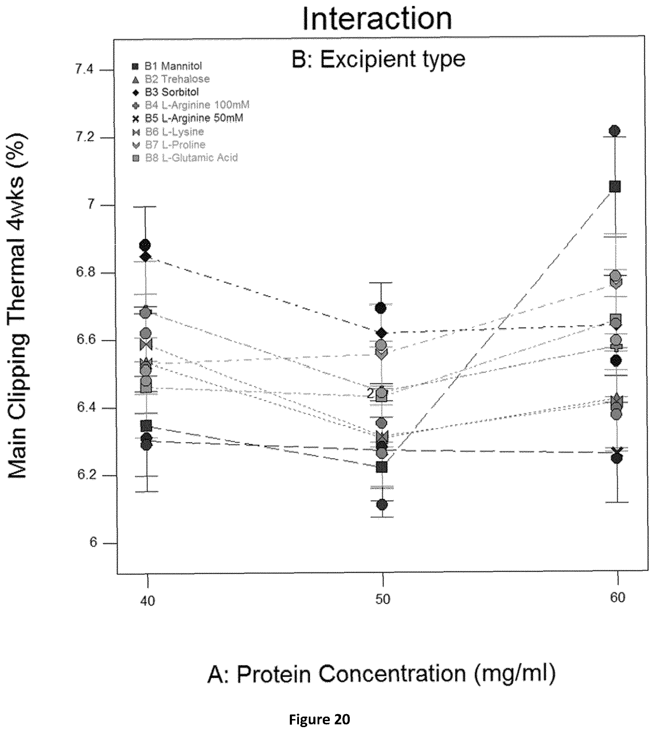

FIG. 20 is a graph showing how Main Clipping, after 4 weeks thermal stress at 40°C, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

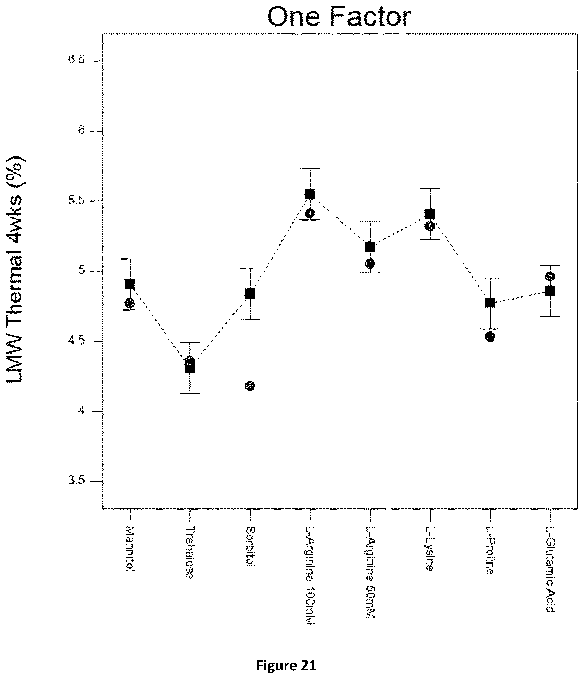

FIG. 21 is a graph showing how LMW%, after 4 weeks thermal stress at 40°C, varies with the "varied excipient" when the surfactant is fixed as polysorbate 20 and ionic strength is fixed at whatever NaCl concentration applies for the particular protein concentration with polysorbate 20 in Table 3J. The measured values are indicated by the circles, whereas the values predicted based on the measured values are indicated by the squares.

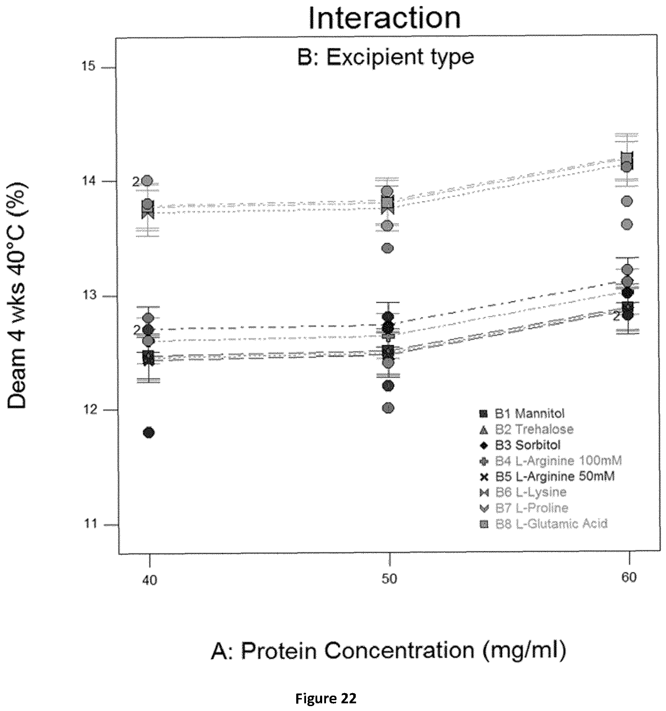

FIG. 22 is a graph showing how % deamidated forms, after 4 weeks at 40°C, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

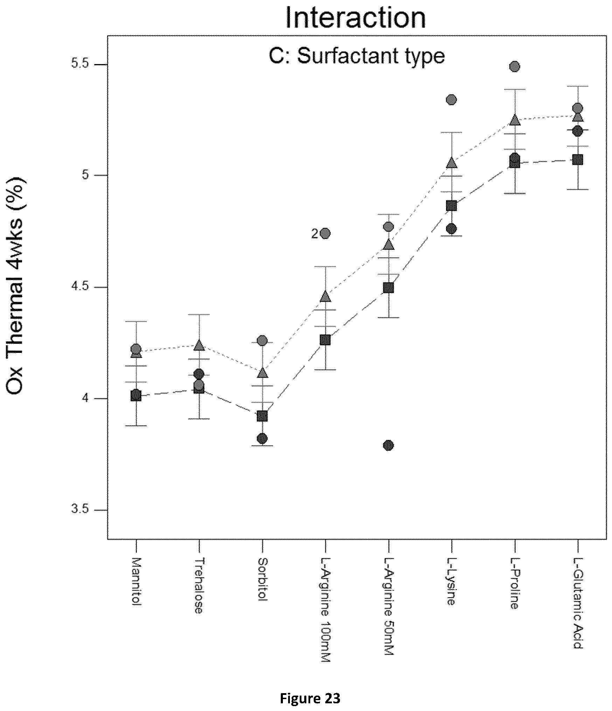

FIG. 23 is a graph showing how % oxidated forms, after 4 weeks at 40°C, varies with excipient when protein (bintrafusp alfa) concentration is fixed at 40 mg/mL, for the surfactants polysorbate 20 (squares indicate the predicted values) and kolliphor 188 (triangles indicate the predicted values based on the measured values, which are represented by circles).

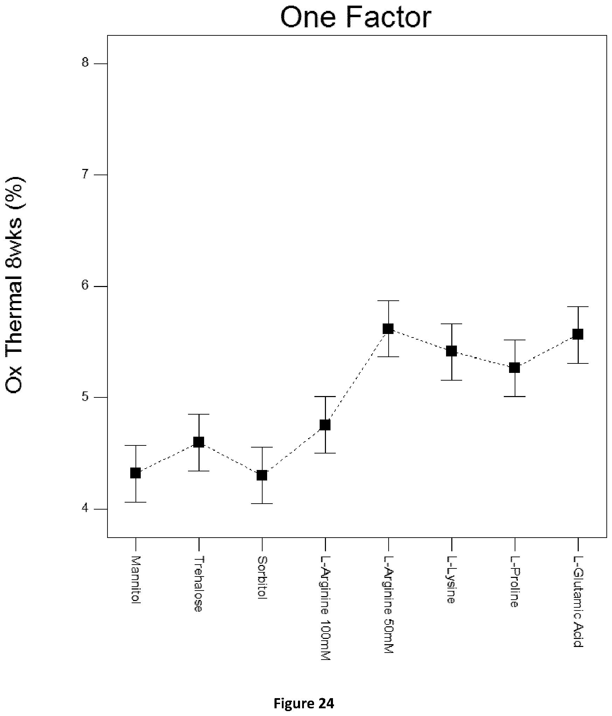

FIG. 24 is a graph showing how % oxidated forms, after 8 weeks at 40°C, varies with excipient when protein (bintrafusp alfa) concentration is fixed at 40 mg/mL.

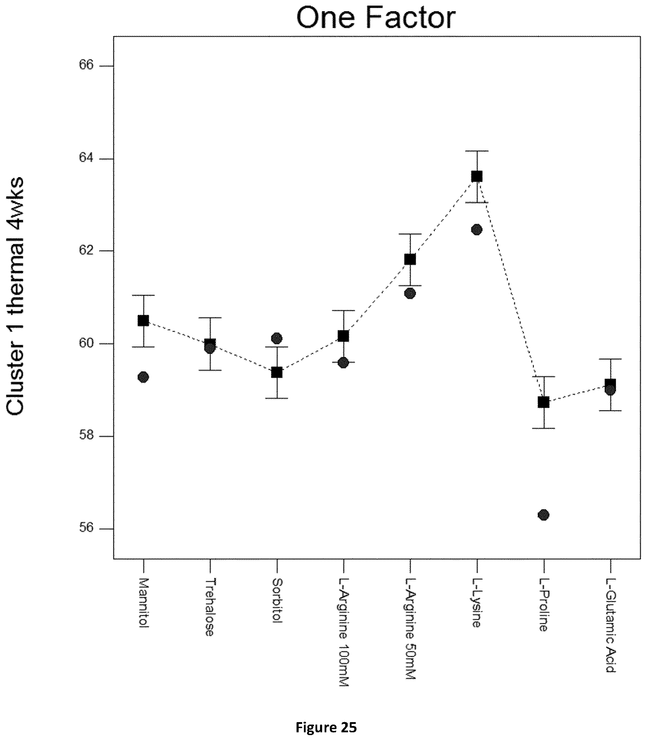

FIG. 25 is a graph showing how % Cluster 1 (by iCE3) varies, after 4 weeks thermal stress at 40°C, with excipient when protein (bintrafusp alfa) concentration is fixed at 40 mg/mL. The circles represent the measured values and the squares represent the predicted values that are based on the measured values.

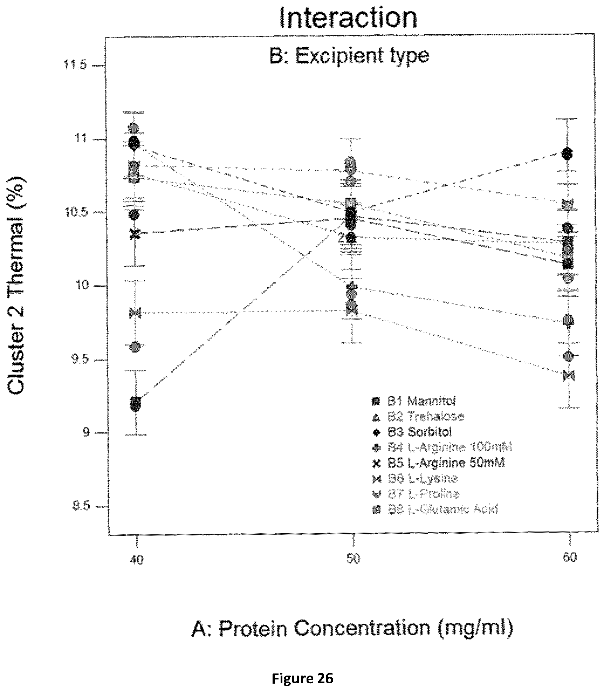

FIG. 26 is a graph showing how % Cluster 2 (by iCE3), after 4 weeks at 40°C, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

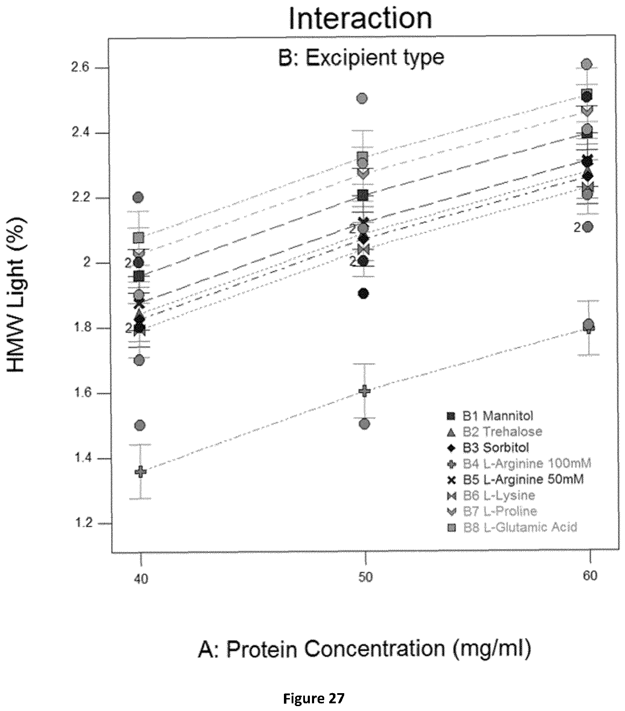

FIG. 27 is a graph showing how %NMW (by SE-UPLC), after light stress, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

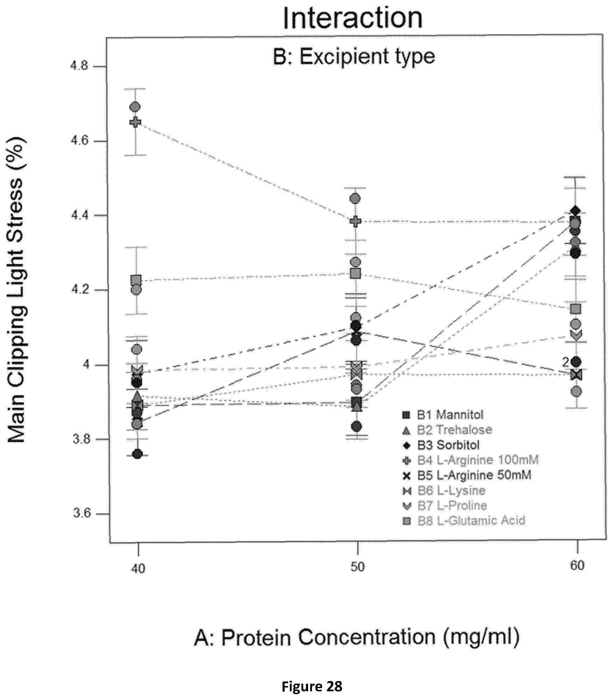

FIG. 28 is a graph showing how Main Clipping (by CGE), after light stress, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

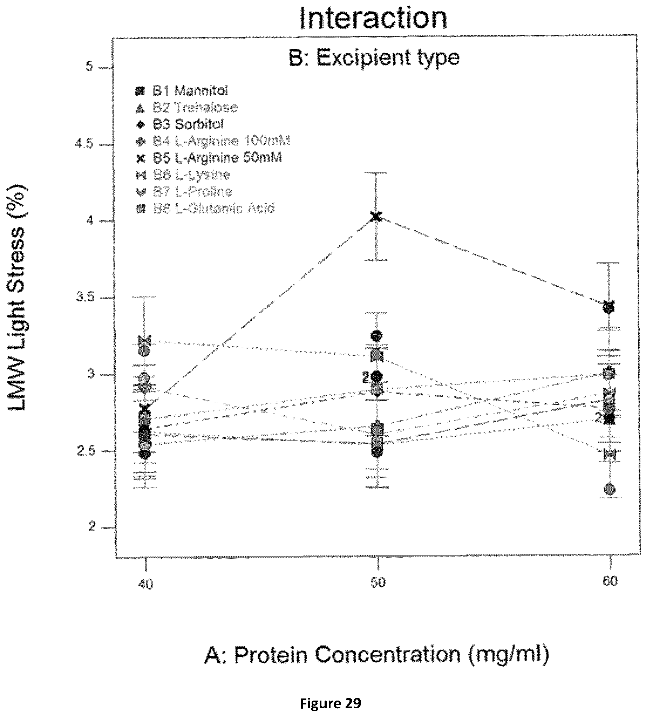

FIG. 29 is a graph showing how %LMW (by CGE), after light stress, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

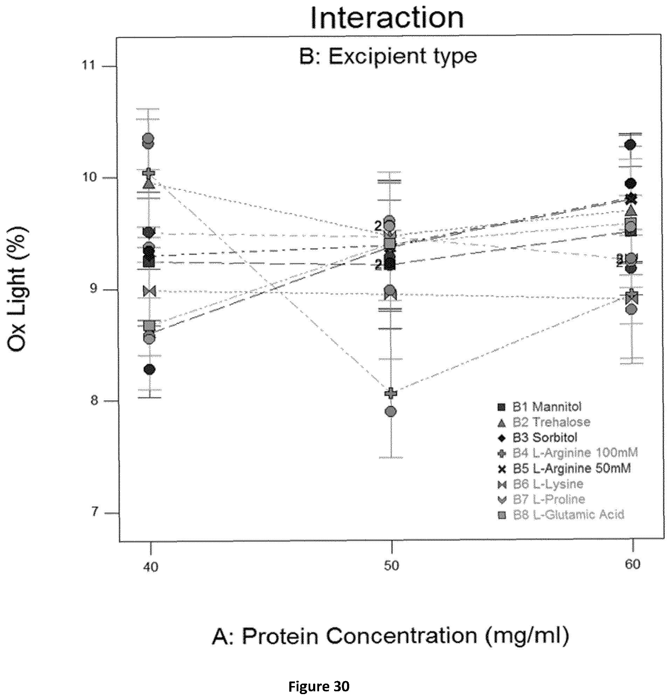

FIG. 30 is a graph showing how % oxidated forms (by RO-UPLC), after light stress, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

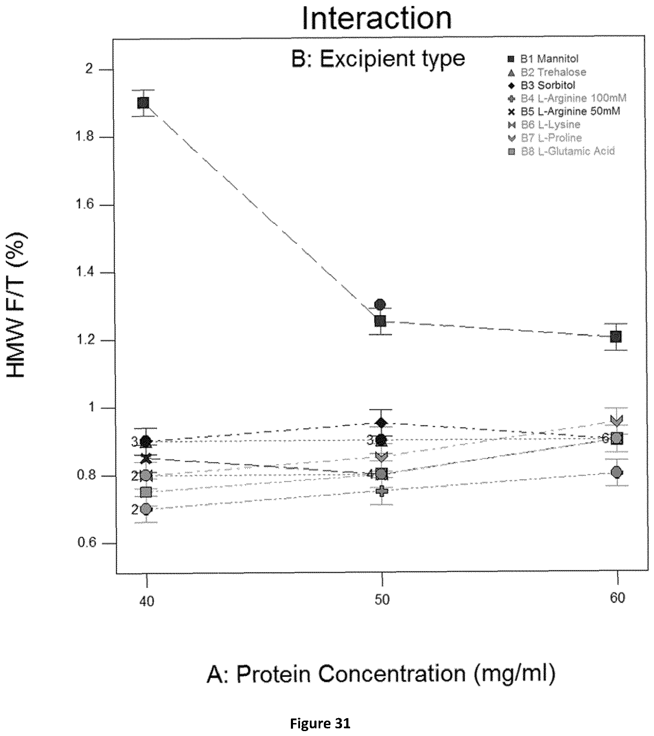

FIG. 31 is a graph showing how HMW%, after 3 freeze thaw cycles, varies with protein concentration when the surfactant is fixed as polysorbate 20 and NaCl concentration (ionic strength) is that used in Table 3J for the particular protein concentration with polysorbate 20, and the "varied excipient" as indicated in the legend (the legend refers to the predicted values based on the measured values, which are indicated by the circles in the graph).

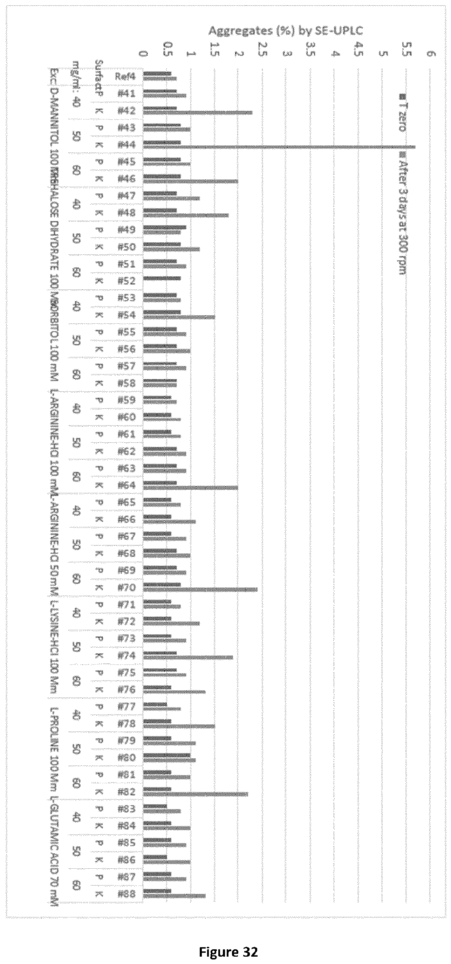

FIG. 32 is a bar chart showing how HMW% (by SE-UPLC), before and after 3 days of mechanical stress (300 rpm), varies with formulation (formulations numbers which correspond to those of Table 3J are given in the horizontal axes).

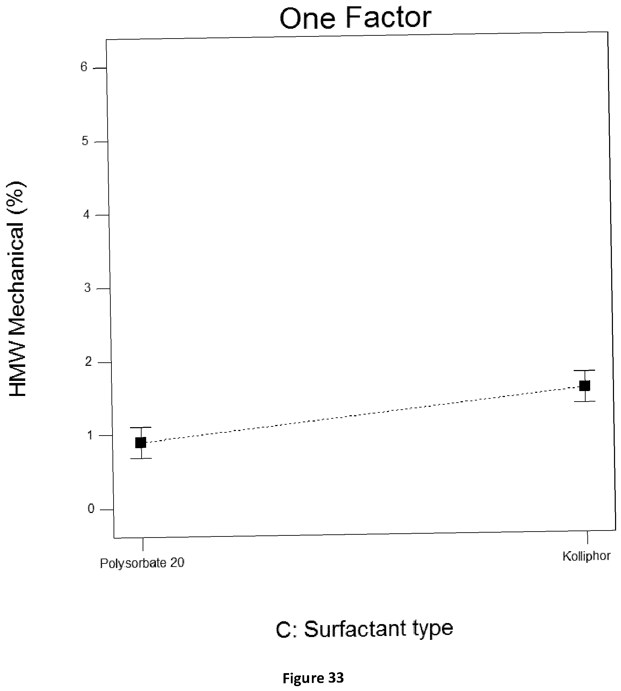

FIG. 33 is a graph showing how the HMW% (after mechanical stress) varies with surfactant when all other factors are averaged.

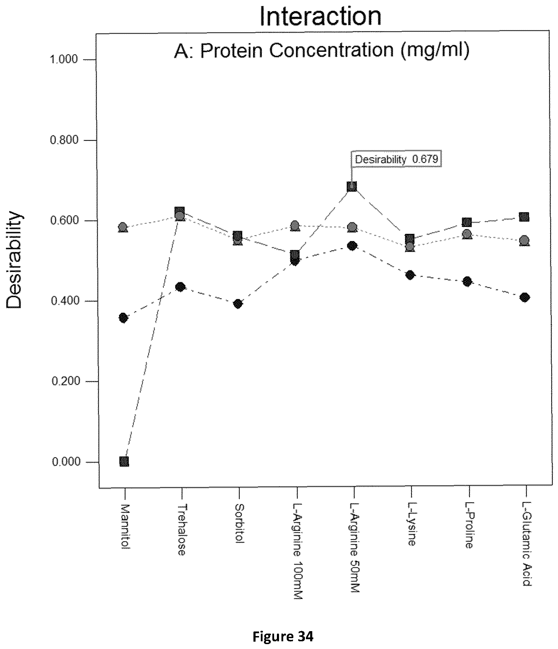

FIG. 34 is a graph showing how overall desirability (based on a balance of factors, stress tests, and results) varies with excipient for protein (bintrafusp alfa) concentrations of 40 mg/mL (predicted values shown as squares, which are based on the measured values shown as circles), 50 mg/mL (predicted values shown as triangles, which are based on the measured values shown as circles), and 60 mg/mL (predicted values shown as diamonds, which are based on the measured values shown as circles).

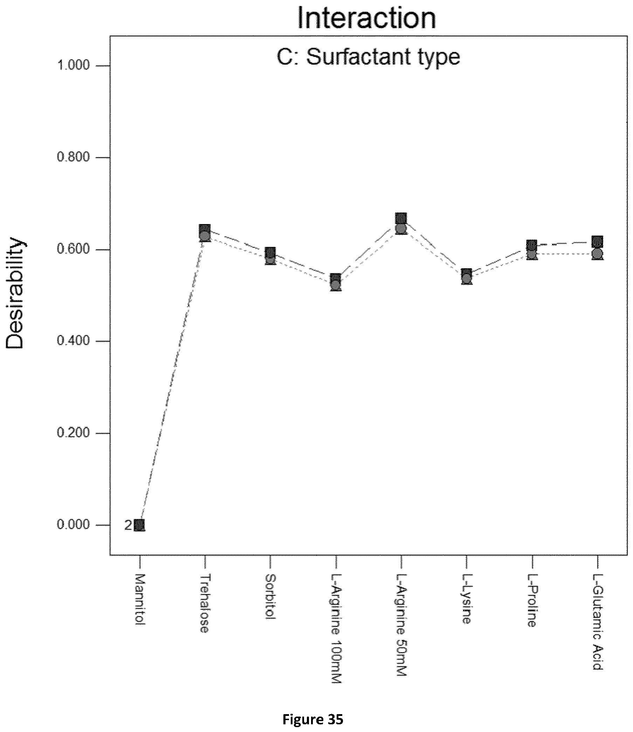

FIG. 35 is a graph showing how overall desirability (based on a balance of factors, stress tests, and results) varies with excipient, when protein concentration is fixed at 40 mg/mL, for the surfactants polysorbate 20 (predicted values shown as squares, which are based on the measured values shown as circles) and kolliphor 188 (predicted values shown as triangles, which are based on the measured values shown as circles).

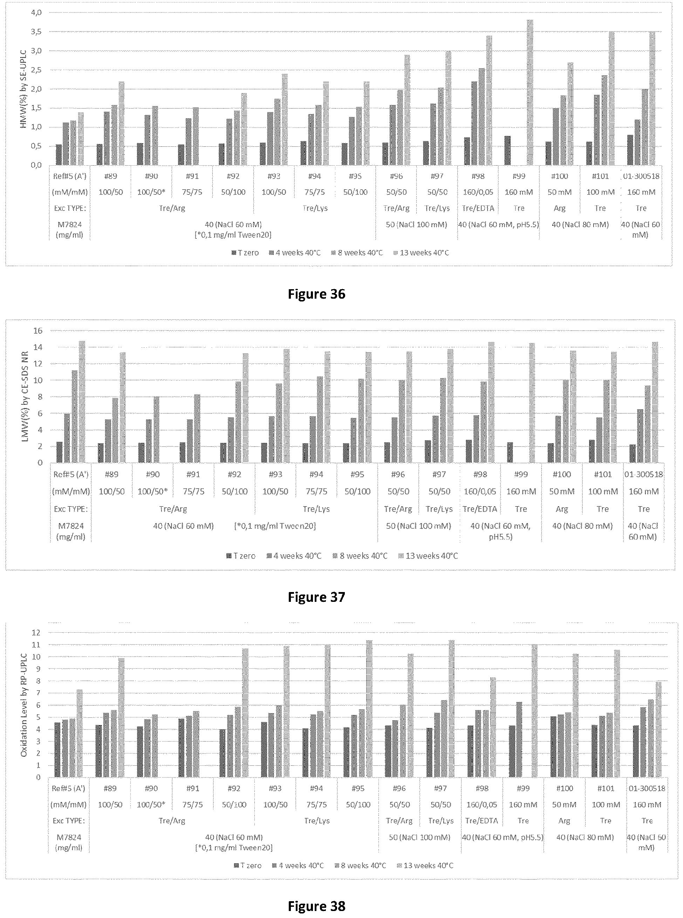

FIG. 36 is a bar chart showing %HMW increases (by SE-UPLC) after heat stress, for each of the formulations of Table 3K and additionally 2A.5 (2A.5) of Table 2A (referred to as "01-300518").

FIG. 37 is a bar chart showing %LMW increases (by CGE-NRED) after heat stress, for each of the formulations of Table 3K and additionally 2A.5 of Table 2A (referred to as "01-300518").

FIG. 38 is a bar chart showing % oxidation (by RP-UPLC) after heat stress, for each of the formulations of Table 3K and additionally 2A.5 of Table 2A (referred to as "01-300518").

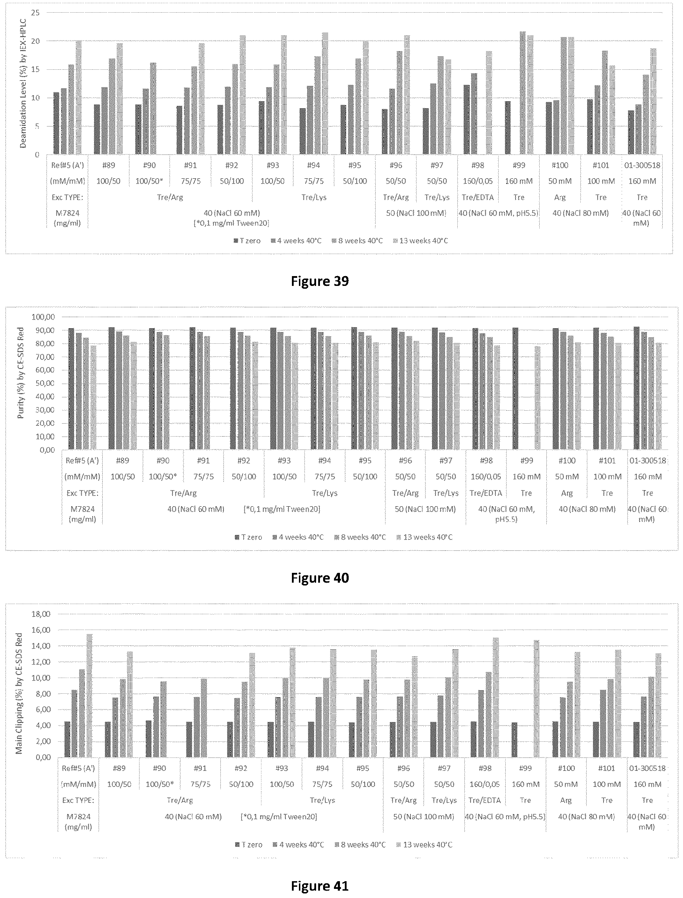

FIG. 39 is a bar chart showing % deamidation (by IEX) after heat stress, for each of the formulations of Table 3K and additionally 2A.5 of Table 2A (referred to as "01-300518").

FIG. 40 is a bar chart showing % purity (by CGE-RED, i.e. CGE under reducing conditions) after heat stress, for each of the formulations of Table 3K and additionally 2A.5 of Table 2A (referred to as "01-300518").

FIG. 41 is a bar chart showing % Main Clipping (by CGE-RED) after heat stress, for each of the formulations of Table 3K and additionally 2A.5 of Table 2A (referred to as "01-300518").

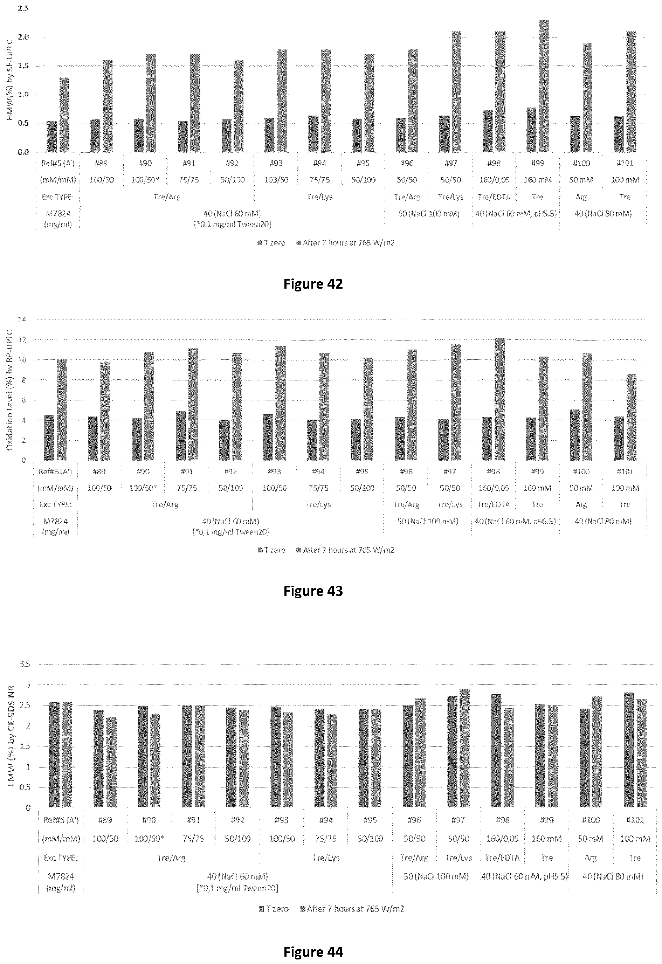

FIG. 42 is a bar chart showing %HMW increases (by SE-UPLC) after light stress, for each of the formulations of Table 3K.

FIG. 43 is a bar chart showing % oxidation (by RP-UPLC) after light stress, for each of the formulations of Table 3K.

FIG. 44 is a bar chart showing % LMW (by CGE-NRED) after light stress, for each of the formulations of Table 3K.

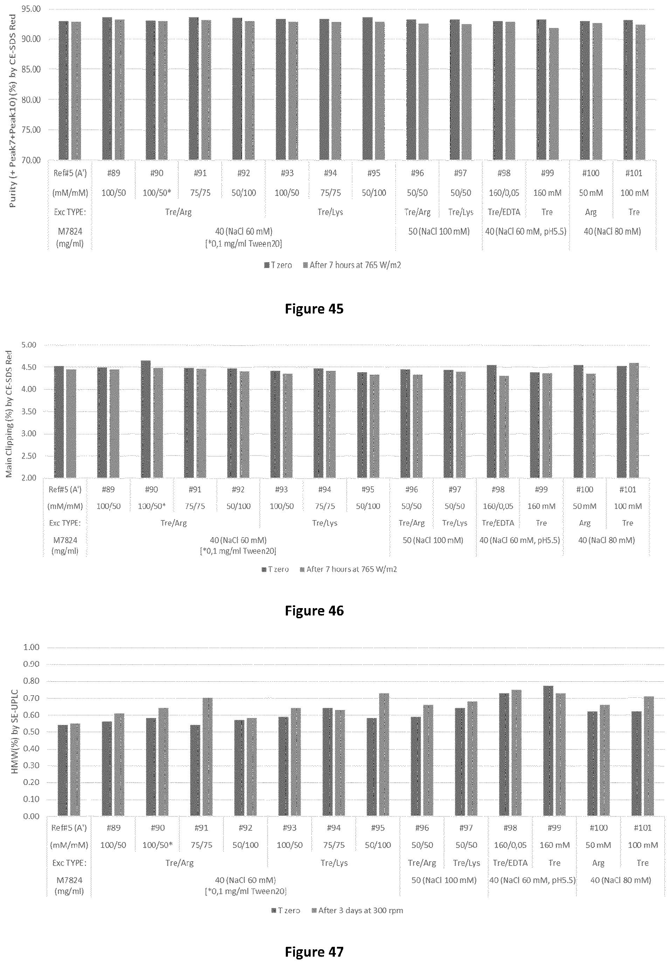

FIG. 45 is a bar chart showing % purity (by CGE-RED) after light stress, for each of the formulations of Table 3K.

FIG. 46 is a bar chart showing % Main Clipping (by CGE-RED) after light stress, for each of the formulations of Table 3K.

FIG. 47 is a bar chart showing %HMW (by SE-UPLC) after meachanical stress, for each of the formulations of Table 3K.

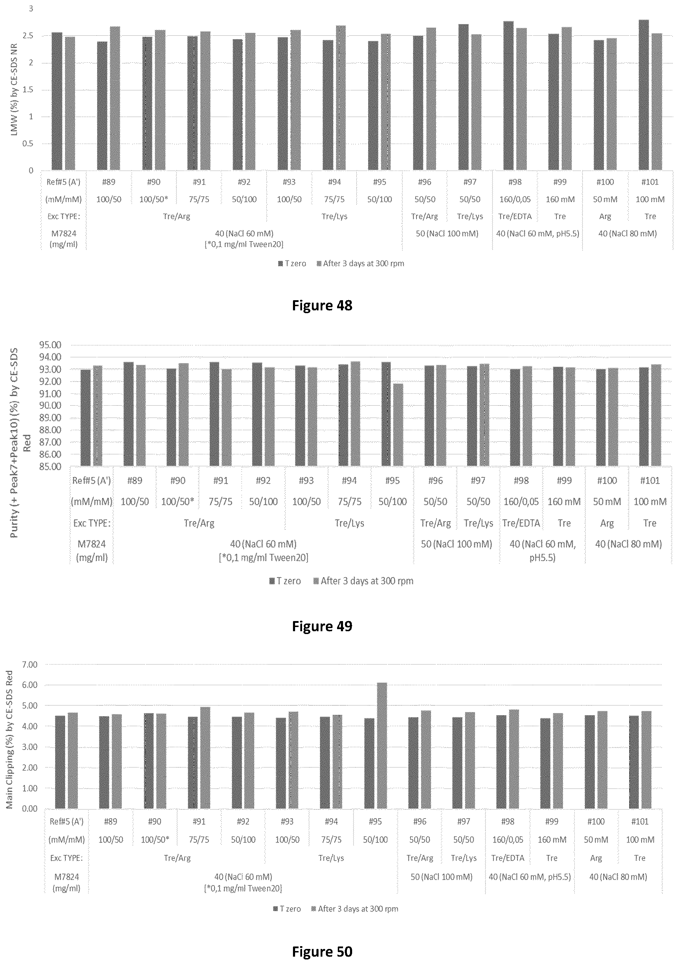

FIG. 48 is a bar chart showing % LMW (by CGE-NRED) after meachanical stress, for each of the formulations of Table 3K.

FIG. 49 is a bar chart showing % purity (by CGE-RED) after meachanical stress, for each of the formulations of Table 3K.

FIG. 50 is a bar chart showing % Main Clipping (by CGE-RED) after meachanical stress, for each of the formulations of Table 3K.

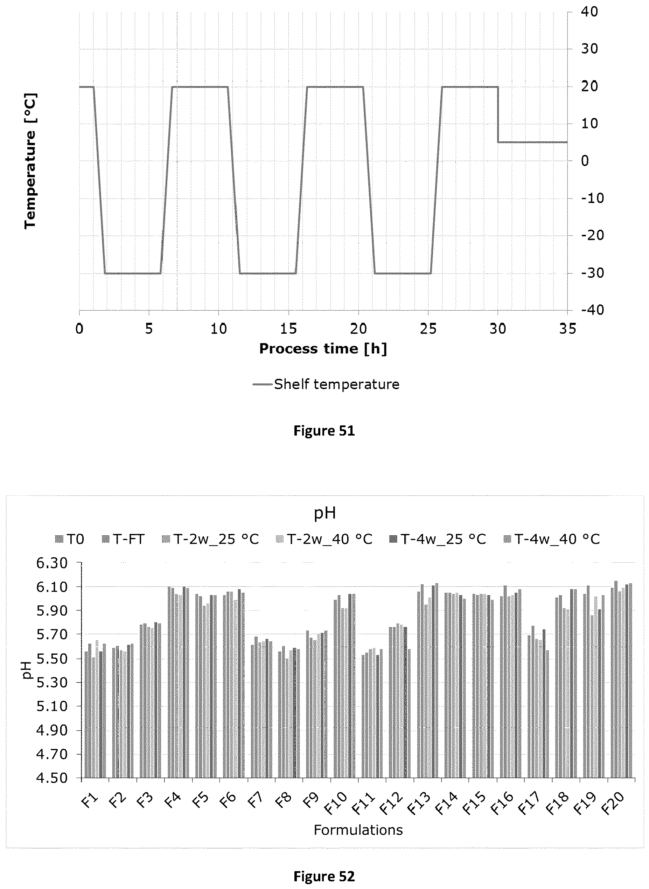

FIG. 51 shows a graph of time vs temperature illustrating how 3 FT cycles were performed.

FIG. 52 is a bar chart showing how pH varies in all of formulations F1-F20 at various time points of stressing testing, including (bars listed from left-to-right for each formulation): time = 0; after 3 FT cycles; after 2 weeks at 25°C, after 2 weeks at 40°C, after 4 weeks at 25°C, after 4 weeks at 40°C.

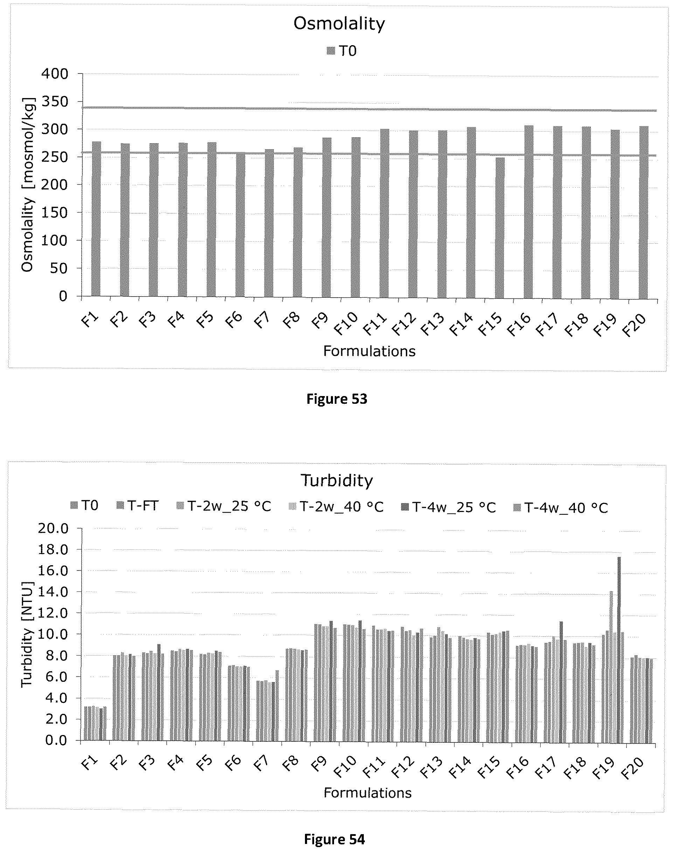

FIG. 53 is a bar chart showing the osmolality for each of the formulations F1-F20.

FIG. 54 is a bar chart showing how turbity varies in all of formulations F1-F20 at various time points of stressing testing, including (bars listed from left-to-right for each formulation): time = 0; after 3 FT cycles; after 2 weeks at 25°C, after 2 weeks at 40°C, after 4 weeks at 25°C, after 4 weeks at 40°C.

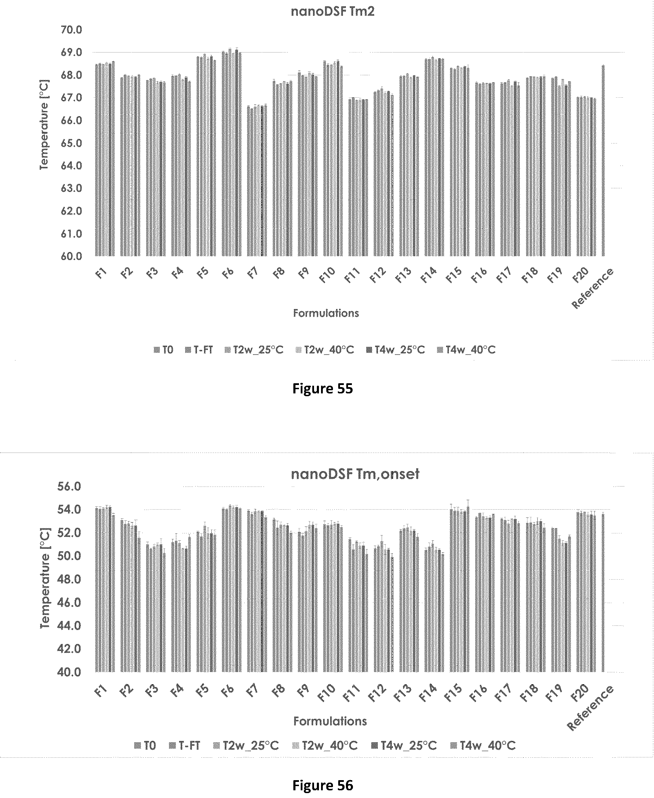

FIG. 55 is a bar chart showing how the temperature of the $T_m2$ peak of nano-DSC traces varies in all of formulations F1-F20 at various time points of stressing testing, including (bars listed from left-to-right for each formulation): time = 0; after 3 FT cycles; after 2 weeks at 25°C, after 2 weeks at 40°C, after 4 weeks at 25°C, after 4 weeks at 40°C.

FIG. 56 is a bar chart showing how the onset temperature $T_m$ of nano-DSC traces varies in all of formulations F1-F20 at various time points of stressing testing, including (bars listed from left-to-right for each formulation): time = 0; after 3 FT cycles; after 2 weeks at 25°C, after 2 weeks at 40°C, after 4 weeks at 25°C, after 4 weeks at 40°C.

## DETAILED DESCRIPTION OF THE INVENTION

### DEFINITIONS

Unless otherwise stated, the following terms used in the specification and claims have the following meanings set out below.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Herein, references to an "antibody", such as IgG (for instance when used in notation such as IgG:TGFβR fusion protein), preferably means the full antibody or an antigen-binding fragment thereof, wherein the antigen-binding fragment preferably comprises at least an Fv region or a ScFv, more preferably comprises at least a Fab region or a F(ab)$_2$ fragment. However, in more preferred embodiments, references to an "antibody", such as IgG (or any of its subtypes, such as IgG1, IgG4), means the full antibody.

A full antibody generally contains in its monomeric form two fragment antigen-binding (Fab) regions (or Fab domains), optionally labelled F(ab)$_2$, connected, typically *via* a hinge region, to a fragment crystaliizable (Fc) region (or Fc domain). Such structures are composed from four polypeptide chains - two identical heavy chains and two identical light chains all interconnected by disulphide bridges. Each Fab domain contains a pairing of part of a single heavy chain (e.g. $V_H$-$C_H1$) and a single light chain (e.g. $V_L$-$C_L$) joined *via* a disulphide bridge. The Fc domain contains a pairing of the two remaining parts of the heavy chains (e.g. $C_H2$-$C_H3$).

A full IgG is a monomeric antibody and has the structure described above in respect of a full antibody. In the case of a full IgG, each identical heavy chain contains, in sequential order starting from the *N*-terminus and finishing at the *C*-terminus, a variable heavy region ($V_H$), a first constant heavy region ($C_H1$), a second constant heavy region ($C_H2$), and a third constant heavy region ($C_H3$). Meanwhile, each identical light chain contains, in sequential order starting from the *N*-terminus and finishing at the *C*-terminus, a variable light region ($V_L$), and a constant light region ($C_L$). Bridged together *via* disulphide bridges, the $V_H$-$C_H1$ part of the heavy chain and the $V_L$-$C_L$ of the light chain form one of a pair of Fab regions of a full IgG. Bridged together *via* disulphide bridges, two identical $C_H2$-$C_H3$ parts of the heavy chain form the Fc region of a full IgG. A full IgG has a molecular weight between 140 kDa and 180 kDa, preferably either a molecular weight of 140-160 kDa (more preferably 144-155 kDa, most preferably about 146 kDa) or a molecular weight of 165-175 kDa (more preferably 168-172 kDa, most preferably about 170 kDa). A full IgG typically has a pI of 6-9.5.

There are four distinct subclasses of full IgG antibodies, namely IgG1, IgG2, IgG3, and IgG4. All subclasses possess the same core regions described above, but vary slightly, especially in terms of the number of amino acids and/or disulphide bonds at the hinge region. IgG1 and IgG4 may be considered most similar from a structural perspective. A full IgG1, IgG2, or an IgG4 typically has a molecular weight of 140-160 kDa (more preferably 144-155 kDa, most preferably about 146 kDa), whereas a full IgG3 typically has a molecular weight of 165-175 kDa (more preferably 168-172 kDa, most preferably about 170 kDa). Typically a full IgG1 has a pI of 8-9.4, more preferably 8.2-9.2. Typically a full IgG2 has a pI of 6.5-8.5, more preferably 7.0-8.0. Typically a full IgG3 has a pI of 7-9.5, more preferably 7.5-9.0. Typically a full IgG4 has a pI of 6-8.5, more preferably 6.4-8.

An antigen-binding fragment may, for instance, include: a Fab region (e.g. $V_H$-$C_H1$ paired with its corresponding light chain $V_L$-$C_L$) or a F(ab)$_2$ region with two connected Fab regions; an Fv region (e.g. $V_H$ paired with its corresponding variable light chain portion, $V_L$); and/or a single-chain variable fragment ScFv domain (e.g. $V_H$ connected to $V_L$ *via* a peptide linker, preferably containing about 25 amino acids). Insofar as the present invention is concerned, references to IgG, for instance in an IgG:TGFβR fusion protein (including more specifically defined fusion proteins such as anti-PD-L1 (IgG):TGFβR2 fusion proteins), may be the full IgG, for instance fused to TGFβR2, or an antigen-binding fragment such as the aforementioned, for instance fused to TGFβR2. Most preferably, however, the full IgG is preferred in such fusion proteins.

The term "TGF-β receptor" (TGFβR), as well as "TGF-β receptor I" (TGFβR1) or "TGF-β receptor II" (TGFβR2), are well known in the art. For the purposes of this disclosure, reference to such receptor includes the full receptor and fragments that are capable of binding TGF-β. Preferably, it is the extracellular domain of the receptor or a fragment of the extracellular domain that is capable of binding TGF-β.

The term "fusion protein" is well understood in the art. An IgG:TGFβR fusion protein is an IgG antibody (preferably a monoclonal antibody, preferably in homodimeric form) fused to a TGF-β receptor. The nomenclature anti-PD-L1 (IgG1):TGFβR2 fusion protein indicates an anti-PD-L1 IgG1 antibody fused to a TGF-β receptor II, preferably a fragment of the extracellular domain thereof that is capable of binding TGF-β.

"Bintrafusp alfa" is well understood in the art. Bintrafusp alfa is an anti-PD-L1 (IgG1):TGFβR2 fusion protein and described under the CAS Registry Number 1918149-01-5. It is also described in WO2015118175 and further elaborated in *Lan et al* (Lan et al, "Enhanced preclinical antitumor activity of M7824, a bifunctional fusion protein simultaneously targeting PD-L1 and TGF-β", Sci. Transl. Med. 10, 2018, p.1-15),. In particular, bintrafusp alfa is a fully human immunoglobin G1 (IgG1) monoclonal antibody against human PD-L1 fused to the extracellular domain of human TGFβ receptor II (TGFβR2). As such, bintrafusp alfa is a bifunctional fusion protein which simultaneously blocks PD-L1 and TGFβ pathways. In particular, WO2015118175 describes bintrafusp alfa on page 34 in Example 1 thereof as follows (bintrafusp alfa is referred to in this passage as "anti-PD-L1/TGFβ Trap"):

"Anti-PD-L1/TGFβ Trap is an anti-PD-L1 antibody-TGFβ Receptor II fusion protein. The light chain of the molecule is identical to the light chain of the anti-PD-L1 antibody (SEQ ID NO: 1). The heavy chain of the molecule (SEQ ID NO:3) is a fusion protein comprising the heavy chain of the anti-PD-L1 antibody (SEQ ID NO: 2) genetically fused to via a flexible (Gly$_4$Ser)$_4$Gly linker (SEQ ID NO:11) to the N-terminus of the soluble TGFβ Receptor II (SEQ ID NO: 10). At the fusion junction, the C-terminal lysine residue of the antibody heavy chain was mutated to alanine to reduce proteolytic cleavage."

For the purposes of this disclosure and molar calculations, the molecular weight ($M_w$) of bintrafusp alfa is considered to be 182 kilodaltons (kDa), or 182,000 g/mol. As such, a liquid pharmaceutical composition containing 10 mg/mL bintrafusp alfa may be considered a 0.055 mM solution thereof, 40 mg/mL as a 0.220 mM solution thereof, 50 mg/mL as a 0.275 mM solution thereof.

References herein to any particular fusion protein, including any of the IgG:TGFβR fusion proteins described herein (especially bintrafusp alfa), include the relevant originator drug substance, whether as commercially available, as described in a patent document, or as described elsewhere in the art, and also biosimilars thereof.

References herein to any particular IgG:TGFβR fusion protein defined by reference to a defined nucleotide or amino acid sequence (especially IgG:TGFβR2 fusion proteins and more particularly an IgG:TGFβR2 fusion protein having the amino acid sequence of bintrafusp alfa) described herein, may include variants, which (1) share at least 70%, at least 80%, at least 90%, at least 95%, at least 98%, or at least 99% amino acid sequence identity with amino acid sequences disclosed in relation to said IgG:TGFβR fusion proteins or (2) have differences in not more than 30%, not more than 20%, not more than 10% or not more than 5% of the amino acid residues disclosed in relation to said IgG:TGFβR fusion proteins. Preferably, references herein to any particular IgG:TGFβR fusion protein defined by reference to a nucleotide or amino acid sequence (especially IgG:TGFβR2 fusion proteins and more particularly an IgG:TGFβR2 fusion protein having the amino acid sequence of bintrafusp alfa) described herein, refer to a protein having such exact sequence without any variants thereof.

The term sequence identity is well known in the art. Sequence identity can be determined using the Needleman-Wunsch algorithm for the global alignment of two sequences (Needleman and Wunsch, "A general method applicable to the search for similarities in the amino acid sequence of two proteins", J Mol Biol 48 (3), 443-53. Mar 1970).

The indication that a protein "differs" in a certain amount of amino acid residues means that such amount of residues is either inserted, deleted and/or substituted as compared to the reference sequence.

Herein, the term "buffer", "buffer system", or "buffered solution" refers to a generally aqueous solution comprising a mixture of an acid (usually a weak acid, e.g. acetic acid, citric acid, imidazolium form of histidine) and its conjugate base (e.g. an acetate or citrate salt, for example, sodium acetate, sodium citrate, or histidine) or alternatively a mixture of a base (usually a weak base, e.g. histidine) and its conjugate acid (e.g. protonated histidine salt). The pH of a "buffered solution" will change only slightly upon addition of a small quantity of strong acid or base due to the "buffering effect" imparted by the buffer system. A buffered solution may comprise one or more buffer systems, though preferably no more than two buffer systems (i.e. a dual-buffer system such as histidine-citrate, histidine-acetate, citrate-phosphate), but most preferably the buffered solution comprises one and at most one buffer system.

Herein, in the context of the present specification, a "strong acid" is preferably one having a $pK_a$ of -1.0 or less, whereas a "weak acid" is preferably one having a $pK_a$ of 2.0 or more. Herein, in the context of the present specification, a "strong base" is preferably one whose conjugate acid has a $pK_a$ of 12 or higher (preferably 14 or higher), whereas a "weak base" is preferably one whose conjugate acid has a $pK_a$ of 10 or less.

Unless stated otherwise, references herein to a "$pK_a$" should be construed as a $pK_a$ value in water at standard ambient temperature and pressure (SATP), preferably of the conjugate acid of the relevant species.

An "amino acid component" is an ingredient or ingredients comprising one or more amino acids, though an amino acid component may consist of a single amino acid.

Unless stated otherwise, references herein to an "amino acid" or "amino acids", whether specific (e.g. arginine, histidine) or general (e.g. any amino acid), in the context of their presence or otherwise within compositions (especially pharmaceutical liquid compositions of the invention) relate to the corresponding free amino acid(s) (regardless of its/their protonation state and/or salt form, though for consistency amounts are preferably calculated by reference to the free amino acid *per se*). This may preferably include natural and/or artificial amino acids. Unless stated to the contrary, such references are not intended to relate to amino acid residue(s) covalently incorporated as part of a larger compound (as opposed to a composition comprising multiple compounds), such as a peptide or protein (where such amino acid residues are linked *via* peptide bonds). As such, though an antibody, as a protein, contains amino acid residues, it is not considered to comprise any "free amino acid(s)". By way of example, a composition defined as being "*free of arginine*" does not contain any free arginine but it may still include one or more proteins (e.g. bintrafusp alfa) which do themselves comprise arginine residues.

Unless stated otherwise, references herein to any one or more "amino acids", whether specific or general, preferably relate to the L- stereoisomers or a racemate thereof, most preferably L-amino acids.

Herein, a "sugar component" is an ingredient or ingredients comprising one or more sugar(s) and/or sugar alcohol(s), though a sugar component may consist of a single sugar or sugar alcohol.

Herein, a "non-reducing sugar" is a sugar without any aldehyde moieties or without the capability of forming an aldehyde moiety (e.g. through isomerism).

Herein, a "tonicity modifier" or "tonicifier" refers to a reagent whose inclusion within a composition contributes to (or increases) the overall osmolality and osmolarity of the composition. Preferably, a tonicifier, as used herein includes an agent which functions to render a solution similar in osmotic characteristics to physiologic fluids.

Herein, an "antioxidant" or "antioxidant component" is an ingredient or ingredients comprising one or more antioxidant compounds, though an antioxidant component may consist of a single antioxidant compound. An antioxidant in the context of the compositions of the invention preferably mitigate oxidation of groups within the fusion protein that might otherwise be vulnerable to oxidation.

"Chelator" is a term of art referring to a compound capable of complexing, preferably in a multidentate manner, with various groups, molecules, atoms, or ions, and may exert an antioxidant effect in its own right.

Herein, wherever a composition is said "to be characterised by an absence of [*a particular component*]", this means that the composition in question is either substantially free or entirely free of said component.

The term "substantially free", when used in relation to a given component of a composition (e.g. "a liquid pharmaceutical composition substantially free of an amino acid component"), refers to a composition to which essentially none of said component has been added. As explained above, such references have no bearing on the presence of amino acid residue(s) within a protein structure. When a composition is "substantially free" of a given component, said composition preferably comprises no more than 0.1 wt% of said component, preferably no more than 0.01 wt% of said component, preferably no more than 0.001 wt% of said component, preferably no more than 0.0001 wt% of said component, preferably no more than 0.00001 wt%, preferably no more than 0.000001 wt%, preferably no more than 0.0000001 wt% thereof, most preferably no more than 0.0001 parts per billion (by weight).

The term "entirely free", when used in relation to a given component of a composition (e.g. "a liquid pharmaceutical composition entirely free of an amino acid component"), refers to a composition containing none of said component. As explained above, such references in relationship to an amino acid present in the pharmaceutical composition have no bearing on the presence of amino acid residue(s) within a protein structure.

Preferably, unless stated otherwise, where reference is made to a parameter (e.g. pH, $pK_a$, etc.) or state of a material (e.g. liquid, gas, etc.) which may depend on pressure and/or temperature, preferably in the absence of further clarification such a reference refers to said parameter at standard ambient temperature and pressure (SATP). SATP is a temperature of 298.15 K (25 °C, 77 °F) and an absolute pressure of 100 kPa (14.504 psi, 0.987 atm).

Herein, references to specific amounts of a given component of a composition, especially a buffering agent or buffer system, surfactant, sugar component, amino acid component, tonicifier, antioxidant, and/or chelator preferably relate to the amounts of the pure anhydrous form of the relevant component (or compositions formed by using said amounts of the pure anhydrous form), even though such a component may be used in a non-anhydrous form when forming the composition. Amounts of any corresponding non-anhydrous forms (e.g. monohydrates, dihydrates, etc.) may be readily calculated by simply using the appropriate multiplier. For instance, unless stated otherwise (as per the Examples, where quantities relate to trehalose dihydrate), amounts stipulated in relation to trehalose refer to the anhydrous form of trehalose (or compositions formed by using the stipulated amounts/concentrations of anhydrous trehalose), which has a molecular weight of 342.296 g/mol, so to calculate the corresponding amount of trehalose dihydrate needed to form the same composition (less water would have to be added) it is necessary to multiply the stipulated amount by 378.33/342.296, since 378.33 is the molecular weight of trehalose dihydrate. The skilled person would readily understand how to judiciously adjust the quantity of diluent/water depending on the form of the components used, in order to derive the target concentrations. Clearly, where molar quantities are stipulated this issue does not apply.

Herein, the term "pharmaceutical composition" refers to a formulation which is suitable for mammalian treatment, e.g., in the sense that it does not include ingredients that are overly toxic. Herein, references to a "composition" generally refer to a pharmaceutical composition as defined herein.

Herein, the term "stable" generally refers to the physical stability and/or chemical stability and/or biological stability of a component, typically an active or composition thereof, during preservation/storage. For aqueous compositions of a biologic, storage stability may preferably mean that the biologic is sufficiently stable (i.e. with prescribed limits for patient safety) when stored at 2-8°C for at least 6 months, preferably at least 12 months, preferably up to 24 months. However, accelerated stability studies may be used to provide relevant stability information.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, *i.e.*, causing regression of the state, disorder or condition or at least one of its clinical or subclinical symptoms.

In the context of the present invention, a "therapeutically effective amount" or "effective amount" of the pharmaceutical composition means an amount that is effective, when administered to a mammal for treating a disease or disorder, in prophylactic and therapeutic aspect and the pharmaceutical composition is effective in treatment of the diseases concerned. The "therapeutically effective amount" will vary depending on the fusion protein, the disease and its severity and the age, weight, etc., of the mammal to be treated.

Herein, amounts stipulated for components and ingredients, whether specified in terms of "parts", ppm (parts per million), percentages (%, e.g. wt%), or ratios, are intended to be by weight, unless stated otherwise.

Where the quantity or concentration of a particular component of a given composition is specified as a weight percentage (wt% or %w/w), said weight percentage refers to the percentage of said component by weight relative to the total weight of the composition as a whole. It will be understood by those skilled in the art that the sum of weight percentages of all components of a composition (whether or not specified) will total 100 wt%. However, where not all components are listed (e.g. where compositions are said to "comprise" one or more particular components), the weight percentage balance may optionally be made up to 100 wt% by unspecified ingredients (e.g. a diluent, such as water, or other non-essentially but suitable additives).

Where a composition is said to comprise a plurality of stipulated ingredients (optionally in stipulated amounts of concentrations), said composition may optionally include additional ingredients other than those stipulated. However, in certain embodiments, a composition said to comprise a plurality of stipulated ingredients may in fact consist essentially of or consist of all the stipulated ingredients, optionally in the amounts specified. In either circumstance, an individual component may itself comprise, consist essentially of, or consist of a sub-component or one or more sub-components. Herein, wherever the term "comprise" is used it may, where compatible with the context, be replaced by "consists essentially of or "consists of".

Herein, where a composition is said to "consist essentially of" a particular component or plurality of components, said composition preferably comprises at least 70 wt% of said component(s), preferably at least 90 wt% thereof, preferably at least 95 wt% thereof, most preferably at least 99 wt% thereof. Preferably, a composition said to "consist essentially of" a particular component consists of said component save for one or more trace impurities.

"About" when used to modify a numerically defined parameter (e.g., a pH value) means that the parameter may vary, e.g., within the experimental accuracy of determining the parameter or by as much as much as 5% below or above the stated numerical value for that parameter, preferably by as much as 2% below or above the stated numerical value for that parameter. In preferred embodiments, a parameter described by the term "about" corresponds to the stated numerical value.

Herein, unless incompatible in a given context, wherever a component is stipulated which is capable of ionization (e.g. protonation or deprotonation), the definition of said component preferably includes any suitable salts thereof, preferably pharmaceutically acceptable salts thereof. For example, this applies to any references herein to buffering agents (e.g. citric acid or citrate), amino acids, and such like. Likewise, unless incompatible in a given context, wherever a component is stipulated which is capable of neutralisation, the definition of said component preferably includes neutralised forms thereof - e.g. citric acid instead of citrate.

An "isoelectric point" (pI) represents a pH at which a given molecule (or portion thereof) is, from a statistical perspective, electrically neutral - i.e. carrying no net electrical charge. pI is of particular relevance to proteins, including fusion proteins of the invention, because they contain functional groups that may be positive, negative, neutral, and polar, depending on the prevailing pH of the local environment. The pI of any given molecule or portion thereof may be experimentally determined by methods well known in the art. However, pI may also be calculated using various methods well known in the art. Preferably, the pI is experimentally determined.

## *GENERAL POINTS AND ADVANTAGES REGARDING THE INVENTION*

When developing viable antibody formulations, and especially antibody-fusion protein formulations, it is generally acknowledged that various factors affect the stability of the formulation. In the present case, the fusion of antibody with receptor further complicates formulation development since both parts of the molecule must be catered for.

The present invention arose following skilful and intuitive targeting and diligent exploration of generally-barren formulation space to reveal which key excipients, and relative amounts thereof, should be present or absent in order to best complement the relevant fusion protein during processing and/or storage. Without such a well-targeted and diligent approach a formulation scientist is unlikely to devise viable formulations.

Many of the advantages of the present invention, and indeed the challenges involved in its conception and development, will be self-evident. The inventive endeavours elucidated in this disclosure represent a significant contribution to the art, a contribution to which the present invention is commensurate in scope given that, notwithstanding the aforesaid unpredictabilities, the present invention underpinned by the examples and data presented herein establishes viable and plausible formulation space.

## *PHARMACEUTICAL COMPOSITION*

The present invention provides a pharmaceutical composition. Preferably, the pharmaceutical composition is a liquid pharmaceutical composition, preferably an aqueous pharmaceutical composition (which thus comprises water, preferably water for injection, as diluent). However, the pharmaceutical composition may also be a lyophilised composition (i.e. and thus preferably a solid lyophilised pharmaceutical composition). Such a lyophilised composition may be preferably reconstituted to provide a liquid pharmaceutical composition, preferably an aqueous pharmaceutical composition. Definitions and amounts given herein may pertain to either or both liquid and/or lyophilised compositions. Where amounts are stipulated in terms of a concentration within a liquid composition (e.g. whether as a weight%, a weight per volume, or a molarity or molality) the same may be converted to concentration ratios of a solid composition (whether weight ratios or molar ratios) *via* simple calculations known in the art, for instance, using a foreknowledge of molecular weights of the relevant ingredients to enable conversion of two common units (e.g. parts by weight or weight%, or moles).

The pharmaceutical composition comprises an IgG:TGFβR fusion protein, and optionally one or more pharmaceutically acceptable excipients and/or carriers. Preferably, the pharmaceutical composition comprises one or more of the following: a buffer system, a surfactant, a sugar component, an amino acid component, a tonicifier, an antioxidant, and a chelator. Liquid pharmaceutical compositions (whether formulated as such or reconstituted form a lyophilised formulation) preferably comprise a diluent, such as water (e.g. water for injection). Preferably, the pharmaceutical composition is characterised by a pH of 4-8. Preferably, the pharmaceutical composition is characterised by an osmolality of 240-640 mOsm/kg, in particular, if the pharmaceutical composition is intended for subcutaneous injection without prior dilution.

In particular embodiments, the pharmaceutical composition comprises (or consists of, optionally along with water for injection in the case of aqueous composition) and/or is characterised by one or more of: an IgG:TGFβR fusion protein; a buffer system; a pH of 4-8; a surfactant; a sugar component; an amino acid component; a tonicifier; an antioxidant; and/or a chelator.

The following numbered paragraphs A1 to A9 disclose specific embodiments of the invention.

A1. A pharmaceutical composition comprising an IgG:TGFβR fusion protein.
A2. The pharmaceutical compositon of A1 further comprising a tonicifier.
A3. The pharmaceutical compositon of A1 or A2, further characterised by a pH of 4-8.
A4. The pharmaceutical compositon of any of A1-A3, further comprising a buffer system.
A5. The pharmaceutical compositon of any of A1-A4, further comprising a surfactant.
A6. The pharmaceutical compositon of any of A1-A5, further comprising a sugar component.
A7. The pharmaceutical compositon of any of A1-A6, further comprising an antioxidant.
A8. The pharmaceutical compositon of any of A1-A7, further comprising an amino acid component.
A9. The pharmaceutical compositon of any of A1-A8, further comprising a chelator.

### *Fusion Protein*

The pharmaceutical composition comprises an IgG:TGFβR fusion protein.

The IgG:TGFβR fusion protein is preferably an IgG:TGFβR fusion protein comprising an IgG fused to a soluble extracellular domain of TGFβR, more preferably a soluble extracellular domain of TGFβR2, or a fragment thereof that is capable of binding TGF-β.

The IgG:TGFβR fusion protein is preferably an IgG:TGFβR fusion protein, wherein the IgG has a pI of 8-10, preferably a pI of 8.5-9.5, whereas the TGFβR has a pI of 4.5-6, preferably a pI of 4.6-5.4.

The IgG of the IgG:TGFβR fusion protein is preferably selected from the group consisting of an anti-PD-L1 (IgG) and an anti-PD-1 (IgG), more preferably it is an anti-PD-L1 (IgG). In some embodiments, the anti-PD-L1 (IgG) is selected from the group consisting of (1) an anti-PD-L1 (IgG) comprising three heavy chain CDRs having amino acid sequences of SEQ ID NO: 19 (CDR1), SEQ ID NO: 20 (CDR2) and SEQ ID NO: 21 (CDR3), and three light chain CDRs having amino acid sequences of SEQ ID NO: 22 (CDR1), SEQ ID NO: 23 (CDR2) and SEQ ID NO: 24 (CDR3), (2) an anti-PD-L1 (IgG) comprising three heavy chain CDRs having amino acid sequences of SEQ ID NO: 1 (CDR1), SEQ ID NO: 2 (CDR2) and SEQ ID NO: 3 (CDR3), and three light chain CDRs having amino acid sequences of SEQ ID NO: 4 (CDR1), SEQ ID NO: 5 (CDR2) and SEQ ID NO: 6 (CDR3), and (3) an anti-PD-L1 (IgG) comprising three heavy chain CDRs having amino acid sequences of SEQ ID NO: 27 (CDR1), SEQ ID NO: 28 (CDR2) and SEQ ID NO: 29 (CDR3), and three light chain CDRs having amino acid sequences of SEQ ID NO: 30 (CDR1), SEQ ID NO: 31 (CDR2) and SEQ ID NO: 32 (CDR3). In preferred embodiments, the anti-PD-L1 (IgG) comprises the heavy chain CDRs having amino acid sequences of SEQ ID NOs: 1, 2 and 3, and the light chain CDRs having amino acid sequences of SEQ ID NOs: 4, 5 and 6. In some embodiments, the light chain variable region and the heavy chain variable region of the anti-PD-L1 (IgG) comprise SEQ ID NO: 25 and SEQ ID NO: 26, respectively. In some embodiments, the light chain sequences and the heavy chain sequences of the anti-PD-L1 (IgG) respectively correspond to (1) SEQ ID NO: 7 and SEQ ID NO: 16, (2) SEQ ID NO: 15 and SEQ ID NO: 14, or (3) SEQ ID NO: 33 and SEQ ID NO: 35. The IgG class of the IgG:TGFβR fusion protein is preferably selected from the group consisting of IgG1, IgG2, and IgG4, more preferably it is IgG1 or IgG4 and most preferably it is IgG1.

Preferably, the $C_H3$ domain of the IgG:TGFβR fusion protein has greater than or equal to 85% sequence identity, greater than or equal to 90% sequence identity, greater than or equal to 95% sequence identity, or at least 96% sequence identity with the amino acid sequence of the $C_H3$ domain of bintrafusp alfa. Preferably, the $C_H3$ domain of the IgG:TGFβR fusion protein has an amino acid sequence with not more than 10, not more than 5, or not more than 4 amino acid residues different from the $C_H3$ domain of bintrafusp alfa.

Preferably, the $C_H1$ domain of the IgG:TGFβR fusion protein has greater than or equal to 80% sequence identity, greater than or equal to 85% sequence identity, greater than or equal to 90% sequence identity, or at least 91% sequence identity with the amino acid sequence of the $C_H1$ domain of bintrafusp alfa. Preferably the $C_H1$ domain of the IgG:TGFβR fusion protein has an amino acid sequence with not more than 20, not more than 10, or not more than 7 amino acid residues different from the $C_H1$ domain of bintrafusp alfa.

Preferably, the $C_H2$ domain of the IgG:TGFβR fusion protein has greater than or equal to 80% sequence identity, greater than or equal to 85% sequence identity, greater than or equal to 90% sequence identity, or at least 91% sequence identity with the amino acid sequence of the $C_H2$ domain of bintrafusp alfa. Preferably the $C_H2$ domain of the IgG:TGFβR fusion protein has an amino acid sequence with not more than 20, not more than 10, or not more than 8 amino acid residues different from the $C_H2$ domain of bintrafusp alfa.

Variability in the variable domains (light and heavy) and the light chain at large may be tolerable.

The TGFβR of the IgG:TGFβR fusion protein is preferably TGFβR1 or TGFβR2, more preferably it is TGFβR2. In a preferred embodiment, it is an IgG:TGFβR2 fusion protein, wherein the IgG has a pI of 8.5-9.5 whereas the TGFβR2 has a pI of 4.6-5.4. In another preferred embodiment, it is an anti-PD-L1 (IgG):TGFβR2 fusion protein, such as an anti-PD-L1 (IgG1):TGFβR2 or an anti-PD-L1 (IgG4):TGFβR2. Most preferably, it is an anti-PD-L1 (IgG1):TGFβR2. Preferably, the TGFβR2 is a soluble extracellular domain of TGFβR2 or a fragment thereof that is capable of binding TGF-β. Preferably, TGFβR2 lacks the cytoplasmic domain of TGFβR2. In some embodiments, the TGFβR2 corresponds to the wild-type human TGFβ Receptor Type 2 Isoform A sequence (e.g., the amino acid sequence of NCBI Reference Sequence (RefSeq) Accession No. NP_001020018 (SEQ ID NO: 9)), or the wild-type human TGFβ Receptor Type 2 Isoform B sequence (e.g., the amino acid sequence of NCBI RefSeq Accession No. NP_003233 (SEQ ID NO: 10)). Preferably, the TGFβR2 comprises or consists of a sequence corresponding to SEQ ID NO: 11 or a fragment thereof capable of binding TGFβ. For instance, the TGFβR2 may correspond to the full-length sequence of SEQ ID NO: 11. Alternatively, it may have an N-terminal deletion. For instance, amino acids 1-26 of the N-terminus of SEQ ID NO: 11 may be deleted, such as 14-21 or 14-26 of the most N-terminal amino acids. In some embodiments, the N-terminal 14, 19 or 21 amino acids of SEQ ID NO: 11 are deleted. Preferably, the TGFβR2 comprises or consists of a sequence selected from the group consisting of SEQ ID NO: 11, SEQ ID NO: 12 and SEQ ID NO: 13. Preferably, the TGFβR2 has at least 80% sequence identity, at least 90% sequence identity, or at least 95% sequence identity to the full-length amino acid sequence of any one of SEQ ID NO: 11, SEQ ID NO: 12 and SEQ ID NO: 13. In another preferred embodiment, the TGFβR2 has at least 80% sequence identity to the full-length amino acid sequence of SEQ ID NO: 11. In a preferred embodiment, the TGFβR2 has an amino acid sequence that does not differ in more than 25 amino acids from SEQ ID NO: 11.

Preferably, the TGFβR of the IgG:TGFβR fusion protein has greater than or equal to 92% sequence identity, greater than or equal to 95% sequence identity, greater than or equal to 99% sequence identity, or 100% sequence identity with the amino acid sequence of the TGFβR of bintrafusp alfa. Preferably, the TGFβR of the IgG:TGFβR fusion protein has an amino acid sequence with not more than 50, not more than 40, or not more than 25 amino acid residues different from the TGFβR of bintrafusp alfa. The TGFβR of the IgG:TGFβR fusion protein preferably has between 100-160 amino acid residues, more preferably 110-140 amino acid residues. In some embodiments, the amino acid sequence of the TGFβR is selected from the group consisting of a sequence corresponding to positions 1-136 of the TGFβR of bintrafusp alfa, a sequence corresponding to positions 20-136 of the TGFβR of bintrafusp alfa and a sequence corresponding to positions 22-136 of the TGFβR of bintrafusp alfa.

Preferably, the TGFβR of the IgG:TGFβR fusion protein has greater than or equal to 98% sequence identity with the amino acid sequence of the TGFβR of bintrafusp alfa, and the $C_H3$ domain of the IgG:TGFβR fusion protein has greater than or equal to 92% sequence identity with the amino acid sequence of the $C_H3$ domain of bintrafusp alfa. Preferably, the TGFβR of the IgG:TGFβR fusion protein has not more than 25 amino acid residues different from the amino acid sequence of the TGFβR of bintrafusp alfa, and the $C_H3$ domain of the IgG:TGFβR fusion protein has not more than 4 amino acid residues different from the amino acid sequence of the $C_H3$ domain of bintrafusp alfa.

Preferably, the IgG:TGFβR fusion protein comprises a linker between the IgG and TGFβR, which linker preferably comprises between 5 and 50 amino acid residues, between 10 and 30 amino acid residues, or between 20 and 27 amino acid residues. Preferably such a linker comprises at most two different types of amino acid residue. Preferably the linker comprises glycine amino acid residues and/or serine amino acid residues. Preferably such a linker is defined by the formula $(Gly_xSer)_yGly$, where x is an integer between 1 and 6, and y is an integer between 2 and 7. Preferably x is 4. Preferably y is 4 or 5. Most preferably, the linker is defined by the formula $(Gly_xSer)_yGly$, wherein x is 4 and y is either 4 or 5.

The IgG:TGFβR fusion protein is preferably an IgG:TGFβR2 fusion protein comprising a TGFβR2 fused at the N-terminus thereof to the C-terminus of an IgG antibody, optionally via a linker.

Preferably, the IgG:TGFβR fusion protein is one of the IgG:TGFβR fusion proteins disclosed in WO 2015/118175 or WO 2018/205985. For instance, the IgG:TGFβR fusion protein may comprise the light chains and heavy chains of SEQ ID NO: 1 and SEQ ID NO: 3 of WO 2015/118175, respectively. In another embodiment, the IgG:TGFβR fusion protein is one of the constructs listed in Table 2 of WO 2018/205985, such as construct 9 or 15 thereof.

Preferably, the light chain sequences and the heavy chain sequences of the IgG:TGFβR fusion protein respectively correspond to (1) SEQ ID NO: 7 and SEQ ID NO: 8, (2) SEQ ID NO: 15 and SEQ ID NO: 17, (3) SEQ ID NO: 15 and SEQ ID NO: 18, or (4) SEQ ID NO: 33 and SEQ ID NO: 34. Preferably, the amino acid sequence of the IgG:TGFβR fusion protein is identical to the amino acid sequence of bintrafusp alfa. Most preferably, the IgG:TGFβR fusion protein is bintrafusp alfa.

In a particular embodiment, the IgG:TGFβR fusion protein is characterised by:

- a TGFβR having greater than or equal to 95% sequence identity with the amino acid sequence of the TGFβR of bintrafusp alfa;

- a $C_H3$ domain having greater than or equal to 92% sequence identity with the amino acid sequence of the $C_H3$ domain of bintrafusp alfa;

- a $C_H1$ domain having greater than or equal to 90% sequence identity with the amino acid sequence of the $C_H1$ domain of bintrafusp alfa; and

- a $C_H2$ domain having greater than or equal to 90% sequence identity with the amino acid sequence of the $C_H2$ domain of bintrafusp alfa.

In a particular embodiment, the IgG:TGFβR fusion protein is characterised by:

- a TGFβR having not more than 25 amino acid residues different from the amino acid sequence of the TGFβR of bintrafusp alfa;

- a $C_H3$ domain having not more than 4 amino acid residues different from the amino acid sequence of the $C_H3$ domain of bintrafusp alfa;

- a $C_H1$ domain having not more than 7 amino acid residues different from the amino acid sequence of the $C_H1$ domain of bintrafusp alfa; and

- a $C_H2$ domain having not more than 8 amino acid residues different from the amino acid sequence of the $C_H2$ domain of bintrafusp alfa.

The pharmaceutical composition preferably comprises the IgG:TGFβR fusion protein (e.g. anti-PD-L1 (IgG):TGFβR2 or IgG:TGFβR having the sequence of bintrafusp alfa) in an amount of 1-200 mg/ml, 5-150 mg/mL, 7-70 mg/mL, 5-15 mg/mL, 15-65 mg/mL, 15-30 mg/mL, 35-65 mg/mL, 35-45 mg/mL, 45-55 mg/mL, 55-65 mg/mL, 40-120 mg/mL, 75-115 mg/mL or 95-105 mg/mL. In some embodiments, the pharmaceutical composition comprises about 10 mg/mL, about 20 mg/mL, about 25 mg/mL, about 40 mg/mL, about 50 mg/mL, about 60 mg/mL or about 100 mg/mL anti-PD-L1 (IgG):TGFβR2 fusion protein.

### *Diluent*

The pharmaceutical composition preferably comprises a diluent. The composition may include one or more pharmaceutically acceptable diluents, or mixtures thereof. However, most preferably the composition is an aqueous composition. Most preferably the diluent is water, and preferably water alone. The water is preferably water for injection (WFI).
Preferably the diluent may constitute the balance of ingredients in any composition, for instance so that the weight percentages of all ingredients total 100%. Preferably any concentrations given herein in relation to any component of the composition represent concentrations of said component in (and preferably dissolved in) the diluent in admixture with any other components.
The composition of the invention is preferably a solution and is preferably (substantially or entirely) free of particulates or precipitates.
In an embodiment, however, the pharmaceutical composition is either free of water or comprises at most 10 wt% water, preferably at most 5 wt% water, preferably at most 2 wt% water, preferably at most 1 wt% water. Such embodiments may be solid pharmaceutical compositions or lyophilised pharmaceutical compositions which may, for instance, be capable of reconstitution (preferably through the addition of water and/ or other relevant diluents, e.g. intravenous fluids or saline solution) prior to use, administration, or (preferably short-term) storage. Such lyophilised formulations may be reconstituted to afford aqueous pharmaceutical compositions disclosed herein (e.g. with ingredients present or absent in the concentrations specified herein).

### *Buffer System*

The pharmaceutical composition preferably comprises a buffer system. The buffer system preferably acts as a pH buffer creating inertia to pH changes when small amounts of (strong) acid or (strong) base are added to (or generated within, potentially as a result of degradation of one or more of the components of the pharmaceutical composition, most likely the fusion protein thereof) the pharmaceutical composition. As such, the buffer system preferably maintains a substantially constant composition pH over time, insodoing preferably mitigating against pH-triggered degradation pathways. Preferably the pharmaceutical composition is sufficiently buffered so as to resist pH changes of greater than or equal to 1 pH unit during 6 months storage at 2-8°C, preferably to resist pH changes of greater than or equal to 0.5 pH units, most preferably to resist pH changes of greater than or equal to 0.2 pH units under the same storage conditions.
Preferably, the composition is a buffered solution whose pH is stabilised by a buffering agent, which is a weak acid or a weak base, in combination with a conjugate acid or conjugate base of the buffering agent, depending on whether the buffering agent is itself a base or acid respectively. Collectively, the buffering agent and its acid/base conjugate may be considered a "buffer system", though in some embodiments where more than one buffer system is present the composition may comprise multiple different buffering agents and corresponding acid/base conjugates. The composition thus preferably comprises a "buffer system" (preferably comprising a buffering agent(s) and an acid/base conjugate(s) thereof), and any concentrations stipulated in relation to the buffer system generally relate to the combined concentrations of the buffering agent(s) and any acid/base conjugate(s) thereof.

The pharmaceutical composition preferably comprises a buffer system comprising one or more buffer systems. For instance, the buffer system could be a dual-buffer system (e.g. histidine-acetate, phosphate-citrate). The pharmaceutical composition most preferably comprises a buffer system comprising only one buffer system. However, the pharmaceutical composition may be characterised by an absence of a buffer system (or of any one or more of those specifically mentioned herein in relation to the buffer system). The pharmaceutical composition may, for example, be sufficiently stable without a buffer system where the fusion protein imparts sufficient self-buffering, which preferably occurs at higher concentrations of fusion protein.

In some embodiments, the buffer system is selected from a monoprotic buffer system (e.g. acetate buffer), a polyprotic buffer system (e.g. phosphate buffer), and an amphoteric buffer system (e.g. amino acid buffer, e.g. histidine), an inorganic buffer system (e.g. ammonium buffer, a bicarbonate buffer, a carbonate buffer, a borate buffer, a phosphate buffer), an organic buffer system (e.g. a carboxylate buffer, an organic ammonium buffer, an alkanolammonium buffer, a zwitterionic buffer, an amino acid buffer, an aromatic nitrogen buffer, a sugar buffer), and any combination thereof.

In other embodiments, the buffer system is selected from a monocarboxylate buffer system (e.g. an acetate buffer, a formate buffer, a lactate buffer, a salicylate buffer, a benzoate buffer), a dicarboxylate buffer system (e.g. a succinate buffer, a maleate buffer, a malate buffer, a fumarate buffer, a tartrate buffer, an adipate buffer, a hexanedioate buffer), a tricarboxylate buffer system (e.g. citrate buffer), a zwitterionic buffer system (e.g. amino acid buffers, zwitterionic sulphonate buffers, e.g. a N-(2-Acetamido)-2-aminoethanesulfonic acid (ACES) buffer, a 2-Aminoethanesulfonic acid (AES) buffer, a N-(1,1-Dimethyl-2-hydroxyethyl)-3-amino-2-hydroxypropanesulfonic acid (AMPSO) buffer, a N,N-Bis-(2-hydroxyethyl)-2-aminoethanesulfonic acid (BES) buffer, a 3-(Cyclohexylamino)-propanesulfonic acid (CAPS)), an amino acid buffer system (e.g. a histidine buffer, a glycine buffer, a lysine buffer, a glycylglycine buffer, a N-[Tris(hydroxymethyl)-methyl]-glycine (tricine) buffer, a glutamate buffer, an asparate buffer, a N,N′-Bis(2-hydroxyethyl)-glycine (Bicine) buffer, a N-(2-Acetamido)-iminodiacetic acid (ADA) buffer), an aromatic nitrogen buffer system (e.g. imidazole, pyridine buffers), and an alkanolammonium buffer system (e.g. an aminomethylpropanol (AMP) buffer, an aminomethyl propanediol (AMPD) buffer, a tris(hydroxymethyl)aminomethane (Tris) buffer, a [Bis-(2-hydroxyethyl)-imino]-tris-(hydroxymethylmethane) (BIS-Tris) buffer, a 1,3-Bis[tris(hydroxymethyl)-methylamino]propane (Bis-tris propane) buffer).

Suitably, the buffer system is a buffer system selected from the group consisting of a histidine buffer, a phosphate buffer, a succinate buffer, a citrate buffer, an acetate buffer, gluconate buffer, a Tris buffer, an aspartate buffer, a glutamate buffer, a tartrate buffer, a malate buffer, a maleate buffer, a fumarate buffer, a histidine-acetate buffer, a phosphate-citrate buffer, and any combination thereof. Preferably, the buffer system is a buffer system selected from the group consisting of a histidine buffer, a phosphate buffer, a succinate buffer, a citrate buffer, and any combination thereof. More preferably, the buffer system is a buffer system comprising a single buffer selected from the group consisting of a histidine buffer, a phosphate buffer, a succinate buffer, and a citrate buffer.

The pharmaceutical composition may comprise 1-100 mM buffer system, 2-70 mM buffer system, 3-50 mM buffer system, 4-30 mM buffer system, 5-20 mM buffer system, 6-14 mM buffer system, or, preferably, about 10 mM buffer system.

The pharmaceutical composition may comprise a buffer system in a molar ratio of buffer system to fusion protein of from 1280:1 to 3:1, of from 370:1 to 9:1, of from 260:1 to 11:1, of from 100:1 to 15:1, or, preferably, of from 70:1 to 20:1.

Most preferably, the buffer system is or comprises a histidine buffer system. For instance, the buffer system is or comprises 5-60 mM histidine buffer system, 5-15 mM histidine buffer system, or, preferably, about 10 mM histidine buffer system. In other embodiments, the composition comprises a histidine buffer system in a molar ratio of histidine buffer system to fusion protein of from 1280:1 to 3:1 or of from 370:1 to 9:1.

Preferably, the buffer system is or comprises a phosphate buffer system. For instance, the buffer system is or comprises 5-60 mM phosphate buffer system or 5-15 mM phosphate buffer system. In other embodiments, the composition comprises a phosphate buffer system in a molar ratio of phosphate buffer system to fusion protein of from 1280:1 to 3:1 or of from 370:1 to 9:1.

Preferably, the buffer system is or comprises a succinate buffer system. For instance, the buffer system is or comprises 5-60 mM succinate buffer system or 5-15 mM succinate buffer system. In other embodiments, the composition comprises a succinate buffer system in a molar ratio of succinate buffer system to fusion protein of from 1280:1 to 3:1 or of from 370:1 to 9:1.

Preferably, the buffer system is or comprises a citrate buffer system. For instance, the buffer system is or comprises 5-60 mM citrate buffer system or 5-15 mM citrate buffer system. In other embodiments, the composition comprises a citrate buffer system in a molar ratio of citrate buffer system to fusion protein of from 1280:1 to 3:1 or of from 370:1 to 9:1.

Preferably, the buffer system does not comprise an acetate buffer.

Amounts and concentrations in relation to the buffer system relate to total amounts and concentrations of all buffer systems, unless only a single buffer system is stipulated. Concentrations of the or any particular buffer system can be replaced by molar ratios.

## *pH*

The pharmaceutical composition preferably has a pH of 4-9. Though the fusion protein, and any excipients, within the pharmaceutical composition may tolerate and remain reasonably stable over a range of pH values, certain pHs are particularly advantageous, especially in the presence of particular excipients or combinations thereof.

In some embodiments, the pharmaceutical composition has a pH of 4-8, a pH of 4.8-7.8, a pH of 5-7, a pH of 4.9-6.8, a pH of 4.5-6.0, a pH of 4.8-6.5, a pH of 5-5.4, a pH of 5.2-6.2, a pH of 5.3-6.3, a pH of 5.2-5.8, a pH of 5.6-5.8, a pH of 5.9-6.1, a pH of 5.2-6.2, a pH of 5.4-6.0, or, preferably, a pH of 5.4-5.6 or a pH of 5.8-6.0. In some embodiments, the pH of the pharmaceutical composition is about 5.5 or about 5.9.

One of the challenges encountered during the formulation of IgG:TGFβR fusion protein compositions was the significant difference between the pI of the IgG moiety (with pIs typically about 8-10, 8.5-9.5, or about 9.1) and the pI of the TGFβR moiety (with pIs typically about 4.5-6, 4.6-5.4, or about 4.9). It is generally desirable to formulate biologics at a pH greater than 1-2 units away from the pI of the biologic or pI of relevant parts of the biologic. Optimising pH for bi-functional biologics whose functional parts exhibit dramatically different pIs is particularly difficult. In the case of IgG:TGFβR fusion proteins, the TGFβR moiety is highly influential in terms of formulation considerations and potentially less stable than the IgG moiety. In particular, the IgG moiety of bintrafusp alfa was found to be rather tolerant to stress conditions and long-term stable even in a liquid/non-lyophilised form. In contrast to this, when the IgG moiety of bintrafusp alfa was fused to the TGFβR moiety, it was found that the molecule precipitates more easily and particle formation, as well as phase separation was observed in the formulation, which suggests that the TGFβR moiety is less stable than the IgG moiety or at least acts destabilizing in conjunction with the IgG moiety. This reinforces that the pH of the composition of an IgG:TGFβR fusion protein should have a pH that is, in particular, remote from the pI of the TGFβR moiety, e.g. have a pH outside 3.9-5.9. Surprisingly, however, it was found that a pharmaceutical composition having a pH close to the pI of the TGFβR moiety of the IgG:TGFβR fusion protein is highly stable.

In light of this, the pharmaceutical composition preferably has a pH within 2 pH units, more preferably within 1 pH unit of the pI of the TGFβR moiety of the fusion protein (i.e. where the difference between the pH of the composition and pI of the TGFβR moiety is less than 2 units or 1 unit, respectively). The pharmaceutical composition preferably has a pH that differs from the pI of the IgG moiety of the fusion protein by greater than or equal to 2 pH units or, more preferably, greater than or equal to 3 pH units. For instance, the composition may have a pH of 5.4-6.0, where the pI of the TGFβR moiety is 4.4-5.0 and the pI of the IgG moiety is 8.4-9.5.

Though the present invention encompasses using any buffer system as defined herein, or none, so long as the pH is as defined herein, a histidine buffer system is the most preferred buffer system, most preferably at a pH of 5.2-6.2.

## *Surfactant*

The pharmaceutical composition preferably comprises a surfactant. The pharmaceutical composition preferably comprises at most one surfactant. In the context of the present invention, a surfactant may inhibit one or more degradation pathways of the fusion protein or its constituent parts, for instance, unfolding (and consequential aggregation), aggregation, and sometimes even fragmentation. The surfactant may facilitate dissolution of the fusion protein. However, the pharmaceutical composition may be characterised by an absence of a surfactant (or of any one or more of those specifically mentioned herein in relation to the surfactant).

Preferably, the surfactant is a non-ionic surfactant, for instance, selected from the group consisting of: a fatty alcohol, a fatty alcohol ether, a fatty acid ester, a fatty acid amide, a polyoxyalkylene alkyl ether, a polyoxyethylene alkyl ether, a non-ionic block copolymer, alpha-tocopherol, and any combination thereof or selected from the group consisting of a sorbitan ester (Span), an ethoxylated sorbitan ester (polysorbate), and a block alkoxylate.

In some embodiments, the surfactant is an ethoxylated fatty acid ester surfactant. In other embodiments, the surfactant is a surfactant selected from the group consisting of polysorbate(s), poloxamer(s), and kolliphor(s). Preferably, it is a kolliphor surfactant or a polysorbate surfactant. Preferably, the kolliphor is kolliphor 188, whereas the polysorbate is a polysorbate 20 or polysorbate 80 surfactant. Most preferably, the surfactant is polysorbate 20.

The pharmaceutical composition preferably comprises 0.01-2 mg/mL surfactant, 0.05-1.5 mg/mL surfactant, 0.1-0.6 mg/mL surfactant, 0.25-0.75 mg/mL surfactant, 0.4-0.6 mg/mL surfactant, 0.4-1.2 mg/mL surfactant or 0.8-1.1 mg/mL surfactant.

The pharmaceutical composition preferably comprises a surfactant in a molar ratio of surfactant to fusion protein of from 30:1 to 1:70, of from 12:1 to 1:3, of from 17:1 to 1:1, of from 5:1 to 1:2, or of from 7:1 to 1:1.

The pharmaceutical composition preferably comprises 0.01-2 mg/mL polysorbate 20, 0.05-1.5 mg/mL polysorbate 20, 0.05-0.3 mg/mL polysorbate 20, 0.05-0.15 mg/mL polysorbate 20, 0.1-0.7 mg/mL polysorbate 20, 0.3-0.7 mg/mL polysorbate 20, 0.4-0.6 mg/mL polysorbate 20, 0.4-1.3 mg/mL polysorbate 20, 0.8-1.2 mg/mL polysorbate 20, 0.9-1.1 mg/mL polysorbate 20, or, most preferably about 0.5 mg/mL polysorbate 20.

The pharmaceutical composition preferably comprises polysorbate 20 in a molar ratio of polysorbate 20 to fusion protein of from 30:1 to 1:70, of from 12:1 to 1:3, of from 17:1 to 1:1, of from 5:1 to 1:2, or of from 7:1 to 1:1.

Amounts and concentrations in relation to the surfactant relate to total amounts and concentrations of all surfactants, unless only a single surfactant is stipulated. Concentrations of the or any particular surfactant can be replaced by molar ratios.

### *Sugar Component*

The pharmaceutical composition preferably comprises a sugar component, such as a lyoprotectant sugar component. In the context of the invention, a sugar component can serve one or more functions within the pharmaceutical composition. For instance, they may serve as a lyoprotectant during freeze drying. A sugar component may impart tonicity, for instance, to bring the osmolality within a desired range (e.g. for isotonicity of a formulation for injection without prior dilution - e.g. an osmolality of 200-400 mOsmol/L, more preferably 250-350 mOsmol/L, most preferably 270-310 mOsmol/L). Using sugar components to contribute towards tonicity can be particularly useful where high ionic strengths are less desirable, though in general compositions of the invention tend to favour higher ionic strengths for higher concentrations of fusion protein. A sugar component may facilitate solubility of the fusion protein and/or other excipients within the pharmaceutical composition. A sugar component may impart a stabilising effect towards the fusion protein of the pharmaceutical composition, for example, improving conformational stability (e.g. reducing protein unfolding events that can increase the likelihood of ultimate aggregation), reducing aggregation, and/or reducing fragmentation. However, in some embodiments, the pharmaceutical composition is characterised by an absence of a sugar component (or of any one or more of those specifically mentioned herein in relation to the sugar component).

In some embodiments, the sugar component is a non-reducing sugar component. The sugar component may also comprise one or more sugar(s) and/or sugar alcohol(s). In some embodiments, the sugar component comprises one or more sugar(s) or one or more sugar alcohol(s). Preferably, the sugar component consists of a single compound. In other embodiments, the sugar component comprises at most one sugar or at most one sugar alcohol.

Preferably, the sugar component is selected from the group consisting of trehalose, sucrose, mannitol, and sorbitol, more preferably from trehalose or sucrose.

In some embodiments, the sugar component is a sugar, which may be selected from the group consisting of a monosaccharide, a disaccharide, a polysaccharide, and a complex carbohydrate. Preferably, the sugar component is a disaccharide, such as, a non-reducing disaccharide. More preferably, the sugar component is a disaccharide selected from trehalose or sucrose. Preferably, the sugar component is sucrose. Preferably, the sugar component is trehalose.

In some embodiments, the sugar component is a sugar alcohol, such as a (3-12C) sugar alcohol, a (3-6C) sugar alcohol, or a (5-6C) sugar alcohol selected from mannitol, sorbitol, arabitol, xylitol, ribitol, and inositol. In some embodiments, the sugar component is a sugar alcohol selected from mannitol or sorbitol.

The pharmaceutical composition preferably comprises 30-400 mM sugar component, 40-300 mM sugar component, 40-100 mM sugar component, 40-60 mM sugar component, 90-290 mM sugar component, 100-200 mM sugar component, 130-170 mM sugar component, 200-300 mM sugar component, 230-270 mM sugar component, about 159 mM sugar component, about 100 mM sugar component or about 50 mM sugar component.

The pharmaceutical composition preferably comprises a sugar component in a molar ratio of sugar component to fusion protein of from 7300:1 to 50:1, of from 3700:1 to 70:1, of from 1000:1 to 100:1, of from 1000:1 to 500:1, of from 500:1 to 180:1, of from 2900:1 to 2700:1, of from 800:1 to 600:1, of from 500:1 to 300:1, or of from 300:1 to 100:1.

The pharmaceutical composition preferably comprises 40-300 mM trehalose, 40-100 mM trehalose, 40-60 mM trehalose, 65-85 mM trehalose, 70-130 mM trehalose, 90-110 mM trehalose, 100-200 mM trehalose, 140-180 mM trehalose, or 150-170 mM trehalose. In some embodiments, the pharmaceutical composition comprises trehalose at a concentration of about 50 mM, about 75 mM, about 100 mM or about 159 mM. The pharmaceutical composition preferably comprises trehalose in a molar ratio of trehalose to fusion protein of from 7300:1 to 50:1, of from 3700:1 to 70:1, of from 1000:1 to 100:1, of from 1000:1 to 500:1, or of from 500:1 to 180:1. Most preferably the pharmaceutical composition comprises trehalose as the sole sugar component, most preferably at a concentration of 40-200 mM.

The pharmaceutical composition preferably comprises 50-300 mM sucrose, 150-290 mM sucrose, 220-280 mM sucrose, or 240-260 mM sucrose.

The pharmaceutical composition preferably comprises 40-300 mM mannitol, 40-100 mM mannitol, 40-60 mM mannitol, 80-120 mM mannitol, 100-200 mM mannitol, 210-290 mM mannitol, or 240-260 mM mannitol.

The pharmaceutical composition preferably comprises 40-300 mM sorbitol, 40-100 mM sorbitol, 40-60 mM sorbitol, 80-120 mM sorbitol, 100-200 mM sorbitol, 210-290 mM sorbitol, or 240-260 mM sorbitol.

Amounts and concentrations in relation to the sugar component relate to total amounts and concentrations of all sugar components, unless only a single sugar component is stipulated. Concentrations of the or any particular sugar component can be replaced by molar ratios.

### *Amino Acid Component*

The pharmaceutical composition preferably comprises an amino acid component. Amino acids may be used to adjust the osmolality of the pharmaceutical composition, for instance, to bring the osmolality within a preferred range (e.g. for isotonicity of a formulation for injection without prior dilution- e.g. an osmolality of 200-400 mOsmol/L, more preferably 250-350 mOsmol/L, most preferably 270-310 mOsmol/L). Amino acids may also facilitate solubility of the fusion protein and/or other excipients within the pharmaceutical composition. Amino acids may also impart a stabilising effect towards the fusion protein of the pharmaceutical composition, for example, improving conformational stability (e.g. reducing protein unfolding events that can increase the likelihood of ultimate aggregation), reducing aggregation, and/or reducing fragmentation. One or more amino acids (e.g. arginine), or any salts thereof (e.g. arginine hydrochloride), may replace some or all of a tonicifier (e.g. NaCl) and/or some or all of a sugar component (e.g. trehalose). Preferably, the pharmaceutical composition comprises an amino acid component in addition to histidine where histidine is used as a or part of a buffer system and/or methionine where methionine is used as a or part of an antioxidant component.

However, the pharmaceutical composition may be characterised by an absence of an amino acid component (or of any one or more of those specifically mentioned herein in relation to the amino acid component), preferably with the optional exception of histidine where histidine is used as a or part of a buffer system or with the optional exception of methionine where methionine is used as a or part of an antioxidant component, preferably with the optional exception of histidine and methionine where these are used as a or part of a buffer system and as a or part of an antioxidant component, respectively.

Preferably, the amino acid component comprises one or more amino acids, in particular one or more amino acids other than histidine where histidine is included, e.g., as a or part of a buffer system and/or one or more amino acids other than methionine where methionine is included, e.g., as a or part of an antioxidant component. Preferably, the amino acid component comprises at most one amino acid or at most one amino acid other than histidine where histidine is included, e.g., as a or part of a buffer system and/or other than methionine where methionine is included, e.g., as a or part of an antioxidant component. Preferably, the amino acid component comprises a single amino acid or a single amino acid other than histidine where histidine is included, e.g., as a or part of a buffer system and/or other than methionine where methionine is included, e.g., as a or part of an antioxidant component. Preferably, the amino acid component comprises an L-amino acid. Preferably the amino acid(s) constituting the amino acid component is/are L-amino acid(s).

The amino acid component, or any amino acids therein, may be pharmaceutically acceptable salts thereof. For instance, in the case of arginine, the arginine may in fact be provided in the form of arginine monohydrochloride.

Preferably, the amino acid component comprises an amino acid selected from the group consisting of arginine, lysine, proline, gluatamic acid, glycine, and any combination thereof. Preferably, the amino acid component comprises an amino acid selected from the group consisting of arginine, lysine, proline, gluatamic acid, and any combination thereof.

Preferably, the amino acid component comprises arginine.

Preferably, the amino acid component comprises lysine.

Preferably, the amino acid component comprises proline.

Preferably, the amino acid component comprises glutamic acid.

The amino acid is preferably a charged amino acid (i.e. where the amino acid bears a net positive or negative charge). Preferably the amino acid component comprises an amino acid salt (e.g. arginine hydrochloride).

The pharmaceutical composition preferably comprises 10-300 mM amino acid component, 20-260 mM amino acid component, 30-110 mM amino acid component, 30-60 mM amino acid component, 35-95 mM amino acid component, 40-170 mM amino acid component, 50-200 mM amino acid component, 60-140 mM amino acid component, 110-190 mM amino acid component, 140-180 mM amino acid component, or about 50 mM amino acid component.

The pharmaceutical composition preferably comprises an amino acid component in a molar ratio of amino acid component to fusion protein of from 5500:1 to 18:1, of from 2000:1 to 54:1, of from 3500:1 to 200:1, of from 500:1 to 100:1, or of from 900:1 to 400:1.

The pharmaceutical composition preferably comprises 20-300 mM arginine, 30-60 mM arginine, 30-50 mM arginine, 40-60 mM arginine, 60-80 mM arginine, 35-95 mM arginine, 80-120 mM arginine, 100-140 mM arginine, 120-180 mM arginine, 150-170 mM arginine, 200-300 mM arginine, or, preferably about 50 mM arginine or about 75 mM arginine. The pharmaceutical composition preferably comprises arginine in a molar ratio of arginine to fusion protein of from 5500:1 to 18:1, of from 2000:1 to 54:1, of from 3500:1 to 200:1, of from 500:1 to 100:1, or of from 900:1 to 400:1.

The pharmaceutical composition preferably comprises 20-200 mM lysine, 30-150 mM lysine, 30-80 mM lysine, 40-60 mM lysine, 60-90 mM lysine, 70-130 mM lysine, or 90-110 mM lysine.

The pharmaceutical composition preferably comprises 20-200 mM proline, 30-150 mM proline, 30-80 mM proline, 40-60 mM proline, 60-90 mM proline, 70-130 mM proline, or 90-110 mM proline.

The pharmaceutical composition preferably comprises 20-200 mM glutamic acid, 30-150 mM glutamic acid, 30-80 mM glutamic acid, 40-60 mM glutamic acid, 60-90 mM glutamic acid, 70-130 mM glutamic acid, or 90-110 mM glutamic acid.

Amounts and concentrations in relation to the amino acid component relate to total amounts and concentrations of all amino acid components, unless only a single amino acid component is stipulated. Concentrations of the or any particular amino acid component can be replaced by molar ratios.

### Ionic Strength

The pharmaceutical composition of the invention preferably has a non-zero ionic strength. It is thought that ionic strength facilitates solubility of the fusion protein, particularly the TGFβR moiety thereof. A degree of ionic strength may reduce fragmentation. A degree of ionic strength may reduce aggregation.

Ionic strength may be preferably defined as "molar ionic strength ($I$)", which is a function of the concentration of ALL ions present within a given composition (which is preferably a solution, preferably an aqueous solution). Molar ionic strength ($I$) may be defined by the following equation:

$$I = \frac{1}{2} \sum_{i=1}^{n} c_i z_i^2$$

where $c_i$ is the molar concentration of ion $i$ (mol/L) and $z_i$ is the charge number of ion $i$. The ½ multiplier accounts for the fact that the summation is taken over ALL ions, thus include both polarities, anion(s) and cation(s).

By way of example, if NaCl is the sole electrolyte contributing towards the ionic strength of the composition, the ionic strength is equal to the concentration of the NaCl (i.e. 100 mM NaCl = 100 mM ionic strength), because each of the cation and anion of NaCl is singly charged - so for a 60 mM NaCl solution the ionic strength is as follows:

$$I = \frac{1}{2} \sum (60 \times 1^2) + (60 \times -1^2) = 60$$

If a salt such as $MgSO_4$ is the sole electrolyte contributing towards the ionic strength of the composition, the ionic strength is four times that of the equivalent molar concentration of sodium chloride because each of the cation and anion is doubly-charge - so for a 60 mM $MgSO_4$ solution the ionic strength is as follows:

$$I = \frac{1}{2} \sum (60 \times 2^2) + (60 \times -2^2) = 240$$

As a consequence, multivalent ions contribute relatively more ionic strength than monovalent ions.

Amino acid salts (e.g. arginine monohydrochloride) may also contribute to ionic strength. Buffers (or the ionic buffering species thereof - e.g. conjugate acid and/or conjugate base) may also contribute to ionic strength.

The ionic strength of the composition is suitably equal to the the sum of ionic contributions (defined by the aforesaid equation) from the tonicifier and, where present, the buffer system. The ionic strength of the composition may be equal to any of the concentrations defined herein in relation to a tonicifier plus the molar concentration of any buffer system.

In some embodiments, compositions of the invention may be characterised by a non-buffering (or tonicifer-based) ionic strength. In such cases, the non-buffering (or tonicifer-based) ionic strength of the composition is preferably equal to any of the concentration defined herein in relation to a tonicifier. Also, in such cases, the ratio of non-buffering (or tonicifer-based) ionic strength to the molar concentration of the fusion protein is preferably the same as any of the molar ratios of tonicifier to fusion protein as defined herein.

The pharmaceutical composition preferably has an ionic strength of 5-250 mM, 10-200 mM, 20-170 mM, 20-80 mM, 20-60 mM, 30-50 mM, 30-130 mM, 80-150 mM, 90-100 mM, 100-170 mM, 110-130 mM, 150-170 mM, 130-170 mM, about 40 mM, about 60 mM, or about 100 mM.

The pharmaceutical composition preferably has an ionic strength in a molar ratio of ionic strength to fusion protein of from 3700:1 to 15:1, of from 1000:1 to 70:1, of from 910:1 to 200:1, of from 910:1 to 540:1, of from 320:1 to 220:1, of from 410:1 to 320:1, of from 520:1 to 390:1, of from 700:1 to 450:1, of from 680:1 to 720:1, of from 460:1 to 420:1, or of from 290:1 to 250:1.

The ionic strength is preferably provided, at least in part or in full, by sodium chloride. The ionic strength is preferably provided, at least in part or in full, by sodium chloride and arginine (or a salt thereof). The ionic strength is preferably provided, at least in part or in full, by sodium chloride and/or an amino acid salt (e.g. arginine or arginine salt).

### *Tonicifier*

The pharmaceutical composition preferably comprises a tonicifier, such as a non-buffering tonicifier (i.e. a tonicifier that does not impart a pH buffering effect, unlike buffering salts which may themselves to an extent contribute to overall tonicity). In the context of the present invention, tonicifiers may be used to adjust the osmolality of the pharmaceutical composition, for instance, to bring the osmolality within a preferred range (e.g. for isotonicity of a formulation for injection without prior dilution - e.g. an osmolality of 200-400 mOsmol/L, more preferably 250-350 mOsmol/L, most preferably 270-310 mOsmol/L). Similarly, tonicifiers, especially ionic tonicifiers (e.g. salt tonicifiers) may be used to impart ionic strength. Tonicifiers may also facilitate solubility of the fusion protein and/or other excipients within the pharmaceutical composition. Tonicifiers may also impart a stabilising effect towards the fusion protein of the pharmaceutical composition, for example, improving conformational stability (e.g. reducing protein unfolding events that can increase the likelihood of ultimate aggregation), reducing aggregation, and/or reducing fragmentation.

However, in some embodiments, the pharmaceutical composition is characterised by an absence of a (non-buffering) tonicifier (or of any one or more of those specifically mentioned herein in relation to the tonicifier). Alternatively, some of the tonicifier, especially a non-buffering salt tonicifier (e.g. NaCl), may be substituted by some amino acid component and/or some sugar component, especially where said components impart their own tonicifying effect.

The tonicifier may be or comprise a tonicifying lyoprotectant. A tonicifying lyoprotectant is a compound or compound(s) that imparts tonicify to a composition whilst also acting as a lyoprotectant, for instance, during freeze drying (in particular the drying phase thereof). A lyoprotectant effect is well known in the art and, for example, may include compound(s) that stabilise an active ingredient during the drying phase of freeze-drying and/or stabilise lyophilised cake (e.g. preventing collapse thereof upon drying). A tonicifying lyoprotectant may comprise or consist of a sugar or sugar polyol, such as a sugar or sugar polyol defined herein in relation to a "sugar component". A tonicifying lyoprotectant may comprise or consist of an amino acid, such as an "amino acid component" as defined herein. Trehalose is an example of a tonicifying lyoprotectant. Arginine may also act as a lyoprotectant. It may be advantageous for compositions to contain a combination of both trehalose and arginine.

Preferably, the tonicifier comprises a tonicifier selected from the group consisting of a salt tonicifier, a metal salt tonicifier, a non-buffering salt tonicifier, a metal halide salt tonicifier, an alkali metal or alkaline earth metal halide salt tonicifier, an alkali metal halide salt tonicifier, and an alkali metal halide salt tonicifier selected from sodium chloride or potassium chloride, preferably, sodium chloride, and any combination thereof.

Preferably, the tonicifier is an ionic tonicifier and, as such, contributes directly to the ionic strength of the composition.

The pharmaceutical composition preferably comprises 5-250 mM tonicifier, 10-200 mM tonicifier, 20-170 mM tonicifier, 20-80 mM tonicifier, 20-60 mM tonicifier, 50-70 mM tonicifier, 30-50 mM tonicifier, 30-130 mM tonicifier, 80-150 mM tonicifier, 90-110 mM tonicifier, 90-100 mM tonicifier, 100-170 mM tonicifier, 110-130 mM tonicifier, 150-170 mM tonicifier, 130-170 mM tonicifier, about 40 mM tonicifier, about 60 mM tonicifer, or about 100 mM tonicifier.

The pharmaceutical composition preferably comprises a tonicifier in a molar ratio of tonicifier to fusion protein of from 3700:1 to 15:1, of from 1000:1 to 70:1, of from 910:1 to 200:1, of from 910:1 to 540:1, of from 320:1 to 220:1, of from 410:1 to 320:1, of from 520:1 to 390:1, of from 700:1 to 450:1, of from 680:1 to 720:1, of from 460:1 to 420:1, or of from 290:1 to 250:1.

The pharmaceutical composition preferably comprises 5-250 mM sodium chloride, 10-200 mM sodium chloride, 20-170 mM sodium chloride, 30-90 mM sodium chloride, 20-60 mM sodium chloride, 30-50 mM sodium chloride, 50-70 mM sodium chloride, 35-45 mM sodium chloride, 30-130 mM sodium chloride, 80-150 mM sodium chloride, 90-110 mM sodium chloride, 100-200 mM sodium chloride, 110-130 mM sodium chloride, 150-170 mM sodium chloride, 130-170 mM sodium chloride, about 40 mM sodium chloride, about 60 mM sodium chloride, or about 100 mM sodium chloride.

The pharmaceutical composition preferably comprises sodium chloride in a molar ratio of sodium chloride to fusion protein of from 3700:1 to 15:1, of from 1000:1 to 70:1, of from 910:1 to 200:1, of from 910:1 to 540:1, of from 600:1 to 250:1, of from 320:1 to 220:1, of from 410:1 to 320:1, of from 520:1 to 390:1, of from 700:1 to 450:1, of from 680:1 to 720:1, of from 460:1 to 420:1, or of from 290:1 to 250:1.

Amounts and concentrations in relation to the tonicifier relate to total amounts and concentrations of all tonicifiers, unless only a single tonicifier is stipulated. Concentrations of the or any particular tonicifier can be replaced by molar ratios.

In general, in the context of formulations of the invention, save for the buffer system the only viable components for imparting ionic strength is the tonicifier(s), such as sodium chloride and/or a charged amino acid (e.g. arginine, or arginine hydrochloride, or other amino acid salt). NaCl as the tonicifier was deemed a particularly advantageous component, since it generally facilitates solubility of the fusion protein in aqueous media, though it may also participate in charge masking, especially of certain groups of the receptor portion of the fusion protein. Experiments often demonstrated that as the concentration of the fusion protein is increased formulations generally benefit from higher concentrations of NaCl. For example, 5-15 mg/mL fusion protein may benefit from 30-50 mM NaCl; 35-55 mg/mL fusion protein may benefit from 50-70 mM NaCl; and 50-70 mg/mL fusion protein may benefit from 130-170 mM NaCl.

### Antioxidant

The pharmaceutical composition preferably comprises an antioxidant. An antioxidant may inhibit oxidative degradation pathways open to the fusion protein and/or other components within the pharmaceutical composition. For instance, an antioxidant may inhibit oxidation of the fusion protein, for example by inhibiting oxidation of certain oxidizable amino acid residues within the fusion protein, and/or inhibit oxidative deamination pathways.

However, in some embodiments, the pharmaceutical composition is characterised by an absence of an antioxidant (or of any one or more of those specifically mentioned herein in relation to the antioxidant).

Preferably, the antioxidant comprises an antioxidant selected from the group consisting of an amino acid or peptide antioxidant, a mineral antioxidant, a vitamin antioxidant, a carotenoid antioxidant, a polyphenol antioxidant, an aromatic or phenolic antioxidant, a chelating agent antioxidant, a thiol antioxidant, and any combination thereof.

Preferably, the antioxidant comprises an antioxidant consisting of a single compound.

Preferably, the antioxidant comprises an amino acid antioxidant, for instance, an amino acid antioxidant selected from the group consisting of: methionine, N-acetyl-l-cysteine, cysteine, and glutathione.

Most preferably, the antioxidant is methionine.

The pharmaceutical composition preferably comprises 0.1-50 mM antioxidant, 1-30 mM antioxidant, 2-20 mM antioxidant, 3-10 mM antioxidant, or 4-6 mM antioxidant.

The pharmaceutical composition preferably comprises an antioxidant in a molar ratio of antioxidant to fusion protein of from 910:1 to 1:6, of from 365:1 to 3:1, of from 182:1 to 5:1, of from 100:1 to 7:1, or of from 50:1 to 10:1.

The pharmaceutical composition preferably comprises 1-30 mM methionine, 2-20 mM methionine, 3-12 mM methionine, 4-6 mM methionine, or about 5 mM methionine.

The pharmaceutical composition preferably comprises methionine in a molar ratio of methionine to fusion protein of from 910:1 to 1:6, of from 365:1 to 3:1, of from 182:1 to 5:1, of from 100:1 to 7:1, or of from 50:1 to 10:1.

Amounts and concentrations in relation to the antioxidant relate to total amounts and concentrations of all antioxidants, unless only a single antioxidant is stipulated. Concentrations of the or any particular antioxidant can be replaced by molar ratios.

### Chelator

The pharmaceutical composition may also comprise a chelator. In the context of the invention, a chelator such as EDTA (other chelators, are well known in the art and may serve a similar or identical function) may increase stability of the pharmaceutical composition, in particular inhibiting degradation of the fusion protein. For instance, chelators can reduce the likelihood of aggregation, and can also act as an antioxidant. Chelators can also sequester residual metals that may otherwise promote degradation of the fusion protein and/or of other components present within the pharmaceutical composition.

However, in some embodiments, the pharmaceutical composition is characterised by an absence of a chelator (or of any one or more of those specifically mentioned herein in relation to the chelator).

Preferably, the chelator comprises EDTA or EGTA, more preferably, the chelator comprises EDTA.

The pharmaceutical composition preferably comprises 0.001-0.5 mM chelator, 0.01-0.2 mM chelator, or 0.025-0.075 mM chelator. The pharmaceutical composition preferably comprises a chelator in a molar ratio of chelator to fusion protein of from 10:1 to 1:550, of from 4:1 to 1:55, or of from 1.5:1 to 1:22.

The pharmaceutical composition preferably comprises 0.001-0.5 mM EDTA, 0.01-0.2 mM EDTA, or 0.025-0.075 mM EDTA. The pharmaceutical composition preferably comprises EDTA in a molar ratio of EDTA to fusion protein of from 10:1 to 1:550, of from 4:1 to 1:55, or of from 1.5:1 to 1:22.

Amounts and concentrations in relation to the chelator relate to total amounts and concentrations of all chelators, unless only a single chelator is stipulated. Concentrations of the or any particular chelator can be replaced by molar ratios.

*Osmolality*

Generally, the osmolality of the pharmaceutical composition is less critical and is determined by the amounts of osmolytes required to stabilize the formulation. In case that the pharmaceutical formulation is intendend for injection without prior dilution, e.g., a subcutaneous injection, the formulation is preferably substantially isotonic to avoid skin irritation. In some embodiments, to afford compositions with the desired osmolality the concentration of the fusion protein may be carefully balanced with concentrations of tonicifiers (e.g. NaCl), amino acid components (e.g. arginine/arginine hydrochloride), sugar components (e.g. trehalose). In some embodiments, the pharmaceutical formulation preferably has an osmolality of 240-640 mOsm/kg, 200-400 mOsm/kg, 250-420 mOsm/kg, 250-350 mOsm/kg, 255-345 mOsm/kg, or 270-310 mOsm/kg.

*Specific Embodiments*

The examples and data presented herein suggest that certain technical effects may arise from certain features or combinations of features, in light of which various specific embodiments are discussed below.

*pH*

Experimental results have shown that a liquid composition having a pH within 2 pH units, suitably within 1.5 pH units, and suitably even within 1 pH unit of the pI of the TGFβR2 moiety of the fusion protein is surprisingly stable. Preferably, the pH is greater than or equal to pH 4, and is more preferably greater than or equal to pH 5. However, high pHs can lead to protein stability and protein solubility issues and, as such, the liquid composition preferably has a pH less than or equal to pH 7, and more preferably less than or equal to pH 6.3. A particularly preferred pH range is pH 5.3-6.3.
The following numbered paragraphs E1 to E12 disclose specific embodiments of the invention.

E1. A pharmaceutical composition comprising an IgG:TGFβR fusion protein and characterised by a pH that is within 2 pH units of the pI of the TGFβR moiety of the fusion protein.
E2. A pharmaceutical composition comprising an IgG:TGFβR fusion protein and characterised by a pH that is within 1.5 pH units of the pI of the TGFβR moiety of the fusion protein.
E3. A pharmaceutical composition comprising an IgG:TGFβR fusion protein and characterised by a pH that is within 1 pH unit of the pI of the TGFβR moiety of the fusion protein.
E4. A pharmaceutical composition comprising an IgG:TGFβR fusion protein and characterised by a pH of pH 4-7.
E5. A pharmaceutical composition comprising an IgG:TGFβR fusion protein and characterised by a pH of pH 5.2-6.1.
E6. The pharmaceutical compositon of any of E1 to E5, wherein the IgG:TGFβR fusion protein is an anti-PD-L1 (IgG):TGFβR2 fusion protein, preferably having an amino acid sequence identical to the amino acid sequence of bintrafusp alfa.
E7. The pharmaceutical compositon of any of E1 to E6 further comprising a tonicifier, preferably sodium chloride.
E8. The pharmaceutical compositon of any of E1 to E7, further comprising a surfactant, preferably polysorbate 20.
E9. The pharmaceutical compositon of any of E1 to E8, further comprising a sugar component, preferably trehalose.
E10. The pharmaceutical compositon of any of E1 to E9, further comprising an amino acid component, preferably arginine and/or lysine.
E11. The pharmaceutical compositon of any of E1 to E10, further comprising a buffer system, preferably a histidine buffer system.
E12. The pharmaceutical compositon of any of E1 to E11, further comprising an antioxidant, preferably methionine.

*Aqueous Formulations and Ionic Strength*

Formulation studies revealed particular aqueous solubility challenges in respect of IgG:TGFβR fusion proteins which, without wishing to be bound by theory, are thought to stem from the significant differences between the pI of the antibody moiety and the pI of the fused TGFβR moiety. Surprisingly, ionic strength can have a dramatic influence on the aqueous solubility of the fusion protein. Ionic strength also appears to play a role in protein stabilisation.
During the early phases of formulation research, lyophilised formulations were thought to be the only viable formulation option from a long-term storage stability perspective and, as discussed below, these appeared to impose certain limitations. However, as studies progressed, liquid formulations were also found ot be viable and, for practical reasons, generally preferred (e.g. it avoids energy-intensive freeze-drying, and manual reconstitution by physicians).
As mentioned above in the section on "Ionic Strength", the composition may be characterised by an ionic strength, which suitably includes contributions from all ionic species within the composition. However, in some embodiments, the compositions may be characterised by a non-buffering (or tonicifier-based) ionic strength, in which case the ionic strength suitably includes contributions from all ionic species except those of the buffer system. Since a buffer system may be used at a relatively low concentration, the embodiments that follow may apply to either definition of ionic strength.

Preferably the ionic strength of the composition is at least 20 mM, whilst, in the case of a pharmaceutical composition that is intended for subcutaneous use without prior dilution, its maximum is preferably governed by osmolality requirements so as to avoid any excessive hypertonicity. In a preferred embodiment, the maximum ionic strength is 200 mM, more preferably 160 mM.

Whilst ionic strength may be imparted by a variety of charged or ionic excipients, the ionic strength is suitably provided by one or more ionic tonicifiers, for example sodium chloride and/or charged amino acids (e.g. arginine or arginine salts), with sodium chloride being most preferred.

Sodium chloride may be the sole non-buffering contributor to ionic strength. In some embodiments, the pharmaceutical composition comprises an amino acid component, which is separately defined from the tonicifier (e.g., NaCl) and which may, for example, include charged or ionic compounds such as arginine or arginine salts. In such instances, it will be understood that the amino acid component contributes to the overall ionic strength and indeed tonicity.

The following numbered paragraphs F1 to F12 disclose specific embodiments of the invention.

F1. A pharmaceutical composition comprising an IgG:TGFβR fusion protein and characterised by an ionic strength (optionally a non-buffering ionic strength) greater than or equal to 20 mM.

F2. A pharmaceutical composition comprising an IgG:TGFβR fusion protein, and characterised by an ionic strength (optionally a non-buffering ionic strength) greater than or equal to 20 mM and by an osmolality of 255-345 mOsm/kg.

F3. A pharmaceutical composition comprising an IgG:TGFβR fusion protein, and characterised by an ionic strength (optionally a non-buffering ionic strength) between 20 and 200 mM.

F4. A pharmaceutical composition comprising an IgG:TGFβR fusion protein, and greater than or equal to 20 mM ionic tonicifier.

F5. A pharmaceutical composition comprising an IgG:TGFβR fusion protein, and greater than or equal to 20 mM sodium chloride.

F6. The pharmaceutical composition of any of F1 to F5 further characterised by a pH as defined in any of E1 to E5.

F7. The pharmaceutical compositon of any of F1 to F6, wherein the IgG:TGFβR fusion protein is an anti-PD-L1 (IgG):TGFβR2 fusion protein, preferably having an amino acid sequence identical to the amino acid sequence of bintrafusp alfa.

F8. The pharmaceutical compositon of any of F1 to F7, further comprising a surfactant, preferably polysorbate 20.

F9. The pharmaceutical compositon of any of F1 to F8, further comprising a sugar component, preferably trehalose.

F10. The pharmaceutical compositon of any of F1 to F9, further comprising an amino acid component, preferably arginine and/or lysine.

F11. The pharmaceutical compositon of any of F1 to F10, further comprising a buffer system, preferably a histidine buffer system.

F12. The pharmaceutical compositon of any of F1 to F11, further comprising an antioxidant, preferably methionine.

*Lyophilisation and Ionic Strength*

Early studies focused on lyophilised formulations which, at the time, were seen as the most viable option from a long-term storage perspective. As such, a balance was sought between solubility and isotonicity (in liquid forms), stability (in lyophilised and reconstituted liquid forms), and freeze-dryability. As per the above section "Aqueous Formulations and Ionic Strength", ionic strength, particularly when provided to the composition in the form of sodium chloride, was instrumental in meeting tonicity, stability, and solubility requirements. However, studies found that using too much sodium chloride compromised freeze-drying and caused lyophilised cakes to collapse. Though incorporation of lyoprotectants, such as trehalose, could mitigate this to an extent, there appeared to be a maximum tolerable amount of sodium chloride for meeting each of the above objectives of a lyophilised formulation. Since, as will be discussed below, higher protein concentrations generally required the support of higher concentrations of sodium chloride, this limitation on the amount of sodium chloride also potentially limited the concentration of the fusion protein.

The following numbered paragraphs G1 to G21 disclose specific embodiments of the invention.

G1. A pharmaceutical composition comprising an IgG:TGFβR fusion protein and characterised by an ionic strength (optionally a non-buffering ionic strength) between 20 and 50 mM.

G2. A pharmaceutical composition comprising an IgG:TGFβR fusion protein, and characterised by an ionic strength (optionally a non-buffering ionic strength) between 20 and 50 mM and by an osmolality of 255-345 mOsm/kg.

G3. A pharmaceutical composition comprising an IgG:TGFβR fusion protein, and between 20 and 50 mM ionic tonicifier.

G4. A pharmaceutical composition comprising an IgG:TGFβR fusion protein, and between 20 and 50 mM sodium chloride.

G5. The pharmaceutical composition of any of G1 to G4, wherein the concentration of the ionic tonicifier (or sodium chloride) is 35-45 mM.

G6. The pharmaceutical composition of any of G1 to G4, wherein the concentration of the ionic tonicifier (or sodium chloride) is about 40 mM.

G7. The pharmaceutical composition of any of G1 to G6, wherein the concentration of the IgG:TGFβR fusion protein is 5-25 mM.

G8. The pharmaceutical composition of any of G1 to G7, wherein the concentration of the IgG:TGFβR fusion protein is about 10 mM.

G9. A pharmaceutical composition, comprising an IgG:TGFβR fusion protein, and an ionic tonicifier in a molar ratio of ionic tonicifier to fusion protein between 180:1 and 9110:1.

G10. A pharmaceutical composition, comprising an IgG:TGFβR fusion protein, and an ionic tonicifier in a molar ratio of ionic tonicifier to fusion protein between 360:1 and 911:1.

G11. A pharmaceutical composition, comprising an IgG:TGFβR fusion protein, and sodium chloride in a molar ratio of sodium chloride to fusion protein between 180:1 and 9110:1.

G12. A pharmaceutical composition, comprising an IgG:TGFβR fusion protein, and sodium chloride in a molar ratio of sodium chloride to fusion protein between 360:1 and 911:1.

G13. The pharmaceutical compositon of any of G1 to G12 further characterised by a pH as defined in any of E1 to E5.

G14. The pharmaceutical compositon of any of G1 to G13, wherein the IgG:TGFβR fusion protein is an anti-PD-L1 (IgG):TGFβR2 fusion protein, preferably having an amino acid sequence identical to the amino acid sequence of bintrafusp alfa.

G15. The pharmaceutical compositon of any of G1 to G14, further comprising a surfactant, preferably polysorbate 20.

G16. The pharmaceutical compositon of any of G1 to G15, further comprising a sugar component, preferably trehalose.

G17. The pharmaceutical compositon of any of G1 to G16, further comprising an amino acid component, preferably arginine and/or lysine.

G18. The pharmaceutical compositon of any of G1 to G17, further comprising a buffer system, preferably a histidine buffer system.

G19. The pharmaceutical compositon of any of G1 to G18, further comprising an antioxidant, preferably methionine.

G20. The pharmaceutical composition of any of G1 to G19, which is a reconstituted lyophilised pharmaceutical composition.

G21. The pharmaceutical composition of any of G1 to G19, which is a solid lyophilised pharmaceutical composition suitable for reconstitution.

*Tonicifying Lyoprotectant*

In the case of lyophilised formulations, it was found that inclusion of a tonicifying lyoprotectant could assist in striking a reasonable balance between solubility and isotonicity (in liquid forms), stability (in lyophilised and reconstituted liquid forms), and freeze-dryability. A tonicifying lyoprotectant could impart tonicity without compromising freeze-dryability, unlike ionic strength providers such as sodium chloride which damaged lyophilised cakes, thereby permitting the use of a minimal but sufficiently high amount of ionic strength provider (e.g. NaCl) to facilitate solubility and stability of the protein. In many cases, using a tonicifying lyoprotectant could counteract, at least to a degree, the negative impacts of an ionic tonicifier (e.g. NaCl) upon freeze-drying.

Suitable tonicifying lyoprotectants include sugar component(s) and/or amino acid component(s), for example trehalose and/or arginine, though a sugar component (preferably a single sugar component) is more preferred. Preferred sugar components are described herein, though most suitably the sugar component is trehalose.

The following numbered paragraphs H1 to H33 disclose specific embodiments of the invention.

H1. A pharmaceutical composition comprising an IgG:TGFβR fusion protein, a tonicifying lyoprotectant (e.g. sugar component and/or amino acid component as defined above), and an ionic tonicifier.

H2. A pharmaceutical composition comprising an IgG:TGFβR fusion protein, a tonicifying lyoprotectant (e.g. sugar component and/or amino acid component as defined above), and an ionic tonicifier in a molar ratio of tonicifying lyoprotectant to ionic tonicifier between 10:1 and 1:2.

H3. A pharmaceutical composition comprising an IgG:TGFβR fusion protein, trehalose, and sodium chloride in a molar ratio of trehalose to sodium chloride between 10:1 and 1:2.

H4. A pharmaceutical composition comprising an IgG:TGFβR fusion protein, a tonicifying lyoprotectant (e.g. sugar component and/or amino acid component as defined above), and between 20 and 50 mM ionic tonicifier.

H5. A pharmaceutical composition comprising an IgG:TGFβR fusion protein, a tonicifying lyoprotectant (e.g. sugar component and/or amino acid component), and between 20 and 50 mM sodium chloride.

H6. A pharmaceutical composition comprising an IgG:TGFβR fusion protein, trehalose, and between 20 and 50 mM ionic tonicifier.

H7. A pharmaceutical composition comprising an IgG:TGFβR fusion protein, trehalose, and between 20 and 50 mM sodium chloride.

H8. The pharmaceutical composition of any of H1 to H7, wherein the tonicifying lyoprotectant is present at a concentration of 100-200 mM, preferably 130-170 mM.

H9. The pharmaceutical composition of any of H1 to H8, wherein the concentration of the IgG:TGFβR fusion protein is 5-25 mM.

H10. The pharmaceutical compositon of any of H1 to H9, wherein the concentration of the IgG:TGFβR fusion protein is about 10 mM.

H11. A pharmaceutical composition, comprising an IgG:TGFβR fusion protein, a tonicifying lyoprotectant (e.g. sugar component and/or amino acid component), and an ionic tonicifier in a molar ratio of ionic tonicifier to fusion protein between 180:1 and 9110:1.

H12. A pharmaceutical composition, comprising an IgG:TGFβR fusion protein, a tonicifying lyoprotectant (e.g. sugar component and/or amino acid component), and an ionic tonicifier in a molar ratio of ionic tonicifier to fusion protein between 360:1 and 911:1.

H13. A pharmaceutical composition, comprising an IgG:TGFβR fusion protein, a tonicifying lyoprotectant (e.g. sugar component and/or amino acid component), and sodium chloride in a molar ratio of sodium chloride to fusion protein between 180:1 and 9110:1.

H14. A pharmaceutical composition, comprising an IgG:TGFβR fusion protein, a tonicifying lyoprotectant (e.g. sugar component and/or amino acid component), and sodium chloride in a molar ratio of sodium chloride to fusion protein between 360:1 and 911:1.

H15. A pharmaceutical composition, comprising an IgG:TGFβR fusion protein, trehalose, and an ionic tonicifier in a molar ratio of ionic tonicifier to fusion protein between 180:1 and 9110:1.

H16. A pharmaceutical composition, comprising an IgG:TGFβR fusion protein, trehalose, and an ionic tonicifier in a molar ratio of ionic tonicifier to fusion protein between 360:1 and 911:1.

H17. A pharmaceutical composition, comprising an IgG:TGFβR fusion protein, trehalose, and sodium chloride in a molar ratio of sodium chloride to fusion protein between 180:1 and 9110:1.

H18. A pharmaceutical composition, comprising an IgG:TGFβR fusion protein, trehalose, and sodium chloride in a molar ratio of sodium chloride to fusion protein between 360:1 and 911:1.

H19. The pharmaceutical compositon of any of H1 to H18, wherein the tonicifying lyoprotectant is selected from the group consisting of trehalose, sucrose, sorbitol, mannitol, arginine, and any combination thereof.

H20. The pharmaceutical compositon of any of H1 to H19, wherein the tonicifying lyoprotectant is a combination of trehalose and arginine.

H21. The pharmaceutical compositon of any of H1 to H20, wherein the tonicifying lyoprotectant is trehalose alone.

H22. The pharmaceutical composition of any of H1 to H21, wherein the molar ratio of tonicifying lyoprotectant to ionic tonicifier to fusion protein is 910-36430 : 180-9110 : 1.

H23. The pharmaceutical composition of any of H1 to H22, wherein the molar ratio of tonicifying lyoprotectant to ionic tonicifier to fusion protein is 1820-3643 : 360-911 : 1.

H24. The pharmaceutical composition of any of H1 to H23, wherein the molar ratio of trehalose to sodium chloride to fusion protein is 1820-3643 : 360-911 : 1.

H25. The pharmaceutical composition of any of H1 to H24 further characterised by a pH as defined in any of E1 to E5.

H26. The pharmaceutical compositon of any of H1 to H25, wherein the IgG:TGFβR fusion protein is an anti-PD-L1 (IgG):TGFβR2 fusion protein, preferably having an amino acid sequence identical to the amino acid sequence of bintrafusp alfa.

H27. The pharmaceutical compositon of any of H1 to H26, further comprising a surfactant, preferably polysorbate 20.

H28. The pharmaceutical compositon of any of H1 to H27, further comprising a sugar component, preferably trehalose.

H29. The pharmaceutical compositon of any of H1 to H28, further comprising an amino acid component, preferably arginine and/or lysine.

H30. The pharmaceutical compositon of any of H1 to H29, further comprising a buffer system, preferably a histidine buffer system.

H31. The pharmaceutical compositon of any of H1 to H30, further comprising an antioxidant, preferably methionine.

H32. The pharmaceutical composition of any of H1 to H31, which is a reconstituted lyophilised pharmaceutical composition.

H33. The pharmaceutical composition of any of H1 to H31, which is a solid lyophilised pharmaceutical composition suitable for reconstitution.

*pH and Ionic Strength*

As aforementioned, ionic strength (especially when provided by NaCl) can improve solubility of the fusion protein at all pHs, but this effect is particularly marked at higher pHs.

The following numbered paragraphs I1 to I13 disclose specific embodiments of the invention.

I1. A pharmaceutical composition comprising an IgG:TGFβR fusion protein, and greater than or equal to 20 mM ionic tonicifier, and characterised by a pH of pH 5-7.

I2. A pharmaceutical composition comprising an IgG:TGFβR fusion protein, and greater than or equal to 20 mM sodium chloride, and characterised by a pH of pH 5-7.

I3. A pharmaceutical composition comprising an IgG:TGFβR fusion protein, and greater than or equal to 20 mM ionic tonicifier, and characterised by a pH of pH 5.4-6.4.

I4. A pharmaceutical composition comprising an IgG:TGFβR fusion protein, and greater than or equal to 20 mM sodium chloride, and characterised by a pH of pH 5.4-6.4.

I5. A pharmaceutical composition comprising an IgG:TGFβR fusion protein, and greater than or equal to 20 mM ionic tonicifier, and characterised by a pH of pH 5.8-6.8.

I6. A pharmaceutical composition comprising an IgG:TGFβR fusion protein, and greater than or equal to 20 mM sodium chloride, and characterised by a pH of pH 5.8-6.8.

I7. The pharmaceutical compositon of any of I1 to I6, wherein the IgG:TGFβR fusion protein is an anti-PD-L1 (IgG):TGFβR2 fusion protein, preferably having an amino acid sequence identical to the amino acid sequence of bintrafusp alfa.

I8. The pharmaceutical compositon of any of I1 to I7, further comprising a surfactant, preferably polysorbate 20.

I9. The pharmaceutical compositon of any of I1 to I8, further comprising a sugar component, preferably trehalose.

I10. The pharmaceutical compositon of any of I1 to I9, further comprising an amino acid component, preferably arginine and/or lysine.

I11. The pharmaceutical compositon of any of I1 to I10, further comprising a buffer system, preferably a histidine buffer system.

I12. The pharmaceutical compositon of any of I1 to I11, further comprising an antioxidant, preferably methionine.

I13. The pharmaceutical composition of any of I1 to I12, which is a liquid pharmaceutical composition.

*Fusion Protein Concentration*

Whilst early studies suggested that fusion protein concentration may be limited in lyophilised formulations by the maximum amount of sodium chloride tolerated by the freeze-drying process, later studies demonstrated the long-term viability of liquid formulations, which in turn unleashed the potential for higher fusion protein concentrations (especially when suitably supported by increases in ionic strength).

The following numbered paragraphs J1 to J12 disclose specific embodiments of the invention.

J1. A pharmaceutical composition comprising more than 20 mg/mL, preferably more than 80 mg/mL IgG:TGFβR fusion protein.

J2. A pharmaceutical composition comprising more than 20 mg/mL, preferably more than 80 mg/mL IgG:TGFβR fusion protein, and greater than or equal to 20 mM ionic tonicifier.

J3. A pharmaceutical composition comprising more than 20 mg/mL, preferably more than 80 mg/mL IgG:TGFβR fusion protein, and greater than or equal to 20 mM ionic tonicifier, and characterised by a pH of pH 4-8.

J4. The pharmaceutical compositon of any of J1 to J3, wherein the IgG:TGFβR fusion protein is an anti-PD-L1 (IgG):TGFβR2 fusion protein, preferably having an amino acid sequence identical to the amino acid sequence of bintrafusp alfa.

J5. The pharmaceutical compositon of any of J1 to J4, characterised by a pH as defined in any of E1 to E5.

J6. The pharmaceutical compositon of any of J1 to J5 further comprising a tonicifier, preferably sodium chloride.

J7. The pharmaceutical compositon of any of J1 to J6, further comprising a surfactant, preferably polysorbate 20.

J8. The pharmaceutical compositon of any of J1 to J7, further comprising a sugar component, preferably trehalose.

J9. The pharmaceutical compositon of any of J1 to J8, further comprising an amino acid component, preferably arginine and/or lysine.

J10. The pharmaceutical compositon of any of J1 to J9, further comprising a buffer system, preferably a histidine buffer system.

J11. The pharmaceutical compositon of any of J1 to J10, further comprising an antioxidant, preferably methionine.

J12. The pharmaceutical composition of any of J1 to J11, which is a liquid pharmaceutical composition.

*Fusion Protein Concentration and Ionic Strength*

Studies found that higher concentrations of fusion protein could be achieved but were preferably supported by an increase in ionic strength. Increasing the ionic strength (especially sodium chloride concentration) in tandem with fusion protein concentration ensured greater protein solubility and stability. In some cases, it was found that a sugar component, such as trehalose, could partially compensate for a lack of NaCl, the presence of an ionic strength provider is generally preferred.

The following numbered paragraphs K1 to K27 disclose specific embodiments of the invention.

K1. A pharmaceutical composition comprising 5-150 mg/mL IgG:TGFβR fusion protein, and further characterised by an ionic strength (optionally a non-buffering ionic strength) of 5-250 mM.

K2. A pharmaceutical composition comprising an IgG:TGFβR fusion protein, and further characterised by an ionic strength in a molar ratio of ionic strength to fusion protein of from 1000:1 to 70:1.

K3. A pharmaceutical composition comprising 5-25 mg/mL IgG:TGFβR fusion protein, and further characterised by an ionic strength (optionally a non-buffering ionic strength) of 20-50 mM.

K4. A pharmaceutical composition comprising 25-45 mg/mL IgG:TGFβR fusion protein, and further characterised by an ionic strength (optionally a non-buffering ionic strength) of 40-80 mM.

K5. A pharmaceutical composition comprising at least 25 mg/mL IgG:TGFβR fusion protein, and further characterised by an ionic strength (optionally a non-buffering ionic strength) of at least 50 mM.

K6. A pharmaceutical composition comprising 45-65 mg/mL IgG:TGFβR fusion protein, and further characterised by an ionic strength (optionally a non-buffering ionic strength) of 80-170 mM.

K7. A pharmaceutical composition comprising 45-65 mg/mL IgG:TGFβR fusion protein, and further characterised by an ionic strength (optionally a non-buffering ionic strength) of 80-120 mM, preferably where a sugar component (most preferably trehalose) is also present.

K8. A pharmaceutical composition comprising 45-65 mg/mL IgG:TGFβR fusion protein, and further characterised by an ionic strength (optionally a non-buffering ionic strength) of 120-170 mM, preferably where a sugar component (most preferably trehalose) is absent.

K9. A pharmaceutical composition comprising 65-115 mg/mL IgG:TGFβR fusion protein, and further characterised by an ionic strength (optionally a non-buffering ionic strength) of 100-200 mM.

K10. A pharmaceutical composition comprising 5-150 mg/mL IgG:TGFβR fusion protein and 10-200 mM tonicifier (preferably 10-200 mM sodium chloride).

K11. A pharmaceutical composition comprising an IgG:TGFβR fusion protein and a tonicifier (preferably sodium chloride) in a molar ratio of tonicifier (preferably sodium chloride) to fusion protein of from 910:1 to 200:1.

K12. A pharmaceutical composition comprising 5-25 mg/mL IgG:TGFβR fusion protein and 20-50 mM tonicifier (preferably 20-50 mM sodium chloride).

K13. A pharmaceutical composition comprising 25-45 mg/mL IgG:TGFβR fusion protein and 40-80 mM tonicifier (preferably 40-80 mM sodium chloride).

K14. A pharmaceutical composition comprising at least 25 mg/mL IgG:TGFβR fusion protein and at least 50 mM tonicifier (preferably at least 50 mM sodium chloride).

K15. A pharmaceutical composition comprising 45-65 mg/mL IgG:TGFβR fusion protein and 80-170 mM tonicifier (preferably 80-170 mM sodium chloride).

K16. A pharmaceutical composition comprising 45-65 mg/mL IgG:TGFβR fusion protein and 80-120 mM tonicifier (preferably 80-120 mM sodium chloride), preferably where a sugar component (most preferably trehalose) is also present.

K17. A pharmaceutical composition comprising 45-65 mg/mL IgG:TGFβR fusion protein and 120-170 mM tonicifier (preferably 120-170 mM mM sodium chloride), preferably where a sugar component (most preferably trehalose) is absent.

K18. A pharmaceutical composition comprising 65-115 mg/mL IgG:TGFβR fusion protein and 100-200 mM tonicifier (preferably 100-200 mM sodium chloride).

K19. The pharmaceutical compositon of any of K1 to K18, wherein the IgG:TGFβR fusion protein is an anti-PD-L1 (IgG):TGFβR2 fusion protein, preferably having an amino acid sequence identical to the amino acid sequence of bintrafusp alfa.

K20. The pharmaceutical compositon of any of K1 to K19, characterised by a pH as defined in any of E1 to E5.

K21. The pharmaceutical compositon of any of K1 to K20 further comprising a tonicifier, preferably sodium chloride.

K22. The pharmaceutical compositon of any of K1 to K21, further comprising a surfactant, preferably polysorbate 20.

K23. The pharmaceutical compositon of any of K1 to K22, further comprising a sugar component, preferably trehalose.

K24. The pharmaceutical compositon of any of K1 to K23, further comprising an amino acid component, preferably arginine and/or lysine.

K25. The pharmaceutical compositon of any of K1 to K24, further comprising a buffer system, preferably a histidine buffer system.

K26. The pharmaceutical compositon of any of K1 to K25, further comprising an antioxidant, preferably methionine.

K27. The pharmaceutical composition of any of K1 to K26, which is a liquid pharmaceutical composition.

### Fusion Protein Concentration and pH

It was discovered that, in general, higher concentrations of fusion protein tend to be best supported by slightly higher pHs, albeit pHs that are still closer to the pl of the TGFβR moiety than might be normally expected.

The following numbered paragraphs L1 to L17 disclose specific embodiments of the invention.

L1. A pharmaceutical composition comprising 5-150 mg/mL IgG:TGFβR fusion protein, and characterised by a pH of pH 5.0-7.5, preferably a pH of pH 5.0-7.0.

L2. A pharmaceutical composition comprising 5-150 mg/mL IgG:TGFβR fusion protein, characterised by a pH of pH 5.3-6.3, and further characterised by an absence of acetate buffers and acetate salts.

L3. A pharmaceutical composition comprising 5-150 mg/mL IgG:TGFβR fusion protein, a histidine buffer system, and characterised by a pH of pH 5.3-6.3.

L4. A pharmaceutical composition comprising 5-25 mg/mL IgG:TGFβR fusion protein, and characterised by a pH of pH 5.0-6.5, preferably a pH of pH 5.0-5.7.

L5. A pharmaceutical composition comprising 25-45 mg/mL IgG:TGFβR fusion protein, and characterised by a pH of pH 5.0-7.5, preferably a pH 5.3-6.2.

L6. A pharmaceutical composition comprising at least 25 mg/mL IgG:TGFβR fusion protein, and characterised by a pH of pH 5.0-7.5, preferably a pH of pH 5.3-6.5.

L7. A pharmaceutical composition comprising at least 35 mg/mL IgG:TGFβR fusion protein, and characterised by a pH of pH 5.0-7.5, preferably a pH of pH 5.4-7.0.

L8. A pharmaceutical composition comprising 45-65 mg/mL IgG:TGFβR fusion protein, and characterised by a pH of pH 5.0-7.5, preferably a pH of pH 5.5-6.5.

L9. A pharmaceutical composition comprising 65-115 mg/mL IgG:TGFβR fusion protein, and characterised by a pH of pH 5.0-8.0, preferably a pH of pH 5.8-6.8.

L10. The pharmaceutical compositon of any of L1 to L9, wherein the IgG:TGFβR fusion protein is an anti-PD-L1 (IgG):TGFβR2 fusion protein, preferably having an amino acid sequence identical to the amino acid sequence of bintrafusp alfa.

L11. The pharmaceutical compositon of any of L1 to L10 further comprising a tonicifier, preferably sodium chloride.

L12. The pharmaceutical compositon of any of L1 to L11, further comprising a surfactant, preferably polysorbate 20.

L13. The pharmaceutical compositon of any of L1 to L12, further comprising a sugar component, preferably trehalose.

L14. The pharmaceutical compositon of any of L1 to L13, further comprising an amino acid component, preferably arginine and/or lysine.

L15. The pharmaceutical compositon of any of L1 to L14, further comprising a buffer system, preferably a histidine buffer system.

L16. The pharmaceutical compositon of any of L1 to L15, further comprising an antioxidant, preferably methionine.

L17. The pharmaceutical composition of any of L1 to L16, which is a liquid pharmaceutical composition.

### Fusion Protein Concentration, Ionic Strength, and pH

As alluded to above, higher concentrations of fusion protein tend to be best supported by slightly higher pHs and slightly higher ionic strengths.

The following numbered paragraphs M1 to M28 disclose specific embodiments of the invention.

M1. A pharmaceutical composition comprising 5-150 mg/mL IgG:TGFβR fusion protein, and further characterised by an ionic strength (optionally a non-buffering ionic strength) of 5-250 mM and a pH of pH 5.0-7.0.

M2. A pharmaceutical composition comprising an IgG:TGFβR fusion protein, and further characterised by an ionic strength in a molar ratio of ionic strength to fusion protein of from 1000:1 to 70:1 and a pH of pH 5.0-6.5.

M3. A pharmaceutical composition comprising 5-150 mg/mL IgG:TGFβR fusion protein, and further characterised by an ionic strength in a molar ratio of ionic strength to fusion protein of from 375:1 to 150:1 and a pH of pH 5.3-6.3.

M4. A pharmaceutical composition comprising 5-25 mg/mL IgG:TGFβR fusion protein, and further characterised by an ionic strength (optionally a non-buffering ionic strength) of 20-50 mM and a pH of pH 5.0-6.5, preferably a pH of pH 5.0-5.7.

M5. A pharmaceutical composition comprising 25-45 mg/mL IgG:TGFβR fusion protein, and further characterised by an ionic strength (optionally a non-buffering ionic strength) of 40-80 mM and a pH of pH 5.0-7.5, preferably a pH 5.3-6.2.

M6. A pharmaceutical composition comprising at least 25 mg/mL IgG:TGFβR fusion protein, and further characterised by an ionic strength (optionally a non-buffering ionic strength) of at least 50 mM and a pH of pH 5.0-7.5, preferably a pH of pH 5.3-6.5.

M7. A pharmaceutical composition comprising 45-65 mg/mL IgG:TGFβR fusion protein, and further characterised by an ionic strength (optionally a non-buffering ionic strength) of 80-170 mM and a pH of pH 5.0-7.5, preferably a pH of pH 5.5-6.5.

M8. A pharmaceutical composition comprising 45-65 mg/mL IgG:TGFβR fusion protein, and further characterised by an ionic strength (optionally a non-buffering ionic strength) of 80-120 mM and a pH of pH 5.0-7.5, preferably a pH of pH 5.5-6.5, preferably where a sugar component (most preferably trehalose) is also present.

M9. A pharmaceutical composition comprising 45-65 mg/mL IgG:TGFβR fusion protein, and further characterised by an ionic strength (optionally a non-buffering ionic strength) of 120-170 mM a pH of pH 5.0-7.5, preferably a pH of pH 5.5-6.5, preferably where a sugar component (most preferably trehalose) is absent.

M10. A pharmaceutical composition comprising 65-115 mg/mL IgG:TGFβR fusion protein, and further characterised by an ionic strength (optionally a non-buffering ionic strength) of 100-200 mM and a pH of pH of pH 5.0-8.0, preferably a pH of pH 5.8-6.8.

M11. A pharmaceutical composition comprising 5-150 mg/mL IgG:TGFβR fusion protein and 10-200 mM tonicifier (preferably 10-200 mM sodium chloride), further characterised by a pH of pH 5-7.

M12. A pharmaceutical composition comprising an IgG:TGFβR fusion protein and a tonicifier (preferably sodium chloride) in a molar ratio of tonicifier (preferably sodium chloride) to fusion protein of from 910:1 to 200:1, further characterised by a pH of pH 5-7.0.

M13. A pharmaceutical composition comprising 5-25 mg/mL IgG:TGFβR fusion protein and 20-50 mM tonicifier (preferably 20-50 mM sodium chloride), further characterised by a pH of pH 5.0-6.5, preferably a pH of pH 5.0-5.7.

M14. A pharmaceutical composition comprising 25-45 mg/mL IgG:TGFβR fusion protein and 40-80 mM tonicifier (preferably 40-80 mM sodium chloride), further characterised by a pH of pH 5.0-7.5, preferably a pH 5.3-6.2.

M15. A pharmaceutical composition comprising at least 25 mg/mL IgG:TGFβR fusion protein and at least 50 mM tonicifier (preferably at least 50 mM sodium chloride), further characterised by a pH of pH 5.0-7.5, preferably a pH of pH 5.3-6.5.

M16. A pharmaceutical composition comprising 45-65 mg/mL IgG:TGFβR fusion protein and 80-170 mM tonicifier (preferably 80-170 mM sodium chloride), further characterised by a pH of pH 5.0-7.5, preferably a pH of pH 5.5-6.5.

M17. A pharmaceutical composition comprising 45-65 mg/mL IgG:TGFβR fusion protein and 80-120 mM tonicifier (preferably 80-120 mM sodium chloride), further characterised by a pH of pH 5.0-7.5, preferably a pH of pH 5.5-6.5, preferably where a sugar component (most preferably trehalose) is also present.

M18. A pharmaceutical composition comprising 45-65 mg/mL IgG:TGFβR fusion protein and 120-170 mM tonicifier (preferably 120-170 mM mM sodium chloride), further characterised by a pH of pH 5.0-7.5, preferably a pH of pH 5.5-6.5, preferably where a sugar component (most preferably trehalose) is absent.

M19. A pharmaceutical composition comprising 65-115 mg/mL IgG:TGFβR fusion protein and 100-200 mM tonicifier (preferably 100-200 mM sodium chloride), further characterised by a pH of pH 5.0-8.0, preferably a pH of pH 5.8-6.8.

M20. The pharmaceutical compositon of any of M1 to M19, wherein the IgG:TGFβR fusion protein is an anti-PD-L1 (IgG):TGFβR2 fusion protein, preferably having an amino acid sequence identical to the amino acid sequence of bintrafusp alfa.

M21. The pharmaceutical compositon of any of M1 to M20, further comprising a tonicifier, preferably sodium chloride.

M22. The pharmaceutical compositon of any of M1 to M21, further comprising a surfactant, preferably polysorbate 20.

M23. The pharmaceutical compositon of any of M1 to M22, further comprising a sugar component, preferably trehalose.

M24. The pharmaceutical compositon of any of M1 to M23, further comprising an amino acid component, preferably arginine and/or lysine.

M25. The pharmaceutical compositon of any of M1 to M24, further comprising a buffer system, preferably a histidine buffer system.

M26. The pharmaceutical compositon of any of M1 to M25, further comprising an antioxidant, preferably methionine.

M27. The pharmaceutical composition of any of M1 to M26, further characterised by an absence of acetate buffer and acetate salts.

M28. The pharmaceutical composition of any of M1 to M27, which is a liquid pharmaceutical composition.

## Sugar Component

Experimental studies generally showed the inclusion of a sugar component to be advantageous, especially a sugar component selected from the group consisting of a non-reducing disaccharide and a non-reduing sugar polyol, with trehalose seemingly outperforming all other sugar components.

The following numbered paragraphs N1 to N24 disclose specific embodiments of the invention.

N1. A pharmaceutical composition comprising an IgG:TGFβR fusion protein and a sugar component.

N2. A pharmaceutical composition comprising an IgG:TGFβR fusion protein and 30-300 mM sugar component.

N3. A pharmaceutical composition comprising an IgG:TGFβR fusion protein and 40-110 mM sugar component.

N4. A pharmaceutical composition comprising an IgG:TGFβR fusion protein and 100-200 mM sugar component.

N5. A pharmaceutical composition comprising an IgG:TGFβR fusion protein and a sugar component in a molar ratio of sugar component to fusion protein of from 3700:1 to 70:1.

N6. A pharmaceutical composition comprising an IgG:TGFβR fusion protein and a sugar component selected from the group consisting of trehalose, sucrose, mannitol, and sorbitol.

N7. A pharmaceutical composition comprising an IgG:TGFβR fusion protein and 30-300 mM sugar component selected from the group consisting of trehalose, sucrose, mannitol, and sorbitol.

N8. A pharmaceutical composition comprising an IgG:TGFβR fusion protein and 40-110 mM sugar component selected from the group consisting of trehalose, sucrose, mannitol, and sorbitol.

N9. A pharmaceutical composition comprising an IgG:TGFβR fusion protein and 100-200 mM sugar component selected from the group consisting of trehalose, sucrose, mannitol, and sorbitol.

N10. A pharmaceutical composition comprising an IgG:TGFβR fusion protein and a sugar component selected from the group consisting of trehalose, sucrose, mannitol, and sorbitol, in a molar ratio of sugar component to fusion protein of from 3700:1 to 70:1.

N11. A pharmaceutical composition comprising an IgG:TGFβR fusion protein and trehalose.

N12. A pharmaceutical composition comprising an IgG:TGFβR fusion protein and 30-300 mM trehalose.

N13. A pharmaceutical composition comprising an IgG:TGFβR fusion protein and 40-110 mM trehalose.

N14. A pharmaceutical composition comprising an IgG:TGFβR fusion protein and 100-200 mM trehalose.

N15. A pharmaceutical composition comprising an IgG:TGFβR fusion protein and a trehalose in a molar ratio of trehalose to fusion protein of from 3700:1 to 70:1.

N16. A pharmaceutical composition comprising an IgG:TGFβR fusion protein and a disaccharide.

N17. A pharmaceutical composition comprising an IgG:TGFβR fusion protein and 40-200 mM disaccharide.

N18. The pharmaceutical composition of any of N1 to N17 further characterised by the features set forth in any of E1 to E5, F1 to F5, G1 to G12, H1 to H24, I1 to I6, J1 to J4, K1 to K18, L1 to L9, and M1 to M19.

N19. The pharmaceutical compositon of any of N1 to N18, wherein the IgG:TGFβR fusion protein is an anti-PD-L1 (IgG):TGFβR2 fusion protein, preferably having an amino acid sequence identical to the amino acid sequence of bintrafusp alfa.

N20. The pharmaceutical compositon of any of N1 to N19 further comprising a tonicifier, preferably sodium chloride.

N21. The pharmaceutical compositon of any of N1 to N20, further comprising a surfactant, preferably polysorbate 20.

N22. The pharmaceutical compositon of any of N1 to N21, further comprising an amino acid component, preferably arginine and/or lysine.

N23. The pharmaceutical compositon of any of N1 to N22, further comprising a buffer system, preferably a histidine buffer system.

N24. The pharmaceutical compositon of any of N1 to N23, further comprising an antioxidant, preferably methionine.

*Amino Acid Component*

Experimental studies generally showed the inclusion of an amino acid component to be advantageous, especially amino acid component component selected from the group consisting of arginine, lysine, proline, gluatamic acid, and glycine, with arginine, and to some extent lysine, seemingly outperforming all other amino acid components.

The following numbered paragraphs O1 to O20 disclose specific embodiments of the invention.

O1. A pharmaceutical composition comprising an IgG:TGFβR fusion protein and an amino acid component.
O2. A pharmaceutical composition comprising an IgG:TGFβR fusion protein and 10-300 mM amino acid component.
O3. A pharmaceutical composition comprising an IgG:TGFβR fusion protein and 30-110 mM amino acid component.
O4. A pharmaceutical composition comprising an IgG:TGFβR fusion protein and a amino acid component in a molar ratio of amino acid component to fusion protein of from 5500:1 to 18:1.
O5. A pharmaceutical composition comprising an IgG:TGFβR fusion protein and an amino acid component selected from the group consisting of arginine, lysine, proline, gluatamic acid, glycine, and any combination thereof.
O6. A pharmaceutical composition comprising an IgG:TGFβR fusion protein and 10-300 mM amino acid component selected from the group consisting of arginine, lysine, proline, gluatamic acid, glycine, and any combination thereof.
O7. A pharmaceutical composition comprising an IgG:TGFβR fusion protein and 30-110 mM amino acid component selected from the group consisting of arginine, lysine, proline, gluatamic acid, glycine, and any combination thereof.
O8. A pharmaceutical composition comprising an IgG:TGFβR fusion protein and an amino acid component selected from the group consisting of arginine, lysine, proline, gluatamic acid, glycine, and any combination thereof in a molar ratio of amino acid component to fusion protein of from 5500:1 to 18:1.
O9. A pharmaceutical composition comprising an IgG:TGFβR fusion protein and arginine.
O10. A pharmaceutical composition comprising an IgG:TGFβR fusion protein and 10-300 mM arginine.
O11. A pharmaceutical composition comprising an IgG:TGFβR fusion protein and 30-110 mM arginine.
O12. A pharmaceutical composition comprising an IgG:TGFβR fusion protein and 30-80 mM arginine.
O13. A pharmaceutical composition comprising an IgG:TGFβR fusion protein and arginine in a molar ratio of arginine to fusion protein of from 5500:1 to 18:1.
O14. The pharmaceutical composition of any of O1 to 013 further characterised by the features set forth in any of E1 to E5, F1 to F5, G1 to G12, H1 to H24, I1 to I6, J1 to J4, K1 to K18, L1 to L9, M1 to M19, and N1 to N17.
O15. The pharmaceutical compositon of any of O1 to 014, wherein the IgG:TGFβR fusion protein is an anti-PD-L1 (IgG):TGFβR2 fusion protein, preferably having an amino acid sequence identical to the amino acid sequence of bintrafusp alfa.
O16. The pharmaceutical compositon of any of O1 to 015 further comprising a tonicifier, preferably sodium chloride.
O17. The pharmaceutical compositon of any of O1 to 016, further comprising a surfactant, preferably polysorbate 20.
O18. The pharmaceutical compositon of any of O1 to 017, further comprising a sugar component, preferably trehalose.
019. The pharmaceutical compositon of any of O1 to 018, further comprising a buffer system, preferably a histidine buffer system.
O20. The pharmaceutical compositon of any of O1 to O19, further comprising an antioxidant, preferably methionine.

*Sugar Component and Amino Acid Component*

Studies showed that arginine, trehalose, and lysine all contributed to formulation stability. Though arginine could potentially replace or at least partially replace trehalose, the combined use of trehalose and arginine was found to stabilize particularly well.

The following numbered paragraphs P1 to P18 disclose specific embodiments of the invention.

P1. A pharmaceutical compositon comprising an IgG:TGFβR fusion protein, a sugar component, and a amino acid component.

P2. A pharmaceutical compositon comprising an IgG:TGFβR fusion protein, 30-300 mM sugar component, and 10-300 mM amino acid component.

P3. A pharmaceutical compositon comprising an IgG:TGFβR fusion protein, 40-110 mM sugar component, and 30-110 mM amino acid component.

P4. A pharmaceutical compositon comprising an IgG:TGFβR fusion protein, a sugar component in a molar ratio of sugar component to fusion protein of from 3700:1 to 70:1, and a amino acid component in a molar ratio of amino acid component to fusion protein of from 5500:1 to 18:1.

P5. A pharmaceutical compositon comprising an IgG:TGFβR fusion protein, a sugar component selected from the group consisting of trehalose, sucrose, mannitol, and sorbitol, and a amino acid component selected from the group consisting of arginine, lysine, proline, gluatamic acid, and glycine.

P6. A pharmaceutical compositon comprising an IgG:TGFβR fusion protein, 30-300 mM sugar component component selected from the group consisting of trehalose, sucrose, mannitol, and sorbitol, and 10-300 mM amino acid component selected from the group consisting of arginine, lysine, proline, gluatamic acid, and glycine.

P7. A pharmaceutical compositon comprising an IgG:TGFβR fusion protein, 40-110 mM sugar component selected from the group consisting of trehalose, sucrose, mannitol, and sorbitol, and 30-110 mM amino acid component selected from the group consisting of arginine, lysine, proline, gluatamic acid, and glycine.

P8. A pharmaceutical compositon comprising an IgG:TGFβR fusion protein, a sugar component selected from the group consisting of trehalose, sucrose, mannitol, and sorbitol in a molar ratio of sugar component to fusion protein of from 3700:1 to 70:1, and a amino acid component selected from the group consisting of arginine, lysine, proline, gluatamic acid, and glycine in a molar ratio of amino acid component to fusion protein of from 5500:1 to 18:1.

P9. A pharmaceutical compositon comprising an IgG:TGFβR fusion protein, trehalose, and arginine.

P10. A pharmaceutical compositon comprising an IgG:TGFβR fusion protein, 30-300 mM trehalose, and 10-300 mM arginine.

P11. A pharmaceutical compositon comprising an IgG:TGFβR fusion protein, 40-110 mM trehalose, and 30-110 mM arginine.

P12. A pharmaceutical compositon comprising an IgG:TGFβR fusion protein, trehalose in a molar ratio of trehalose to fusion protein of from 3700:1 to 70:1, and arginine in a molar ratio of arginine to fusion protein of from 5500:1 to 18:1.

P13. The pharmaceutical composition of any of P1 to P12 further characterised by the features set forth in any of E1 to E5, F1 to F5, G1 to G12, H1 to H24, I1 to I6, J1 to J4, K1 to K18, L1 to L9, and M1 to M19.

P14. The pharmaceutical compositon of any of P1 to P13, wherein the IgG:TGFβR fusion protein is an anti-PD-L1 (IgG):TGFβR2 fusion protein, preferably having an amino acid sequence identical to the amino acid sequence of bintrafusp alfa.

P15. The pharmaceutical compositon of any of P1 to P14, further comprising a tonicifier, preferably sodium chloride.

P16. The pharmaceutical compositon of any of P1 to P15, further comprising a surfactant, preferably polysorbate 20.

P17. The pharmaceutical compositon of any of P1 to P16, further comprising a buffer system, preferably a histidine buffer system.

P18. The pharmaceutical compositon of any of P1 to P17, further comprising an antioxidant, preferably methionine.

*Sugar Component and Amino Acid Component as Alternatives to Sodium Chloride*

Later studies suggest that arginine, and to an extent trehalose, may potentially replace or partially replace NaCl as a source of ionic strength.

The following numbered paragraphs Q1 to Q17 disclose specific embodiments of the invention.

Q1. A pharmaceutical composition comprising an IgG:TGFβR fusion protein, and one or more or all of sodium chloride, a sugar component, and an amino acid component.

Q2. A pharmaceutical composition comprising an IgG:TGFβR fusion protein, and one or more or all of sodium chloride and an amino acid component.

Q3. A pharmaceutical composition comprising an IgG:TGFβR fusion protein, and one or more or all of sodium chloride, a sugar component, and an amino acid component; wherein the total molar concentration of the combination of one or more or all of sodium chloride, a sugar component, and an amino acid component, is between 150 mM and 250 mM.

Q4. A pharmaceutical composition comprising an IgG:TGFβR fusion protein, and one or more or all of sodium chloride, a sugar component, and an amino acid component; wherein the total molar concentration of the combination of one or more or all of sodium chloride, a sugar component, and an amino acid component, is between 180 mM and 220 mM.

Q5. A pharmaceutical composition comprising an IgG:TGFβR fusion protein, and one or more or all of sodium chloride, a sugar component selected from the group consisting of trehalose, sucrose, mannitol, and sorbitol, and an amino acid component selected from the group consisting of arginine, lysine, proline, gluatamic acid, and glycine.

Q6. A pharmaceutical composition comprising an IgG:TGFβR fusion protein, sodium chloride, and an amino acid component selected from the group consisting of arginine, lysine, proline, gluatamic acid, and glycine.

Q7. A pharmaceutical composition comprising an IgG:TGFβR fusion protein, and one or more or all of sodium chloride, a sugar component selected from the group consisting of trehalose, sucrose, mannitol, and sorbitol, and an amino acid component selected from the group consisting of arginine, lysine, proline, gluatamic acid, and glycine; wherein the total molar concentration of the combination of one or more or all of sodium chloride, a sugar component, and an amino acid component, is between 150 mM and 250 mM.

Q8. A pharmaceutical composition comprising an IgG:TGFβR fusion protein, and one or more or all of sodium chloride, a sugar component selected from the group consisting of trehalose, sucrose, mannitol, and sorbitol, and an amino acid component selected from the group consisting of arginine, lysine, proline, gluatamic acid, and glycine; wherein the total molar concentration of the combination of one or more or all of sodium chloride, a sugar component, and an amino acid component, is between 180 mM and 220 mM.

Q9. A pharmaceutical composition comprising an IgG:TGFβR fusion protein, and one or more or all of sodium chloride, trehalose, and arginine.

Q10. A pharmaceutical composition comprising an IgG:TGFβR fusion protein, sodium chloride, and arginine.

Q11. A pharmaceutical composition comprising an IgG:TGFβR fusion protein, and one or more or all of sodium chloride, trehalose, and arginine; wherein the total molar concentration of the combination of one or more or all of sodium chloride, trehalose, and arginine, is between 150 mM and 250 mM.

Q12. A pharmaceutical composition comprising an IgG:TGFβR fusion protein, and one or more or all of sodium chloride, trehalose, and arginine; wherein the total molar concentration of the combination of one or more or all of sodium chloride, trehalose, and arginine, is between 180 mM and 220 mM.

Q13. The pharmaceutical composition of any of Q1 to Q12 further characterised by the features set forth in any of E1 to E5, F1 to F5, G1 to G12, H1 to H24, I1 to I6, J1 to J4, K1 to K18, L1 to L9, M1 to M19, and N1 to N17.

Q14. The pharmaceutical compositon of any of Q1 to Q13, wherein the IgG:TGFβR fusion protein is an anti-PD-L1 (IgG):TGFβR2 fusion protein, preferably having an amino acid sequence identical to the amino acid sequence of bintrafusp alfa.

Q15. The pharmaceutical compositon of any of Q1 to Q14, further comprising a surfactant, preferably polysorbate 20.

Q16. The pharmaceutical compositon of any of Q1 to Q15, further comprising a buffer system, preferably a histidine buffer system.

Q17. The pharmaceutical compositon of any of Q1 to Q16, further comprising an antioxidant, preferably methionine.

*Surfactant*

Studies demonstrated that a surfactant was desirable, with polysorbate surfactants, especially polysorbate 20, being the top performers. Surfactants were particularly useful for mitigating aggregation.

The following numbered paragraphs R1 to R34 disclose specific embodiments of the invention.

R1. A pharmaceutical composition comprising an IgG:TGFβR fusion protein, and a surfactant.

R2. A pharmaceutical composition comprising an IgG:TGFβR fusion protein, and 0.01-2 mg/mL surfactant.

R3. A pharmaceutical composition comprising an IgG:TGFβR fusion protein, and 0.05-1.5 mg/mL surfactant.

R4. A pharmaceutical composition comprising an IgG:TGFβR fusion protein, and 0.4-1.2 mg/mL surfactant.

R5. A pharmaceutical composition comprising an IgG:TGFβR fusion protein, and 0.4-0.6 mg/mL surfactant.

R6. A pharmaceutical composition comprising an IgG:TGFβR fusion protein, and about 0.5 mg/mL surfactant.

R7. A pharmaceutical composition comprising an IgG:TGFβR fusion protein, and a surfactant in a molar ratio of surfactant to fusion protein of from 30:1 to 1:70.

R8. A pharmaceutical composition comprising an IgG:TGFβR fusion protein, and a surfactant in a molar ratio of surfactant to fusion protein of from 12:1 to 1:3.

R9. A pharmaceutical composition comprising an IgG:TGFβR fusion protein, and a surfactant in a molar ratio of surfactant to fusion protein of from 8:1 to 1:2.

R10. A pharmaceutical composition comprising an IgG:TGFβR fusion protein, and a surfactant selected from polysorbate 20 or polysorbate 80.

R11. A pharmaceutical composition comprising an IgG:TGFβR fusion protein, and 0.01-2 mg/mL surfactant selected from polysorbate 20 or polysorbate 80.

R12. A pharmaceutical composition comprising an IgG:TGFβR fusion protein, and 0.05-1.5 mg/mL surfactant selected from polysorbate 20 or polysorbate 80.

R13. A pharmaceutical composition comprising an IgG:TGFβR fusion protein, and 0.4-1.2 mg/mL surfactant selected from polysorbate 20 or polysorbate 80.

R14. A pharmaceutical composition comprising an IgG:TGFβR fusion protein, and 0.4-0.6 mg/mL surfactant selected from polysorbate 20 or polysorbate 80.

R15. A pharmaceutical composition comprising an IgG:TGFβR fusion protein, and about 0.5 mg/mL surfactant selected from polysorbate 20 or polysorbate 80.

R16. A pharmaceutical composition comprising an IgG:TGFβR fusion protein, and a surfactant selected from polysorbate 20 or polysorbate 80 in a molar ratio of surfactant to fusion protein of from 30:1 to 1:70.

R17. A pharmaceutical composition comprising an IgG:TGFβR fusion protein, and a surfactant selected from polysorbate 20 or polysorbate 80 in a molar ratio of surfactant to fusion protein of from 12:1 to 1:3.

R18. A pharmaceutical composition comprising an IgG:TGFβR fusion protein, and a surfactant selected from polysorbate 20 or polysorbate 80 in a molar ratio of surfactant to fusion protein of from 8:1 to 1:2.

R19. A pharmaceutical composition comprising an IgG:TGFβR fusion protein, and polysorbate 20.

R20. A pharmaceutical composition comprising an IgG:TGFβR fusion protein, and 0.01-2 mg/mL polysorbate 20.

R21. A pharmaceutical composition comprising an IgG:TGFβR fusion protein, and 0.05-1.5 mg/mL polysorbate 20.

R22. A pharmaceutical composition comprising an IgG:TGFβR fusion protein, and 0.4-1.2 mg/mL polysorbate 20.

R23. A pharmaceutical composition comprising an IgG:TGFβR fusion protein, and 0.4-0.6 mg/mL polysorbate 20.

R24. A pharmaceutical composition comprising an IgG:TGFβR fusion protein, and about 0.5 mg/mL polysorbate 20.

R25. A pharmaceutical composition comprising an IgG:TGFβR fusion protein, and a polysorbate 20 in a molar ratio of polysorbate 20 to fusion protein of from 30:1 to 1:70.

R26. A pharmaceutical composition comprising an IgG:TGFβR fusion protein, and a polysorbate 20 in a molar ratio of polysorbate 20 to fusion protein of from 12:1 to 1:3.

R27. A pharmaceutical composition comprising an IgG:TGFβR fusion protein, and a polysorbate 20 in a molar ratio of polysorbate 20 to fusion protein of from 8:1 to 1:2.

R28. The pharmaceutical composition of any of R1 to R27 further characterised by the features set forth in any of E1 to E5, F1 to F5, G1 to G12, H1 to H24, I1 to I6, J1 to J4, K1 to K18, L1 to L9, M1 to M19, N1 to N17, O1 to O13, P1 to P12, and Q1 to Q12.

R29. The pharmaceutical compositon of any of R1 to R28, wherein the IgG:TGFβR fusion protein is an anti-PD-L1 (IgG):TGFβR2 fusion protein, preferably having an amino acid sequence identical to the amino acid sequence of bintrafusp alfa.

R30. The pharmaceutical compositon of any of R1 to R29, further comprising a tonicifier, preferably sodium chloride.

R31. The pharmaceutical compositon of any of R1 to R30, further comprising a sugar component, preferably trehalose.

R32. The pharmaceutical compositon of any of R1 to R31, further comprising an amino acid component, preferably arginine and/or lysine.

R33. The pharmaceutical compositon of any of R1 to R32, further comprising a buffer system, preferably a histidine buffer system.

R34. The pharmaceutical compositon of any of R1 to R33, further comprising an antioxidant, preferably methionine.

*Buffers*

As buffer-free compositions appear to be less favoured than those including a buffer, pharmaceutical compositions of the invention preferably comprise a buffer system. Though a number of buffer systems may be used to buffer pharmaceutical compositions of the invention at an appropriate pH (suitably as defined herein), certain buffers appear more preferred than others. However, studies showed that buffer concentration appears less critical.

Overall, histidine buffer systems performed best. Though an acetate buffer may be used, it is suitably excluded due to potential for skin irritation. Similarly, citrate buffers may advantageously be absent from pharmaceutical compositions of the invention, though citrate buffers can be useful at higher pHs.

The following numbered paragraphs S1 to S18 disclose specific embodiments of the invention.

S1. A pharmaceutical composition comprising an IgG:TGFβR fusion protein, and a buffer system.

S2. A pharmaceutical composition comprising an IgG:TGFβR fusion protein, and a buffer system selected from the group consisting of a histidine buffer, a phosphate buffer, a succinate buffer, a citrate buffer, and any combination thereof.

S3. A pharmaceutical composition comprising an IgG:TGFβR fusion protein, and a buffer system selected from the group consisting of a histidine buffer, a phosphate buffer, a succinate buffer, and a citrate buffer.

S4. A pharmaceutical composition comprising an IgG:TGFβR fusion protein, and a histidine buffer system.

S5. The pharmaceutical composition of any of S1 to S4 wherein the buffer system is present at a concentration of 2-70 mM.

S6. The pharmaceutical composition of any of S1 to S4 wherein the buffer system is present at a concentration of 4-30 mM.

S7. The pharmaceutical composition of any of S1 to S4 wherein the buffer system is present at a concentration of 5-15 mM.

S8. The pharmaceutical composition of any of S1 to S4 wherein the buffer system is present at a molar ratio of buffer system to fusion protein of from 1280:1 to 3:1.

S9. The pharmaceutical composition of any of S1 to S4 wherein the buffer system is present at a molar ratio of buffer system to fusion protein of from 370:1 to 9:1.

S10. The pharmaceutical composition of any of S1 to S4 wherein the buffer system is present at a molar ratio of buffer system to fusion protein of from 100:1 to 15:1.

S11. The pharmaceutical composition of any of S1 to S4; wherein the composition is further characterised by an absence of acetate buffers and acetate salts.

S12. The pharmaceutical composition of any of S1 to S11 further characterised by the features set forth in any of E1 to E5, F1 to F5, G1 to G12, H1 to H24, I1 to I6, J1 to J4, K1 to K18, L1 to L9, M1 to M19, N1 to N17, O1 to O13, P1 to P12, Q1 to Q12, and R1 to R27.

S13. The pharmaceutical compositon of any of S1 to S12, wherein the IgG:TGFβR fusion protein is an anti-PD-L1 (IgG):TGFβR2 fusion protein, preferably having an amino acid sequence identical to the amino acid sequence of bintrafusp alfa.

S14. The pharmaceutical compositon of any of S1 to S13, further comprising a tonicifier, preferably sodium chloride.

S15. The pharmaceutical compositon of any of S1 to S14, further comprising a surfactant, preferably polysorbate 20.

S16. The pharmaceutical compositon of any of S1 to S15, further comprising a sugar component, preferably trehalose.

S17. The pharmaceutical compositon of any of S1 to S16, further comprising an amino acid component, preferably arginine and/or lysine.

S18. The pharmaceutical compositon of any of S1 to S17, further comprising an antioxidant, preferably methionine.

In light of the experimental studies, the following pharmaceutical compositions disclosed in T1 to T19 turned out to be particularly stable:

T1. A pharmaceutical composition comprising an IgG:TGFβR fusion protein, a buffer system, NaCl; a sugar component, a non-ionic surfactant, and optionally an amino acid antioxidant, and characterised by a pH of 5.0-6.0.

T2. The pharmaceutical composition of T1 comprising 2-30 mg/mL IgG:TGFβR fusion protein, 2-30 mM buffer system, 20-70 mM NaCl; 100-200 mM sugar component, 0.1-1.1 mg/mL non-ionic surfactant, and optionally 1-20 mM amino acid antioxidant, and characterised by a pH of 5.0-6.0.

T3. The pharmaceutical composition of T1 comprising 5-15 mg/mL IgG:TGFβR fusion protein, 5-15 mM buffer system, 30-50 mM NaCl; 150-170 mM sugar component, 0.3-0.7 mg/mL non-ionic surfactant, and optionally 2-8 mM amino acid antioxidant, and characterised by a pH of 5.3-5.7.

T4. The pharmaceutical composition of T1 comprising about 10 mg/mL IgG:TGFβR fusion protein, about 10 mM buffer system, about 40 mM NaCl; about 159 mM sugar component, about 0.5 mg/mL non-ionic surfactant, and optionally about 5 mM amino acid antioxidant, and characterised by a pH of about 5.5.

T5. The pharmaceutical composition of T1 comprising 25-100 mg/mL IgG:TGFβR fusion protein, 2-30 mM buffer system, 40-100 mM NaCl; 100-200 mM sugar component, 0.1-1.1 mg/mL non-ionic surfactant, and optionally 1-20 mM amino acid antioxidant, and characterised by a pH of 5.0-6.0.

T6. The pharmaceutical composition of T1 comprising 35-45 mg/mL IgG:TGFβR fusion protein, 5-15 mM buffer system, 50-70 mM NaCl; 150-170 mM sugar component, 0.3-0.7 mg/mL non-ionic surfactant, and optionally 2-8 mM amino acid antioxidant, and characterised by a pH of 5.3-5.7.

T7. The pharmaceutical composition of T1 comprising about 40 mg/mL IgG:TGFβR fusion protein, about 10 mM buffer system, about 60 mM NaCl; about 159 mM sugar component, about 0.5 mg/mL non-ionic surfactant, and optionally about 5 mM amino acid antioxidant, and characterised by a pH of about 5.5.

T8. The pharmaceutical composition of any of T1 to T7 wherein the IgG:TGFβR fusion protein has an amino acid sequence identical to the amino acid sequence of bintrafusp alfa, the buffer system is a histidine buffer system, the sugar component is trehalose, the non-ionic surfactant is polysorbate 20 and the amino acid antioxidant is methionine.

T9. A pharmaceutical composition comprising an IgG:TGFβR fusion protein, a buffer system, NaCl; a sugar component, an amino acid component, a non-ionic surfactant, and optionally an amino acid antioxidant, and characterised by a pH of 5.4-6.4.

T10. The pharmaceutical composition of T9 comprising 25-100 mg/mL IgG:TGFβR fusion protein, 2-30 mM buffer system, 40-100 mM NaCl; 40-160 mM sugar component, 10-100 mM amino acid component, 0.1-1.1 mg/mL non-ionic surfactant, and optionally 1-20 mM amino acid antioxidant, and characterised by a pH of 5.4-6.4.

T11. The pharmaceutical composition of T9 comprising 35-45 mg/mL IgG:TGFβR fusion protein, 5-15 mM buffer system, 50-70 mM NaCl; 90-110 mM sugar component, 40-60 mM amino acid component, 0.3-0.7 mg/mL non-ionic surfactant, and optionally 2-8 mM amino acid antioxidant, and characterised by a pH of 5.7-6.1.

T12. The pharmaceutical composition of T9 comprising about 40 mg/mL IgG:TGFβR fusion protein, about 10 mM buffer system, about 60 mM NaCl; about 100 mM sugar component, about 50 mM amino acid component, about 0.5 mg/mL non-ionic surfactant, and optionally about 5 mM amino acid antioxidant, and characterised by a pH of about 5.9.

T13. The pharmaceutical composition of T9 comprising 25-100 mg/mL IgG:TGFβR fusion protein, 2-30 mM buffer system, 40-100 mM NaCl; 25-125 mM sugar component, 25-125 mM amino acid component, 0.1-1.1 mg/mL non-ionic surfactant, and optionally 1-20 mM amino acid antioxidant, and characterised by a pH of 5.4-6.4.

T14. The pharmaceutical composition of T9 comprising 35-45 mg/mL IgG:TGFβR fusion protein, 5-15 mM buffer system, 50-70 mM NaCl; 65-85 mM sugar component, 65-85 mM amino acid component, 0.3-0.7 mg/mL non-ionic surfactant, and optionally 2-8 mM amino acid antioxidant, and characterised by a pH of 5.7-6.1.

T15. The pharmaceutical composition of T9 comprising about 40 mg/mL IgG:TGFβR fusion protein, about 10 mM buffer system, about 60 mM NaCl; about 75 mM sugar component, about 75 mM amino acid component, about 0.5 mg/mL non-ionic surfactant, and optionally about 5 mM amino acid antioxidant, and characterised by a pH of about 5.9.

T16. The pharmaceutical composition of T9 comprising 35-120 mg/mL IgG:TGFβR fusion protein, 2-30 mM buffer system, 50-150 mM NaCl; 10-100 mM sugar component, 10-100 mM amino acid component, 0.1-1.1 mg/mL non-ionic surfactant, and optionally 1-20 mM amino acid antioxidant, and characterised by a pH of 5.4-6.4.

T17. The pharmaceutical composition of T9 comprising 45-55 mg/mL IgG:TGFβR fusion protein, 5-15 mM buffer system, 90-110 mM NaCl; 40-60 mM sugar component, 40-60 mM amino acid component, 0.3-0.7 mg/mL non-ionic surfactant, and optionally 2-8 mM amino acid antioxidant, and characterised by a pH of 5.7-6.1.

T18. The pharmaceutical composition of T9 comprising about 50 mg/mL IgG:TGFβR fusion protein, about 10 mM buffer system, about 100 mM NaCl; about 50 mM sugar component, about 50 mM amino acid component, about 0.5 mg/mL non-ionic surfactant, and optionally about 5 mM amino acid antioxidant, and characterised by a pH of about 5.9.

T19. The pharmaceutical composition of any of T1 to T18 wherein the IgG:TGFβR fusion protein has an amino acid sequence identical to the amino acid sequence of bintrafusp alfa, the buffer system is a histidine buffer system, the sugar component is trehalose, the amino acid component is arginine, the non-ionic surfactant is polysorbate 20 ad the amino acid antioxidant is methionine.

Further to the above, the following embodiments U1 to U19 are particularly preferred:

U1. A pharmaceutical composition comprising an IgG:TGFβR fusion protein, an ionic tonicifier, and one or both of a disaccharide and an amino acid component.

U2. The pharmaceutical composition of U1, wherein the composition comprises a disaccharide.

U3. The pharmaceutical composition of U1 or U2, wherein the ionic tonicifier is sodium chloride.

U4. The pharmaceutical composition of any one of U1 to U3, wherein the disaccharide is trehalose or sucrose.

U5. The pharmaceutical composition of any one of U1 to U4, wherein the disaccharide is trehalose.

U6. The pharmaceutical composition of any one of U1 to U5, wherein the amino acid component is arginine or lysine.

U7. The pharmaceutical composition of any one of U1 to U6, wherein the amino acid component is arginine.

U8. The pharmaceutical composition of any one of U1 to U7, wherein the ionic tonicifier is sodium chloride, the disaccharide is trehalose and the amino acid component is arginine.

U9. The pharmaceutical composition of any one of U1 to U8, further comprising a surfactant.

U10. The pharmaceutical composition of any one of U1 to U9, wherein the composition has a pH of 5.0 to 6.5.

U11. The pharmaceutical composition of any one of U1 to U10, wherein the IgG:TGFβR fusion protein is an anti-PD-1 (IgG):TGFβR fusion protein or anti-PD-L1 (IgG):TGFβR fusion protein, preferably an anti-PD-L1 (IgG):TGFβR fusion protein.

U12. The pharmaceutical composition of U11, wherein the IgG:TGFβR fusion protein is an anti-PD-L1 (IgG):TGFβR fusion protein and the anti-PD-L1 (IgG) is selected from the group consisting of (1) an anti-PD-L1 (IgG) comprising three heavy chain CDRs having amino acid sequences of SEQ ID NO: 19 (CDR1), SEQ ID NO: 20 (CDR2) and SEQ ID NO: 21 (CDR3), and three light chain CDRs having amino acid sequences of SEQ ID NO: 22 (CDR1), SEQ ID NO: 23 (CDR2) and SEQ ID NO: 24 (CDR3), (2) an anti-PD-L1 (IgG) comprising three heavy chain CDRs having amino acid sequences of SEQ ID NO: 1 (CDR1), SEQ ID NO: 2 (CDR2) and SEQ ID NO: 3 (CDR3), and three light chain CDRs having amino acid sequences of SEQ ID NO: 4 (CDR1), SEQ ID NO: 5 (CDR2) and SEQ ID NO: 6 (CDR3), and (3) an anti-PD-L1 (IgG) comprising three heavy chain CDRs having amino acid sequences of SEQ ID NO: 27 (CDR1), SEQ ID NO: 28 (CDR2) and SEQ ID NO: 29 (CDR3), and three light chain CDRs having amino acid sequences of SEQ ID NO: 30 (CDR1), SEQ ID NO: 31 (CDR2) and SEQ ID NO: 32 (CDR3).

U13. The pharmaceutical composition of U12, wherein the light chain sequences and the heavy chain sequences of the anti-PD-L1 (IgG) respectively correspond to (1) SEQ ID NO: 7 and SEQ ID NO: 16, (2) SEQ ID NO: 15 and SEQ ID NO: 14, or (3) SEQ ID NO: 33 and SEQ ID NO: 35.

U14. The pharmaceutical composition of any one of U1 to U13, wherein the TGFβR of the IgG:TGFβR fusion protein is a soluble extracellular domain of TGFβR2 or a fragment thereof that is capable of binding TGF-β.

U15. The pharmaceutical composition of U14, wherein the TGFβR2 comprises or consists of a sequence selected from the group consisting of SEQ ID NO: 11, SEQ ID NO: 12 and SEQ ID NO: 13.

U16. The pharmaceutical composition of any one of U1 to U15, wherein the light chain sequences and the heavy chain sequences of the IgG:TGFβR fusion protein respectively correspond to (1) SEQ ID NO: 7 and SEQ ID NO: 8, (2) SEQ ID NO: 15 and SEQ ID NO: 17, (3) SEQ ID NO: 15 and SEQ ID NO: 18, or (4) SEQ ID NO: 33 and SEQ ID NO: 34.

U17. The pharmaceutical composition of any one of U1 to U16, wherein the IgG:TGFβR fusion protein has the amino acid sequence of bintrafusp alfa.

U18. The pharmaceutical composition of any one of U1 to U17, wherein the composition is charactarised by a pH of 5.0 to 6.5 and comprises a buffer system, an ionic tonicifier, a disaccharide, a non-ionic surfactant, optionally an amino acid component and further optionally an antioxidant.

U19. The pharmaceutical composition of U18, wherein the buffer system is a histidine buffer system, the ionic tonicifier is sodium chloride, the disaccharide is trehalose, the amino acid component is arginine, the antioxidant is methionine, and the non-ionic surfactant is polysorbate 20.

U19. The pharmaceutical composition of U18 or U19, wherein the composition is selected from the following group of compositions:

a. a pharmaceutical composition characterised by a pH of 5.3-5.7 and comprising 5-15 mg/mL IgG:TGFβR fusion protein, 5-15 mM buffer system, 30-50 mM ionic tonicifier; 150-170 mM disaccharide, 0.3-0.7 mg/mL non-ionic surfactant, and optionally 2-8 mM antioxidant;

b. a pharmaceutical composition characterised by a pH of 5.3-5.7 and comprising 35-45 mg/mL IgG:TGFβR fusion protein, 5-15 mM buffer system, 50-70 mM ionic tonicifier; 150-170 mM disaccharide, 0.3-0.7 mg/mL non-ionic surfactant, and optionally 2-8 mM antioxidant;

c. a pharmaceutical composition characterised by a pH of 5.7-6.1 and comprising 35-45 mg/mL IgG:TGFβR fusion protein, 5-15 mM buffer system, 50-70 mM ionic tonicifier; 90-110 mM disaccharide, 40-60 mM amino acid component, 0.3-0.7 mg/mL non-ionic surfactant, and optionally 2-8 mM antioxidant;

d. a pharmaceutical composition characterised by a pH of 5.7-6.1 and comprising 35-45 mg/mL IgG:TGFβR fusion protein, 5-15 mM buffer system, 50-70 mM ionic tonicifier; 65-85 mM disaccharide, 65-85 mM amino acid component, 0.3-0.7 mg/mL non-ionic surfactant, and optionally 2-8 mM antioxidant; and

e. a pharmaceutical composition characterised by a pH of 5.7-6.1 and comprising 45-55 mg/mL IgG:TGFβR fusion protein, 5-15 mM buffer system, 90-110 mM ionic tonicifier; 40-60 mM disaccharide, 40-60 mM amino acid component, 0.3-0.7 mg/mL non-ionic surfactant, and optionally 2-8 mM antioxidant.

## CONTAINER

The present invention provides a container comprising a pharmaceutical composition as defined herein. Preferably the container comprises a drug delivery device as defined herein, preferably a plurality of drug delivery devices.
The present invention provides a method of manufacturing a container, the method comprising incorporating a pharmaceutical composition as defined herein within a container. Preferably this is achieved by incorporating said pharmaceutical composition within one or more drug delivery devices, and thereafter incorporating the one or more pre-filled drug delivery devices into a container.

## DRUG-DELIVERY DEVICE

The present invention provides a drug delivery device comprising a pharmaceutical composition as defined herein. Preferably the drug delivery device comprises a chamber within which the, preferably liquid (most preferably aqueous), pharmaceutical composition resides. Preferably the drug delivery device is sterile.
The drug delivery device may be a vial, ampoule, syringe, injection pen (e.g. essentially incorporating a syringe), autoinjector, or intravenous bag. Most preferably the drug delivery device is a syringe. Preferably the syringe is a glass syringe. Preferably the syringe comprises a needle.
The present invention provides a method of manufacturing a drug delivery device, preferably as defined herein, the method comprising incorporating a pharmaceutical composition as defined herein within a drug delivery device. Such manufacture typically involves charging the pharmaceutical composition as defined herein to a syringe, preferably via a needle affixed thereto. The needle may thereafter be removed, replaced, or remain.

## KIT OF PARTS

The present invention provides a kit of parts comprising a drug delivery device (without the pharmaceutical composition incorporated therein), a pharmaceutical composition as defined herein (optionally contained in a separate package or container), and optionally a set of instructions with directions regarding the administration (e.g. intravenous, sub-cutaneous) of the pharmaceutical composition. The user may then fill the drug delivery device with the pharmaceutical composition (which may be provided in a vial or ampoule or such like) prior to administration.

## METHOD OF MANUFACTURE

The present invention provides a method of manufacturing a pharmaceutical composition as defined herein.

The method preferably comprises mixing together, in any particular order deemed appropriate, any relevant components required to form a composition as defined herein. The skilled person may refer to the Examples or techniques well known in the art for forming pharmaceutical compositions (especially those for injection *via* syringe, especially those for intravenous injection). Different embodiments will preferably require different combinations of components to be mixed, potentially in different amounts. The skilled person can readily deduce such combinations and amounts by reference to the foregoing disclosure relating to the pharmaceutical composition.

Preferably the method involves mixing together the relevant components, preferably in a diluent (e.g. water), preferably so that all of the components are (substantially or entirely) dissolved in the diluent.

The method may involve first preparing a concentrated pre-mixture (or pre-solution) of some or all components (optionally with some or all of the diluent) including the fusion protein, and the and the pre-mixture (or pre-solution) may then be diluted with diluent, preferably water, to afford the pharmaceutical composition, or a composition to which final components are then added to furnish the final pharmaceutical composition. Preferably the aforementioned pre-mixture is prepared with the desired pH for the final formulation.

The method may alternatively or in addition involve first preparing a pre-mixture (or pre-solution) of some or all components (optionally with some or all of the diluent) excluding the fusion protein, and the fusion protein may then itself (optionally with or pre-dissolved in some of the diluent) be mixed with the pre-mixture (or pre-solution) to afford the pharmaceutical composition, or a composition to which final components are then added to furnish the final pharmaceutical composition. Preferably, the pre-mixture contains all components except for the fusion protein and optionally also some diluent (which may be used to pre-dissolve the fusion protein), preferably so that the fusion protein is added to a mixture which offers optimal stabilisation of the fusion protein. Preferably the aforementioned pre-mixture is prepared with the desired pH for the final formulation.

Preferably, the method involves forming a buffer system, preferably a buffer system comprising a buffering agent as defined herein. The buffer system may preferably have a pH as defined herein in relation to the buffer system *per se.* The buffer system is preferably formed in a pre-mixture prior to the addition of the fusion protein, though the buffer system may optionally be formed with the fusion protein present. The buffer system may be formed through simply mixing the buffering agent (supplied ready-made) with its acid/base conjugate (preferably in appropriate relative quantities to provide the desired pH - this can be determined by the skilled person either theoretically or experimentally). In the case of an acetate buffer system, this means mixing sodium acetate with acetic acid. Alternatively, the buffer system may be formed through adding a strong acid (e.g. HCl) to the buffering agent (e.g. sodium acetate) in order to form *in situ* the acid/base conjugate (e.g. acetic acid) (again preferably in appropriate relative quantities to provide the desired pH). Alternatively, the buffer system may be formed through adding a strong base (e.g. sodium hydroxide) to the acid/base conjugate (e.g. acetic acid) of the buffering agent (e.g. sodium acetate) in order to form *in situ* the buffering agent (again preferably in appropriate relative quantities to provide the desired pH). The pH of either the pre-mixture of final composition may be judiciously adjusted by adding the required quantity of strong base or strong acid, or even a quantity of buffering agent or acid/base conjugate. The same protocols may be used in relation to other buffer systems, such as histidine buffer systems, which may be formed using a combination of free (neutral) histidine with the imidazolium form of histidine, or using either an adjusting the pH accordingly (e.g. with base or acid as required).

In certain embodiments, the buffering agent and/or buffer system is pre-formed as a separate mixture, and the buffer system is transferred to a precursor of the composition (comprising some or all components save for the buffering agent and/or buffer system, preferably comprising the fusion protein) *via* buffer exchange (e.g. using diafiltration until the relevant concentrations or osmolality is reached). Additional excipients may be added thereafter if necessary in order to produce the final composition. The pH may be adjusted once or before all the components are present.

Any, some, or all components may be pre-dissolved or pre-mixed with a diluent prior to mixing with other components.

The final composition may be filtered, preferably to remove particulate matter. Preferably filtration is through filters sized at or below 1 $\mu$m, preferably at 0.22$\mu$m. Preferably, filtration is through either PES filters or PVDF filters, preferably with 0.22 $\mu$m PES filters.

It will be appreciated that pharmaceutical compositions of the invention may be provided, and preferably duly stored, as a liquid (preferably aqueous) pharmaceutical composition. However, the pharmaceutical compositions of the invention may be provided, and preferably duly stored, as a lyophilised pharmaceutical composition. Such a lyophilised pharmaceutical composition may be formed by freeze-drying a liquid (e.g. aqueous) pharmaceutical composition as defined herein. However, most preferably any such lyophilised pharmaceutical compositions will be reconstituted prior to use, administration, or even (potentially short-term) storage to afford a liquid or aqueous pharmaceutical composition as defined herein. Such reconstitution preferably comprises dissolving the lyophilised composition in water (for injection), preferably to afford a solution of a desired concentration of the fusion protein.

## METHOD OF TREATMENT

The present invention provides methods of treating a disease or medical disorder by administering a pharmaceutical composition as defined herein. Such methods preferably comprise administering, to a subject in need thereof, a therapeutically effective amount of the pharmaceutical composition. Administering may preferably comprise parenteral administration, which preferably includes any form of administration other than enteral and topical administration, usually by injection, and preferably includes, without limitation, intravenous, intravitreal, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion. Such methods preferably comprise subcutaneous or intravenous delivery of a therapeutically effective amount of the pharmaceutical composition. Such methods preferably comprise intravenously-administering a therapeutically effective amount of the pharmaceutical composition, preferably *via* intravenous injection, preferably along with other intravenous fluids (e.g. saline).

Where the pharmaceutical composition is a lyophilised pharmaceutical composition, prior to administration said composition is preferably reconstituted in water for injection or an appropriate intravenous fluid, preferably to afford an aqueous pharmaceutical composition as defined herein. The aqueous pharmaceutical composition is then preferably administered as defined herein, preferably *via* intravenous injection, preferably alongside other intravenous fluids (e.g. saline).

The present invention provides a pharmaceutical composition as defined herein, for use in treating a disease or medical disorder in a patient in need of such treatment. The present invention also provides a use of a pharmaceutical composition as defined herein, in the manufacture of a medicament for the treatment of a disease or disorder.

The present invention provides a method of treating a disease or medical disorder, a pharmaceutical composition for use in treating a disease or medical disorder, and a use of a pharmaceutical composition in the manufacture of a medicament for a treatment of a disease or disorder as defined herein, wherein the disease or medical disorder is a PD-L1- and/or TGFβ-related disease, preferably a PD-L1- and/or TGFβ-related proliferative disease, more preferably a PD-L1- and/or TGFβ-related cancer. In such aspects of the invention, an anti-PD-L1 antigen-binding portion of an anti-LD-L1(IgG):TGFβR fusion protein may bind a tumour borne PD-L1, thereby disrupting the PD-1 / PD-L1 pathway whilst localising the TGFβ receptor portion of the fusion protein within the tumour microenvironment where it can most-effectively target and/or sequester autocrine or pancrine TGFβ. In such a manner, multiple mechanisms of action are disrupted to thereby block growth of and/or survival pathways for cancer cells.

The present invention provides a method of treating a disease or medical disorder, a pharmaceutical composition for use in treating a disease or medical disorder, and a use of a pharmaceutical composition in the manufacture of a medicament for a treatment of a disease or disorder as defined herein, wherein the disease or medical disorder is a proliferative disease or disorder. The proliferative disease or disorder is preferably cancer. The cancer is preferably manifest in one or more solid tumours. The cancer is preferably selected from carcinoma, lymphoma, leukemia, blastoma, and sarcoma. More particular examples of such cancers include squamous cell carcinoma, myeloma, small-cell lung cancer, non-small cell lung cancer, glioma, Hodgkin's lymphoma, non-Hodgkin's lymphoma, acute myeloid leukemia, multiple myeloma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma, cervical cancer, brain cancer, stomach cancer, bladder cancer, hepatoma, breast cancer, colon carcinoma, biliary tract cancer, and head and neck cancer. The disease or medical disorder in question may preferably be selected from any of those disclosed in WO2015118175, WO2018029367, WO2018208720, PCT/US18/12604, PCT/US19/47734, PCT/US19/40129, PCT/US19/36725, PCT/US19/732271, PCT/US19/38600, and PCT/EP2019/061558.

The present invention provides a method of treating a disease or medical disorder, a pharmaceutical composition for use in treating a disease or medical disorder, and a use of a pharmaceutical composition in the manufacture of a medicament for a treatment of a disease or disorder as defined herein, wherein the disease or medical disorder is preferably as defined herein, and wherein the treating or treatment involves combination therapy whereby the pharmaceutical composition is administered in combination with one or more other pharmaceutical or biopharmaceutical active(s); wherein said combination therapy involves simultaneous, sequential or separate dosing of the individual components of the treatment. In some embodiments, the additional pharmaceutical or biopharmaceutical active may be present within any of the pharmaceutical compositions as defined herein.

All the references cited herein are incorporated by reference in the disclosure of the invention hereby.

## EXAMPLES

### Anti-PD-L1 biological activity assay protocol

The biological activity of the anti-PD-L1 (IgG):TGFβRII fusion protein was evaluated through a cell-based assay able to measure its capability to bind the human PD-L1 receptor over-expressed on a recombinant HEK-293 (hPD-L1) cell line. To this end, the anti-PD-L1 (IgG):TGFβRII fusion protein was allowed to bind to a protein A-coated 96-well plate for 30 minutes at room temperature under swirling. Then, 50,000 HEK-293 (hPD-L1) cells were added per well of the anti-PD-L1 (IgG):TGFβRII coated plate and allowed to bind to anti-PD-L1 (IgG):TGFβRII for 1 hour at 37°C, 5% CO2. The unbound cells were washed away and presence of the bound cells was confirmed in each well by the ATPlite 1step (Perkin Elmer). Counts per seconds were then plotted against the Log transformed anti-PD-L1 (IgG):TGFβRII concentration and fitted by 4PL (sigmoidal dose response curve). For each data set, the concentration of anti-PD-L1 (IgG):TGFβRII able to bind at 50% of the maximum possible PDL-1 binding capacity (EC50) is calculated. The biological activity of a sample is expressed as % activity with respect to reference material and is the percentage expression of the potency ratio (i.e., the EC50 ratio of equipotent doses of standard and unknown preparations). The potency is the average result coming from three independent assays.

### TGFβRII biological activity assay protocol

The TGFβ-binding activity of the anti-PD-L1 (IgG):TGFβRII fusion protein was evaluated with the reporter cell line 4T1 pSmad sLuc, which is stably transfected with a luciferase reporter gene that is controlled by SMAD binding elements. Upon binding of TGFβ to the TGFβ receptors on the cell surface of the cell line, a signalling cascade is initiated that results in the formation of the SMAD complex. The SMAD complex then translocates into the nucleus where it binds to the SMAD binding elements and induces transcription of the luciferase gene.

For each bioassay, 50'000 4T1 cells/well were loaded in a 96-well plate and allowed to adhere to the plate for 4 hours at 37°C, 5% CO2. Then, the plate was incubated overnight at 37°C, 5% CO2 with different concentrations of the anti-PD-L1 (IgG):TGFβRII fusion protein and a fixed amount of human TGFβ to be able to generate a dose-response curve. Luciferase expression was evaluated thereafter by using a luciferin-based detection dye. The emitted light is inversely correlated with the TGFβ-binding activity of the anti-PD-L1 (IgG):TGFβRII fusion protein, as the anti-PD-L1 (IgG):TGFβRII fusion protein traps and neutralizes soluble TGFβ.

For each data set, the concentration of anti-PD-L1 (IgG):TGFβRII able to bind at 50% of the maximum possible TGFβ binding capacity (EC50) is calculated. The biological activity of a sample is expressed as % activity with respect to reference material and is the percentage expression of the potency ratio (i.e., the EC50 ratio of equipotent doses of standard and unknown preparations). The potency is the average result coming from three independent assays.

### Stress Testing Protocols

The following stress testing protocols were used in the examples that follow, albeit variations therefrom will be apparent from the context.

Thermal stability

The thermal stability of the formulations was examined over a period of time (e.g. 8, 9 or 13 weeks) of storage at $40 \pm 2°C$ (75% R.H.). Not only were samples analysed at Time = 0 and Time = Whole Period (e.g. 8 weeks), but also at intermediate intervals (e.g. Time = 2 weeks, Time = 4 weeks). Sometimes, back-up samples were placed at $25 \pm 5°C$ for similar periods to allow analysis of samples which at $40 \pm 2°C$ degraded too quickly or otherwise yielded unusual results. The thermal stability samples were generally analyzed for the following:

- Protein content by optical density

- Aggregation index: calculated by optical density to track aggregation and formation of HMW impurities

- HMW content by SE-UPLC (to track HMW impurity generation and hence monitor aggregations)

- Turbidity by nephelometry (also related to solubility and aggregate formation)

- Visual inspection for presence of visible particles and/or changes in degree of colouration

- Charge isoforms by iso-electric capillary electrophoresis (to monitor charge variants)

- pH

- LMW content by bioanalyzer or CGE-NR (to track fragmentation)

- Sub-visible particles by light obscuration

- Fluorescence (selected samples) to monitor changes in the tertiary structure of the molecule

Light stress

Photosensitivity of the IgG:TGFβR2 fusion protein in various formulations was also investigated. Formulations were generally exposed to 7 hours of light at an intensity of 765 W/m$^2$, which satisfies ICHQ1B guideline requirements. The light-stressed formulations were generally analyzed by the following techniques:

- Protein content by optical density

- Aggregation Index: calculated by OD, measures the extent of aggregate formation which results from light stress

- Turbidity measured by nephelometry

- Visual Inspection: for presence of visible particles resulting from aggregation

- SE-UPLC: quantitation of HMW impurities resulting from aggregation

- Charge isoforms by iso-electric capillary electrophoresis (to monitor charge variants)

- Fluorescence (selected samples): to observe any change in tertiary structure which could arise from oxidation of aromatic amino acid side chains

Shaking stress

Mechanical (shaking) stress is often associated with a production of aggregates due to protein self-association and interaction among hydrophobic regions of the protein in solution. Various formulations of the IgG:TGFβR2 fusion protein were examined for resistance to shaking stress, typically as 200mg/vial and 600mg/vial stirred at 200rpm (or 300 rpm) at room temperature for 24 hours in both upright and oblique positions. The shaking stress formulations were generally analyzed as follows:

- Protein content by optical density

- Aggregation index: calculated by optical density to track aggregation and formation of HMW impurities

- Turbidity by nephelometry (also related to solubility and aggregate formation)

- Visual inspection for presence of visual particles and/or degree of colouration

- HMW content by SE-UPLC (to track HMW impurity generation and hence monitor aggregations)

- LMW content by bioanalyzer or CGE-NR (to track fragmentation)

- Sub-visible particles by light obscuration

- pH

Freeze/thaw stress

As a protein formulation freezes, an interface is formed as micro-regions within the solution begin solidify. In these micro-environments there is a change in polarity as different component of the formulation buffer are excluded or included from the liquid matrix that is solidifying. What results is precipitation of protein as hydrophilic/hydrophobic interactions are forced upon the molecules in these changing micro-environments. To ascertain the effectiveness of the various excipients and conditions, various formulations of the IgG:TGFβR2 fusion protein were exposed to three cycles of freeze-thawing (each cycle from -25°C to RT), typically as 200mg/vial or 600mg/vial samples. The samples were then generally examined by the following analyses to determine their resistance to precipitation/aggregation/degradation by freeze-thawing:

- Protein content by optical density

- Aggregation index: calculated by optical density to track aggregation and formation of HMW impurities

- Charge isoforms by iso-electric capillary electrophoresis (to monitor charge variants)

- Turbidity by nephelometry (also related to solubility and aggregate formation)

- Visual inspection for presence of visual particles

- HMW content by SE-UPLC (to track HMW impurity generation and hence monitor aggregations)

- LMW content by bioanalyzer or CGE-NR (to track fragmentation)

- Sub-visible particles

- pH

### Results Analysis and Data Handling Protocols

Some DoEs were set up using the Response Surface Model option in Design-Expert software, selecting the D-Optimal Design with three numeric factors (buffer strength, pH, surfactant strength) and one categorical factor (excipient type). To ascertain whether there is a positive response to a given stress the data was statistically evaluated by ANOVA using a Response Surface Linear Model. Design expert software was utilized to explore the relationship between all the variables in the DoE and the results were illustrated in 3D surface plots to illustrate the significant contributing factors in response to a given stress. This way a statistically significant link can be established between a given factor and the response to stress.

### Formulation Preparation Protocols

Bintrafusp alfa drug substance (an example of an IgG:TGFβR2 fusion protein) can be manufactured as set forth in WO2015118175, the Examples of which describe its production. The full heavy chain of the IgG connected to TGFβR2 extracellular domain *via* a linker is given as SEQ NO. 3. The TGFβR2 extracellular domain corresponds with SEQ NO. 10 whereas the linker corresponds with SEQ NO. 11. The light chain of bintrafusp alfa corresponds to SEQ NO. 1 of WO2015118175.

FIG. 1 shows one half of the bintrafusp alfa fusion protein, containing a light chain (with designated $V_L$ and $C_L$ regions) and a heavy chain (with designated $V_H$, $C_H1$, $C_H2$, and $C_H3$ regions) connected *via* the designated linker to the extracellular domain (ECD) of a TGFβ Receptor II.

Purified bintrafusp alfa was stored in a pre-formulated state at -70°C, and thawed prior to use.

In one method, aqueous formulations were prepared by mixing a pre-prepared excipient-containing solution (at a designated pH where applicable) with an appropriate weighted amount of bintrafusp alfa to furnish a desired formulation in terms of concentrations of bintrafusp alfa and excipients. A final pH adjustment is made in some cases to ensure the pH is as desired. In other methods, aqueous formulations may be produced by dissolving or mixing bintrafusp alfa in water, optionally containing some excipients, and then adding further excipients (optionally pre-dissolved in water) to furnish the final desired formulation - again final pH adjustments may be made.

In some methods, stock solutions of bintrafusp alfa were produced (e.g. via the above methods) and potentially stored at reduced temperature prior to use / processing. Such stock solutions could then be processed, to furnish the desired formulations, for example by performing buffer exchanges, concentrations, dilutions, etc.

In the Examples that follow, it would be self-evident to the skilled person how the relevant formulations are produced.

### EXAMPLE 1 -Lyophilised and Liquid formulations of an IgG:TGFβR2 fusion protein

### General Overview

The preparation of a stable formulation for the IgG:TGFβR2 fusion protein is particularly challenging, in part due to the two fused entities, the IgG portion and the TGFβR2 portion, being a bad fit regarding pI and colloidal stability - the pI of the TGFβR2 region is radically different to that of the IgG portion, and yet it is generally recognised as important to adopt a pH that is at least 1-2 pH unit away from the pI of each major protein moiety.

*List of Tested Formulations*

An initial screen was performed to establish viable pHs and optimal pHs, which focused on colloidal and conformational stability. Formulations for this initial screen are shown in Table 1A.

**Table 1A** - Formulations for initial pH screen

| Formulation | bintrafusp alfa | pH | Excipients |
|---|---|---|---|
| 1A.1 | 5 mg/mL | 4.0 | Buffer*, 200 mM NaCl |
| 1A.2 | 5 mg/mL | 5.5 | Buffer*, 75 mM NaCl |
| 1A.3 | 5 mg/mL | 5.0 | Buffer*, 200 mM NaCl |
| 1A.4 | 5 mg/mL | 5.5 | Buffer*, 200 mM NaCl |
| 1A.5 | 5 mg/mL | 6.0 | Buffer*, 200 mM NaCl |
| 1A.6 | 5 mg/mL | 7.0 | Buffer*, 200 mM NaCl |
| 1A.7 | 5 mg/mL | 8.0 | Buffer*, 200 mM NaCl |
| 1A.8 | 5 mg/mL | 9.0 | Buffer*, 200 mM NaCl |
| *the buffer used in this example was a polybuffer system comprising 10 mM sodium phosphate, 10 mM TRIS and 10 mM citrate | | | |

Further experiments were performed to establish the impact of sodium chloride on solubility and dialysis recovery. These formulations are shown in Table 1B.

**Table 1B** Formulations for initial sodium chloride and solubility screen

| Formulation | bintrafusp alfa | pH | Excipients |
|---|---|---|---|
| 1B.1 | 1.5 mg/mL | 5.5 | - |
| 1B.2 | 2.5 mg/mL | 5.5 | - |
| 1B.3 | 5 mg/mL | 5.5 | - |
| 1B.4 | 10 mg/mL | 5.5 | - |
| 1B.5 | 1.5 mg/mL | 7.0 | - |
| 1B.6 | 2.5 mg/mL | 7.0 | - |
| 1B.7 | 5 mg/mL | 7.0 | - |
| 1B.8 | 10 mg/mL | 7.0 | - |
| 1B.9 | 10 mg/mL | 5.5 | 0 mM NaCl |
| 1B.10 | 10 mg/mL | 5.5 | 40 mM NaCl |
| 1B.11 | 10 mg/mL | 5.5 | 50 mM NaCl |
| 1B.12 | 10 mg/mL | 5.5 | 60 mM NaCl |
| 1B.13 | 10 mg/mL | 5.5 | 75 mM NaCl |
| 1B.14 | 10 mg/mL | 7.0 | 0 mM NaCl |
| 1B.15 | 10 mg/mL | 7.0 | 40 mM NaCl |
| 1B.16 | 10 mg/mL | 7.0 | 50 mM NaCl |
| 1B.17 | 10 mg/mL | 7.0 | 60 mM NaCl |

(continued)

| Formulation | bintrafusp alfa | pH | Excipients |
|---|---|---|---|
| 1B.18 | 10 mg/mL | 7.0 | 160 mM NaCl |

A further screen for freeze-dryability and osmolality was then performed upon 18 formulations, of pH 5.5, containing various concentrations of sodium chloride and trehalose (wt% amounts of trehalose relate to wt% trehalose dihydrate) in order to assess lyophilisation performance. The API was excluded from these formulations because it has little impact on the factors (appearance of lyophilizates) under observation owing to its negligible impact on osmolality. Such placebo formulations can thus be regarded as a worst case regarding the appearance of lyophilizates. These formulations are shown in Table 1C.

Table 1C - Formulations of sodium chloride and lyoprotectant for freeze-dryability and osmolality screen

| Formulation | pH | NaCl | Trehalose |
|---|---|---|---|
| 1C.1 | 5.5 | 160 mM | 8 wt% |
| 1C.2 | 5.5 | 80 mM | 8 wt% |
| 1C.3 | 5.5 | 40 mM | 8 wt% |
| 1C.4 | 5.5 | 20 mM | 8 wt% |
| 1C.5 | 5.5 | 10 mM | 8 wt% |
| 1C.6 | 5.5 | 0 mM | 8 wt% |
| 1C.7 | 5.5 | 160 mM | 4 wt% |
| 1C.8 | 5.5 | 80 mM | 4 wt% |
| 1C.9 | 5.5 | 40 mM | 4 wt% |
| 1C.10 | 5.5 | 20 mM | 4 wt% |
| 1C.11 | 5.5 | 10 mM | 4 wt% |
| 1C.12 | 5.5 | 0 mM | 4 wt% |
| 1C.13 | 5.5 | 160 mM | 2 wt% |
| 1C.14 | 5.5 | 80 mM | 2 wt% |
| 1C.15 | 5.5 | 40 mM | 2 wt% |
| 1C.16 | 5.5 | 20 mM | 2 wt% |
| 1C.17 | 5.5 | 10 mM | 2 wt% |
| 1C.18 | 5.5 | 0 mM | 2 wt% |

A further screen for freeze-dryability and osmolality was then performed upon 6 substantially isotonic formulations, of pH 5.5, containing refined concentrations of sodium chloride and trehalose. These formulations are shown in Table 1D. Again, as per Table 1C the API was excluded for the same reasons.

Table 1D - Further Formulations of sodium chloride and lyoprotectant for freeze-dryability and osmolality screens

| Formulation | pH | NaCl | Trehalose | Osmolality |
|---|---|---|---|---|
| 1D.1 | 5.5 | 40 mM | 5.5 wt% | 264 mOsm/kg |
| 1D.2 | 5.5 | 40 mM | 6.0 wt% | 281 mOsm/kg |
| 1D.3 | 5.5 | 50 mM | 5.0 wt% | 269 mOsm/kg |
| 1D.4 | 5.5 | 50 mM | 5.8 wt% | 295 mOsm/kg |
| 1D.5 | 5.5 | 60 mM | 4.4 wt% | 268 mOsm/kg |
| 1D.6 | 5.5 | 60 mM | 5.5 wt% | 306 mOsm/kg |

A further screen was then performed upon 2 substantially isotonic formulations, of pH 5.5, containing 10 mg/mL bintrafusp alfa and refined concentrations of sodium chloride and trehalose. These formulations are shown in Table 1E.

**Table 1E** - Formulations for quick stability screen

| Formulation | bintrafusp alfa | pH | L-histidine buffer | Methionine | NaCl | Trehalose | Tween 20 |
|---|---|---|---|---|---|---|---|
| 1E.1 | 10 mg/mL | 5.5 | 10 mM | 5 mM | 40 mM | 6.0 wt% | 0.05 wt% |
| 1E.2 | 10 mg/mL | 5.5 | 10 mM | 5 mM | 75 mM | 4.0 wt% | 0.05 wt% |

*1A Formulations - Results and Analysis*

Nano-DSC measurements performed upon Formulations 1A.1-1A.8 (see Table 1A above) to measure the conformational stability of these formulations and the results are presented in Table 1F below.

**Table 1F** - DSC results showing protein unfolding events at $T_m1$ and $T_m2$ temperature peaks

| Formulation | Conditions | $T_M1$ (°C) | $T_M2$ (°C) |
|---|---|---|---|
| 1A.1 | pH 4.0 | 46.8 | 62.4 |
| 1A.2 | pH 5.5 + 75 mM NaCl | 66.8 | 68.5 |
| 1A.3 | pH 5.0 | 61.0 | 69.6 |
| 1A.4 | pH 5.5 | 65.8 | 71.7 |
| 1A.5 | pH 6.0 | 66.2 | 69.7 |
| 1A.6 | pH 7.0 | 67.6 | 71.4 |
| 1A.7 | pH 8.0 | 66.2 | 71.3 |
| 1A.8 | pH 9.0 | 65.6 | 70.8 |

The $T_m$ measurements above suggest that, from a conformational stability standpoint, optimal pHs lie between 5.5 and 9. Furthermore, also from a conformational stability standpoint, the addition of NaCl is not detrimental but does not in itself improve conformational stability. At pHs 4 and 5, without any NaCl $T_m$ values were slightly lower, suggesting a decrease in conformational stability.

*1B Formulations - Results and Analysis*

The formulations of Table 1B above (Formulations 1B.1-1B.18) were subjected to a solubility assay in which % protein recovery was measured following reformulation / rebuffering in order to determine the colloidal stability of these formulations. The results are presented in Table 1G below.

**Table 1G** - Solubility assay: Results showing protein recovery after reformulation/rebuffering

| Formulation | bintrafusp alfa (conc. before dialysis) | pH | Excipients | % Protein Recovery |
|---|---|---|---|---|
| 1B.1 | 1.5 mg/mL | 5.5 | - | 57.7% |
| 1B.2 | 2.5 mg/mL | 5.5 | - | 71.3% |
| 1B.3 | 5 mg/mL | 5.5 | - | 73.1% |
| 1B.4 | 10 mg/mL | 5.5 | - | 76.9% |
| 1B.5 | 1.5 mg/mL | 7.0 | - | 14.8% |
| 1B.6 | 2.5 mg/mL | 7.0 | - | 14.6% |
| 1B.7 | 5 mg/mL | 7.0 | - | 13.4% |
| 1B.8 | 10 mg/mL | 7.0 | - | 0 |
| 1B.9 | 10 mg/mL | 5.5 | 0 mM NaCl | 45.0% |

(continued)

| Formulation | bintrafusp alfa (conc. before dialysis) | pH | Excipients | % Protein Recovery |
|---|---|---|---|---|
| 1B.10 | 10 mg/mL | 5.5 | 40 mM NaCl | 79.5% |
| 1B.11 | 10 mg/mL | 5.5 | 50 mM NaCl | 73.2% |
| 1B.12 | 10 mg/mL | 5.5 | 60 mM NaCl | 77.2% |
| 1B.13 | 10 mg/mL | 5.5 | 75 mM NaCl | 79.3% |
| 1B.14 | 10 mg/mL | 7.0 | 0 mM NaCl | 4.0% |
| 1B.15 | 10 mg/mL | 7.0 | 40 mM NaCl | 78.0% |
| 1B.16 | 10 mg/mL | 7.0 | 50 mM NaCl | 77.9% |
| 1B.17 | 10 mg/mL | 7.0 | 60 mM NaCl | 73.9% |
| 1B.18 | 10 mg/mL | 7.0 | 160 mM NaCl | 76.2% |

Overall, pH5.5 showed a better solubility profile than pH7 at concentrations of 1 - 10 mg/mL. However, NaCl had a marked impact on the % recovery of protein - this was particularly pronounced at higher pHs.

*1C Formulations - Results and Analysis*

The formulations of Table 1C (Formulations 1C.1 to 1C.18), all of which had a pH of 5.5 and contained 10 mg/mL bintrafusp alfa, were tested in the context of freeze-drying (*via* standard freeze-thaw cycles), and also in terms of osmolality (using the freezing-point depression method commonly deployed in the pharmaceutical industry) when constituted at 10 mg/mL bintrafusp alfa.

The results are shown in Table 1H below.

**Table 1H** - Freeze-dryability and osmolality assays

| Formulation | NaCl | Trehalose | Osmolality (mOsm/kg)[†] | Appearance upon freeze-drying [‡] |
|---|---|---|---|---|
| 1C.1 | 160 mM | 8 wt% | 570 | 2 |
| 1C.2 | 80 mM | 8 wt% | 418 | 2 |
| 1C.3 | 40 mM | 8 wt% | 350 | 1 |
| 1C.4 | 20 mM | 8 wt% | 313 | 1 |
| 1C.5 | 10 mM | 8 wt% | 291 | 1 |
| 1C.6 | 0 mM | 8 wt% | 271 | 1 |
| 1C.7 | 160 mM | 4 wt% | 449 | 5 |
| 1C.8 | 80 mM | 4 wt% | 304 | 2 |
| 1C.9 | 40 mM | 4 wt% | 224 | 1 |
| 1C.10 | 20 mM | 4 wt% | 191 | 1 |
| 1C.11 | 10 mM | 4 wt% | 166 | 1 |
| 1C.12 | 0 mM | 4 wt% | 145 | 1 |
| 1C.13 | 160 mM | 2 wt% | 385 | 5 |
| 1C.14 | 80 mM | 2 wt% | 236 | 5 |
| 1C.15 | 40 mM | 2 wt% | 162 | 3 |
| 1C.16 | 20 mM | 2 wt% | 126 | 1 |
| 1C.17 | 10 mM | 2 wt% | 105 | 1 |

(continued)

| Formulation | NaCl | Trehalose | Osmolality (mOsm/kg)† | Appearance upon freeze-drying ‡ |
|---|---|---|---|---|
| 1C.18 | 0 mM | 2 wt% | 84 | 1 |

† - This was the osmolality of a formulation prior to bintrafusp alfa being added thereto.

‡ - Appearance is on a scale of 1-5 (good to bad), where: 1 = completely intact lyophilizate; 2 - slight shrinkage in diameter on bottom (excess of Tg'); 3 - more pronounced shrinkage in diameter on bottom (excess of Tg'); 4 - collapse at the bottom (potentially incomplete primary drying); 5- total collapse.

FIG. 2 shows a graphical representation of the relationship between Tg' (in °C), as determined by nano-differential-scanning calorimetry, and NaCl concentration for formulations containing 8% trehalose (diamonds), 4% trehalose (squares), and 2% trehalose (triangles). It is undesirable for Tg' values to be below -40°C from the point of view of practicalities when freeze drying.

The data indicates, from the perspective of balancing freeze-drying viability and osmolality (i.e. substantially isotonic formulations), there is a promising window around 40 mM NaCl. Moreover, a trehalose concentration between 4% and 6% would appear to be desirable from the perspective of freeze-drying. More generally, a molar ratio of tonicifying lyoprotectant to ionic tonicifier of 10:1 to 1:2, more preferably of 5:1 to 2:1, appears to be particularly suitable in terms of freeze-dryability.

*1D Formulations - Results and Analysis*

Following on from the experiments with Formulations 1A-1C, a formulation window was identified that provided a favourable balance in terms of isotonicity, solubility, and freeze-dryability. The formulations of Table 1D (Formulations 1D.1-1D.6), all of which had a pH of 5.5 and contained 10 mg/mL bintrafusp alfa, were tested in the same manner as Formulations 1C, for freeze-dryability and osmolality, with the results given in Table 1I below.

**Table 1I** - Further Freeze-dryability and osmolality assays (fine tuning)

| Formulation | NaCl | Trehalose | Osmolality (mOsm/kg)† | Appearance upon freeze-drying ‡ |
|---|---|---|---|---|
| 1D.1 | 40 mM | 5.5 wt% | 264 mOsm/kg | 1 |
| 1D.2 | 40 mM | 6.0 wt% | 281 mOsm/kg | 1 |
| 1D.3 | 50 mM | 5.0 wt% | 269 mOsm/kg | 1 |
| 1D.4 | 50 mM | 5.8 wt% | 295 mOsm/kg | 1 |
| 1D.5 | 60 mM | 4.4 wt% | 268 mOsm/kg | 2 |
| 1D.6 | 60 mM | 5.5 wt% | 306 mOsm/kg | 2 |

† - This was the osmolality of a formulation prior to bintrafusp alfa being added thereto.

‡ - Appearance is on a scale of 1-5 (good to bad), where: 1 = completely intact lyophilizate; 2 - slight shrinkage in diameter on bottom (excess of Tg'); 3 - more pronounced shrinkage in diameter on bottom (excess of Tg'); 4 - collapse at the bottom (potentially incomplete primary drying); 5- total collapse.

The results suggest formulations with around 40mM NaCl, or 20-50 mM NaCl were optimal for 10 mg/mL bintrafusp alfa formulations, since freeze-dryability is somewhat compromised at 60mM NaCl, whereas bintrafusp alfa begins to precipitate at around 20 mM NaCl, as observed during dilution of samples for analytical purposes. In addition, a sugar, such as trehalose (sucrose is also a valid substitute) exhibiting different mode of action (cryo/lyophilization protector, lyophilization bulking agent and tonicifier) at around 6%(w/v) (certainly ≥4.4% (w/v) is preferred) is useful to formulate bintrafusp alfa at 10 mg/mL as a lyophilized dosage form.

*1E Formulations - Results and Analysis*

Having established, from experiments with Formulations 1A-1D, a formulation window providing a favourable balance in terms of isotonicity and freeze-dryability, it was further examined whether such a window also provides sufficient stability for the protein. To this end, the 2 formulations from Table 1E were subjected to short-term (3 weeks) stability tests (at 5°C, 25°C, and 40°C), which revealed that, although both formulations (in both lyophilised and liquid states) passed the stability tests, the formulation with 75 mM NaCl and 4% trehalose at pH 5.5 was not sufficiently freeze-dryable for lyophilised formulations. A liquid formulation of the drug (10mg/mL bintrafusp alfa, 10mM L-Histidine, 5 mM Methionine, 40mM NaCl, 6% (w/v) Trehalose, 0.05%(w/v) Polysorbate 20 at pH 5.5) did exhibit good stability (3 month at 5°C without significant changes in quality attributes; and though at 6 months there was an increase in subvisible particles, other quality attributes remained well within expected ranges).

However, these early stability tests suggested that liquid formulations of bintrafusp alfa may not exhibit long-term stability, even when cooled at 5°C, and thus it was envisaged that lyophilised formulations would provide the solution to this problem. However, use of increasing quantities of NaCl to solubilise high concentrations of bintrafusp alfa was known to detrimentally effect freeze-drying, so a number of problems remained outstanding in order to permit formulations of higher drug concentration.

That said, these studies demonstrate that lyophilised formulations offer a potential solution where formulations exhibit certain solution-phase storage stability challenges.

*Conclusions for this Example*

Other formulation parameters, such as buffer system, surfactant, and other excipients, can apparently be varied significantly without unfavourably compromising basic stability, solubility, freeze-dryability, and osmolality, if pH and/or NaCl concentrations are maintained within an appropriate window for a given concentration of bintrafusp alfa. For 10 mg/mL bintrafusp alfa, the following formulation was confirmed to be particularly suitable:

| Composition | Concentration (mM) | Concentration (mg/ml) |
|---|---|---|
| Bintrafusp alfa | - | 10 |
| Histidine | 10 | 1.55 |
| Sodium chloride | 40 | 2.34 |
| Trehalose Dihydrate | 159 | 60.00 |
| Polysorbate20 | - | 0.5 |
| L-Methionine | 5 | 0.75 |
| WFI and diluted HCl/NaOH | q.b. to adjust pH to 5.5 | |

Surprisingly, the best pH (pH 5.5) was within 1 pH unit of the pl of the TGFβR2 moiety. However, a broader pH range, e.g., between pH 5-9, appears acceptable, in particular, upon the addition of sodium chloride.

Sodium chloride facilitated solubility of the drug substance, but too much NaCl could compromise lyophilisability.

However, at this stage lyophilized formulations were seen as most likely to succeed. In fact, lyophilised formulations offer a potential solution where formulations exhibit certain solution-phase storage stability challenges. However, if sodium chloride is chosen as the provider of ionic strength, lyophilised formulations may be limited in terms of maximum drug concentrations, since the higher the drug concentration the more NaCl is required to facilitate solubility and stability, but high concentrations of salt compromise lyophilisability somewhat.

**EXAMPLE 2 -Higher Concentration Liquid Formulations of an IgG:TGFIβR2 fusion protein**

*General Overview*

Though Example 1 provided viable pharmaceutical compositions, it was desirable to increase the concentration of the IgG:TGFβR2 fusion protein, be it for lyophilised compositions or liquid compositions, and to investigate whether these could withstand longer term storage. To this end, further formulation studies were conducted.

*List of Tested Formulations*

Stability results showed that the candidate formulation from Example 1 (2A.1) was also stable in liquid form and this formulation therefore served as a reference sample in Example 2. A formulation screen was then performed upon 4 additional formulations (2A.2-2A.5) in order to assess feasibility of upconcentrating bintrafusp alfa. These formulations are shown in Table 2A.

**Table 2A** - Up-concentrated Formulations

| Formulation | bintrafusp alfa | Other Excipients and Conditions |
|---|---|---|
| 2A.1 | 10 mg/mL | 10mM L-Histidine, 5 mM Methionine, 40mM NaCl, 6% (w/v) Trehalose, 0.05%(w/v) Polysorbate 20 at pH 5.5 |
| 2A.2 | 20 mg/mL | 10mM L-Histidine, 5 mM Methionine, 40mM NaCl, 6% (w/v) Trehalose, 0.05%(w/v) Polysorbate 20 at pH 5.5 |
| 2A.3 | 35 mg/mL | 10mM L-Histidine, 5 mM Methionine, 40mM NaCl, 6% (w/v) Trehalose, 0.05%(w/v) Polysorbate 20 at pH 5.5 |
| 2A.4 | 40 mg/mL | 10mM L-Histidine, 5 mM Methionine, 40mM NaCl, 6% (w/v) Trehalose, 0.05%(w/v) Polysorbate 20 at pH 5.5 |
| 2A.5 | 40 mg/mL | 10mM L-Histidine, 5 mM Methionine, **60mM** NaCl, 6% (w/v) Trehalose, 0.05%(w/v) Polysorbate 20 at pH 5.5 |

_Results and Analysis_

The formulations listed in Table 2A were the subject of long term stability studies (up to 12 months at 5°C), accelerated stability studies (up to 6 months at 25°C), and stressed stability studies (up to 3 months at 40°C). Table 2B shows the analytical results pertaining to long-term stability studies (at 5°C) with Formulation 2A.2, which had a drug concentration of 20 mg/mL.

**Table 2B** - Results of Long-term stability studies of Formulation 2A.2

| Test | Time at 5°C | | | | | |
|---|---|---|---|---|---|---|
| | T=0 | 1 M (4 wk) | 2 M (8 wk) | 3 M (13 wk) | 6 M (26 wk) | 12 M (52 wk) |
| **Visible Particles** | FVP | VP | VP | VP | VP | VP |
| **Degree of colouration** | <BY6 | <BY6 | <BY6 | <BY6 | <BY6 | <BY6 |
| **Clarity and degree of opalescence (NTU)** | 8 | 8 | 8 | 8 | 8 | 8 |
| **pH** | 5.4 | 5.4 | 5.4 | 5.5 | 5.6 | 5.5 |
| **Protein content (mg/mL)** | 20.4 | 20.5 | 19.6 | 21.1 | 20.3 | 21.9 |
| **CA % LC** | 26.3 | 26.7 | 26.1 | 26.6 | 28.0 | 26.4 |
| **Main Clipping (peak 6)** | 4.5 | 4.4 | 4.4 | 4.4 | 4.3 | 4.6 |
| **Additive shoulder (Peak 7)** | 0.2 | 0.3 | 0.3 | 0.2 | 0.3 | 0.2 |
| **CA% HC-Gly Var** | 27.6 | 26.9 | 27.3 | 27.5 | 27.2 | 27.5 |
| **CA% HC** | 39.2 | 39.6 | 39.6 | 40.0 | 39.4 | 39.0 |
| **Purity (+ peak 7)** | 93.4 | 93.6 | 93.3 | 94.3 | 94.9 | 93.1 |
| **Purity %** | 93.2 | 93.3 | 93.0 | 94.1 | 94.6 | 92.9 |
| **Fragmentation (LMW%)** | 2.7 | 2.9 | 2.8 | 2.7 | 2.7 | 2.6 |
| **Aggregation (HMW%)** | 0.4 | 0.4 | 0.5 | 0.6 | 0.5 | 0.7 |
| **Oxidation (%)** | 4.1 | 4.0 | 5.5 | 5.5 | 5,5 | 4.1 |
| **Deamidation (%)** | 8.1 | 8.4 | 8.4 | 7.3 | 7,1 | 8.4 |
| **Particulate contamination: Sub-visible particles > 2$\mu$m** | 4588 | 7859 | 721 | 4736 | 34356 | 5007 |
| **Particulate contamination: Sub-visible particles > 5$\mu$m** | 419 | 553 | 41 | 454 | 7272 | 1312 |
| **Particulate contamination: Sub-visible particles > 10$\mu$m** | 49 | 74 | 3 | 44 | 1663 | 281 |

(continued)

| Test | Time at 5°C | | | | | |
|---|---|---|---|---|---|---|
| | T=0 | 1 M (4 wk) | 2 M (8 wk) | 3 M (13 wk) | 6 M (26 wk) | 12 M (52 wk) |
| Particulate contamination: Sub-visible particles > 25μm | 0 | 0 | 0 | 5 | 202 | 10 |
| Biological Activity (Anti PD-L1) | N.T. | 100 | 102[1] | 109 | 100 | 104 |
| Biological Activity (TGF) | N.T. | 94 | 97 | 105 | 95 | 101 |

Table 2C shows the analytical results pertaining to long-term stability studies (at 5°C) with Formulation 2A.3, which had a drug concentration of 35 mg/mL.

**Table 2C** - Results of Long-term stability studies of Formulation 2A.3

| Test | Time at 5°C | | | | | |
|---|---|---|---|---|---|---|
| | T=0 | 1M (4 wk) | 2 M (8 wk) | 3 M (13 wk) | 6 M (26 wk) | 12 M (52 wk) |
| Visible Particles | FVP | VP | VP | FVP | VP | VP |
| Degree of colouration | <BY5 | <BY5 | <BY5 | <BY5 | <BY5 | <BY5 |
| Clarity and degree of opalescence (NTU) | 12 | 15 | 10 | 14 | 12 | 13 |
| pH | 5.4 | 5.4 | 5.4 | 5.5 | 5.5 | 5.4 |
| Protein content (mg/mL) | 35.6 | 35.9 | 34.4 | 36.0 | 34.8 | 35.7 |
| CA % LC | 26.4 | 26.6 | 26.5 | 26.5 | 28.1 | 26.5 |
| Main Clipping (peak 6) | 4.4 | 4.2 | 4.4 | 4.4 | 4.4 | 4.9 |
| Additive shoulder (Peak 7) | 0.2 | 0.3 | 0.2 | 0.2 | 0.2 | 0.2 |
| CA% HC-Gly Var | 27.5 | 27.0 | 27.4 | 27.4 | 27.0 | 27.4 |
| CA% HC | 39.3 | 39.8 | 39.6 | 40.3 | 39.4 | 38.7 |
| Purity (+ peak 7) | 93.4 | 93.6 | 93.6 | 94.4 | 94.7 | 92.7 |
| Purity % | 93.2 | 93.3 | 93.4 | 94.2 | 94.5 | 92.6 |
| Fragmentation (LMW%) | 2.7 | 2.8 | 2.7 | 2.6 | 3.0 | 3.2 |
| Aggregation (HMW%) | 0.5 | 0.5 | 0.6 | 0.7 | 0.6 | 0.8 |
| Oxidation (%) | 4.1 | 4.0 | 5.5 | 5.6 | 5,6 | 4.1 |
| Deamidation (%) | 8.2 | 8.4 | 8.5 | 8.2 | 7,0 | 8.5 |
| Particulate contamination: Sub-visible particles > 2μm | 15024 | 11501 | 480 | 9494 | 14261 | 2232 |
| Particulate contamination: Sub-visible particles > 5μm | 974 | 547 | 20 | 936 | 2736 | 662 |
| Particulate contamination: Sub-visible particles > 10μm | 125 | 34 | 2 | 72 | 446 | 187 |
| Particulate contamination: Sub-visible particles > 25μm | 5 | 5 | 0 | 0 | 62 | 29 |
| Biological Activity (Anti PD-L1) | N.T. | 103 | 95 | 103 | 104 | 120 |
| Biological Activity (TGF) | N.T. | 90 | 99 | 105 | 97 | 93 |

Table 2D shows the analytical results pertaining to long-term stability studies (at 5°C) with Formulation 2A.4, which had a drug concentration of 40 mg/mL.

51

**Table 2D** - Results of Long-term stability studies of Formulation 2A.4

| Test | Time at 5°C | | | | | |
|---|---|---|---|---|---|---|
| | T=0 | 1 M (4 wk) | 2 M (8 wk) | 3 M (13 wk) | 6 M (26 wk) | 12 M (52 wk) |
| Visible Particles | FVP | VP | VP | FVP | FVP | FVP |
| Degree of colouration | <BY5 | <BY5 | <BY5 | <BY5 | <BY5 | <BY5 |
| Clarity and degree of opalescence (NTU) | 13 | 13 | 13 | 13 | 15 | 14 |
| pH | 5.4 | 5.4 | 5.3 | 5.4 | 5.4 | 5.3 |
| Protein content (mg/mL) | 39.6 | 42.7 | 39.5 | 40.6 | 40.9 | 40.3 |
| CA % LC | 26.1 | 26.9 | 26.3 | 26.6 | 27.8 | 26.2 |
| Main Clipping (peak 6) | 4.5 | 4.1 | 4.5 | 4.3 | 4.4 | 4.6 |
| Additive shoulder (Peak 7) | 0.2 | 0.4 | 0.2 | 0.2 | 0.3 | 0.2 |
| CA% HC-Gly Var | 27.5 | 26.8 | 27.5 | 27.3 | 27.2 | 27.5 |
| CA% HC | 39.5 | 39.6 | 39.6 | 40.4 | 39.5 | 38.9 |
| Purity % (+ peak 7) | 93.3 | 93.8 | 93.6 | 94.4 | 94.8 | 92.8 |
| Purity % | 93.1 | 93.4 | 93.4 | 94.2 | 94.5 | 92.6 |
| Fragmentation (LMW%) | 2.8 | 2.7 | 2.5 | 2.6 | 2.9 | 3.1 |
| Aggregation (HMW%) | 0.5 | 0.5 | 0.6 | 0.7 | 0.7 | 0.9 |
| Oxidation (%) | 4.1 | 4.0 | 5.5 | 5.2 | 5.2 | 4.2 |
| Deamidation (%) | 8.3 | 8.4 | 8.9 | 8.3 | 7.1 | 8.8 |
| Particulate contamination: Sub-visible particles > 2$\mu$m | 12797 | 12831 | 647 | 5405 | 14440 | 5729 |
| Particulate contamination: Sub-visible particles > 5$\mu$m | 651 | 500 | 41 | 403 | 1831 | 1554 |
| Particulate contamination: Sub-visible particles > 10$\mu$m | 67 | 42 | 7 | 139 | 302 | 449 |
| Particulate contamination: Sub-visible particles > 25$\mu$m | 4 | 4 | 0 | 4 | 21 | 50 |
| Biological Activity (Anti PD-L1) | N.T. | 101 | 104[1] | 99 | 88 | 97 |
| Biological Activity (TGF) | N.T. | 93 | 98 | 103 | 90 | 96 |

Table 2E shows the analytical results pertaining to long-term stability studies (at 5°C) and accelerated stability studies (at 25°C) with Formulation 2A.5, which had a drug concentration of 40 mg/mL but an elevated sodium chloride concentration of 60 mM.

**Table 2E** - Results of Long-term and Accelerated stability studies of Formulation 2A.5

| Test | T=0 | 1M (4 wk) | 2 M (8 wk) | 3 M (13 wk) | 6 M (26 wk) |
|---|---|---|---|---|---|
| Visible Particles | FVP | FVP | FVP | FVP | FVP |
| Degree of colouration | < BY5 | < BY5 | < BY5 | < BY5 | < BY5 |
| Clarity and degree of opalescence (NTU) | 15 | 11 | 11 | 12 | 12 |
| pH | 5.5 | 5.5 | 5.5 | 5.5 | 5.5 |
| Protein content (mg/mL) | 41.3 | 40.7 | 38.8 | 38.6 | 39.0 |
| CA % LC | 27.60 | 28.33 | 26.56 | 27.01 | 26.83 |

(continued)

| Test | T=0 | 1M (4 wk) | 2 M (8 wk) | 3 M (13 wk) | 6 M (26 wk) |
|---|---|---|---|---|---|
| Main Clipping (peak 6) | 4.45 | 4.51 | 4.50 | 4.32 | 4.50 |
| Additive shoulder (Peak 7) | 0.17 | 0.13 | 0.23 | 0.16 | 0.24 |
| CA% HC-Gly Var | 27.20 | 26.91 | 27.39 | 26.98 | 27.42 |
| CA % HC | 38.82 | 38.29 | 39.11 | 39.38 | 38.64 |
| Purity (+ peak 7) | 93.79 | 93.21 | 92.33 | 93.53 | 93.13 |
| Purity % | 93.62 | 93.53 | 93.06 | 93.37 | 92.89 |
| Fragmentation (LMW%) | 2.23 | 2.40 | 2,.0 | 2,.6 | 2.70 |
| Aggregation (HMW%) | 0.8 | 0.4 | 0.5 | 0.7 | 0.7 |
| Oxidation (%) | 4.33 | 4.33 | 3.89 | 4.50 | 3.55 |
| Deamidation (%) | 7.8 | 11.7 | 7.8 | 9.4 | 9.8 |
| Particulate contamination: Sub-visible particles > 2μm | 6271 | 1940 | 35175 | 21815 | 1088 |
| Particulate contamination: Sub-visible particles > 5μm | 2029 | 596 | 3980 | 4158 | 139 |
| Particulate contamination: Sub-visible particles > 10μm | 899 | 202 | 872 | 932 | 42 |
| Particulate contamination: Sub-visible particles > 25μm | 101 | 25 | 63 | 97 | 0 |
| Biological Activity (Anti PD-L1) | 107 | 101 | 112 | 104 | 113 |
| Biological Activity (TGF) | 101 | 106 | 110 | 100 | 97 |

Table 2F shows protein stability by optical density for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. No particular decrease in protein concentration was observed during these stability studies for any of the analyzed batches.

**Table 2F** - Protein Concentration by Optical Density for Formulations 2A.2-2A.5 at various time points after storage at 5°C, 25°C, and 40°C

| Protein content by OD | 5°C | | | | | |
|---|---|---|---|---|---|---|
|  | T0 | 1M | 2M | 3M | 6M | 12M |
| 2A.2 | 20.4 | 20.5 | 19.6 | 21.1 | 20.3 | 21.9 |
| 2A.3 | 35.6 | 35.9 | 34.5 | 36.0 | 34.8 | 35.7 |
| 2A.4 | 39.6 | 42.7 | 39.5 | 40.6 | 40.9 | 40.3 |
| 2A.5 | 41.3 | 40.7 | 38.8 | 38.6 | 39.0 | NA |
|  | 25°C | | | | | |
| 2A.2 | 20.4 | 20.8 | 19.8 | 20.9 | 20.5 | |
| 2A.3 | 35.6 | 36.4 | 34.3 | 35.1 | 35.4 | |
| 2A.4 | 39.6 | 41.7 | 40.0 | 40.1 | 40.3 | |
| 2A.5 | 41.3 | 40.5 | 38.8 | 38.7 | 39.5 | |
|  | 40°C | | | | | |
| 2A.2 | 20.4 | 21.2 | 19.8 | 20.5 | | |

(continued)

|  | 40°C |  |  |  |  |
|---|---|---|---|---|---|
| 2A.3 | 35.6 | 35.7 | 34.4 | 35.7 |  |
| 2A.4 | 39.6 | 42.3 | 39.2 | 41.0 |  |
| 2A.5 | 41.3 | 40.4 | 38.9 | 39.5 |  |

Table 2G shows % high molecular weight (%HMW) species by SE-UPLC for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. The higher the concentration the higher the %HMWs, except for 2A.5 (60 mM NaCl), which appeared more stable than the corresponding formulation with 40 mM NaCl (which also contained 40 mg/mL bintrafusp alfa). This suggests that higher API concentrations require more NaCl to mitigate aggregation.

Table 2G - %HMW for Formulations 2A.2-2A.5 at various time points after storage at 5°C, 25°C, and 40°C

|  | 5°C |  |  |  |  |  |
|---|---|---|---|---|---|---|
| Aggregation (HMW%) | T0 | 1M | 2M | 3M | 6M | 12M |
| 2A.2 | 0.4 | 0.4 | 0.5 | 0.6 | 0.5 | 0.7 |
| 2A.3 | 0.5 | 0.5 | 0.6 | 0.7 | 0.6 | 0.8 |
| 2A.4 | 0.5 | 0.5 | 0.6 | 0.7 | 0.7 | 0.9 |
| 2A.5 | 0.8 | 0.4 | 0.5 | 0.7 | 0.7 | NA |
|  | 25°C |  |  |  |  |  |
| 2A.2 | 0.4 | 0.5 | 0.7 | 0.8 | 0.7 |  |
| 2A.3 | 0.5 | 0.7 | 0.9 | 1.0 | 1.0 |  |
| 2A.4 | 0.5 | 0.7 | 0.9 | 1.1 | 1.1 |  |
| 2A.5 | 0.8 | 0.5 | 0.7 | 1.0 | 1.2 |  |
|  | 40°C |  |  |  |  |  |
| 2A.2 | 0.4 | 1.2 | 1.9 | 2.5 |  |  |
| 2A.3 | 0.5 | 1.8 | 2.8 | 3.7 |  |  |
| 2A.4 | 0.5 | 2.0 | 3.1 | 4.3 |  |  |
| 2A.5 | 0.8 | 1.2 | 2.0 | 3.5 |  |  |

Table 2H shows % low molecular weight (%LMW) species by CE-SDS for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. The %LMWs is lower for 2A.5 at all stability conditions, which potentially indicates that NaCl may mitigate fragmentation in samples of higher drug concentration.

Table 2H - %LMW for Formulations 2A.2-2A.5 at various time points after storage at 5°C, 25°C, and 40°C

|  | 5°C |  |  |  |  |  |
|---|---|---|---|---|---|---|
| Fragmentation (LMW%) | T0 | 1M | 2M | 3M | 6M | 12M |
| 2A.2 | 2.7 | 2.9 | 2.8 | 2.7 | 2.7 | 2.6 |
| 2A.3 | 2.7 | 2.8 | 2.7 | 2.6 | 3.0 | 3.2 |
| 2A.4 | 2.8 | 2.7 | 2.5 | 2.6 | 2.9 | 3.1 |
| 2A.5 | 2.2 | 2.4 | 2.2 | 2.3 | 2.7 | NA |

(continued)

| | 25°C | | | | | |
|---|---|---|---|---|---|---|
| 2A.2 | 2.7 | 3.0 | 3.5 | 3.7 | 5.4 | |
| 2A.3 | 2.7 | 3.0 | 3.4 | 3.6 | 5.3 | |
| 2A.4 | 2.8 | 2.9 | 3.5 | 3.7 | 5.6 | |
| 2A.5 | 2.2 | 2.7 | 2.9 | 3.7 | 4.3 | |
| | 40°C | | | | | |
| 2A.2 | 2.7 | 6.0 | 11.2 | 14.6 | | |
| 2A.3 | 2.7 | 6.1 | 10.6 | 13.9 | | |
| 2A.4 | 2.8 | 5.8 | 12.1 | 14.3 | | |
| 2A.5 | 2.2 | 6.5 | 9.4 | 14.7 | | |

Table 2I shows % oxidation level by RP-UPLC for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. For all lots, similar oxidation levels (%) were observed over stability at 5°C±3°C and 25°C±2°C (taking in consideration the oxidation level of IRS reported as comparison in each session), while an increase is observed at 40°C±2°C. High values for 2-6M at 5°C and 25°C may be misleading due to analytical session variability.

Table 2I - %Oxidation for Formulations 2A.2-2A.5 at various time points after storage at 5°C, 25°C, and 40°C

| | 5°C | | | | | |
|---|---|---|---|---|---|---|
| Oxidation level (%Ox) | T0 | 1M | 2M | 3M | 6M | 12M |
| 2A.2 | 4.1 | 4.0 | 5.5 | 5.5 | 5.5 | 4.1 |
| 2A.3 | 4.1 | 4.0 | 5.5 | 5.6 | 5.6 | 4.1 |
| 2A.4 | 4.1 | 4.0 | 5.5 | 5.2 | 5.2 | 4.2 |
| 2A.5 | 4.3 | 4.3 | 3.9 | 4.5 | 3.6 | NA |
| | 25°C | | | | | |
| 2A.2 | 4.1 | 4.2 | 5.5 | 5.6 | 6.0 | |
| 2A.3 | 4.1 | 4.3 | 5.5 | 5.2 | 5.9 | |
| 2A.4 | 4.1 | 4.2 | 5.8 | 5.5 | 6.3 | |
| 2A.5 | 4.3 | 4.2 | 4.3 | 4.6 | 4.4 | |
| | 40°C | | | | | |
| 2A.2 | 4.1 | 5.0 | 7.6 | 8.9 | | |
| 2A.3 | 4.1 | 5.4 | 7.4 | 8.7 | | |
| 2A.4 | 4.1 | 4.9 | 7.5 | 8.5 | | |
| 2A.5 | 4.3 | 5.8 | 6.5 | 7.9 | | |

Table 2J shows % deaminated forms (LC) by IEX-HPLC for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. At 5°C±3°C and 25°C±2°C, all tested samples remain stable over time, with only a minor increase for 2A.5 in the last time point. At 40°C ±2°C, the %LC increase over time. The levels of deamidation is comparable for all samples, and apparently independent of API concentration within the formulations.

**Table 2J** - % deamination (LC) for Formulations 2A.2-2A.5 at various time points after storage at 5°C, 25°C, and 40°C

|  | 5°C | | | | | |
|---|---|---|---|---|---|---|
| **Deamidation level** | **T0** | **1M** | **2M** | **3M** | **6M** | **12M** |
| **2A.2** | 8.1 | 8.4 | 8.4 | 7.3 | 7.1 | 8.4 |
| **2A.3** | 8.2 | 8.4 | 8.5 | 8.2 | 7.0 | 8.5 |
| **2A.4** | 8.3 | 8.4 | 8.9 | 8.3 | 7.1 | 8.8 |
| **2A.5** | 7.8 | 11.7 | 7.8 | 9.4 | 9.8 | NA |
|  | 25°C | | | | | |
| **2A.2** | 8.1 | 8.2 | 9.1 | 8.4 | 9.0 | |
| **2A.3** | 8.1 | 8.2 | 9.3 | 8.3 | 9.1 | |
| **2A.4** | 8.1 | 8.2 | 9.7 | 8.3 | 9.1 | |
| **2A.5** | 7.8 | 9.0 | 8.6 | 9.3 | 8.5 | |
|  | 40°C | | | | | |
| **2A.2** | 8.1 | 10.4 | 14.1 | 14.2 | | |
| **2A.3** | 8.2 | 10.8 | 14.2 | 15.9 | | |
| **2A.4** | 8.3 | 10.7 | 14.2 | 14.9 | | |
| **2A.5** | 7.8 | 8.9 | 14.1 | 18.7 | | |

Table 2K shows % purity by CE-SDS RED for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. Slight decreases in purity were observed at $25°C \pm 2°C$, with more significant decreases being apparent at $40°C \pm 2°C$. However, the trends were comparable across all formulations and seemingly concentration independent.

**Table 2K** - % purity for Formulations 2A.2-2A.5 at various time points after storage at 5°C, 25°C, and 40°C

|  | 5°C | | | | | |
|---|---|---|---|---|---|---|
| **%purity by CGE-SDS** | **T0** | **1M** | **2M** | **3M** | **6M** | **12M** |
| **2A.2** | 93.2 | 93.3 | 93.0 | 93.6 | 94.6 | 92.9 |
| **2A.3** | 93.2 | 93.3 | 93.4 | 93.7 | 94.5 | 92.6 |
| **2A.4** | 93.1 | 93.4 | 93.4 | 93.6 | 94.5 | 92.6 |
| **2A.5** | 93.6 | 93.5 | 93.1 | 93.4 | 92.9 | NA |
|  | 25°C | | | | | |
| **2A.2** | 93.2 | 92.8 | 92.1 | 92.0 | 91.7 | |
| **2A.3** | 93.2 | 93.0 | 92.6 | 91.8 | 91.8 | |
| **2A.4** | 93.1 | 92.7 | 92.0 | 91.8 | 91.9 | |
| **2A.5** | 93.6 | 92.9 | 92.0 | 92.0 | 90.3 | |
|  | 40°C | | | | | |
| **2A.2** | 93.2 | 89.0 | 83.9 | 79.6 | | |
| **2A.3** | 93.2 | 88.6 | 84.1 | 79.5 | | |
| **2A.4** | 93.1 | 89.0 | 82.5 | 79.0 | | |
| **2A.5** | 93.6 | 89.3 | 85.5 | 81.5 | | |

Table 2L shows % main clipping by CE-SDS RED for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. Increases in % Main clipping were similar for all formulations at all timepoints under all conditions, and thus appears to be concentration independent.

**Table 2L** - % main clipping for Formulations 2A.2-2A.5 at various time points after storage at 5°C, 25°C, and 40°C

| main clipping by CGE-SDS | 5°C | | | | | |
| --- | --- | --- | --- | --- | --- | --- |
| | T0 | 1M | 2M | 3M | 6M | 12M |
| 2A.2 | 4.5 | 4.4 | 4.4 | 4.3 | 4.3 | 4.6 |
| 2A.3 | 4.4 | 4.2 | 4.4 | 4.4 | 4.4 | 4.9 |
| 2A.4 | 4.5 | 4.1 | 4.5 | 4.3 | 4.4 | 4.6 |
| 2A.5 | 4.5 | 4.5 | 4.5 | 4.3 | 4.5 | NA |
| | 25°C | | | | | |
| 2A.2 | 4.5 | 4.6 | 5.1 | 5.6 | 6.8 | |
| 2A.3 | 4.4 | 4.5 | 5.0 | 5.6 | 6.8 | |
| 2A.4 | 4.5 | 4.6 | 5.2 | 5.5 | 6.8 | |
| 2A.5 | 4.5 | 4.8 | 5.3 | 5.4 | 6.5 | |
| | 40°C | | | | | |
| 2A.2 | 4.5 | 7.3 | 10.4 | 12.6 | | |
| 2A.3 | 4.4 | 7.4 | 10.4 | 14.4 | | |
| 2A.4 | 4.5 | 7.4 | 10.8 | 14.2 | | |
| 2A.5 | 4.5 | 7.6 | 10.1 | 13.1 | | |

Table 2M shows % anti-PD-L1 biological activity for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. No significant variation in the biological activity for the anti-PD-L1 moiety was observed over time in all stability conditions.

**Table 2M -** % anti-PD-L1 biological activity for Formulations 2A.2-2A.5 at various time points after storage at 5°C, 25°C, and 40°C

| Anti PD-L1 moiety by in vitro bioassay | 5°C | | | | | |
| --- | --- | --- | --- | --- | --- | --- |
| | T0 | 1M | 2M | 3M | 6M | 12M |
| 2A.2 | N.T. | 100 | 102 | 109 | 100 | 104 |
| 2A.3 | N.T. | 103 | 95 | 103 | 104 | 120 |
| 2A.4 | N.T. | 101 | 104 | 99 | 88 | 97 |
| 2A.5 | 107 | 101 | 112 | 104 | 113 | NA |
| | 25°C | | | | | |
| 2A.2 | N.T. | 101 | 116 | 109 | 107 | |
| 2A.3 | N.T. | 104 | 123 | 106 | 105 | |
| 2A.4 | N.T. | 100 | 110 | 111 | 101 | |
| 2A.5 | 107 | 106 | 112 | 115 | 103 | |
| | 40°C | | | | | |
| 2A.2 | N.T. | 103 | 122 | 116 | | |

(continued)

|  | 40°C |  |  |  |  |
|---|---|---|---|---|---|
| 2A.3 | N.T. | 106 | 113 | 125 |  |
| 2A.4 | N.T. | 101 | 121 | 108 |  |
| 2A.5 | 107 | 118 | 121 | 117 |  |

Table 2N shows % TGF-β biological activity for all 4 up-concentrated formulations (2A.2-2A.5) from Table 2A under long-term stability testing (5°C), accelerated stability testing (25°C), and stressed stability testing (40°C), at various intervals ranging from Time = 0 to 12 months. Significant decreases in the biological activity for TGF-β moiety was observed over time at 40°C±2°C, to a similar extent for all formulations.

Table 2N - % TGF-β biological activity for Formulations 2A.2-2A.5 at various time points after storage at 5°C, 25°C, and 40°C

|  | 5°C |  |  |  |  |  |
|---|---|---|---|---|---|---|
| TGF moiety by in vitro bioassay | T0 | 1M | 2M | 3M | 6M | 12M |
| 2A.2 | N.T. | 94 | 97 | 105 | 95 | 101 |
| 2A.3 | N.T. | 90 | 99 | 105 | 97 | 93 |
| 2A.4 | N.T. | 93 | 98 | 103 | 90 | 96 |
| 2A.5 | 101 | 106 | 110 | 100 | 97 | NA |
|  | 25°C |  |  |  |  |  |
| 2A.2 | N.T. | 92 | 102 | 98 | 93 |  |
| 2A.3 | N.T. | 91 | 99 | 100 | 93 |  |
| 2A.4 | N.T. | 93 | 103 | 95 | 88 |  |
| 2A.5 | 101 | 102 | 105 | 98 | 90 |  |
|  | 40°C |  |  |  |  |  |
| 2A.2 | N.T. | 87 | 64 | 52 |  |  |
| 2A.3 | N.T. | 87 | 65 | 60 |  |  |
| 2A.4 | N.T. | 82 | 66 | 59 |  |  |
| 2A.5 | 101 | 86 | 72 | 68 |  |  |

*Conclusions*

Table 2O summarises the trends observed throughout the aforementioned stability studies.

Table 2O - Analytical protocols used to test Formulations of Table 2A, and general observations

| Parameter | Long term +5°C | Accelerated +25°C | Stress +40°C |
|---|---|---|---|
| HMW species | Very slight increase for all formulations in the last time points | Increase over time: the highest the concentration, the highest the increase, except for 2A.5 | Significant increase over time, in relation with protein concentration. 2A.5 at 40 mg/ml is comparable to formulations at lower concentration |
| LMW species by CE-SDS | Stable over time with slight increase only at 6 and 12 M. 2A.5 showed the lowest values | Increase only at 6M with the least increase for 2A.5. | Significant increase comparable in all formulations |

(continued)

| Parameter | Long term +5°C | Accelerated +25°C | Stress +40°C |
|---|---|---|---|
| Main Clipping by CE-SDS | Stable over time and similar in all formulations | Slight increase similar for all formulations | Increase over time similar for all formulations, slightly better for 2A.5 |
| Electrophoretic Purity by CE-SDS | Stable over time and similar in all formulations | Slight decrease only after 6 M, similar in all formulations | Decrease over time similar in all formulations |
| Deamidation | Stable over time and similar in all formulations | Stable over time and similar in all formulations | Increase over time similar in all formulations |
| Oxidation | Stable over time and similar in all formulations | Stable over time and similar in all formulations | Increase over time similar in all formulations |
| Biological Activity Anti PDL1 | Stable over time and similar in all formulations | Stable over time and similar in all formulations | Stable over time and similar in all formulations |
| Biological Activity TGF | Stable over time and similar in all formulations | Stable over time and similar in all formulations | Decrease over time similar in all formulations |

Again it is envisaged that other formulation parameters, such as buffer system, surfactant, and other excipients, can be varied significantly without unfavourably compromising basic stability, solubility, freeze-dryability, and osmolality, if pH and/or NaCl concentrations are maintained within an appropriate window for a given concentration of bintrafusp alfa. For 40 mg/mL bintrafusp alfa, the following formulation was confirmed to be particularly suitable:

| Composition | Concentration (mM) | Concentration (mg/ml) |
|---|---|---|
| Bintrafusp alfa | - | 40 |
| Histidine | 10 | 1.55 |
| Sodium chloride | 60 | 3.51 |
| Trehalose Dihydrate | 159 | 60.00 |
| Polysorbate20 | - | 0.5 |
| L-Methionine | 5 | 0.75 |
| WFI and diluted HCl/NaOH | q.b. to adjust pH to 5.5 | |

The main difference from the lead candidate identified in Example 1 is the higher concentrations of bintrafusp alfa, at 40 mg/mL rather than 10 mg/mL, and a raised amount of sodium chloride, from 40 mM to 60 mM. Moreover, these studies suggested that liquid formulations of Example 1 and of this Example are in fact viable, and that lyophilisation / reconstitution may not be strictly necessary if storage of liquid formulations is considered more practical.

Further studies also showed that optimal amounts of NaCl for given concentrations of bintrafusp alfa were as follows:

- 10 - 20mg/mL API can be stabilized by 40mM NaCl

- 40mg/mL generally preferred 60mM NaCl

- 60mg/mL with Trehalose generally preferred 100mM NaCl

- 60mg/mL w/o Trehalose generally preferred 150mM NaCl

These studies also suggested that liquid formulations having high concentrations of IgG:TGFβR2 could indeed be viable, and possibly even preferable to lyophilised formulations, especially where higher concentrations of sodium chloride are used.

### EXAMPLE 3A - Further screening studies - Step 1A (pH, ionic strength, and protein concentration screen)

#### General Overview

Further formulation studies were performed to assess variations in pH (pH 5.0-7.5), ionic strengths (40-150 mM ionic salts, e.g. sodium chloride), and protein concentrations (20, 40, and 60 mg/mL), in a 10 mM histidine buffer system, which served as an exemplary buffer system.

#### List of Tested Formulations

An initial screen was performed upon 20 formulations to establish viable pHs and optimal pHs, viable and optimal ionic strength, and viable and optimal protein concentrations. Formulations for this initial screen are shown in Table 3A.

**Table 3A** - Formulations for initial pH, ionic strength, and protein concentration screen

| Run | pH | bintrafusp alfa (mg/mL) | Ionic strength (mM of NaCl) |
|---|---|---|---|
| 1 | 5.7 | 60 | 150 |
| 2 | 6.8 | 40 | 150 |
| 3 | 6.6 | 20 | 150 |
| 4 | 5.0 | 40 | 70 |
| 5 | 5.0 | 60 | 110 |
| 6 | 7.5 | 20 | 40 |
| 7 | 6.1 | 40 | 50 |
| 8 | 7.5 | 60 | 150 |
| 9 | 5.5 | 60 | 100 |
| 10 | 7.1 | 20 | 100 |
| 11 | 6.1 | 40 | 50 |
| 12 | 5.5 | 20 | 150 |
| 13 | 6.2 | 20 | 70 |
| 14 | 5.8 | 40 | 115 |
| 15 | 7.5 | 60 | 100 |
| 16 | 7.0 | 60 | 100 |
| 17 | 6.1 | 60 | 100 |
| 18 | 7.5 | 40 | 100 |
| 19 | 5.3 | 40 | 150 |
| 20 | 5.3 | 20 | 70 |
| Reference | 5.5 | 10 | 40 |

#### Results and Analysis

The formulations in Table 3A were evaluated in a DoE study. Specifically, their unfolding temperature was examined. In addition, they were exposed to thermal stress (40°C for 4 weeks) and light stress (7 hours at 765 W/m$^2$) and the stressed samples were analysed *via* various analytical techniques.

With respect to thermally-stressed samples, significant models could be constructed by reference to the following analyses, though other additional analytical techniques were also used during preliminary analysis:

- HMWs by SE-UPLC

- Isoforms profiles by ICE3

- Purity/LMW by CGE-SDS (Red/NoRed)

- Oxidized Forms by RP-UPLC

- Deamidated Forms by IEX-HPLC

- Unfolding Temperature by nano-DSC

With respect to light-stressed samples, significant models could be constructed by reference to the following analyses, though again other additional analytical techniques were also used during preliminary analysis:

- HMWs by SE-UPLC

- Oxidized Forms by RP-UPLC

- Isoforms profiles by iCE3

*Unfolding temperatures*

Together, FIG. 3 and FIG. 4 suggest that a pH in the range 5.7-6.2 may be optimal from the perspective of unfolding temperatures, and that higher protein concentrations require higher ionic strengths to impart conformational stability.

Table 3B shows unfolding temperatures by nano-DSC for the formulations of Table 3A.

**Table 3B** - Unfolding temperatures ($T_m1$-$T_m4$) for the formulations of Table 3A

| Protein Concentration, pH, Ionic strength | Sample | Tm1 | Tm2 | Tm3 | Tm4 |
|---|---|---|---|---|---|
| 60 mg/ml, pH 5.7, NaCl 150 mM | #1 | 63.08 | - | 71.84 | 80.24 |
| 40 mg/ml, pH 6.8, NaCl 150 mM | #2 | 62.67 | 65.86 | 70.94 | 79.27 |
| 20 mg/ml, pH 6.6, NaCl 150 mM | #3 | 63.00 | 65.19 | 70.09 | 81.00 |
| 40 mg/ml, pH 5.0, NaCl 70 mM | #4 | 61.97 | 68.55 | 72.29 | 79.68 |
| 60 mg/ml, pH 5.0, NaCl 110 mM | #5 | 62.47 | - | 71.96 | 80.34 |
| 20 mg/ml, pH 7.5, NaCl 40 mM | #6 | 62.46 | - | 70.38 | 81.01 |
| 40 mg/ml, pH 6.1, NaCl 50 mM | #7 | 62.02 | 66.76 | 72.72 | 80.82 |
| 60 mg/ml, pH 7.5, NaCl 150 mM | #8 | 62.02 | 65.67 | 71.67 | 80.67 |
| 60 mg/ml, pH 5.5, NaCl 100 mM | #9 | 61.98 | 68.05 | 72.44 | 80.51 |
| 20 mg/ml, pH 7.1, NaCl 100 mM | #10 | 63.04 | - | 69.98 | 81.20 |
| 40 mg/ml, pH 6.1, NaCl 50 mM | #11 | 62.27 | 66.20 | 72.37 | 80.81 |
| 20 mg/ml, pH 5.5, NaCl 150 mM | #12 | 63.35 | 66.23 | 70.63 | 80.00 |
| 20 mg/ml, pH 6.2, NaCl 70 mM | #13 | 62.74 | 65.79 | 71.27 | 81.00 |
| 40 mg/ml, pH 5.8, NaCl 115 mM | #14 | 62.36 | 66.85 | 72.01 | 80.46 |
| 60 mg/ml, pH 7.5, NaCl 100 mM | #15 | 62.41 | 66.17 | 72.03 | 81.53 |
| 60 mg/ml, pH 7.0, NaCl 100 mM | #16 | 62.26 | 66.14 | 72.28 | 81.38 |
| 60 mg/ml, pH 6.1, NaCl 100 mM | #17 | 62.46 | 66.65 | 72.41 | 81.35 |
| 40 mg/ml, pH 7.5, NaCl 100 mM | #18 | 62.72 | - | 70.26 | 81.85 |
| 40 mg/ml, pH 5.3, NaCl 150 mM | #19 | 63.07 | 66.17 | 71.34 | 80.46 |
| 20 mg/ml, pH 5.3, NaCl 70 mM | #20 | 62.96 | 66.54 | 71.78 | 80.49 |

(continued)

| Protein Concentration, pH, Ionic strength | Sample | Tm1 | Tm2 | Tm3 | Tm4 |
|---|---|---|---|---|---|
| 10 mg/ml, pH 5.5, NaCl 40 mM | Reference#1 | 61.62 | 65.00 | 71.68 | 80.26 |

*Thermal stress*

Table 3C shows turbidity, as characterised by a high value of opalescence (ranging from 7 NTU to 18 NTU), upon thermal stress testing of the formulations of Table 3A. No significant changes were observed for any of the samples after 4 weeks of incubation at +40°C±2°C.

**Table 3C** - Turbidity of Formulations of Table 3A before and after thermal stress at 40°C for 4 weeks

| Protein Concentration, pH, Ionic strenght | Sample | Stress Stability at 40°C | |
|---|---|---|---|
| | | Time 0 | 4 weeks 40°C |
| 60 mg/ml, pH 5.7, NaCl 150 mM | #1 | 11 | 12 |
| 40 mg/ml, pH 6.8, NaCl 150 mM | #2 | 12 | 11 |
| 20 mg/ml, pH 6.6, NaCl 150 mM | #3 | 7 | 7 |
| 40 mg/ml, pH 5.0, NaCl 70 mM | #4 | 13 | 13 |
| 60 mg/ml, pH 5.0, NaCl 110 mM | #5 | 14 | 14 |
| 20 mg/ml, pH 7.5, NaCl 40 mM | #6 | 10 | 10 |
| 40 mg/ml, pH 6.1, NaCl 50 mM | #7 | 17 | 18 |
| 60 mg/ml, pH 7.5, NaCl 150 mM | #8 | 12 | 12 |
| 60 mg/ml, pH 5.5, NaCl 100 mM | #9 | 14 | 15 |
| 20 mg/ml, pH 7.1, NaCl 100 mM | #10 | 8 | 7 |
| 40 mg/ml, pH 6.1, NaCl 50 mM | #11 | 18 | 18 |
| 20 mg/ml, pH 5.5, NaCl 150 mM | #12 | 7 | 7 |
| 20 mg/ml, pH 6.2, NaCl 70 mM | #13 | 8 | 8 |
| 40 mg/ml, pH 5.8, NaCl 115 mM | #14 | 11 | 12 |
| 60 mg/ml, pH 7.5, NaCl 100 mM | #15 | 13 | 14 |
| 60 mg/ml, pH 7.0, NaCl 100 mM | #16 | 15 | 14 |
| 60 mg/ml, pH 6.1, NaCl 100 mM | #17 | 14 | 15 |
| 40 mg/ml, pH 7.5, NaCl 100 mM | #18 | 14 | 12 |
| 40 mg/ml, pH 5.3, NaCl 150 mM | #19 | 13 | 11 |
| 20 mg/ml, pH 5.3, NaCl 70 mM | #20 | 7 | 8 |
| 10 mg/ml, pH 5.5, NaCl 40 mM | Reference#1 | 6 | 5 |

Table 3D shows % deamidated forms upon thermal stress testing of the formulations of Table 3A. At **T0** no significant differences are observed for any of the samples, compared to the standard, except for samples of high pH and relatively low ionic strength relative to the concentration of API - e.g. sample #15, which has a value of % Deamidated forms higher than others, probably due to the synergistic effects of high protein concentration, high pH and low ionic strength. Meanwhile, after thermal stress for 4 Weeks at +40°C±2°C increasing pH correlates to increasing %deamidated forms. Overall, ionic strength and protein concentration impacted little on % deamidation. A pH in the range of 5.0-6.2 appears optimal, whilst formulations with a pH between 7.1 and 7.5 perform less well within this context.

**Table 3D** - % deamidated forms of Formulations of Table 3A before and after thermal stress at 40°C for 4 weeks

| Protein Concentration, pH, Ionic strength | Sample | Stress Stability at 40°C | |
|---|---|---|---|
| | | Time 0 | 4 weeks 40°C |
| 60 mg/ml, pH 5.7, NaCl 150 mM | #1 | 8.5 | 10.7 |
| 40 mg/ml, pH 6.8, NaCl 150 mM | #2 | 8.3 | 23.5 |
| 20 mg/ml, pH 6.6, NaCl 150 mM | #3 | 8.6 | 17.8 |
| 40 mg/ml, pH 5.0, NaCl 70 mM | #4 | 7.8 | 9.9 |
| 60 mg/ml, pH 5.0, NaCl 110 mM | #5 | 8.2 | 9.6 |
| 20 mg/ml, pH 7.5, NaCl 40 mM | #6 | 9.0 | 51.9 |
| 40 mg/ml, pH 6.1, NaCl 50 mM | #7 | 8.0 | 13.7 |
| 60 mg/ml, pH 7.5, NaCl 150 mM | #8 | 9.2 | 47.9 |
| 60 mg/ml, pH 5.5, NaCl 100 mM | #9 | 8.4 | 10.3 |
| 20 mg/ml, pH 7.1, NaCl 100 mM | #10 | 8.7 | 32.6 |
| 40 mg/ml, pH 6.1, NaCl 50 mM | #11 | 8.5 | 14.2 |
| 20 mg/ml, pH 5.5, NaCl 150 mM | #12 | 8.1 | 10.3 |
| 20 mg/ml, pH 6.2, NaCl 70 mM | #13 | 8.3 | 14.5 |
| 40 mg/ml, pH 5.8, NaCl 115 mM | #14 | 7.9 | 11.7 |
| 60 mg/ml, pH 7.5, NaCl 100 mM | #15 | 12.0 | 49.8 |
| 60 mg/ml, pH 7.0, NaCl 100 mM | #16 | 8.6 | 29.3 |
| 60 mg/ml, pH 6.1, NaCl 100 mM | #17 | 9.0 | 13.1 |
| 40 mg/ml, pH 7.5, NaCl 100 mM | #18 | 8.8 | 52.1 |
| 40 mg/ml, pH 5.3, NaCl 150 mM | #19 | 8.4 | 10.6 |
| 20 mg/ml, pH 5.3, NaCl 70 mM | #20 | 8.8 | 9.8 |
| 10 mg/ml, pH 5.5, NaCl 40 mM | Reference#1 | 7.4 | 9.5 |

Together, FIG. 5 and FIG. 6 suggest ionic strength and protein concentration have little impact on %LMW species after thermal stress, in contrast to pH which has a significant impact, with the pH region between pH 5.3-6.7 apparently optimal in this context. However, a low ionic strength correlates to fragmentation at a similar level of pH.

Together, FIG. 7 and FIG. 8 suggest that ionic strength should be increased as protein concentration increases to avoid aggregates formation and eventually protein precipitation. Moreover, a pH between pH 5.0-6.6 is apparently optimal in the context of %HMW species after thermal stress.

At Time = 0 in all species monitored by CGE (under reducing conditions), there were no significant differences between the samples in terms of purity, except for sample #19 with a slight increase in main clipping.

Upon thermal stress for 4 weeks at 40°C, all samples with pH >7 or pH < 5.5 showed lower overall purity, independently from the ionic strength or protein concentration. A low pH (pH 5.0 and to a lesser extent also pH 5.3) caused an increase of peak 7 impurity and an increase in main clipping (peak 6) was also observed under low pH conditions (< pH 5.5).

At Time = 0, cIEF showed no significant differences between the samples.

The data from FIG. 9 and elsewhere suggest the impact of ionic strength and protein concentration on isoforms to be insignificant after thermal stess, while isoform distribution is impacted by pH. The data from FIG. 9 suggest a pH of 5.3-5.9 would be optimal in the context of isoforms.

Table 3E shows Isoform clusters after thermal stress at 40°C for 4 weeks.

**Table 3E** - Isoform clusters after thermal stress at 40°C for 4 weeks

| Protein concentration, pH, ionic strenght | sample | Cluster 1 | | Cluster 2 | | Cluster 3 | | Cluster 4 | |
|---|---|---|---|---|---|---|---|---|---|
| | | T0 | 4 wks 40°C | T0 | 4 wks 40°C | T0 | 4 wks 40°C | T0 | 4 wks 40°C |
| 60 mg/ml. pH 5.7. NaCl 150 mM | #1 | 53.26 | 56.68 | 12.32 | 11.26 | 19.90 | 21.54 | 14.54 | 10.53 |
| 40 mg/ml. pH 6.8. NaCl 150 mM | #2 | 53.93 | 68.60 | 11.74 | 7.49 | 19.82 | 15.49 | 14.52 | 8.43 |
| 20 mg/ml. pH 6.6. NaCl 150 mM | #3 | 53.83 | 66.48 | 11.92 | 8.51 | 19.98 | 16.49 | 14.27 | 8.53 |
| 40 mg/ml. pH 5.0. NaCl 70 mM | #4 | 54.11 | 40.07 | 11.47 | 12.98 | 20.19 | 31.31 | 14.23 | 15.65 |
| 60 mg/ml. pH 5.0. NaCl 110 mM | #5 | 54.07 | 42.53 | 12.28 | 12.97 | 20.09 | 29.76 | 13.56 | 14.76 |
| 20 mg/ml. pH 7.5. NaCl 40 mM | #6 | 55.30 | 73.29 | 11.40 | 5.50 | 19.25 | 16.17 | 14.06 | 5.55 |
| 40 mg/ml. pH 6.1. NaCl 50 mM | #7 | 54.76 | 59.63 | 12.22 | 10.40 | 19.85 | 19.98 | 13.18 | 9.99 |
| 60 mg/ml. pH 7.5. NaCl 150 mM | #8 | 53.97 | 73.01 | 12.47 | 5.64 | 19.17 | 45.39 | 14.41 | 5.97 |
| 60 mg/ml. pH 5.5. NaCl 100 mM | #9 | 53.90 | 54.54 | 12.52 | 11.58 | 19.57 | 23.86 | 14.03 | 10.03 |
| 20 mg/ml. pH 7.1. NaCl 100 mM | #10 | 55.12 | 70.57 | 11.89 | 6.54 | 18.87 | 15.38 | 14.12 | 7.52 |
| 40 mg/ml. pH 6.1. NaCl 50 mM | #11 | 54.65 | 60.76 | 11.73 | 9.72 | 19.36 | 19.34 | 14.27 | 10.19 |
| 20 mg/ml. pH 5.5. NaCl 150 mM | #12 | 54.89 | 56.08 | 12.40 | 11.03 | 19.32 | 22.40 | 13.41 | 10.50 |
| 20 mg/ml. pH 6.2. NaCl 70 mM | #13 | 54.55 | 62.81 | 11.82 | 9.58 | 21.58 | 19.01 | 12.06 | 8.61 |
| 40 mg/ml. pH 5.8. NaCl 115 mM | #14 | 53.45 | 58.04 | 14.28 | 10.61 | 21.55 | 20.19 | 10.73 | 11.17 |
| 60 mg/ml. pH 7.5. NaCl 100 mM | #15 | 56.34 | 71.28 | 13.28 | 5.74 | 20.81 | 16.43 | 9.58 | 6.56 |
| 60 mg/ml. pH 7.0. NaCl 100 mM | #16 | 53.95 | 68.13 | 12.80 | 7.32 | 20.74 | 16.89 | 11.48 | 7.64 |
| 60 mg/ml. pH 6.1. NaCl 100 mM | #17 | 53.46 | 59.76 | 13.82 | 10.80 | 21.71 | 20.48 | 11.03 | 8.98 |
| 40 mg/ml. pH 7.5. NaCl 100 mM | #18 | 55.86 | 71.51 | 13.65 | 5.77 | 21.20 | 17.23 | 9.60 | 5.50 |
| 40 mg/ml. pH 5.3. NaCl 150 mM | #19 | 52.29 | 48.87 | 13.42 | 12.67 | 21.75 | 26.74 | 12.54 | 11.73 |
| 20 mg/ml. pH 5.3. NaCl 70 mM | #20 | 52.68 | 48.65 | 13.37 | 13.68 | 21.75 | 25.31 | 12.45 | 12.37 |
| 10 mg/ml. pH 5.5. NaCl 40 mM | Reference #1 | 54.25 | 49.40 | 12.52 | 12.36 | 19.95 | 25.20 | 13.29 | 13.05 |

Together, FIG. 10 and FIG. 11 suggest that ionic strength and protein concentration have little impact on oxidation levels and only a pH above 7.0 shows a significant impact on oxidation levels. However, a pH in the range of 5.3-6.6 (most preferably 5.7-6.2) appears most favourable in the context of thermally-promoted oxidation.

*Light stress*

Table 3F shows %HMW species before and after light stressing. Light stress increases %HMW for all samples, although there appears to be a somewhat lower level of aggregates between pH 5.2 and 6.6 and a significant effect of ionic strength.

**Table 3F** - %HMW species before and after light stress (7 hours at 765 W/m$^2$)

| Protein Concentration, pH, Ionic strenght | Sample | Light stress (7 hours at 765 W/m2) | |
|---|---|---|---|
| | | Time 0 | After stress |
| 60 mg/ml, pH 5.7, NaCl 150 mM | #1 | 1,0 | 3,4 |
| 40 mg/ml, pH 6.8, NaCl 150 mM | #2 | 1,8 | 4,8 |
| 20 mg/ml, pH 6.6, NaCl 150 mM | #3 | 1,1 | 2,3 |
| 40 mg/ml, pH 5.0, NaCl 70 mM | #4 | 0,8 | 4,5 |
| 60 mg/ml, pH 5.0, NaCl 110 mM | #5 | 0,9 | 4,7 |
| 20 mg/ml, pH 7.5, NaCl 40 mM | #6 | 2,1 | 5,1 |
| 40 mg/ml, pH 6.1, NaCl 50 mM | #7 | 1,5 | 4,4 |
| 60 mg/ml, pH 7.5, NaCl 150 mM | #8 | 2,9 | 7,8 |
| 60 mg/ml, pH 5.5, NaCl 100 mM | #9 | 0,9 | 4,0 |
| 20 mg/ml, pH 7.1, NaCl 100 mM | #10 | 1,5 | 3,4 |
| 40 mg/ml, pH 6.1, NaCl 50 mM | #11 | 1,7 | 4,6 |
| 20 mg/ml, pH 5.5, NaCl 150 mM | #12 | 0,6 | 1,6 |
| 20 mg/ml, pH 6.2, NaCl 70 mM | #13 | 1,0 | 2,4 |
| 40 mg/ml, pH 5.8, NaCl 115 mM | #14 | 1,0 | 3,4 |
| 60 mg/ml, pH 7.5, NaCl 100 mM | #15 | 3,1 | 9,4 |
| 60 mg/ml, pH 7.0, NaCl 100 mM | #16 | 2,5 | 7,1 |
| 60 mg/ml, pH 6.1, NaCl 100 mM | #17 | 1,5 | 4,9 |
| 40 mg/ml, pH 7.5, NaCl 100 mM | #18 | 2,8 | 7,6 |
| 40 mg/ml, pH 5.3, NaCl 150 mM | #19 | 0,8 | 3,6 |
| 20 mg/ml, pH 5.3, NaCl 70 mM | #20 | 0,7 | 2,7 |
| 10 mg/ml, pH 5.5, NaCl 40 mM | Reference#1 | 0,7 | 1,9 |

cIEF measurements of samples exposed to light stress suggest no significant differences compared to the reference standard, except for samples 8, 15, 16, 18, and 19.

Table 3G shows Isoform clusters after light stress.

**Table 3G** - Isoform clusters after light stress (7 hours at 765 W/m$^2$)

| Protein concentration, pH, ionic strength | sample | Cluster 1 | | Cluster 2 | | Cluster 3 | | Cluster 4 | |
|---|---|---|---|---|---|---|---|---|---|
| | | T0 | light | T0 | light | T0 | light | T0 | light |
| 60 mg/ml. pH 5.7. NaCl 150 mM | #1 | 53.26 | 58.07 | 12.32 | 11.66 | 19.90 | 20.11 | 14.54 | 10.17 |

(continued)

| Protein concentration, pH, ionic strength | sample | Cluster 1 | | Cluster 2 | | Cluster 3 | | Cluster 4 | |
|---|---|---|---|---|---|---|---|---|---|
| | | T0 | light | T0 | light | T0 | light | T0 | light |
| 40 mg/ml. pH 6.8. NaCl 150 mM | #2 | 53.93 | 59.24 | 11.74 | 13.54 | 19.82 | 18.09 | 14.52 | 9.14 |
| 20 mg/ml. pH 6.6. NaCl 150 mM | #3 | 53.83 | 61.35 | 11.92 | 11.15 | 19.98 | 19.06 | 14.27 | 8.44 |
| 40 mg/ml. pH 5.0. NaCl 70 mM | #4 | 54.11 | 55.40 | 11.47 | 13.08 | 20.19 | 20.68 | 14.23 | 10.85 |
| 60 mg/ml. pH 5.0. NaCl 110 mM | #5 | 54.07 | 54.83 | 12.28 | 13.82 | 20.09 | 20.63 | 13.56 | 10.73 |
| 20 mg/ml. pH 7.5. NaCl 40 mM | #6 | 55.30 | 61.90 | 11.40 | 10.69 | 19.25 | 17.22 | 14.06 | 10.19 |
| 40 mg/ml. pH 6.1. NaCl 50 mM | #7 | 54.76 | 58.48 | 12.22 | 11.60 | 19.85 | 19.26 | 13.18 | 10.66 |
| 60 mg/ml. pH 7.5. NaCl 150 mM | #8 | 53.97 | na | 12.47 | na | 19.17 | na | 14.41 | na |
| 60 mg/ml. pH 5.5. NaCl 100 mM | #9 | 53.90 | 55.65 | 12.52 | 13.82 | 19.57 | 19.61 | 14.03 | 10.58 |
| 20 mg/ml. pH 7.1. NaCl 100 mM | #10 | 55.12 | 59.52 | 11.89 | 12.04 | 18.87 | 18.06 | 14.12 | 10.39 |
| 40 mg/ml. pH 6.1. NaCl 50 mM | #11 | 54.65 | 58.54 | 11.73 | 11.91 | 19.36 | 19.81 | 14.27 | 9.76 |
| 20 mg/ml. pH 5.5. NaCl 150 mM | #12 | 54.89 | 54.69 | 12.40 | 13.25 | 19.32 | 21.28 | 13.41 | 10.80 |
| 20 mg/ml. pH 6.2. NaCl 70 mM | #13 | 54.55 | 57.04 | 11.82 | 12.37 | 21.58 | 19.95 | 12.06 | 53.97 |
| 40 mg/ml. pH 5.8. NaCl 115 mM | #14 | 53.45 | 56.26 | 14.28 | 12.70 | 21.55 | 19.82 | 10.73 | 11.23 |
| 60 mg/ml. pH 7.5. NaCl 100 mM | #15 | 56.34 | na | 13.28 | na | 20.81 | na | 9.58 | na |
| 60 mg/ml. pH 7.0. NaCl 100 mM | #16 | 53.95 | na | 12.80 | na | 20.74 | na | 11.48 | na |
| 60 mg/ml. pH 6.1. NaCl 100 mM | #17 | 53.46 | 58.30 | 13.82 | 12.48 | 21.71 | 19.46 | 11.03 | 9.77 |
| 40 mg/ml. pH 7.5. NaCl 100 mM | #18 | 55.86 | na | 13.65 | na | 21.20 | na | 9.60 | na |
| 40 mg/ml. pH 5.3. NaCl 150 mM | #19 | 52.29 | na | 13.42 | na | 21.75 | na | 12.54 | na |
| 20 mg/ml. pH 5.3. NaCl 70 mM | #20 | 52.68 | 54.80 | 13.37 | 13.19 | 21.75 | 20.61 | 12.45 | 11.41 |
| 10 mg/ml. pH 5.5. NaCl 40 mM | Reference #1 | 54.25 | 54.48 | 12.52 | 13.43 | 19.95 | 21.50 | 13.29 | 10.60 |

Together FIG. 12, FIG. 13, and FIG. 14 suggest that neither ionic strength, protein concentration, nor pH make a significant impact on % oxidation under light stressed conditions.

*Conclusions*

FIG. 15 is a 2D and a 3D contour plot with a desirability parameter (reflecting a balance of factors in the overall response evaluation) represented as contours (in the 2D plot) and curved surface (in the 3D plot) within a formulation space (having a fixed protein concentration of 20 mg/mL) with variable pH and ionic strength (given as mM NaCl). This suggests optimal conditions at this concentration of IgG:TGFβR2 are pH 5.4-6.1, such as pH 5.7, and an ionic strength of 40-60 mM NaCl, such as 40 mM NaCl. Accordingly, for a pharmaceutical composition comprising 10-30 mg/mL IgG:TGFβR2 fusion protein, a pH of 5.2-6.3 is desirable together with a minimal ionic strength, such as 10 mM NaCl, and preferably an ionic strength in the range of 10-80 mM NaCl.

FIG. 16 is a 2D and a 3D contour plot with a desirability parameter (reflecting a balance of factors in the overall response evaluation) represented as contours (in the 2D plot) and curved surface (in the 3D plot) within a formulation space (having a fixed protein concentration of 40 mg/mL) with variable pH and ionic strength (given as mM NaCl). This suggests optimal conditions at this concentration of IgG:TGFβR2 are pH 5.7-6.1, such as pH 5.9, and an ionic strength of 50-70 mM NaCl, such as 60 mM NaCl. Accordingly, for a pharmaceutical composition comprising 30-50 mg/mL IgG:TGFβR2 fusion protein, a pH of 5.5-6.5 is desirable together with a minimal ionic strength, such as 20 mM NaCl, and preferably an ionic strength in the range of 30-90 mM NaCl.

FIG. 17 is a 2D and a 3D contour plot with a desirability parameter (reflecting a balance of factors in the overall response evaluation) represented as contours (in the 2D plot) and curved surface (in the 3D plot) within a formulation space (having a fixed protein concentration of 60 mg/mL) with variable pH and ionic strength (given as mM NaCl). This suggests optimal conditions at this concentration of IgG:TGFβR2 are pH 5.7-6.1, such as pH 5.9, and an ionic strength of 130-150 mM NaCl, such as 150 mM NaCl. Optimal conditions at this concentration of IgG:TGFβR2 are also found at pH 5.7-6.1, such as pH 5.9, and an ionic strength of 80-90 mM NaCl, such as 80 mM NaCl. Accordingly, for a pharmaceutical composition comprising 50-70 mg/mL IgG:TGFβR2 fusion protein, a pH of 5.5-6.5 is desirable together with a minimal ionic strength, such as 50 mM NaCl, and preferably an ionic strength in the range of 60-200 mM NaCl.

### EXAMPLE 3B - *Further screening studies - Step 1B (buffer screen)*

*General Overview*

Following on from the results of Example 3A, experiments were performed to both confirm the results of Example 3A and explore alternative buffers. As such, three buffer types have been selected, to cover the pH range 5.7 - 6.2. Ionic strength was optimized based on protein concentration, as per results from Example 3A.

*List of Tested Formulations*

Table 3H shows the 12 formulations, plus a reference formulation, tested in this particular screen..

**Table 3H** - Formulations for testing

| Buffer Type/pH range | Formulation No | Protein conc. (mg/ml) | Salt conc. NaCl (mM) | pH selected |
|---|---|---|---|---|
| 10 mM Histidine (suitable pH range for histidine is generally 5.5-7.4) | 33 | 20 | 40 | 5.7 |
| | 34 | 30 | 50 | 5.8 |
| | 35 | 40 | 60 | 5.9 |
| | 36 | 60 | 150 | 5.9 |
| 10 mM Succinate (suitable pH range for succinate is generally 3.6 - 6.2) | 37 | 20 | 40 | 5.7 |
| | 38 | 30 | 50 | 5.8 |
| | 39 | 40 | 60 | 5.9 |
| | 40 | 60 | 150 | 5.9 |

(continued)

| Buffer Type/pH range | Formulation No | Protein conc. (mg/ml) | Salt conc. NaCl (mM) | pH selected |
|---|---|---|---|---|
| 10 mM Citrate (suitable pH range for citrate is generally pH 2.7 - 7.0) | 25 | 20 | 40 | 5.7 |
| | 26 | 30 | 50 | 5.8 |
| | 27 | 40 | 60 | 5.9 |
| | 28 | 60 | 150 | 5.9 |
| 10 mM Histidine (ref.) | ref2 | 10 | 40 | 5.5 |

*Results and Analysis*

The formulations listed in Table 3H were investigated under thermal stressing conditions (4 weeks at 40°C) and light-stress conditions (7 hours at 765 W/m$^2$). Thermally stressed samples were examined by the following methods, though only the bold-typed methods revealed significant differences:

| Methods used | Methods revealing significant differents |
|---|---|
| Protein content by OD | HMWs by SE-UPLC |
| HMWs by SE-UPLC | Isoforms profiles by ICE3 |
| Isoforms profiles by ICE3 | Purity/LMW by CGE-SDS (Red/NoRed) |
| Purity/LMW by CGE-SDS (Red/NoRed) | Deamidated Forms by IEX-HPLC |
| Oxidized Forms by RP-UPLC | |
| Deamidated Forms by IEX-HPLC | |
| Turbidity by Nephelometry | |
| Appearance by Visual Inspection | |
| Unfolding Temperature by nano-DSC | |

Light-stressed samples were meanwhile examined by:

- HMWs by SE-UPLC

- Isoforms profiles by ICE3

- Oxidized Forms by RP-UPLC

- Purity/LMW by CGE-SDS (Red/NoRed)

Qualitative results combining both thermal and light-stress conditions are shown in Table below.
Table 3I shows qualitative results (pass or fail) of both thermal and light-stress tests on all of the formulations of Table 3H.

**Table 3I** - Qualitative results combining thermal and light-stress of formulations of Table 3H (N.T. = not tested)

| protein concentration, pH, Ionic strenght | Sample | %HMWs by SEC | Isoform by iCE | % Purity by CGE | % peak 7 by CGE | %LMWs by CGE | % Deam. by IEX |
|---|---|---|---|---|---|---|---|
| 20 mg/ml, histidine 10 mM, pH 5.7, NaCl 40 mM | #33 | pass | pass | pass | pass | pass | pass |

(continued)

| protein concentration, pH, Ionic strenght | Sample | %HMWs by SEC | Isoform by iCE | % Purity by CGE | % peak 7 by CGE | %LMWs by CGE | % Deam. by IEX |
|---|---|---|---|---|---|---|---|
| 30 mg/ml, histidine 10 mM, pH 5.8, NaCl 50 mM | #34 | pass | pass | pass | pass | pass | pass |
| 40 mg/ml, histidine 10 mM, pH 5.9, NaCl 60 mM | #35 | pass | pass | pass | fail | pass | pass |
| 60 mg/ml, histidine 10 mM, pH 5.9, NaCl 150 mM | #36 | pass | pass | pass | pass | pass | pass |
| 20 mg/ml, citrate 10 mM, pH 5.7, NaCl 40 mM | #25 | fail | fail | pass | fail | N.T. | fail |
| 30 mg/ml, citrate 10 mM, pH 5.8, NaCl 50 mM | #26 | fail | fail | pass | fail | N.T. | fail |
| 40 mg/ml, citrate 10 mM, pH 5.9, NaCl 60 mM | #27 | fail | fail | pass | pass | N.T. | fail |
| 60 mg/ml, citrate 10 mM, pH 5.9, NaCl 150 mM | #28 | fail | fail | pass | fail | N.T. | fail |
| 20 mg/ml, succinate 10 mM, pH 5.7, NaCl 40 mM | #37 | pass | pass | pass | pass | pass | pass |
| 30 mg/ml, succinate 10 mM, pH 5.8, NaCl 50 mM | #38 | pass | fail | pass | pass | pass | pass |
| 40 mg/ml, succinate 10 mM, pH 5.9, NaCl 60 mM | #39 | fail | fail | pass | fail | pass | fail |
| 60 mg/ml, succinate 10 mM, pH 5.9, NaCl 150 mM | #40 | fail | fail | fail | pass | fail | fail |

*Conclusions*

The results suggest histidine is the most stabilizing buffer of the three tested buffers, including at high protein concentrations, though other buffers, especially succinate buffer, are suitable too under appropriate conditions. Moreover, these tests underscore the feasibility of operating at higher protein concentrations, such as 40 and 60 mg/ml.

*EXAMPLE 3C - Further screening studies - Step 2A (excipient screening)*

*General Overview*

Following on from the results of Example 3B, a screen of excipients was performed keeping the buffer fixed as 10 mM histidine, pH 5.9, whilst still varying the ionic strength between 60 mM and 100 mM and the protein (bintrafusp alfa) concentration between 40-60 mg/mL. 5 mM methionine was also included in all formulations in this example in order to mitigate oxidation effects self-evident from the previous analyses.

The excipients to be screened were:

- Sugar polyols, such as Mannitol, Trehalose, Sorbitol;

- Amino acids, such as: Arginine, Lysine, Proline, Glutamic acid; and

- Surfactants, such as: Tween 20 and Kolliphor 188

The relevant formulations would be stress tested in the following manner before being subject to further analysis:

- Thermal Stress (40°C up to 12 weeks)

- Light Stress (after 7 hours at 765 W/m$^2$)

- F/T Stress (after 3X Cycles -25°C to RT)

- Mechanical Stress (after 3 days at 300 rpm)

*List of Tested Formulations*

Table 3J shows the 48 formulations tested in this particular screen

**Table 3J** - Formulations for testing

| No. | bintrafusp alfa | Buffer | pH | Antioxidant | Surfactant | NaCl | Varied Excipient |
|---|---|---|---|---|---|---|---|
| #41 | 40 mg/ml | 10 mM histidine | 5.9 | 5mM methionine | 0.1 mg/ml polysorbate 20 | 60 mM | 100 mM Mannitol |
| #42 | 40 mg/ml | 10 mM histidine | 5.9 | 5mM methionine | 0.1 mg/ml kolliphor 188 | 60 mM | 100 mM Mannitol |
| #43 | 50 mg/ml | 10 mM histidine | 5.9 | 5mM methionine | 0.1 mg/ml polysorbate 20 | 100 m M | 100 mM Mannitol |
| #44 | 50 mg/ml | 10 mM histidine | 5.9 | 5mM methionine | 0.1 mg/ml kolliphor 188 | 100 mM | 100 mM Mannitol |
| #45 | 60 mg/ml | 10 mM histidine | 5.9 | 5 mM methionine | 0.1 mg/ml polysorbate 20 | 100 m M | 100 mM Mannitol |
| #46 | 60 mg/ml | 10 mM histidine | 5.9 | 5 mM methionine | 0.1 mg/ml kolliphor 188 | 100 m M | 100 mM Mannitol |
| #47 | 40 mg/ml | 10 mM histidine | 5.9 | 5 mM methionine | 0.1 mg/ml polysorbate 20 | 60 mM | 100 mM trehalose dihydrate |
| #48 | 40 mg/ml | 10 mM histidine | 5.9 | 5 mM methionine | 0.1 mg/ml kolliphor 188 | 60 mM | 100 mM trehalose dihydrate |
| #49 | 50 mg/ml | 10 mM histidine | 5.9 | 5 mM methionine | 0.1 mg/ml polysorbate 20 | 100 m M | 100 mM trehalose dihydrate |
| #50 | 50 mg/ml | 10 mM histidine | 5.9 | 5 mM methionine | 0.1 mg/ml kolliphor 188 | 100 m M | 100 mM trehalose dihydrate |
| #51 | 60 mg/ml | 10 mM histidine | 5.9 | 5 mM methionine | 0.1 mg/ml polysorbate 20 | 100 m M | 100 m M trehalose dihydrate |
| #52 | 60 mg/ml | 10 mM histidine | 5.9 | 5 mM methionine | 0.1 mg/ml kolliphor 188 | 100 mM | 100 mM trehalose dihydrate |
| #53 | 40 mg/ml | 10 mM histidine | 5.9 | 5 mM methionine | 0.1 mg/ml polysorbate 20 | 60 mM | 100 mM sorbitol |
| #54 | 40 mg/ml | 10 mM histidine | 5.9 | 5 mM methionine | 0.1 mg/ml kolliphor 188 | 60 mM | 100 mM sorbitol |
| #55 | 50 mg/ml | 10 mM histidine | 5.9 | 5 mM methionine | 0.1 mg/ml polysorbate 20 | 100 m M | 100 mM sorbitol |
| #56 | 50 mg/ml | 10 mM histidine | 5.9 | 5 mM methionine | 0.1 mg/ml kolliphor 188 | 100 m M | 100 mM sorbitol |
| #57 | 60 mg/ml | 10 mM histidine | 5.9 | 5 mM methionine | 0.1 mg/ml polysorbate 20 | 100 m M | 100 mM sorbitol |

(continued)

| No. | bintrafusp alfa | Buffer | pH | Antioxidant | Surfactant | NaCl | Varied Excipient |
|-----|-----------------|--------|-----|-------------|-----------|------|------------------|
| #58 | 60 mg/ml | 10 mM histidine | 5.9 | 5 mM methionine | 0.1 mg/ml kolliphor 188 | 100 m M | 100 mM sorbitol |
| #59 | 40 mg/ml | 10 mM histidine | 5.9 | 5 mM methionine | 0.1 mg/ml polysorbate 20 | 60 mM | 100 mM arginine monohydrochloride |
| #60 | 40 mg/ml | 10 mM histidine | 5.9 | 5 mM methionine | 0.1 mg/ml kolliphor 188 | 60 mM | 100 mM arginine monohydrochloride |
| #61 | 50 mg/ml | 10 mM histidine | 5.9 | 5 mM methionine | 0.1 mg/ml polysorbate 20 | 100 m M | 100 mM arginine monohydrochloride |
| #62 | 50 mg/ml | 10 mM histidine | 5.9 | 5 mM methionine | 0.1 mg/ml kolliphor 188 | 100 m M | 100 mM arginine monohydrochloride |
| #63 | 60 mg/ml | 10 mM histidine | 5.9 | 5 mM methionine | 0.1 mg/ml polysorbate 20 | 100 m M | 100 mM arginine monohydrochloride |
| #64 | 60 mg/ml | 10 mM histidine | 5.9 | 5 mM methionine | 0.1 mg/ml kolliphor 188 | 100 m M | 100 mM arginine monohydrochloride |
| #65 | 40 mg/ml | 10 mM histidine | 5.9 | 5 mM methionine | 0.1 mg/ml polysorbate 20 | 60 mM | 50 mM arginine monohydrochloride |
| #66 | 40 mg/ml | 10 mM histidine | 5.9 | 5 mM methionine | 0.1 mg/ml kolliphor 188 | 60 mM | 50 mM arginine monohydrochloride |
| #67 | 50 mg/ml | 10 mM histidine | 5.9 | 5 mM methionine | 0.1 mg/ml polysorbate 20 | 100 m M | 50 mM arginine monohydrochloride |
| #68 | 50 mg/ml | 10 mM histidine | 5.9 | 5 mM methionine | 0.1 mg/ml kolliphor 188 | 100 m M | 50 mM arginine monohydrochloride |
| #69 | 60 mg/ml | 10 mM histidine | 5.9 | 5 mM methionine | 0.1 mg/ml polysorbate 20 | 100 m M | 50 mM arginine monohydrochloride |
| #70 | 60 mg/ml | 10 mM histidine | 5.9 | 5 mM methionine | 0.1 mg/ml kolliphor 188 | 100 m M | 50 mM arginine monohydrochloride |
| #71 | 40 mg/ml | 10 m M histidine | 5.9 | 5 mM methionine | 0.1 mg/ml polysorbate 20 | 60 mM | 100 mM lysine monohydrochloride |
| #72 | 40 mg/ml | 10 mM histidine | 5.9 | 5 mM methionine | 0.1 mg/ml kolliphor 188 | 60 mM | 100 mM lysine monohydrochloride |
| #73 | 50 mg/ml | 10 mM histidine | 5.9 | 5 mM methionine | 0.1 mg/ml polysorbate 20 | 100 m M | 100 mM lysine monohydrochloride |
| #74 | 50 mg/ml | 10 mM histidine | 5.9 | 5 mM methionine | 0.1 mg/ml kolliphor 188 | 100 m M | 100 mM lysine monohydrochloride |
| #75 | 60 mg/ml | 10 mM histidine | 5.9 | 5 mM methionine | 0.1 mg/ml polysorbate 20 | 100 m M | 100 mM lysine monohydrochloride |
| #76 | 60 mg/ml | 10 mM histidine | 5.9 | 5 mM methionine | 0.1 mg/ml kolliphor 188 | 100 m M | 100 mM lysine monohydrochloride |
| #77 | 40 mg/ml | 10 mM histidine | 5.9 | 5 mM methionine | 0.1 mg/ml polysorbate 20 | 60 mM | 100 mM proline |
| #78 | 40 mg/ml | 10 mM histidine | 5.9 | 5 mM methionine | 0.1 mg/ml kolliphor 188 | 60 mM | 100 mM proline |
| #79 | 50 mg/ml | 10 mM histidine | 5.9 | 5 mM methionine | 0.1 mg/ml polysorbate 20 | 100 m M | 100 mM proline |

(continued)

| No. | bintrafusp alfa | Buffer | pH | Antioxidant | Surfactant | NaCl | Varied Excipient |
|-----|-----------------|--------|----|-----|-----|-----|-----|
| #80 | 50 mg/ml | 10 mM histidine | 5.9 | 5 mM methionine | 0.1 mg/ml kolliphor 188 | 100 m M | 100 mM proline |
| #81 | 60 mg/ml | 10 mM histidine | 5.9 | 5 mM methionine | 0.1 mg/ml polysorbate 20 | 100 m M | 100 mM proline |
| #82 | 60 mg/ml | 10 mM histidine | 5.9 | 5 mM methionine | 0.1 mg/ml kolliphor 188 | 100 m M | 100 mM proline |
| #83 | 40 mg/ml | 10 mM histidine | 5.9 | 5 mM methionine | 0.1 mg/ml polysorbate 20 | 60 mM | 70 mM glutamic acid |
| #84 | 40 mg/ml | 10 mM histidine | 5.9 | 5 mM methionine | 0.1 mg/ml kolliphor 188 | 60 mM | 70 mM glutamic acid |
| #85 | 50 mg/ml | 10 mM histidine | 5.9 | 5 mM methionine | 0.1 mg/ml polysorbate 20 | 100 m M | 70 mM glutamic acid |
| #86 | 50 mg/ml | 10 mM histidine | 5.9 | 5 mM methionine | 0.1 mg/ml kolliphor 188 | 100 m M | 70 mM glutamic acid |
| #87 | 60 mg/ml | 10 mM histidine | 5.9 | 5 mM methionine | 0.1 mg/ml polysorbate 20 | 100 m M | 70 mM glutamic acid |
| #88 | 60 mg/ml | 10 mM histidine | 5.9 | 5 mM methionine | 0.1 mg/ml kolliphor 188 | 100 m M | 70 mM glutamic acid |

## Results and Analysis

The formulations listed in Table 3J were investigated under thermal stressing conditions (4 weeks and 8 weeks at 40°C), light-stress conditions (7 hours at 765 W/m$^2$), freeze-thaw stress (3 F/T cycles, -25°C to room temperature), and mechanical stress (300 rpm for 3 days).

Thermally stressed samples were examined by the following methods:

| Methods Used | Methods revealing significant differents |
|-----|-----|
| Protein content by OD | HMWs by SE-UPLC |
| HMWs by SE-UPLC | Isoforms profiles by ICE3 |
| Isoforms profiles by ICE3 | Purity/LMW by CGE-SDS (Red/NoRed) |
| Purity/LMW by CGE-SDS (Red/NoRed) | Oxidized Forms by RP-UPLC |
| Oxidized Forms by RP-UPLC | Deamidated Forms by IEX-HPLC |
| Deamidated Forms by IEX-HPLC | |
| Turbidity by Nephelometry | |
| Appearance by Visual Inspection | |
| Unfolding Temperature by nano-DSC | |

Light stressed samples were examined by the following methods:

| Methods Used | Methods revealing significant differents |
|-----|-----|
| HMWs by SE-UPLC | HMWs by SE-UPLC |
| Isoforms profiles by ICE3 | Oxidized Forms by RP-UPLC |
| Oxidized Forms by RP-UPLC | Purity/LMW by CGE-SDS (Red/NoRed) |

(continued)

| Methods Used | Methods revealing significant differents |
|---|---|
| Purity/LMW by CGE-SDS (Red/NoRed) | |

F/T and mechanically stressed samples were examined by the following methods:

- HMWs by SE-UPLC

- Purity/LMW by CGE-SDS (Red/NoRed)

*Thermal stress*

Together, FIG. 18 and FIG. 19 suggest that protein concentration has a significant effect on %HMW under thermal stress, but that 50 mM arginine and 100 mM lysine are the best performing "varied excipients" over 4 weeks whilst 100 mM arginine and 100 mM lysine are the best performers over 8 weeks thermal stress.
Together FIG. 20 and FIG. 21 suggest that sorbitol (at 40 and 50 mg/mL protein concentration) and mannitol (at 60 mg/mL protein concentration) show the highest main clipping, that 100 mM arginine exibits the highest LMW% and trehalose the lowest, and that protein concentration has no significant effect after 4 weeks under thermal stress.
FIG. 22 suggests trehalose and 50 mM arginine exhibit the lowest % deamination under thermal stress, while L-glutamic acid, lysine and proline showed the highest one.
Together, FIG. 23 and 24 suggest that polysorbate 20 exhibits superior performance to Kolliphor 188 and that sorbitol in fact demonstrates the lowest % oxidated forms after 4 weeks of thermal stress. Meanwhile, 50 mM L-arginine would appear to show highest oxidated forms upon thermal stress for 8 weeks (protein concentration was fixed, as it was shown to have no impact).
FIG. 25 suggests that proline is closest to the target % cluster 1 upon thermal stress, which is 52% (protein concentration was fixed, as it was shown to have no impact).
FIG. 26 suggests that mannitol and lysine exhibit lower % cluster 2 than the target, which is 12%, upon thermal stress.

*Light stress*

FIG. 27 suggests 100 mM arginine and lysine exhibit the lowest %HMW, and that protein concentration significantly impacts on %HMW under light-stressed conditions.
Together FIG. 28 and FIG. 29 suggest 100 mM arginine, sorbitol, and mannitol (the latter two only at 60 mg/mL protein concentration) exhibit the highest % main clipping, whereas 50 mM arginine exhibits the highest %LMW under light stressed conditions.
FIG. 30 suggests that 100 mM arginine provides the lowest % oxidation under light-stressed conditions.

*Freeze-thaw stress*

FIG. 31 suggests that mannitol exhibits the highest %HMW, and that protein concentration has no significant impact on %HMW under freeze-thaw stress.

*Mechanical stress*

Together, FIG. 32 and FIG. 33 suggest that polysorbate 20 is superior to kolliphor 188 in terms of preventing aggregation under mechanical stress (especially in combination with sorbitol, arginine, and lysine).

*Overall desirability*

The overall desirability of the different excipients was calculated based on a balance of factors, stress tests, and results.
FIG. 34 suggests an overall higher desirability for formulations having protein concentrations of 40 or 50 mg/mL relative to those at 60 mg/mL.
For formulations having 40 mg/mL protein concentration, a particularly desirable polyol is trehalose and particularly desirable amino acids are 50 mM arginine, and lysine.

For formulations having 50 mg/mL protein concentration, a particularly desirable polyol is trehalose and particularly desirable amino acids are 50 mM arginine, and lysine.

For formulations having 60 mg/mL protein concentration, a particularly desirable polyol is trehalose and particularly desirable amino acids are 50 mM arginine, and lysine.

FIG. 35 suggests a slightly higher desirability of polysorbate 20 across substantially all conditions, as compared to kolliphor 188.

### *EXAMPLE 3D -Step 2B (excipient combination and fine-tuning)*

#### *General Overview*

Based on the overall desirability results of Step 2A, it was decided to continue with a protein concentration of 40 and 50 mg/mL and to investigate the synergistic effect between a sugar and an aminoacid, preparing formulations that combine trehalose and arginine/lysine.

#### *List of Tested Formulations*

Table 3K shows the 12 formulations (+ reference) tested in this particular screen.

**Table 3K** - Formulations for testing

| No | bintrafusp alfa Conc. (mg/ml) | Histidine Conc. (mM) | pH | Methionine Conc. (mM) | NaCl (mM) | Excipient Type | Polysorbate 20 Conc. (mg/ml) | Rationale |
|---|---|---|---|---|---|---|---|---|
| #89 | 40 | 10 | 5.9 | 5 | 60 | Trehalose 100 mM/L-Arginine 50 mM | 0.5 | Combination of the two best performing excipients from step 2A |
| #90 | 40 | 10 | 5.9 | 5 | 60 | Trehalose 100 mM/L-Arginine 50 mM | 0.1 | Same than previous, with surfactant concentration from step 2A |
| #91 | 40 | 10 | 5.9 | 5 | 60 | Trehalose 75 mM/L-Arginine 75 mM | 0.5 | Changed ratio between the two excipients, both intermediate |
| #92 | 40 | 10 | 5.9 | 5 | 60 | Trehalose 50 mM/L-Arginine 100 mM | 0.5 | Changed ratio between the two excipients |
| #93 | 40 | 10 | 5.9 | 5 | 60 | Trehalose 100 mM/L-Lysine 50 mM | 0.5 | Same than formulation 89, with Lysine instead of Arginine |
| #94 | 40 | 10 | 5.9 | 5 | 60 | Trehalose 75 mM/L-Lysine 75 mM | 0.5 | Same than formulation 91, with Lysine instead of Arginine |
| #95 | 40 | 10 | 5.9 | 5 | 60 | Trehalose 50 mM/L-Lysine 100 mM | 0.5 | Same than formulation 92, with Lysine instead of Arginine |
| #96 | 50 | 10 | 5.9 | 5 | 100 | Trehalose 50 mM/L-Arginine 50 mM | 0.5 | Combination of the two best performing excipients from step 2A, with increased protein concentration and adjusted NaCl and excipient conc to meet osmolality |
| #97 | 50 | 10 | 5.9 | 5 | 100 | Trehalose 50 mM/L-Lysine 50 mM | 0.5 | Same than 96, with Lysine instead of Arginine |
| #98 | 40 | 10 | 5.5 | 5 | 60 | Trehalose 160mM/EDTA 0.05mM | 0.5 | Trehalose as fragmentation preventing agent and EDTA as aggregates preventing agent |

(continued)

| No | bintrafusp alfa Conc. (mg/ml) | Histidine Conc. (mM) | pH | Methionine Conc. (mM) | NaCl (mM) | Excipient Type | Polysorbate 20 Conc. (mg/ml) | Rationale |
|---|---|---|---|---|---|---|---|---|
| #99 | 40 | 10 | 5.5 | 5 | 60 | Trehalose 160mM | 0.5 | Reference of formulation 98, without EDTA |
| #100 | 40 | 10 | 5.9 | 5 | 80 | L-Arginine 50mM | 0.5 | Best performing aminoacid with increased NaCl concentration to meet osmolality |
| #101 | 40 | 10 | 5.9 | 5 | 80 | Trehalose 100 mM | 0.5 | Best performing polyol with increased NaCl concentration to meet osmolality |
| #Ref5 | 10 | 10 | 5.5 | 5 | 40 | Trehalose 160 mM | 0.5 | - |

*Results and Analysis*

The formulations listed in Table 3K were investigated under thermal stressing conditions (4 weeks, 8 weeks, and 12 weeks at 40°C), light-stress conditions (7 hours at 765 W/m$^2$), and mechanical stress (300 rpm for 3 days).

Thermally stressed samples were examined by the following methods:

| Methods used | Methods revealing significant differences |
|---|---|
| Protein content by OD | HMWs by SE-UPLC |
| HMWs by SE-UPLC | Isoforms profiles by ICE3 |
| Isoforms profiles by ICE3 | Purity/LMW by CGE-SDS (Red/NoRed) |
| Purity/LMW by CGE-SDS (Red/NoRed) | Oxidized Forms by RP-UPLC |
| Oxidized Forms by RP-UPLC | Deamidated Forms by IEX-HPLC |
| Deamidated Forms by IEX-HPLC | |
| Turbidity by Nephelometry | |
| Appearance by Visual Inspection | |
| Unfolding Temperature by nano-DSC | |

Light stressed samples were examined by the following methods:

| Methods used | Methods revealing significant differences |
|---|---|
| HMWs by SE-UPLC | HMWs by SE-UPLC |
| Isoforms profiles by ICE3 | Oxidized Forms by RP-UPLC |
| Oxidized Forms by RP-UPLC | Purity/LMW by CGE-SDS (Red/NoRed) |
| Purity/LMW by CGE-SDS (Red/NoRed) | |

Mechanically stressed samples were examined by the following methods:

- HMWs by SE-UPLC

- Purity/LMW by CGE-SDS (Red/NoRed)

Table 3L shows the nano-DSC results for the 12 formulations (+ reference) tested in this particular screen.

**Table 3L** - Nano-DSC results of formulations

| For all DPs (if not differently specified): 10 mM Histidine, 5mM Methionine, 0.5 mg/ml Tween20, pH 5.9 | | | | Main Tm |
|---|---|---|---|---|
| | Exc TYPE: | (mM/mM) | Formulation | |
| 40 mg/mL bintrafusp alfa, 60 mM NaCl, 0.1 mg/ml Tween20 | Tre/Arg | 100/50 | #89 | 69.38 |
| | | 100/50* | #90 | 67.80 |
| | | 75/75 | #91 | 69.13 |
| | | 50/100 | #92 | 66.98 |
| | Tre/Lys | 100/50 | #93 | 67.64 |
| | | 75/75 | #94 | 67.60 |
| | | 50/100 | #95 | 67.62 |

(continued)

| For all DPs (if not differently specified): 10 mM Histidine, 5mM Methionine, 0.5 mg/ml Tween20, pH 5.9 | | | | Main Tm |
|---|---|---|---|---|
| | Exc TYPE: | (mM/mM) | Formulation | |
| 50 mg/mL bintrafusp alfa, 100 mM NaCl | Tre/Arg | 50/50 | #96 | 68.72 |
| | Tre/Lys | 50/50 | #97 | 67.21 |
| 40 mg/mL bintrafusp alfa, 60 mM NaCl, pH5.5 | Tre/EDTA | 160/0,05 | #98 | 67.24 |
| | Tre | 160 mM | #99 | 67.47 |
| 40 mg/mL bintrafusp alfa, 80 mM NaCl | Arg | 50 mM | #100 | 66.90 |
| | Tre | 100 mM | #101 | 67.14 |
| 10 mg/mL bintrafusp alfa, 40 mM NaCl, pH 5.5 | - | - | Ref#5 (A') | 68.29 |

Table 3L suggests that formulations #89 and #91 have higher $T_m$ values, indicating higher conformational stability for these formulations. Comparing #89 and #90, it appears that an increased Tween 20 concentration yields a stabilizing effect.

FIG.36 suggests that: the increases in %HMW is comparable in combinations containing L-Arginine and containing L-Lysine; slightly higher values have been observed for formulations at 50 mg/ml protein concentration, with a combination of excipients (#96, #97); even higher values have been obtained for formulations with only 1 excipient (#100 and #101), and somewhat larger increases for formulations contaning EDTA. The results for formulations #89-#95 are better than those of 2A.5 of Table 2A (same protein concentration).

FIG. 37 suggests that the increases in %LMW are comparable in almost all formulations, with slightly higher values exhibited in formulations containing EDTA.

FIG. 38 suggests no significant differences for oxidation after 13 weeks of thermal stress, with only slightly lower value for formulation containing EDTA.

FIG. 39 suggests that all formulations exhibited similar deamidation after thermal stress.

FIG. 40 suggests slight decreases in purity across all formulations, albeit comparable for each.

FIG. 41 suggests an increase in the Main clipping (peak 6) in all formulations after thermal stress, with the highest increases observed for the formulation containing EDTA (#98), and formulations containing only trehalose with a lower amound of NaCl (#99 and reference). These same formulations are characterized also by a higher value of peak 7 impurity.

FIG. 42 suggests higher increases in %HMW are observed after light stress for formulations: with lysine at higher protein concentration of 50 mg/ml; with EDTA; and with only 1 excipient.

FIG. 43 suggests that % oxidation increases after light stress for all formulations, with slightly higher values observed in formulations containing EDTA, and slightly lower values for formulations containing only Trehalose (#101).

FIG. 44 suggests no significant differences in the level of LMWs after light stress for all formulations.

FIG. 45 suggests no significant changes in purity after light stressing, with the exception of formulation #99 which exhibited a slightly larger decrease after stress.

FIG. 46 suggests no significant changes occur in the level of Main clipping after light stress.

FIG. 47 suggests there to be only very slight variations in %HMWs after mechanical stress, albeit the starting value was relatively high for formulations containing EDTA, and more significant increases were observed in the cases of formulations #91 and #95.

FIG. 48 suggests there to be no significant variations in %LMWs after mechanical stress, with only moderate LMW increases observed in formulations #89 and #94.

FIG. 49 suggests a significant % purity decrease after mechanical stress for formulation #95, whilst only slight variations were observed for the other formulations.

FIG. 50 suggest no significant variations in % main clipping after mechanical stress, except for formulation #95 which contained the highest amount of Lysine.

*Conclusions*

From the data collected in this formulation screen, the best performing formulations under all conditions are those with a combination of excipients, in particular with Trehalose in combination with L-Arginine. The 2 formulations at a protein concentration of 50 mg/ml show comparable behaviour to those at 40 mg/ml, indicating that higher concentrations are indeed feasible. 2A.5 of Table 2A, containing 40 mg/ml bintrafusp alfa and 60 mM NaCl, which was used as comparison in the thermal stress tests, appeared to be largely comparable to the formulations with excipient combinations.

Overall, 2A.5 of Table 2A, formulation #89, formulation #91, and formulation #96 were considered particularly promising candidate formulations.

## *EXAMPLE 4 - Further formulation studies*

### *General Overview*

The general plan for this example was to initially screen samples at 20-25 mg/mL bintrafusp alfa concentration in order to assess pH, buffers, and excipients. Thereafter up-concentration and short-term stability testing have been performed for a selection of formulations, before subsequent surfactant screening, and eventual short-term stability testing of 4-5 formulations at high concentration.

### *List of Tested Formulations*

Table 4A shows 20 formulations (F1-F20), including a reference formulation (F1) to be tested in this particular screen.

**Table 4A** - Formulations for testing

| Formulation number | DS [mg/ml] | pH | Buffer system | Salt | Excipient | Antioxidant | PS20 | Rationale |
|---|---|---|---|---|---|---|---|---|
| F1 | 10 | 5.5 | 10 mM His | 40 mM NaCl | 160 mM Trehalose | 5 mM L-Met | 0.5 mg/ml | Early formulation of Example 1 |
| F2 | 25 | 5.5 | 10 mM His | 40 mM NaCl | 160 mM Trehalose | 5 mM L-Met | 0.5 mg/ml | Early formulation, higher concentration |
| F3 | 25 | 5.7 | 10 mM His | 40 mM NaCl | 160 mM Trehalose | 5 mM L-Met | 0.5 mg/ml | Buffer, pH screening |
| F4 | 25 | 6.0 | 10 mM His | 40 mM NaCl | 160 mM Trehalose | 5 mM L-Met | 0.5 mg/ml | |
| F5 | 25 | 6.0 | 10 mM Pho | 40 mM NaCl | 160 mM Trehalose | 5 mM L-Met | 0.5 mg/ml | |
| F6 | 25 | 6.0 | 10 mM Citrate | 40 mM NaCl | 160 mM Trehalose | 5 mM L-Met | 0.5 mg/ml | |
| F7 | 25 | 5.5 | 10 mM His | 40 mM NaCl | 90 mM Arginine | 5 mM L-Met | 0.5 mg/ml | Arginine |
| F8 | 25 | 5.5 | 10 mM His | 40 mM NaCl | 160 mM Sorbitol | 5 mM L-Met | 0.5 mg/ml | Sorbitol |
| F9 | 50 | 5.7 | - | 120 mM NaCl | 50 mM Trehalose | 5 mM L-Met | 0.5 mg/ml | Buffer free |
| F10 | 50 | 6.0 | - | 120 mM NaCl | 50 mM Trehalose | 5 mM L-Met | 0.5 mg/ml | |

(continued)

| Formulation number | DS [mg/ml] | pH | Buffer system | Salt | Excipient | Antioxidant | PS20 | Rationale |
|---|---|---|---|---|---|---|---|---|
| F11 | 50 | 5.5 | 10 mM His | 120 mM NaCl | 50 mM Trehalose | 5 mM L-Met | 0.5 mg/ml | Buffer, pH screening |
| F12 | 50 | 5.7 | 10 mM His | 120 mM NaCl | 50 mM Trehalose | 5 mM L-Met | 0.5 mg/ml | |
| F13 | 50 | 6.0 | 10 mM His | 120 mM NaCl | 50 mM Trehalose | 5 mM L-Met | 0.5 mg/ml | |
| F14 | 50 | 6.0 | 10 mM Pho | 120 mM NaCl | 50 mM Trehalose | 5 mM L-Met | 0.5 mg/ml | |
| F15 | 50 | 6.0 | 10 mM Citrate | 120 mM NaCl | 50 mM Trehalose | 5 mM L-Met | 0.5 mg/ml | |
| F16 | 50 | 6.0 | 10 mM His | 120 mM NaCl | 40 mM Arginine | 5 mM L-Met | 0.5 mg/ml | Arginine |
| F17 | 50 | 5.7 | - | 120 mM NaCl | 40 mM Arginine | 5 mM L-Met | 0.5 mg/ml | Arginine, buffer free, different pH |
| F18 | 50 | 6.0 | - | 120 mM NaCl | 40 mM Arginine | 5 mM L-Met | 0.5 mg/ml | |
| F19 | 50 | 6.0 | 10 mM His | 120 mM NaCl | 50 mM Sorbitol | 5 mM L-Met | 0.5 mg/ml | Sorbitol |
| F20 | 50 | 6.0 | 10 mM His | 40 mM NaCl | 120 mM Arginine | 5 mM L-met | 0.5 mg/ml | higher Arginine, lower salt concentration |

*Results and Analysis*

Formulations were stored and tested in accordance with the following summary set forth in Table 4B (tests that were performed are marked with an "x").

**Table 4B**

| Time point | T0 | T-FT | T-2w (25°C or 40°C) | T-4w (25°C or 40°C) |
|---|---|---|---|---|
| **Number of formulations** | 20 | 20 | 20 | 20 |
| **Analytical methods** | | | | |
| **HP-SEC** | x | x | x | x |
| **RP-UPLC oxidation method** | x | | | x (40°C) |
| **IEX** | x | x | x | x |
| **iCE3** | x | x | x | x |
| **UV spectroscopy** | x | x | x | x |

(continued)

| Analytical methods | | | | |
|---|---|---|---|---|
| MFI | x | x | x | x |
| Visual inspection | x | x | x | x |
| pH | x | x | x | x |
| Osmolality | x | | | |
| nDSF | x | x | x | x |
| Viscosity | x | | | |
| Turbidity | x | x | x | x |
| SDScGE | x | | | x (40°C) |

In this context, T0 is at time 0; T-FT is after 3 freeze-thaw cycles (FIG. 51) ; T-2w and T-4w means after 2 and 4 weeks respectively at either 25°C or 40°C.

FIG. 52 is a bar chart showing how pH varies in all of formulations F1-F20 at various time points of stress testing, including (bars listed from left-to-right for each formulation): time = 0; after 3 FT cycles; after 2 weeks at 25°C, after 2 weeks at 40°C, after 4 weeks at 25°C, after 4 weeks at 40°C.

The target pH for each formulation is set forth in Table 4A, and FIG. 52 illustrates how this changes.

For all formulations the target pH value ($\pm$ 0.1) was reached at T0.

After 4 weeks of storage pH values did not change for most of the formulations.

Lower pH values were detected for three formulations with a protein concentration of 50 mg/ml:

- F12 (His buffer pH 5.7) stored at 40 °C for 4 weeks.

- F17 (buffer free pH 5.7) stored at 40 °C for 4 weeks.

- F19 (containing sorbitol, pH 6.0) stored at 25 °C for 2 and 4 weeks.

FIG. 53 is a bar chart showing the osmolality for each of the formulations F1-F20.

Osmolality values within the specification range of 260 - 340 mOsmol/kg (green lines) were reached for all formulations - except for F15 (a lower osmolality value of 254 mOsmol/kg was detected).

FIG. 54 is a bar chart showing how turbidity varies in all of formulations F1-F20 at various time points of stress testing, including (bars listed from left-to-right for each formulation): time = 0; after 3 FT cycles; after 2 weeks at 25°C, after 2 weeks at 40°C, after 4 weeks at 25°C, after 4 weeks at 40°C.

Turbidity seems to be dependent on protein concentration - higher turbidity values were detected in samples of higher protein concentration. Turbidity values were generally unchanged for most of the formulations, though an increase of 0.5 to 2.0 was observed for the following formulations:

- F3 (His buffer pH 5.7) at T4w-25°C

- F13 (His buffer pH 6.0) at T2w-25°C and T2w-40°C

- F17 (buffer free pH 5.7) at T2w-25°C and T4w-25°C

An increase of 4.1 to 7.4 was observed for the following formulations:

- F19 (Sorbitol containing pH 6.0) at T2w-25°C and T4w-25°C

Visual inspections were performed and reported based on the codes set forth in Table 4C and Table 4D.

Table 4C - Coding for visibility of particles

| Score for visible particles | Description |
|---|---|
| 0 | No particles visible within 5 sec |

(continued)

| Score for visible particles | Description |
|---|---|
| 1 | Few particles visible within 5 sec |
| 2 | Medium number of particles visible within 5 sec |
| 10 | Large number of particles directly visible |

**Table 4D** - Coding for visible features

| Letter | Description |
|---|---|
| A | Air bubbles |
| C | Color |
| F | Fiber (single) |
| FF | Multiple fibers (more than one) |
| H | Hurricane, tornado, e.g., because of sedimenting or floating particles |
| L | Particles that are on the limit of being visible as distinct particles |
| S | Schlieren, phase separation |
| T | Turbidity, opalescence, cloudiness, haziness |
| V | Viscosity |
| X | Non-inherent particles: metal, glitter, rubber parts, glass sheds |

The results of visual inspections under the various above-mentioned stressing conditions and timepoints are set forth below in Table 4E, Table 4F, and Table 4G.

**Table 4E** - Visual inspection results at Time = 0 and after 3 FT cycles

| Formulation | T0 | | | T-FT | | |
|---|---|---|---|---|---|---|
| | Examiner1 | Examiner2 | Letter code | Examiner1 | Examiner2 | Letter code |
| F1 | 1 | 1 | FC | 0 | 0 | C |
| F2 | 0 | 0 | FTC | 0 | 0 | TCS |
| F3 | 0 | 0 | FTC | 1 | 0 | FTCS |
| F4 | 0 | 0 | FTC | 0 | 0 | TCS |
| F5 | 1 | 1 | FTC | 1 | 1 | FTCS |
| F6 | 0 | 0 | FTC | 0 | 0 | FTC |
| F7 | 1 | 1 | FTC | 0 | 0 | FTCS |
| F8 | 0 | 0 | TC | 1 | 1 | TC |
| F9 | 0 | 0 | FTC | 0 | 0 | TCS |
| F10 | 1 | 1 | TC | 0 | 0 | TCS |
| F11 | 0 | 0 | TC | 0 | 0 | TCS |
| F12 | 0 | 0 | TC | 0 | 0 | FTCS |
| F13 | 0 | 0 | TC | 0 | 0 | TC |
| F14 | 0 | 0 | TC | 0 | 0 | FTCS |
| F15 | 0 | 0 | FTC | 0 | 0 | FTCS |
| F16 | 0 | 0 | TC | 0 | 0 | TC |

(continued)

| Formulation | T0 | | | T-FT | | |
|---|---|---|---|---|---|---|
| | Examiner1 | Examiner2 | Letter code | Examiner1 | Examiner2 | Letter code |
| F17 | 0 | 0 | FTC | 0 | 0 | TCS |
| F18 | 0 | 0 | TC | 0 | 0 | TCS |
| F19 | 0 | 0 | FTC | 0 | 0 | FFTC |
| F20 | 0 | 0 | TC | 0 | 0 | TC |

**Table 4F** - Visual inspect results after 2 weeks at 25°C and 40°C

| Formulation | T-2w_25 °C | | | T-2w_40 °C | | |
|---|---|---|---|---|---|---|
| | Examiner1 | Examiner2 | Letter code | Examiner1 | Examiner2 | Letter code |
| F1 | 0 | 0 | FC | 0 | 0 | FC |
| F2 | 0 | 0 | TC | 0 | 0 | FTC |
| F3 | 1 | 1 | FFTC | 0 | 0 | FTC |
| F4 | 0 | 0 | TC | 0 | 0 | FTC |
| F5 | 0 | 0 | FTC | 0 | 0 | TC |
| F6 | 0 | 0 | TC | 0 | 0 | TC |
| F7 | 0 | 0 | FTC | 0 | 0 | TC |
| F8 | 0 | 0 | TC | 0 | 0 | TC |
| F9 | 0 | 0 | FTC | 0 | 0 | FTC |
| F10 | 0 | 0 | TC | 0 | 0 | TC |
| F11 | 1 | 1 | TC | 0 | 0 | FTC |
| F12 | 0 | 0 | TC | 0 | 0 | TC |
| F13 | 1 | 1 | FTC | 0 | 0 | TC |
| F14 | 0 | 0 | TC | 0 | 0 | TC |
| F15 | 0 | 0 | TC | 0 | 0 | TC |
| F16 | 0 | 0 | FTC | 0 | 0 | FTC |
| F17 | 0 | 0 | FTC | 0 | 0 | TC |
| F18 | 1 | 1 | CT | 0 | 0 | TC |
| F19 | 0 | 1 | FTC | 0 | 0 | FTC |
| F20 | 0 | 0 | FTC | 0 | 0 | TC |

**Table 4G** - Visual inspect results after 4 weeks at 25°C and 40°C

| Formulation | T-4w_25 °C | | | T-4w_40 °C | | |
|---|---|---|---|---|---|---|
| | Examiner1 | Examiner2 | Letter code | Examiner1 | Examiner2 | Letter code |
| F1 | 0 | 0 | TC | 1 | 1 | FC |
| F2 | 1 | 1 | FFTC | 1 | 1 | FTC |
| F3 | 1 | 1 | TC | 2 | 1 | FTC |
| F4 | 1 | 1 | TC | 0 | 0 | TC |

(continued)

| Formulation | T-4w_25 °C | | | T-4w_40 °C | | |
|---|---|---|---|---|---|---|
| | Examiner1 | Examiner2 | Letter code | Examiner1 | Examiner2 | Letter code |
| F5 | 0 | 0 | FFTC | 1 | 1 | FFTC |
| F6 | 0 | 0 | FTC | 0 | 0 | TC |
| F7 | 0 | 0 | FTC | 1 | 1 | FTC |
| F8 | 0 | 0 | TC | 1 | 1 | FTC |
| F9 | 2 | 2 | FTC | 2 | 1 | FTC |
| F10 | 1 | 1 | FTC | 1 | 1 | FTC |
| F11 | 0 | 0 | FTC | 0 | 0 | TC |
| F12 | 0 | 0 | TC | 0 | 1 | TC |
| F13 | 0 | 0 | TC | 0 | 0 | TC |
| F14 | 0 | 0 | TC | 1 | 0 | TC |
| F15 | 0 | 0 | TC | 1 | 1 | TC |
| F16 | 0 | 0 | TC | 1 | 1 | FTC |
| F17 | 1 | 1 | HTC | 0 | 0 | TC |
| F18 | 1 | 1 | FTC | 0 | 0 | FTC |
| F19 | 1 | 2 | HTC | 0 | 0 | TC |
| F20 | 1 | 1 | TC | 0 | 0 | TC |

The results of the analyses performed in accordance with Table 4A are shown in Table 4H.

Table 4H Results of analyses of formulations of Table 4A before and after stressing (a classification of each result is shown superscript according to Table 4J)

| Time point | Formulation | pH | Osmolality [mOsmol/kg] | UV [mg/ml] | Viscosity [mPa*s] | Visual inspection | Turbidity [NTU] | MFI [particle/ml] | HP-SEC [monomer] | ICE [cluster 2] | IEX (deamid) | cGE Red [Purity %] | cGE NR [Intact molecule %] | RP-UPLC Oxidized Met516 content [%] |
|---|---|---|---|---|---|---|---|---|---|---|---|---|---|---|
| T0 | | 5.56 | 278[1] | 10.6[2] | 1.0[1] | 2[2] | 2.8[1] | 55[1] | 99.3[1] | 10.7[1] | 93.9[1] | 93.86 | 97.20 | 3.3[1] |
| T-FT | | 5.62[1] | | 10.2[1] | | 0[1] | 2.8[1] | 179[1] | 99.3[1] | 10.1[1] | 93.4[1] | | | |
| T2w_25 °C | | 5.51[1] | | 10.2[1] | | 0[1] | 2.9[1] | 9[1] | 99.3[1] | 10.9[1] | 93.6[1] | | | |
| T2w_40 °C | F1 | 5.65[1] | | 10.2[1] | | 0[1] | 2.8[1] | 61[1] | 99.1[1] | 10.5[1] | 92.5[2] | | | |
| T4w_25 °C | | 5.56[1] | | 10.2[1] | | 0[1] | 2.7[1] | 64[1] | 99.3[1] | 11.6[1] | 93.6[1] | | | |
| T4w_40°C | | 5.62[1] | | 10.2[1] | | 2[2] | 2.8[1] | 139[1] | 99.0[1] | 9.6[2] | 91.8[2] | 90.29[3] | 94.53[2] | 3.9[2] |
| T0 | | 5.59 | 275[1] | 25.3[1] | 1.3[1] | 0[1] | 6.7[1] | 39[1] | 99.2[1] | 11.2[1] | 93.7[1] | 93.69 | 97.17 | 3.9[1] |
| T-FT | | 5.60[1] | | 24.8[1] | | 0[1] | 6.7[1] | 97[1] | 99.3[1] | 9.9[2] | 93.4[1] | | | |
| T2w_25 °C | | 5.57[1] | | 25.1[1] | | 0[1] | 6.9[1] | 136[1] | 99.1[1] | 11.1[1] | 93.5[1] | | | |
| T2w_40 °C | F2 | 5.56[1] | | 25.0[1] | | 0[1] | 6.7[1] | 103[1] | 98.8[2] | 9.8[2] | 92.3[2] | | | |
| T4w_25 °C | | 5.61[1] | | 24.8[1] | | 2[2] | 6.8[1] | 91[1] | 99.2[1] | 10.9[1] | 93.2[1] | | | |
| T4w_40°C | | 5.62[1] | | 24.9[1] | | 2[2] | 6.7[1] | 91[1] | 98.5[2] | 9.5[2] | 91.6[2] | 90.34[2] | 94.14[3] | 2.9[1] |
| T0 | | 5.78 | 276[1] | 25.3[1] | 1.3[1] | 0[1] | 6.9[1] | 76[1] | 99.0[1] | 11.1[1] | 93.7[1] | 93.99 | 97.28 | 2.8[1] |
| T-FT | | 5.79[1] | | 24.7[1] | | 1[2] | 6.9[1] | 85[1] | 99.2[1] | 10.0[1] | 93.3[1] | | | |
| T2w_25 °C | | 5.76[1] | | 25.0[1] | | 2[2] | 7.1[2] | 112[1] | 99.0[1] | 10.5[1] | 93.3[1] | | | |
| T2w_40 °C | F3 | 5.75[1] | | 25.0[1] | | 0[1] | 6.9[1] | 533[1] | 98.7[2] | 9.9[2] | 91.8[2] | | | |
| T4w_25 °C | | 5.80[1] | | 25.0[1] | | 2[2] | 7.6[2] | 312[1] | 99.0[1] | 11.2[1] | 93.1[1] | | | |
| T4w_40°C | | 5.79[1] | | 25.0[1] | | 3[3] | 6.8[1] | 730[1] | 98.4[3] | 9.2[2] | 90.8[2] | 90.82[2] | 94.91[2] | 3.7[2] |
| T0 | | 6.10 | 277[1] | 25.4[1] | 1.3[1] | 0[1] | 7.1[2] | 42[1] | 98.9[2] | 10.7[1] | 93.6[1] | 94.02 | 97.12 | 2.7[1] |
| T-FT | | 6.09[1] | | 24.7[1] | | 0[1] | 7.0[1] | 45[1] | 99.0[1] | 10.4[1] | 93.1[1] | | | |
| T2w_25 °C | | 6.04[1] | | 24.8[1] | | 0[1] | 7.2[2] | 154[1] | 98.9[2] | 10.6[1] | 93.0[1] | | | |
| T2w_40 °C | F4 | 6.03[1] | | 24.9[1] | | 0[1] | 7.1[2] | 139[1] | 98.5[2] | 10.0[1] | 90.9[2] | | | |
| T4w_25 °C | | 6.10[1] | | 24.9[1] | | 2[2] | 7.2[2] | 118[1] | 98.8[3] | 10.8[1] | 92.6[2] | | | |
| T4w_40°C | | 6.09[1] | | 24.9[1] | | 0[1] | 7.1[2] | 48[1] | 98.2[3] | 9.6[2] | 89.1[3] | 90.63[2] | 94.57[2] | 4.0[2] |
| T0 | | 6.04 | 278[1] | 25.1[1] | 1.3[1] | 2[2] | 6.8[1] | 318[1] | 99.1[1] | 11.0[1] | 93.7[1] | 93.99 | 97.07 | 2.9[1] |
| T-FT | | 6.02[1] | | 24.8[1] | | 2[2] | 6.8[1] | 345[1] | 99.1[1] | 10.4[1] | 93.3[1] | | | |
| T2w_25 °C | F5 | 5.94[1] | | 25.1[1] | | 0[1] | 6.9[1] | 209[1] | 98.9[2] | 10.5[1] | 93.4[1] | | | |
| T2w_40 °C | | 5.96[1] | | 25.2[1] | | 0[1] | 6.9[1] | 127[1] | 98.5[2] | 9.4[2] | 90.4[2] | | | |

| Condition | Form. | | | | | | | | | | | | | |
|---|---|---|---|---|---|---|---|---|---|---|---|---|---|---|
| T4w_25 °C | | 6.03[1] | | 25.0[1] | | 0[1] | 7.1[2] | 4,071[2] | 98.8[2] | 11.0[1] | 93.1[1] | | | |
| T4w_40°C | | 6.03[1] | | 25.0[1] | | 2[2] | 7.0[1] | 836[1] | 98.2[3] | 8.1[2] | 87.8[3] | 90.88[2] | 95.27[1] | 3.1[1] |
| T0 | | 6.03 | 260[1] | 25.0[1] | 1.3[1] | 0[1] | 5.9[1] | 48[1] | 99.3[1] | 10.2[1] | 93.7[1] | 93.82 | 97.15 | 2.9[1] |
| T-FT | | 6.06[1] | | 24.6[1] | | 0[1] | 6.0[1] | 127[1] | 99.2[1] | 9.8[2] | 93.1[1] | | | |
| T2w_25 °C | | 6.06[1] | | 24.7[1] | | 0[1] | 5.9[1] | 100[1] | 99.1[1] | 10.9[1] | 93.0[1] | | | |
| T2w_40 °C | F6 | 5.99[1] | | 24.7[1] | | 0[1] | 5.9[1] | 179[1] | 98.8[2] | 8.6[2] | 90.3[2] | | | |
| T4w_25 °C | | 6.08[1] | | 24.7[1] | | 0[1] | 5.9[1] | 324[1] | 99.0[1] | 11.2[1] | 92.8[2] | | | |
| T4w_40°C | | 6.05[1] | | 24.9[1] | | 0[1] | 5.9[1] | 321[1] | 98.6[2] | 8.2[2] | 87.4[3] | 90.95[2] | 95.47[1] | 2.3[1] |
| T0 | | 5.61 | 266[1] | 25.0[1] | 1.1[1] | 2[2] | 4.8[1] | 64[1] | 99.3[1] | 9.5[2] | 93.7[1] | 94.05 | 97.45 | 2.7[1] |
| T-FT | | 5.68[1] | | 24.9[1] | | 0[1] | 4.8[1] | 76[1] | 99.3[1] | 10.1[1] | 93.4[1] | | | |
| T2w_25 °C | | 5.63[1] | | 24.9[1] | | 0[1] | 4.8[1] | 173[1] | 99.2[1] | 11.6[1] | 93.5[1] | | | |
| T2w_40 °C | F7 | 5.64[1] | | 24.7[1] | | 0[1] | 4.7[1] | 197[1] | 99.1[1] | 9.2[2] | 92.6[2] | | | |
| T4w_25 °C | | 5.66[1] | | 24.9[1] | | 0[1] | 4.7[1] | 103[1] | 99.3[1] | 11.0[1] | 93.1[1] | | | |
| T4w_40°C | | 5.64[1] | | 25.2[1] | | 2[2] | 5.6[1] | 176[1] | 99.1[1] | 10.2[1] | 91.6[2] | 90.76[2] | 94.89[2] | 3.4[1] |
| T0 | | 5.56 | 269[1] | 25.1[1] | 1.1[1] | 0[1] | 7.3[1] | 103[1] | 99.4[1] | 9.9[2] | 93.7[1] | 94.08 | 97.26 | 2.7[1] |
| T-FT | | 5.60[1] | | 24.7[1] | | 2[2] | 7.3[1] | 206[1] | 99.2[1] | 10.2[1] | 93.2[1] | | | |
| T2w_25 °C | | 5.50[1] | | 24.8[1] | | 0[1] | 7.3[1] | 145[1] | 98.7[2] | 11.1[1] | 93.2[1] | | | |
| T2w_40 °C | F8 | 5.57[1] | | 25.0[1] | | 0[1] | 7.2[1] | 5,886[3] | 98.7[2] | 9.5[2] | 92.3[2] | | | |
| T4w_25 °C | | 5.59[1] | | 25.0[1] | | 0[1] | 7.1[1] | 615[1] | 99.2[1] | 10.8[1] | 93.1[1] | | | |
| T4w_40°C | | 5.58[1] | | 25.2[1] | | 2[2] | 7.2[1] | 4877[2] | 98.6[2] | 10.3[1] | 91.6[2] | 90.17[3] | 94.58[2] | 2.4[1] |
| T0 | | 5.73 | 287[1] | 50.2[1] | 1.7[1] | 0[1] | 9.1[1] | 130[1] | 99.0[1] | 10.8[1] | 93.6 | 94.04 | 97.18 | 2.5[1] |
| T-FT | | 5.67[1] | | 49.2[1] | | 0[1] | 9.1[1] | 151[1] | 99.0[1] | 10.8[1] | 92.8[2] | | | |
| T2w_25 °C | | 5.65[1] | | 50.4[1] | | 0[1] | 8.9[1] | 106[1] | 98.8[2] | 10.2[1] | 92.9[2] | | | |
| T2w_40 °C | F9 | 5.70[1] | | 50.6[1] | | 0[1] | 9.0[1] | 85[1] | 98.2[3] | 10.6[1] | 91.6[2] | | | |
| T4w_25 °C | | 5.71[1] | | 50.0[1] | | 4[3] | 9.4[1] | 364[1] | 98.7[2] | 10.5[1] | 92.7[2] | | | |
| T4w_40°C | | 5.73[1] | | 49.8[1] | | 3[3] | 8.8[1] | 176[1] | 97.8[3] | 9.6[2] | 90.4[2] | 90.87[2] | 94.87[2] | 3.9[2] |
| T0 | | 5.99 | 288[1] | 50.4[1] | 1.7[1] | 2[2] | 9.1[1] | 130[1] | 98.6[2] | 11.7[1] | 93.5[1] | 94.18 | 97.18 | 2.9[1] |
| T-FT | | 6.03[1] | | 49.4[1] | | 0[1] | 9.1[1] | 88[1] | 98.9[2] | 10.7[1] | 92.8[2] | | | |
| T2w_25 °C | F10 | 5.92[1] | | 50.1[1] | | 0[1] | 9.0[1] | 85[1] | 98.6[2] | 10.7[1] | 92.8[2] | | | |
| T2w_40 °C | | 5.92[1] | | 50.4[1] | | 0[1] | 8.8[1] | 51[1] | 98.1[3] | 10.3[1] | 90.6[2] | | | |

| | | | | | | | | | | | | | | |
|---|---|---|---|---|---|---|---|---|---|---|---|---|---|---|
| T4w_25°C | | 6.04[1] | | 50.2[1] | | 2[2] | 9.4[1] | 148[1] | 98.6[2] | 9.7[2] | 93.1[1] | | | |
| T4w_40°C | | 6.04[1] | | 50.2[1] | | 2[2] | 8.7[1] | 176[1] | 97.7[3] | 10.3[1] | 88.7[3] | 91.36[2] | 95.04[2] | 3.0[1] |
| T0 | | 5.53 | 304[1] | 50.0[1] | 1.7[1] | 0[1] | 9.0[1] | 36[1] | 99.1[1] | 11.1[1] | 93.8[1] | 93.97 | 96.88 | 3.0[1] |
| T-FT | | 5.55[1] | | 50.2[1] | | 0[1] | 8.7[1] | 103[1] | 99.2[1] | 10.8[1] | 93.3[1] | | | |
| T2w_25°C | | 5.58[1] | | 50.0[1] | | 2[2] | 8.7[1] | 52[1] | 99.0[1] | 11.3[1] | 93.4[1] | | | |
| T2w_40°C | F11 | 5.59[1] | | 50.3[1] | | 0[1] | 8.8[1] | 82[1] | 98.5[2] | 10.4[1] | 92.4[2] | | | |
| T4w_25°C | | 5.53[1] | | 50.3[1] | | 0[1] | 8.6[1] | 85[1] | 99.0[1] | 17.8[1] | 93.0[1] | | | |
| T4w_40°C | | 5.58[1] | | 50.8[1] | | 0[1] | 8.6[1] | 209[1] | 98.2[3] | 11.1[1] | 91.6[2] | 90.58[2] | 94.56[2] | 2.8[1] |
| T0 | | 5.76 | 300.5[1] | 49.9[1] | 1.7[1] | 0[1] | 8.9[1] | 76[1] | 98.7[2] | 10.3[1] | 93.8[1] | 94.15 | 97.18 | 2.8[1] |
| T-FT | | 5.76[1] | | 49.7[1] | | 0[1] | 8.6[1] | 139[1] | 99.2[1] | 10.8[1] | 93.2[1] | | | |
| T2w_25°C | | 5.79[1] | | 50.7[1] | | 0[1] | 8.7[1] | 24[1] | 99.0[1] | 11.1[1] | 93.2[1] | | | |
| T2w_40°C | F12 | 5.78[1] | | 50.5[1] | | 0[1] | 8.3[1] | 39[1] | 98.5[2] | 10.7[1] | 92.0[2] | | | |
| T4w_25°C | | 5.76[1] | | 49.9[1] | | 0[1] | 8.5[1] | 127[1] | 98.9[2] | 10.8[1] | 92.9[2] | | | |
| T4w_40°C | | 5.58[3] | | 50.5[1] | | 1[2] | 8.8[1] | 67[1] | 98.2[3] | 10.4[1] | 91.2[2] | 88.92[3] | 93.32[3] | 2.9[1] |
| T0 | | 6.06 | 302[1] | 48.6[1] | 1.7[1] | 0[1] | 8.2[1] | 109[1] | 99.3[1] | 10.2[1] | 93.7[1] | 94.31 | 97.09 | 3.2[1] |
| T-FT | | 6.12[1] | | 48.5[1] | | 0[1] | 8.3[1] | 145[1] | 99.2[1] | 11.0[1] | 93.1[1] | | | |
| T2w_25°C | | 5.95[1] | | 48.0[1] | | 2[2] | 8.9[1] | 64[1] | 99.0[1] | 10.3[1] | 93.0[1] | | | |
| T2w_40°C | F13 | 6.01[1] | | 48.4[1] | | 0[1] | 8.6[1] | 82[1] | 98.6[2] | 9.3[2] | 91.3[2] | | | |
| T4w_25°C | | 6.11[1] | | 48.8[1] | | 0[1] | 8.4[1] | 130 [1] | 99.0[1] | 9.3[2] | 92.6[2] | | | |
| T4w_40°C | | 6.13[1] | | 49.2[1] | | 0[1] | 8.1[1] | 94[1] | 98.3[3] | 8.8[2] | 89.3[3] | 91.31[2] | 94.73[2] | 3.2[1] |
| T0 | | 6.05 | 307.5[1] | 48.9[1] | 1.7[1] | 0[1] | 8.3[1] | 58[1] | 99.1[1] | 10.3[1] | 93.7[1] | 94.13 | 97.25 | 3.1[1] |
| T-FT | | 6.05[1] | | 48.9[1] | | 0[1] | 8.2[1] | 36[1] | 99.0[1] | 10.6[1] | 93.1[1] | | | |
| T2w_25°C | | 6.04[1] | | 49.6[1] | | 0[1] | 8.1[1] | 30[1] | 98.8[2] | 10.6[1] | 93.5[1] | | | |
| T2w_40°C | F14 | 6.05[1] | | 49.2[1] | | 0[1] | 8.0[1] | 30[1] | 98.3[3] | 9.7[2] | 90.9[2] | | | |
| T4w_25°C | | 6.03[1] | | 49.6[1] | | 0[1] | 8.1[1] | 97[1] | 98.7[2] | 11.6[1] | 92.9[2] | | | |
| T4w_40°C | | 6.00[1] | | 49.1[1] | | 1[2] | 8.1[1] | 85[1] | 98.0[3] | 8.3[2] | 88.3[3] | 91.41[2] | 95.43[1] | 2.5[1] |
| T0 | | 6.04 | 254[2] | 48.2[1] | 1.6[1] | 0[1] | 8.5[1] | 1,490[2] | 98.8[2] | 10.9[1] | 93.7[1] | 94.28 | 97.21 | 2.9[1] |
| T-FT | | 6.03[1] | | 48.4[1] | | 0[1] | 8.4[1] | 173[1] | 98.9[2] | 10.9[1] | 92.9[2] | | | |
| T2w_25°C | | 6.04[1] | | 48.9[1] | | 0[1] | 8.4[1] | 145[1] | 98.6[2] | 10.8[1] | 92.9[2] | | | |
| T2w_40°C | F15 | 6.04[1] | | 48.4[1] | | 0[1] | 8.6[1] | 424[1] | 98.2[3] | 10.0[1] | 90.7[2] | | | |
| T4w_25°C | | 6.03[1] | | 48.9[1] | | 0[1] | 8.6[1] | 161[1] | 98.6[2] | 11.6[1] | 92.7[2] | | | |
| T4w_40°C | | 5.99[1] | | 49.0[1] | | 2[2] | 8.7[1] | 463[1] | 97.9[3] | 9.4[2] | 88.6[3] | 91.60[2] | 95.49[1] | 4.6[3] |
| T0 | F16 | 6.02 | 311[1] | 50.0[1] | 1.6[1] | 0[1] | 7.6[1] | 61[1] | 99.0[1] | 10.1[1] | 93.7[1] | 94.31 | 97.23 | 2.9[1] |
| T-FT | | 6.11[1] | | 50.1[1] | | 0[1] | 7.7[1] | 212[1] | 99.2[1] | 10.3[1] | 93.1[1] | | | |

| | | | | | | | | | | | | | |
|---|---|---|---|---|---|---|---|---|---|---|---|---|---|
| T2w_25 °C | | 6.02[1] | | 49.7[1] | | 0[1] | 7.6[1] | 64[1] | 98.8[2] | 10.9[1] | 93.1[1] | | | |
| T2w_40 °C | | 6.03[1] | | 49.9[1] | | 0[1] | 7.7[1] | 136[1] | 98.8[2] | 8.4[2] | 91.3[2] | | | |
| T4w_25 °C | | 6.05[1] | | 50.6[1] | | 0[1] | 7.5[1] | 91[1] | 99.1[1] | 10.3[1] | 92.7[2] | | | |
| T4w_40°C | | 6.08[1] | | 50.5[1] | | 2[2] | 7.5[1] | 85[1] | 98.5[2] | 8.1[2] | 89.3[3] | 92.01[1] | 94.71[2] | 2.4[1] |
| T0 | | 5.69 | 310[1] | 50.3[1] | 1.6[1] | 0[1] | 7.8[1] | 657[1] | 99.2[1] | 10.1[1] | 93.5[1] | 94.02 | 96.83 | 2.2[1] |
| T-FT | | 5.77[1] | | 49.6[1] | | 0[1] | 7.9[1] | 209[1] | 99.1[1] | 10.4[1] | 93.0[1] | | | |
| T2w_25 °C | F17 | 5.66[1] | | 49.5[1] | | 0[1] | 8.3[1] | 2,178[2] | 99.0[1] | 10.9[1] | 92.9[2] | | | |
| T2w_40 °C | | 5.65[1] | | 49.4[1] | | 0[1] | 8.1[1] | 197[1] | 98.6[2] | 9.7[2] | 91.8[2] | | | |
| T4w_25 °C | | 5.74[1] | | 50.3[1] | | 2[2] | 9.4[1] | 3462[2] | 99.0[1] | 9.8[2] | 92.6[2] | | | |
| T4w_40°C | | 5.57[2] | | 50.8[1] | | 0[1] | 8[1] | 191[1] | 98.4[3] | 9.1[2] | 90.7[2] | 90.34[3] | 94.02[2] | 2.9[1] |
| T0 | | 6.01 | 310[1] | 51.9[1] | 1.6[1] | 0[1] | 7.8[1] | 24[1] | 99.1[1] | 10.5[1] | 93.4[1] | 93.87 | 96.31 | 2.4[1] |
| T-FT | | 6.03[1] | | 50.6[1] | | 0[1] | 7.8[1] | 85[1] | 99.1[1] | 10.2[1] | 92.9[2] | | | |
| T2w_25 °C | F18 | 5.92[1] | | 49.5[1] | | 2[2] | 7.8[1] | 242[1] | 98.9[2] | 9.5[2] | 92.8[2] | | | |
| T2w_40 °C | | 5.91[1] | | 49.6[1] | | 0[1] | 7.5[1] | 221[1] | 98.4[3] | 9.3[2] | 90.8[2] | | | |
| T4w_25 °C | | 6.08[1] | | 50.9[1] | | 2[2] | 7.8[1] | 91[1] | 98.8[2] | 9.6[2] | 92.3[2] | | | |
| T4w_40°C | | 6.08[1] | | 50.5[1] | | 0[1] | 7.7[1] | 85[1] | 98.3[3] | 8.3[2] | 88.7[3] | 91.74[1] | 94.36[1] | 2.9[1] |
| T0 | | 6.04 | 305[1] | 49.1[1] | 1.6[1] | 0[1] | 8.4[1] | 188[1] | 99.2[1] | 10.4[1] | 93.6[1] | 93.81 | 97.16 | 2.5[1] |
| T-FT | | 6.11[1] | | 48.8[1] | | 0[1] | 8.7[1] | 70[1] | 99.1[1] | 10.2[1] | 93.1[1] | | | |
| T2w_25 °C | F19 | 5.86[3] | | 48.2[1] | | 1[2] | 11.7[2] | 6,770[3] | 99.0[1] | 9.7[2] | 92.8[2] | | | |
| T2w_40 °C | | 6.02[1] | | 48.0[1] | | 0[1] | 8.6[1] | 212[1] | 98.6[2] | 9.6[2] | 91.4[2] | | | |
| T4w_25 °C | | 5.91[2] | | 49.5[1] | | 3[3] | 14.3[3] | 2699[2] | 99.0[1] | 8.0[2] | 92.6[2] | | | |
| T4w_40°C | | 6.03[1] | | 48.8[1] | | 0[1] | 8.6[1] | 121[1] | 98.3[3] | 8.5[2] | 89.6[3] | 90.48[2] | 94.07[3] | 2.9[1] |
| T0 | | 6.09 | 311[1] | 49.7[1] | 1.6[1] | 0[1] | 6.8[1] | 70[1] | 99.2[1] | 10.1[1] | 93.6[1] | 94.08 | 97.18 | 2.9[1] |
| T-FT | | 6.15[1] | | 49.5[1] | | 0[1] | 6.9[1] | 127[1] | 99.2[1] | 10.6[1] | 93.2[1] | | | |
| T2w_25 °C | F20 | 6.06[1] | | 49.7[1] | | 0[1] | 6.7[1] | 103[1] | 98.9[2] | 10.9[1] | 93.1[1] | | | |
| T2w_40 °C | | 6.09[1] | | 50.0[1] | | 0[1] | 6.7[1] | 161[1] | 98.9[2] | 9.0[2] | 91.3[2] | | | |
| T4w_25 °C | | 6.12[1] | | 50.2[1] | | 2[2] | 6.7[1] | 133[1] | 99.2[1] | 10.5[1] | 92.6[2] | | | |
| T4w_40°C | | 6.13[1] | | 50.6[1] | | 0[1] | 6.6[1] | 85[1] | 98.8[2] | 7.7[3] | 89.1[3] | 91.63[1] | 94.70[2] | 2.5[1] |

The analytical results of Table 4H were classified based on the thresholds defined in Table 4J (see the superscript numbers in Table 4H).

**Table 4I** - Threshold values applicable to the classes specified in Table 4H

| Class | 1 | 2 | 3 |
|---|---|---|---|
| pH variation according to T0 | ≤ 0.1 deviation | ≤ 0.15 deviation | > 0.15 deviation |

(continued)

| Class | | 1 | 2 | 3 |
|---|---|---|---|---|
| **Osmolality** | | 260-340 mOsmol/kg | +/- 10 mOsmol/kg deviation | > 10 mOsmol/kg deviation |
| **Visual inspection Score (Ex1+Ex2)** | | Score: 0 | Score: 1, 2 | Score: >2 |
| **UV spectroscopy** | | $\leq$ 5 % deviation from target value | $\leq$ 10 % deviation from target value | 10 % deviation from target value |
| **MFI (particles => 5$\mu$m)** | | $\leq$ 1,000 | $\leq$ 5,000 | > 5,000 |
| **Viscosity** | | <2 | 2 $\leq$ viscosity $\leq$ 5 | > 5 |
| **HP-SEC** | | $\geq$ 99% monomer content | $\geq$ 98.5% monomer content | < 98.5% monomer content |
| **Turbidity** | | DS 10 mg/ml $\leq$ 3<br>DS 25 mg/ml $\leq$ 7<br>DS 50 mg/ml $\leq$ 10 | DS 10 mg/ml $\leq$ 5<br>DS 25 mg/ml $\leq$ 9<br>DS 50 mg/ml $\leq$ 12 | DS 10 mg/ml > 7<br>DS 25 mg/ml > 11<br>DS 50 mg/ml > 14 |
| **iCE** | | 10% $\leq$ Cluster 2 $\leq$ 13% | 8% $\leq$ cluster 2 < 10% and cluster 2 > 13% | Cluster 2 < 8% |
| **IEX** | | relative peak area peak 2 $\geq$ 93% | 90% $\leq$ relative peak area 2 < 93 % | relative peak area 2 < 90% |
| **cGE** | **Reducing (Purity T0 [%] - Purity [%])** | $\leq$ 2.5% | > 2.5% and < 3.5% | $\geq$ 3.5% |
| | **Non-reducing (Intact molecule T0 [%] - Intact molecule [%])** | $\leq$ 2.0% | > 2.0% and < 3.0% | $\geq$ 3.0% |
| **RP-UPLC oxidation method** | | Relative peak area of oxidized Met516 $\leq$ 3.5 % | 3.5% < Oxidized Met516 content $\leq$ 4.5 % | Relative peak area of oxidized Met516 > 4.5% |

In terms the UV spectroscopic (SoloVPE) analysis to determine bintrafusp alfa concentration, it is noted that all target concentrations were reached at Time = 0. No significant changes were observed after stressing.

A relatively low viscosity of about 1.0 - 1.7 mPa*s was detected for all formulations, with the higher viscosity values (1.6 - 1.7 mPa*s) being observed for formulations containing a protein concentration of 50 mg/ml (F9-F20).

Minimal subvisible particles were detected in all formulations at T0. After 2 and 4 weeks an increase of subvisible particles was observed for F17 (buffer free system, pH 5.7) and F19 (sorbitol-containing formulation) stored at 25 °C and F8 (sorbitol containing formulation) stored at 40 °C. After 4 weeks storage at 25 °C and 40°C F5 showed the largest increase in subvisible particles. For the majority of the formulations a low particle concentration was detected (below 1,000 particles $\geq$ 5 $\mu$m).

In terms of HP-SEC results, it was noted that a LMW shoulder was evolving for F19 after storage at 25°C for 4 weeks. LMW were not, however, integrated in the overall monomer content results, unlike HMW. Generally, a decrease of the monomer content and an increase of the HMW content was observed after storage at 25 °C and 40 °C for up to 4 weeks for all formulations. Formulations containing arginine showed less decrease of the monomer content compared to other formulations with higher protein concentrations. At higher protein concentrations, pH 6.0 seems to be superior to pH 5.5 and 5.7 (tested for His as buffer system). Comparison of F16 and F20 suggests that more arginine and less NaCl might have a stabilizing effect on the protein.

In terms of deamidation (assessed by IEX-HPLC, peak 2 content % - peak 2 was the largest peak, and any decreases in the size of peak 2 indicate deamidation), a relative peak area of > 93% was measured for peak 2 at T0 for all formulations. A decreasing relative peak area of peak 2 was observed for all formulations after storage, which was more pronounced at 40 °C than at 25 °C - accordingly an increase of peak area of the 'LC Deam peak' group (this is an amalgamation of all peaks other than peak 2) was observed.

After FT, also a slight increase of relative peak area of the 'LC Deam peak' was observed, which was comparable to storage at 25 °C for 2 weeks. For more highly concentrated formulations, the most promising results were obtained for:

- 25 mg/ml protein concentration:

  ◦ His buffer system pH 5.5 and 5.7 (F2 and F3)

  ◦ Arginine and Sorbitol containing formulations (F7 and F8)

- 50 mg/ml protein concentration: His buffer system pH 5.5 and 5.7 (F11 and F12)

FIG. 55 is a bar chart showing how the temperature of the $T_m2$ peak of nano-DSC traces varies in all of formulations F1-F20 at various time points of stressing testing, including (bars listed from left-to-right for each formulation): time = 0; after 3 FT cycles; after 2 weeks at 25°C, after 2 weeks at 40°C, after 4 weeks at 25°C, after 4 weeks at 40°C.

FIG. 56 is a bar chart showing how the onset temperature $T_m$ of nano-DSC traces varies in all of formulations F1-F20 at various time points of stressing testing, including (bars listed from left-to-right for each formulation): time = 0; after 3 FT cycles; after 2 weeks at 25°C, after 2 weeks at 40°C, after 4 weeks at 25°C, after 4 weeks at 40°C.

Together FIG. 55 and FIG. 56 suggest that $T_m2$ values above 66 °C were detected for all formulations, and no significant changes were observed during stress or storage.

In terms of isoform profiles, there were no significant changes. At Time = 0, the following relative peak areas were detected:

→ Cluster 1 about 56%

→ Cluster 2 about 11%

→ Cluster 3 about 21%

→ Cluster 4 about 12%

Freeze-thawing seems to have no influence on the isoform profiles of the samples. After 4 weeks of storage at 40 °C the following was noted:

→ a decrease of relative peak area of cluster 2

→ an increase of relative peak area of cluster 4

cGE was performed under both reducing and non-reducing conditions, the results of which are tabulated in Table 4H. Under reducing conditions, very similar results were obtained for all formulations at Time = 0 (0.6% difference between maximum and minimum purity [%]). The purity of all formulations decreased after storage at 40 °C for 4 weeks, though such decreases were least pronounced for F16, F18 and F20 and most pronounced for F1, F8, F12 and F17. Decreases in purity was mainly accompanied by an increased main clipping [%]. Additionally, whilst the content of other impurities increased, these had a minor impact on the overall purity of the formulations.

For cGE under non-reducing conditions, very similar results were observed for all formulations at Time = 0 (1.1% difference between maximum and minimum of corrected area [%] of the intact molecule). Relative peak area of the intact molecule of all formulations decreased after storage at 40 °C for 4 weeks, though such decreases were least pronounced for F5, F6, F14, and F15 and most pronounced for F2, F19 and F12. Decreases of the corrected peak area of the intact molecule were accompanied by an increase of the relative peak area of the LMW species (T4w_40°C).

Oxidation, as measured by RP-UPLC, is recorded in Table 4H. A decrease of *intact Met516* content accompanied by an increase of *oxidized Met516* content was observed for most of the samples after storage for 4 weeks at 40 °C, but was most pronounced for F4, F9 and F15.

*Conclusions*

At Time = 0, target pH, concentration, and osmolality values were reached in respect of all formulations F1-F20. Low numbers of visible and subvisible particles were observed, and the viscosity was relatively low for all formulations. By HP-SEC, the main peak accounted for 98% in all formulations. By cGE, purity was about 94% for all formulations under reducing conditions, and greater than 96% for all formulations under non-reducing conditions.

Freeze-thaw (FT) stress showed no significant impact on the stability of the formulations. Furthermore, pH, concentration, number of visible and subvisible particles, turbidity, and monomer content remained substantially unchanged after FT stress.

Samples stored at 25°C and 40°C for up to 4 weeks exhibited the following characteristics:

- pH was substantially unchanged

- Turbidity values increased, higher values were observed for samples stored at 25 °C compared to samples stored at 40 °C

- More visible particles were observed after 4 weeks of storage at 25°C and 40 °C

- Concentration of protein remained unchanged

- Relatively high numbers of subvisible particles were observed for F5, F8, F17 and F19

- Formulations containing arginine showed less decrease of the monomer content over time (HP-SEC)

- cGE: Purity (%, reducing conditions) as well as the content of intact molecule (%, non-reducing conditions) of all formulations decreased after storage at 40 °C for 4 weeks → F12 showed worst performance for both parameters.

Finally, % oxidation as measured by RP-UPLC showed that intact Met516 was between 96-97% for all formulations at Time = 0, and that a decrease of the intact Met516 and an increase of the oxidized Met516 was observed after storage for 4 weeks at 40 °C.
Formulations (F2-F8) containing 25 mg/mL bintrafusp alfa appeared to perform best overall with a pH of 5.5, though it was noted that citrate buffer performed best at pH 6.0. At this concentration beneficial effects were also observed when arginine was included within the formulation.
Formulations (F9-F20) containing 50 mg/mL bintrafusp alfa appeared to perform best overall with a pH of 5.5, though at this concentration histidine buffers appeared to perform best at pH 6.0 relative to other buffers. As per the lower concentration, arginine was also noted to be beneficial.
Further testing is currently underway for the following promising candidates, F13, F16, F20, and F21-F23, as detailed in Table 4K.

**Table 4K** - Candidate formulations arising from this Example

| Formulation number | [mg/ml] | bintrafusp alfa pH | Buffer system | Salt | Excipient | Anti-oxidant | Surfactant | Rationale |
|---|---|---|---|---|---|---|---|---|
| F13 | 50 | 6.0 | 10 mM His | 120 mM NaCl | 50 mM Trehalose | 5mM L-Met | 0.5 mg/ml PS20 | At higher concentrations a good performance of pH 6.0 and NaCl prevents aggregation |
| F16 | 50 | 6.0 | 10 mM His | 120 mM NaCl | 40 mM Arginine | 5mM L-Met | 0.5 mg/ml PS20 | Arginine instead of Trehalose |
| F20 | 50 | 6.0 | 10 mM His | 40 mM NaCl | 120 mM Arginine | 5mM L-Met | 0.5 mg/ml PS20 | More Arginine, less NaCl |
| F21 | 100 | 6.0 | 10 mM His | 120 mM NaCl | 50 mM Trehalose | 5mM L-Met | 0.5 mg/ml PS20 | At higher concentrations a good performance of pH 6.0 and NaCl prevents aggregation |
| F22 | 100 | 6.0 | 10 mM His | 120 mM NaCl | 40 mM Arginine | 5mM L-Met | 0.5 mg/ml PS20 | Arginine instead of Trehalose |
| F23 | 100 | 6.0 | 10 mM His | 40 mM NaCl | 120 mM Arginine | 5mM L-Met | 0.5 mg/ml PS20 | More Arginine, less NaCl |

## *OVERALL SUMMMARY OF CONCLUSIONS*

Example 1 suggests:

- A pH relatively close to the pI of one of the key protein domains (i.e. TGFβR2 moiety) is surprisingly advantageous for protein solubility and stability.

- A minimum ionic strength (suitably provided by sodium chloride) is preferred to facilitate solubility of the protein.

- Higher ionic strengths are generally required to support high protein concentrations, from at least a protein solubility perspective.

- Lyophilisation facilitates long-term storage of formulations and lyophilised formulations can be readily reconstituted.

- Trehalose facilitates lyophilisation, though alternatives might be considered.

- If too much sodium chloride is used to achieve higher ionic strengths, for instance to support higher protein concentrations, lyophilisation becomes more challenging. Alternative ionic strength providers may be considered in such case.

Example 2 suggests:

- The formulations of Example 1, when formulated in liquid form, could actually be viable from a long-term storage stability standpoint.

- The protein concentration can be increased, especially in liquid formulations, whilst maintaining viability in terms of long-term storage stability.

- Formulations with higher concentrations of the relevant protein tend to benefit from higher ionic strengths (e.g. higher NaCl concentrations).

- Surprisingly, NaCl also supports protein solubility and protein stabilisation.

- Histidine is evidently a good buffer for these formulations, though alternatives might be considered.

Example 3 suggests:

- Higher protein concentrations generally require the support of higher ionic strengths, reinforcing previous findings.

- Higher protein concentrations may benefit from slightly higher pHs, although still closer to the pI of the TGFβR2 group than would be normally expected.

- Various buffers are viable, so long as pH is appropriate for the protein and the buffer, though the best pH can differ slightly for different buffers. Histidine buffer appears to provide the best performance.

- For different stressing conditions (thermal, light, mechanical, freeze-thaw), some excipients are better than others, and this can change for different factors such as fragmentation, conformational stability, aggregation, isoforms, deamidation, oxidation, turbity, and so on.

- Arginine, trehalose, and lysine appeared to be the top performing excipients, albeit their relative ranking to each other changes depending on protein concentration.

- Trehalose generally outperforms other sugar components, such as mannitol and sorbitol.

- Arginine proved to be an excellent stabiliser, especially against aggregation.

- Polysorbate 20 appeared slightly superior to kolliphor 188 surfactants, particular in respect of aggregation mitigation.

- Overall, combinations of trehalose and arginine at different relative molar ratios proved advantageous.

Example 4 suggests:

Arginine could potentially (partially) replace NaCl, as a source of ionic strength.

- Buffer free systems appeared to be less favoured.

Slightly higher pHs appear to work better for high protein concentrations.

## Claims

1. A pharmaceutical composition comprising an IgG:TGFβR fusion protein, an ionic tonicifier, and one or both of a disaccharide and an amino acid component.

2. The pharmaceutical composition of claim 1, wherein the composition comprises a disaccharide.

3. The pharmaceutical composition of claim 1 or 2, wherein the ionic tonicifier is sodium chloride.

4. The pharmaceutical composition of any one of claims 1 to 3, wherein the disaccharide is trehalose or sucrose.

5. The pharmaceutical composition of any one of claims 1 to 4, wherein the disaccharide is trehalose.

6. The pharmaceutical composition of any one of claims 1 to 5, wherein the amino acid component is arginine or lysine.

7. The pharmaceutical composition of any one of claims 1 to 6, wherein the amino acid component is arginine.

8. The pharmaceutical composition of any one of claims 1 to 7, wherein the ionic tonicifier is sodium chloride, the disaccharide is trehalose and the amino acid component is arginine.

9. The pharmaceutical composition of any one of claims 1 to 8, further comprising a surfactant.

10. The pharmaceutical composition of any one of claims 1 to 9, wherein the composition has a pH of 5.0 to 6.5.

11. The pharmaceutical composition of any one of claims 1 to 10, wherein the light chain sequences and the heavy chain sequences of the IgG:TGFβR fusion protein respectively correspond to (1) SEQ ID NO: 7 and SEQ ID NO: 8, (2) SEQ ID NO: 15 and SEQ ID NO: 17, (3) SEQ ID NO: 15 and SEQ ID NO: 18, or (4) SEQ ID NO: 33 and SEQ ID NO: 34.

12. The pharmaceutical composition of any one of claims 1 to 11, wherein the IgG:TGFβR fusion protein has the amino acid sequence of bintrafusp alfa.

13. The pharmaceutical composition of any one of claims 1 to 12, wherein the composition is charactarised by a pH of 5.0 to 6.5 and comprises a buffer system, an ionic tonicifier, a disaccharide, a non-ionic surfactant, optionally an amino acid component and further optionally an antioxidant.

14. The pharmaceutical composition of claim 13, wherein the buffer system is a histidine buffer system, the ionic tonicifier is sodium chloride, the disaccharide is trehalose, the amino acid component is arginine, the antioxidant is methionine, and the non-ionic surfactant is polysorbate 20.

15. The pharmaceutical composition of claim 13 or 14, wherein the composition is selected from the following group of compositions:

    a. a pharmaceutical composition **characterised by** a pH of 5.3-5.7 and comprising 5-15 mg/mL IgG:TGFβR fusion protein, 5-15 mM buffer system, 30-50 mM ionic tonicifier; 150-170 mM disaccharide, 0.3-0.7 mg/mL non-ionic surfactant, and optionally 2-8 mM antioxidant;
    b. a pharmaceutical composition **characterised by** a pH of 5.3-5.7 and comprising 35-45 mg/mL IgG:TGFβR fusion protein, 5-15 mM buffer system, 50-70 mM ionic tonicifier; 150-170 mM disaccharide, 0.3-0.7 mg/mL non-ionic surfactant, and optionally 2-8 mM antioxidant;
    c. a pharmaceutical composition **characterised by** a pH of 5.7-6.1 and comprising 35-45 mg/mL IgG:TGFβR fusion protein, 5-15 mM buffer system, 50-70 mM ionic tonicifier; 90-110 mM disaccharide, 40-60 mM amino acid component, 0.3-0.7 mg/mL non-ionic surfactant, and optionally 2-8 mM antioxidant;
    d. a pharmaceutical composition **characterised by** a pH of 5.7-6.1 and comprising 35-45 mg/mL IgG:TGFβR fusion protein, 5-15 mM buffer system, 50-70 mM ionic tonicifier; 65-85 mM disaccharide, 65-85 mM amino acid component, 0.3-0.7 mg/mL non-ionic surfactant, and optionally 2-8 mM antioxidant; and

e. a pharmaceutical composition **characterised by** a pH of 5.7-6.1 and comprising 45-55 mg/mL IgG:TGFβR fusion protein, 5-15 mM buffer system, 90-110 mM ionic tonicifier; 40-60 mM disaccharide, 40-60 mM amino acid component, 0.3-0.7 mg/mL non-ionic surfactant, and optionally 2-8 mM antioxidant.

**VH**

EVQLLESGGGLVQPGGSLRLS
CAASGFTFSSYIMMWVRQA
PGKGLEWVSSIYPSGGITFYA
DTVKGRFTISRDNSKNTLYLQ
MNSLRAEDTAVYYCARIKLGT
VTTVDYWGQGTLVTVSS...

**VL**

QSALTQPASVSGSPGQ
SITISCTGTSSDVGGYN
YVSWYQQHPGKAPKL
MIYDVSNRPSGVSNRF
SGSKSGNTASLTISGLQ
AEDEADYYCSSYTSSST
RVFGTGTKVTVLGQP...

**CH1**

...ASTKGPSVFPLAPSSKSTSG
GTAALGCLVKDYFPEPVTVSW
NSGALTSGVHTFPAVLQSSGL
YSLSSVVTVPSSSLGTQTYICN
VNHKPSNTKVDKRV...

**Hinge**

...EPKSCDKTHTCP...

**CL**

...KANPTVTLFPPSSEE
LQANKATLVCLISDFY
PGAVTVAWKADGSP
VKAGVETTKPSKQSN
NKYAASSYLSLTPEQ
WKSHRSYSCQVTHE
GSTVEKTVAPTECS...

**CH2**

...PCPAPELLGGPSVFLF
PPKPKDTLMISRTPEVT
CVVVDVSHEDPEVKFN
WYVDGVEVHNAKTKP
REEQYNSTYRVVSVLTV
LHQDWLNGKEYKCKVS
NKALPAPIEKTISKAK...

**CH3**

...GQPREPQVYTLPPSR
EEMTKNQVSLTCLVKG
FYPSDIAVEWESNGQP
ENNYKTTPPVLDSDGSF
FLYSKLTVDKSRWQQG
NVFSCSVMHEALHNHY
TQKSLSLSPGA...

**Linker**

...GGGGSGGGGSGGGGSGGGGSG...

**ECD-TGFβR2**

...IPPHVQKSVNNDMIVTDNNGA
VKFPQLCKFCDVRFSTCDNQKSC
MSNCSITSICEKPQEVCVAVWRKN
DENITLETVCHDPKLPYHDFILEDA
ASPKCIMKEKKKPGETFFMCSCSS
DECNDNIIFSEEYNTSNPD

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

**Figure 24**

Figure 25

Figure 26

## Interaction

**B: Excipient type**

B1 Mannitol
B2 Trehalose
B3 Sorbitol
B4 L-Arginine 100mM
B5 L-Arginine 50mM
B6 L-Lysine
B7 L-Proline
B8 L-Glutamic Acid

A: Protein Concentration (mg/ml)

**Figure 27**

Figure 28

Figure 29

Figure 30

Figure 31

Figure 32

Figure 33

Figure 34

Figure 35

Figure 36

Figure 37

Figure 38

Figure 39

Figure 40

Figure 41

EP 3 838 260 A1

Figure 42

Figure 43

Figure 44

147

**Figure 45**

**Figure 46**

**Figure 47**

**Figure 48**

**Figure 49**

**Figure 50**

Figure 51

Figure 52

Figure 53

Figure 54

**Figure 55**

**Figure 56**

Europäisches
Patentamt

European
Patent Office

Office européen
des brevets

**EUROPEAN SEARCH REPORT**

Application Number

EP 19 21 9008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/205985 A1 (JIANGSU HENGRUI MEDICINE CO [CN] ET AL.) 15 November 2018 (2018-11-15) | 1-5, 8-10,13 | INV. A61K9/00 A61K9/06 |
| Y | * paragraph [0108] - paragraph [0120] * * example 9 * | 1-15 | A61K47/02 A61K47/14 |
| E | & EP 3 623 389 A1 (JIANGSU HENGRUI MEDICINE CO [CN] ET AL.) 18 March 2020 (2020-03-18) * example 9 * * tables 8, 9 * | 1-5, 8-10,13 | A61K47/18 A61K47/26 A61K38/00 A61P35/00 |
| A | WO 2018/029367 A1 (MERCK PATENT GMBH [DE]) 15 February 2018 (2018-02-15) * claims 1-44 * * paragraph [0008] - paragraph [0140] * | 1-15 | |
| Y | WO 2016/208989 A1 (ALTEOGEN INC [KR]) 29 December 2016 (2016-12-29) * claims 1-14 * * example 1 * | 1-15 | |

TECHNICAL FIELDS SEARCHED (IPC)

A61K
A61P

The present search report has been drawn up for all claims

| Place of search | Date of completion of the search | Examiner |
|---|---|---|
| Munich | 3 August 2020 | Schifferer, Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 21 9008

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2020

| Patent document cited in search report | | Publication date | Patent family member(s) | | | Publication date |
|---|---|---|---|---|---|---|
| WO 2018205985 | A1 | 15-11-2018 | AU | 2018264455 | A1 | 14-11-2019 |
| | | | BR | 112019023184 | A2 | 19-05-2020 |
| | | | CA | 3061791 | A1 | 29-10-2019 |
| | | | CN | 110050000 | A | 23-07-2019 |
| | | | EP | 3623389 | A1 | 18-03-2020 |
| | | | JP | 2020519289 | A | 02-07-2020 |
| | | | KR | 20200004801 | A | 14-01-2020 |
| | | | TW | 201900674 | A | 01-01-2019 |
| | | | US | 2020157180 | A1 | 21-05-2020 |
| | | | WO | 2018205985 | A1 | 15-11-2018 |
| WO 2018029367 | A1 | 15-02-2018 | AU | 2017310027 | A1 | 31-01-2019 |
| | | | BR | 112019001818 | A2 | 07-05-2019 |
| | | | CA | 3031168 | A1 | 15-02-2018 |
| | | | CL | 2019000277 | A1 | 05-07-2019 |
| | | | CN | 109641963 | A | 16-04-2019 |
| | | | EP | 3497130 | A1 | 19-06-2019 |
| | | | JP | 2019527706 | A | 03-10-2019 |
| | | | KR | 20190039200 | A | 10-04-2019 |
| | | | PH | 12019500270 | A1 | 01-07-2019 |
| | | | SG | 11201901126U | A | 28-03-2019 |
| | | | US | 2018118832 | A1 | 03-05-2018 |
| | | | WO | 2018029367 | A1 | 15-02-2018 |
| WO 2016208989 | A1 | 29-12-2016 | US | 2016376342 | A1 | 29-12-2016 |
| | | | WO | 2016208989 | A1 | 29-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015118175 A
- WO 2018205985 A
- WO 2018029367 A
- WO 2018208720 A
- US 1812604 W
- US 1947734 W
- US 1940129 W
- US 1936725 W
- US 19732271 W
- US 1938600 W
- EP 2019061558 W

### Non-patent literature cited in the description

- **LAN et al.** Enhanced preclinical antitumor activity of M7824, a bifunctional fusion protein simultaneously targeting PD-L1 and TGF-β. *Sci. Transl. Med.,* 2018, vol. 10, 1-15
- **NEEDLEMAN ; WUNSCH.** A general method applicable to the search for similarities in the amino acid sequence of two proteins. *J Mol Biol,* March 1970, vol. 48 (3), 443-53